(19) Europäisches Patentamt
European Patent Office
Office européen des brevets

(11) EP 4 570 800 A1

(12) **EUROPEAN PATENT APPLICATION**
published in accordance with Art. 153(4) EPC

(43) Date of publication:
**18.06.2025 Bulletin 2025/25**

(21) Application number: **23851836.9**

(22) Date of filing: **08.08.2023**

(51) International Patent Classification (IPC):
***C07D 471/04*** *(2006.01)* ***A61K 31/519*** *(2006.01)*
***A61P 35/00*** *(2006.01)*

(52) Cooperative Patent Classification (CPC):
**A61K 31/519; A61K 45/06; A61P 9/00; A61P 17/00; A61P 35/00; A61P 35/02; C07D 471/04; G01N 5/04; G01N 23/2055; G01N 25/20; G01N 30/02; G01N 30/06; G01N 30/34; G01N 30/74; G01N 30/86;** (Cont.)

(86) International application number:
**PCT/CN2023/111760**

(87) International publication number:
**WO 2024/032615 (15.02.2024 Gazette 2024/07)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **08.08.2022 CN 202210946052**

(71) Applicant: **Wuhan Humanwell Innovative Drug Research and Development Center Limited Company**
**Wuhan, Hubei 430075 (CN)**

(72) Inventors:
- **ZHANG, Xuejun**
  **Wuhan, Hubei 430075 (CN)**
- **ZANG, Yang**
  **Wuhan, Hubei 430075 (CN)**
- **LI, Qun**
  **Wuhan, Hubei 430075 (CN)**
- **HU, Wenbing**
  **Wuhan, Hubei 430075 (CN)**
- **ZHANG, Xin**
  **Wuhan, Hubei 430075 (CN)**
- **ZONG, Qiao**
  **Wuhan, Hubei 430075 (CN)**
- **XIA, Qingfeng**
  **Wuhan, Hubei 430075 (CN)**
- **YUE, Yang**
  **Wuhan, Hubei 430075 (CN)**
- **LI, Lie**
  **Wuhan, Hubei 430075 (CN)**
- **YANG, Jun**
  **Wuhan, Hubei 430075 (CN)**

(74) Representative: **Murgitroyd & Company**
**165-169 Scotland Street**
**Glasgow G5 8PL (GB)**

# (54) CRYSTAL FORM OF PYRIDINOPYRIMIDINONE COMPOUND, ACID SALT THEREOF, CRYSTAL FORM OF ACID SALT THEREOF, AND USE

(57) Disclosed are a crystal form of a pyridinopyrimidinone compound of formula I, a pharmaceutically acceptable salt thereof, and a crystal form of the pharmaceutically acceptable salt thereof, which have significant inhibitory effects on the binding of RAS to SOS1, have ideal physicochemical properties, such as relatively high stability, solubility and bioavailability, and relatively low hygroscopicity, and are suitable for preparing ideal pharmaceutical formulations.

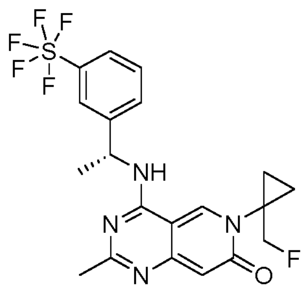

(I)

EP 4 570 800 A1

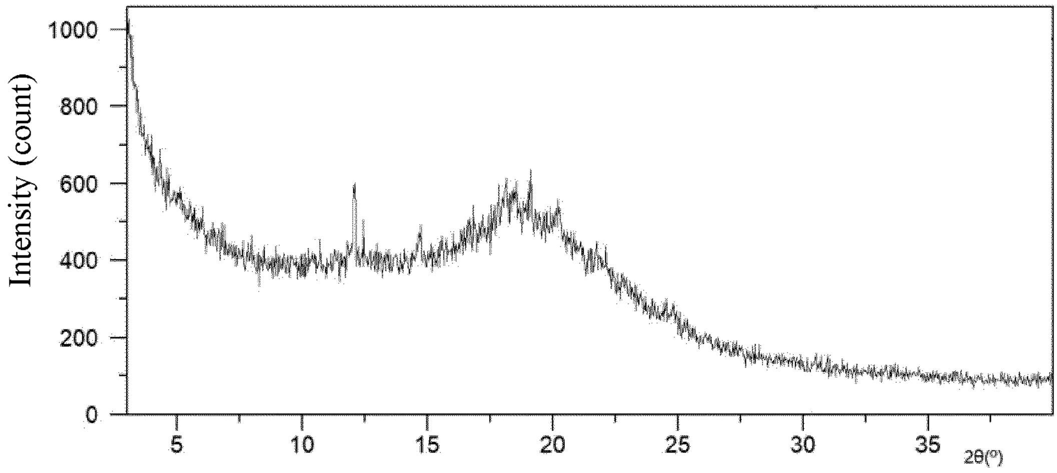


FIG. 1

(52) Cooperative Patent Classification (CPC): (Cont.)
**G01N 30/88**

## Description

The present application claims the right of the priority of Chinese patent application 2022109460522 filed on August 8, 2022. The content of the above Chinese patent application is incorporated herein by reference in its entirety.

## TECHNICAL FIELD

The present disclosure relates to a crystal form of a pyridinopyrimidinone compound, an acid salt thereof, a crystal form of the acid salt thereof, and a use thereof.

## BACKGROUND

RAS protein is a membrane-bound protein with intrinsic GTPase activity that can be activated by many extracellular stimuli, and cycles between a GDP-bound (off) state and a GTP-bound (on) state. When the RAS protein is in the GTP-bound (on) state, it can activate downstream pathways and promote a series of processes such as cell proliferation, differentiation, migration, and immunity.

The RAS protein family comprises three highly homologous isoforms: KRAS (Kirsten rat sarcoma virus oncogene), HRAS (Harvey rat sarcoma virus oncogene), and NRAS (Neuroblastoma ras oncogene), and KRAS comprises two variable splice variants: KRAS4A and KRAS4B. RAS family proteins have weak endogenous GTPase activity and slow nucleotide exchange rate.

Activation of RAS gene mutation is a significant cause of tumorigenesis, with RAS mutation occurring in 27% of all tumor patients. Among them, the mutation frequency of KRAS is the highest, accounting for 86%. KRAS-4B mutation is found in approximately 90% of pancreatic cancers, 30% to 40% of colon cancers, and 15% to 20% of lung cancers. Such mutation is also found in biliary tract malignancies, endometrial cancers, cervical cancers, bladder cancers, liver cancers, myeloid leukemia, and breast cancers. Point mutation is the most common KRAS gene mutation, with KRAS-G12D (41%), KRAS-G12V (28%), and KRAS-G12C (14%) mutations being common. Mutant KRAS affects its ability to bind to GTPase activating protein (GAP), thereby inhibiting GAP-induced GTP hydrolysis. As the hydrolysis ability of GTPase decreases, GTP gradually accumulates, and KRAS is more likely to bind to GTP, such that KRAS is mostly activated, which induces the occurrence and development of malignancies.

The transition of RAS protein from inactivated state to activated state involves the release of GDP and the binding of GTP. The release of GDP requires the involvement of GMP exchange factor (GEF), such as SOS (Son of Sevenless) protein. SOS protein, first identified in Drosophila in 1992, is the GEF of RAS and Rac proteins, and plays an important role in the RAS and Rac signaling pathways. There are two human SOS homologs, SOS1 and SOS2, which are highly similar in structure and sequence with 70% homology, but differ in biological function. SOS1 protein consists of 1300 amino acid residues with a proline-rich C-terminal domain, which can interact with growth factor receptor-bound protein 2 (Grb2) in the RAS pathway. Grb2 binds to SOS1 to form a complex that can bring SOS1 to the vicinity of the cell membrane RAS protein. The interaction between SOS1 and RAS involves two domains of SOS1: CDC25 domain and REM domain. The CDC25 domain has an active site for nucleotide exchange, and the REM domain contains a site that can bind RAS-GTP and cause allosteric activation of the CDC25 domain. SOS1 can convert GDP to GTP through catalytic exchange. GTP is hydrolyzed by RAS, and then activates downstream signals, causing a series of corresponding biological effects.

Specific SOS1 inhibitors can inhibit the interaction between SOS1 and KRAS-GDP, thereby reducing the formation of activated KRAS-GTP. A reduction in KRAS-GTP levels leads to a reduction in downstream MAPK signaling, which plays a role in both wild-type and multiple KRAS mutant types. The SOS1 small molecule inhibitor BAY-293 can effectively reduce the activity of mutated KRAS and wild-type KRAS in tumor cells. The SOS1 inhibitors BI-3406 and BI-1701963 developed by Boehringer Ingelheim can bind to the catalytic domain of SOS1, prevent its interaction with KRAS, reduce the formation of KRAS-GTP, and inhibit KRAS-driven proliferation of various cancer cells. The combination of SOS1 inhibitors with MEK inhibitors significantly reduces KRAS signaling and enhances antitumor activity through a complementary mechanism. According to Boehringer Ingelheim, BI-3406 inhibits cytochrome P450 3A4 (CYP3A4) in a time-dependent manner with a potential drug-drug interaction (DDI) risk, so the development of cytochrome P450-free inhibition has the advantage that SOS1 inhibitors without CYP3A4 inhibition are more clinically valuable, and BI-1701963 as well as a combination therapy of BI-1701963 and MEK inhibitor Trametinib has entered clinical research.

In addition to cancer, SOS1 gene mutation and abnormal expression are also closely related to the occurrence of several genetic diseases. Noonan syndrome (NS) is an autosomal dominant genetic disease in which about 20% of NS patients have SOS1 mutations distributed in six domains of SOS1. Patients with SOS1 mutations exhibit phenotypic features of curly hair and abnormal ectoderm. Mutations in the CDC25 domain can directly increase the GEF activity of SOS1 and induce hyperactivation of the RAS/ERK pathway. Cardio-facio-cutaneous syndrome is one of renin-angiotensin system (RAS) cardiomyopathy group, and it has been reported that there is a SOS1 mutation in the disease. Hereditary gingival fibromatosis type 1 is an autosomal dominant genetic disease whose etiology is associated with

mutations in the proline-rich domain of SOS1.

A compound of formula I, having a structure shown in formula I

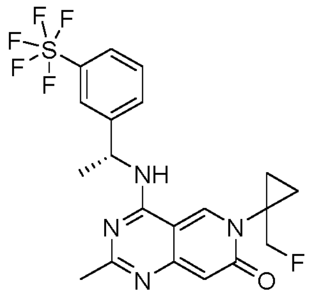

I .

This compound has been recorded in patent CN202210117751.6 as an inhibitor used as an inhibitor of the interaction of the catalytic site of SOS1 with RAS family proteins involved in the regulation of cellular proliferation, and can be used in the treatment of diseases with excessive or abnormal cellular proliferation.

The phenomenon that a substance can exist in two or more different crystal structures is called polymorphism. For drugs, this polymorphism may affect the absorption of the drug, thereby affecting the bioavailability of the drug, thereby showing different clinical efficacy and toxic side effects. In view of this, it is of great significance to develop advantageous crystal forms of compounds of formula I and salts thereof with advantageous properties.

CONTENT OF THE PRESENT INVENTION

The present disclosure provides a crystal form of a compound of formula I, an acid salt thereof, and a crystal form of the acid salt thereof. The preparation method for the above crystal form is simple and suitable for industrial production. Moreover, the above crystal form is not easily hygroscopic, and has good stability and solubility, which is of great value to the optimization and development of drugs.

The present disclosure provides a crystal form A of a compound of formula I, which has an X-ray powder diffraction pattern expressed by 2θ angles comprising diffraction peaks at: 12.06 ± 0.2°, 14.68 ± 0.2°, 18.13 ± 0.2°, 19.12 ± 0.2°, 20.25 ± 0.2°, 22.09 ± 0.2°, and 24.75 ± 0.2°;

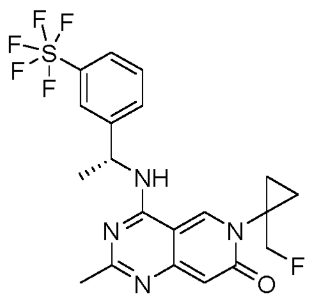

I .

Further, the crystal form A of the compound of formula I has an X-ray powder diffraction pattern expressed by 2θ angles comprising diffraction peaks at: 10.30 ± 0.2°, 12.06 ± 0.2°, 12.42 ± 0.2°, 14.68 ± 0.2°, 15.10 ± 0.2°, 17.78 ± 0.2°, 18.13 ± 0.2°, 19.12 ± 0.2°, 20.25 ± 0.2°, 21.76 ± 0.2°, 22.09 ± 0.2°, and 24.75 ± 0.2°;

further, the crystal form A of the compound of formula I has an X-ray powder diffraction pattern expressed by 2θ angles comprising diffraction peaks at: 6.02 ± 0.2°, 7.78 ± 0.2°, 10.30 ± 0.2°, 11.42 ± 0.2°, 12.06 ± 0.2°, 12.42 ± 0.2°, 14.68 ± 0.2°, 15.10 ± 0.2°, 16.61 ± 0.2°, 17.78 ± 0.2°, 18.13 ± 0.2°, 18.48 ± 0.2°, 19.12 ± 0.2°, 19.98 ± 0.2°, 20.25 ± 0.2°, 21.76 ± 0.2°, 22.09 ± 0.2°, and 24.75 ± 0.2°;

even further, the crystal form A of the compound of formula I has an X-ray powder diffraction pattern expressed by 2θ angles, and diffraction peaks and relative intensities of the X-ray powder diffraction pattern can further be shown in Table 8;

the X-ray powder diffraction pattern of the crystal form A of the compound of formula I expressed by 2θ angles can further be basically shown in FIG. 4.

In a certain preferred embodiment, the crystal form A of the compound of formula I has a thermogravimetric analysis (TGA) pattern with a weight loss when heated from an onset to 120 ± 5°C; preferably, the weight loss is 2% to 4% (such as 3.24%) (the percentage of the weight loss is the percentage of the weight reduction of the sample relative to the weight of the sample prior to this weight loss).

In a certain preferred embodiment, the crystal form A of the compound of formula I has a differential scanning calorimetry (DSC) pattern, and the differential scanning calorimetry pattern can have major endothermic peaks at 73.3 ± 3°C and/or 178.0 ± 3°C.

In a certain preferred embodiment, the DSC pattern of the crystal form A of the compound of formula I can further be basically shown in FIG. 5.

In a certain preferred embodiment, the TGA pattern of the crystal form A of the compound of formula I can further be basically shown in FIG. 6.

In a certain preferred embodiment, the crystal form A of the compound of formula I is a hydrate of the compound of formula I, wherein the compound of formula I and water have a molar ratio of 1:(0.5 to 1.5), preferably 1:(1 to 1.5); such as 1:0.5, 1:0.6, 1:0.7, 1:0.8, 1:0.9, 1:1.0, 1:1, 1:1.1, 1:1.2, 1:1.3, 1:1.4, or 1:1.5; most preferably 1:1 or 1:1.5.

The present disclosure provides a crystal form B of the compound of formula I, which has an X-ray powder diffraction pattern expressed by 2θ angles comprising diffraction peaks at: 12.30 ± 0.2°, 12.89 ± 0.2°, 14.49 ± 0.2°, 18.10 ± 0.2°, 18.70 ± 0.2°, 20.33 ± 0.2°, and 21.66 ± 0.2°.

Further, the crystal form B of the compound of formula I has an X-ray powder diffraction pattern expressed by 2θ angles comprising diffraction peaks at: 12.30 ± 0.2°, 12.89 ± 0.2°, 14.49 ± 0.2°, 15.32 ± 0.2°, 17.45 ± 0.2°, 18.10 ± 0.2°, 18.70 ± 0.2°, 20.33 ± 0.2°, 21.66 ± 0.2°, 22.01 ± 0.2°, 22.52 ± 0.2°, 23.23 ± 0.2°, 24.35 ± 0.2°, and 24.69 ± 0.2°;

further, the crystal form B of the compound of formula I has an X-ray powder diffraction pattern expressed by 2θ angles comprising diffraction peaks at: 10.08 ± 0.2°, 10.30 ± 0.2°, 11.58 ± 0.2°, 12.30 ± 0.2°, 12.89 ± 0.2°, 14.49 ± 0.2°, 15.32 ± 0.2°, 16.39 ± 0.2°, 16.89 ± 0.2°, 17.45 ± 0.2°, 18.10 ± 0.2°, 18.21 ± 0.2°, 18.70 ± 0.2°, 19.84 ± 0.2°, 20.33 ± 0.2°, 21.05 ± 0.2°, 21.66 ± 0.2°, 22.01 ± 0.2°, 22.52 ± 0.2°, 23.23 ± 0.2°, 24.35 ± 0.2°, and 24.69 ± 0.2°;
even further, the crystal form B of the compound of formula I has an X-ray powder diffraction pattern expressed by 2θ angles, and diffraction peaks and relative intensities of the X-ray powder diffraction pattern can further be shown in Table 10;
the X-ray powder diffraction pattern of the crystal form B of the compound of formula I expressed by 2θ angles can further be basically shown in FIG. 10.

The present disclosure further provides a pharmaceutically acceptable salt of the compound of formula I; the pharmaceutically acceptable salt is a salt formed by the compound of formula I and an acid; the acid is hydrochloric acid, sulfuric acid, maleic acid, aspartic acid, phosphoric acid, fumaric acid, tartaric acid, citric acid, glucuronic acid, glycolic acid, malic acid, hippuric acid, gluconic acid, lactic acid, succinic acid, ascorbic acid, adipic acid, p-toluenesulfonic acid, methanesulfonic acid, benzenesulfonic acid, oxalic acid, 2-hydroxyethanesulfonic acid, ethanesulfonic acid, gentisic acid, or benzoic acid.

In a certain preferred embodiment, the pharmaceutically acceptable salt of the compound of formula I is in crystalline form.

In a certain preferred embodiment, the acid is fumaric acid, citric acid, methanesulfonic acid, ethanesulfonic acid, maleic acid, L-tartaric acid, glycolic acid, L-malic acid, hippuric acid, succinic acid, ascorbic acid, adipic acid, p-toluenesulfonic acid, benzenesulfonic acid, oxalic acid, or 2-hydroxyethanesulfonic acid.

In a certain preferred embodiment, the compound of formula I and the acid have a molar ratio of 1:(0.5 to 1.2), such as 1:0.5, 1:0.6, 1:0.7, 1:0.8, 1:0.9, 1:1, 1: 1.1, or 1:1.2.

In a certain preferred embodiment, the pharmaceutically acceptable salt of the compound of formula I is any one of the following pharmaceutically acceptable salts:

(1) a fumarate salt of the compound of formula I; wherein the compound of formula I and fumaric acid have a molar ratio of 1:1;
(2) a citrate salt of the compound of formula I; wherein the compound of formula I and citric acid have a molar ratio of 1:(1 to 1.2), such as 1:1, 1:1.1, or 1: 1.2;
(3) a methanesulfonate salt of the compound of formula I; wherein the compound of formula I and methanesulfonic acid have a molar ratio of 1:(0.8 to 1), such as 1:1, 1:0.9, or 1:0.8;
(4) an ethanesulfonate salt of the compound of formula I; wherein the compound of formula I and ethanesulfonic acid have a molar ratio of 1:1;
(5) a maleate salt of the compound of formula I; wherein the compound of formula I and maleic acid have a molar ratio of 1:1;
(6) an L-tartrate salt of the compound of formula I; wherein the compound of formula I and L-tartaric acid have a molar

ratio of 1:(0.5 to 1.1), such as 1:0.5, 1:0.6, 1:0.7, 1:0.8, 1:0.9, 1:1, or 1:1.1;
(7) a glycolate salt of the compound of formula I; wherein the compound of formula I and glycolic acid have a molar ratio of 1:(1 to 1.1), such as 1: 1 or 1:1.1;
(8) an L-malate salt of the compound of formula I; wherein the compound of formula I and L-malic acid have a molar ratio of 1:(1 to 1.1), such as 1: 1 or 1:1.1;
(9) a hippurate salt of the compound of formula I; wherein the compound of formula I and hippuric acid have a molar ratio of 1:1;
(10) a succinate salt of the compound of formula I; wherein the compound of formula I and succinic acid have a molar ratio of 1:(0.8 to 1.2), such as 1:0.8, 1:0.9, 1:1, or 1: 1.2;
(11) an ascorbate salt of the compound of formula I; wherein the compound of formula I and ascorbic acid have a molar ratio of 1:(0.5 to 1.2), such as 1:0.5, 1:0.6, 1:0.7, 1:0.8, 1:0.9, 1:1, or 1: 1.2;
(12) an adipate salt of the compound of formula I; wherein the compound of formula I and adipic acid have a molar ratio of 1:1;
(13) a *p*-toluenesulfonate salt of the compound of formula I; wherein the compound of formula I and *p*-toluenesulfonic acid have a molar ratio of 1:1;
(14) a benzenesulfonate salt of the compound of formula I; wherein the compound of formula I and benzenesulfonic acid have a molar ratio of 1:1;
(15) an oxalate salt of the compound of formula I; wherein the compound of formula I and oxalic acid have a molar ratio of 1:1;
(16) a 2-hydroxyethanesulfonate salt of the compound of formula I; wherein the compound of formula I and 2-hydroxyethanesulfonic acid have a molar ratio of 1:(1 to 1.1), such as 1:1 or 1:1.1.

The present disclosure provides a preparation method for the pharmaceutically acceptable salt of the compound of formula I, comprising: carrying out a salt-forming reaction of the compound of formula I with an acid in a solvent to obtain the pharmaceutically acceptable salt of the compound of formula I; wherein the acid is hydrochloric acid, sulfuric acid, maleic acid, aspartic acid, phosphoric acid, fumaric acid, tartaric acid, citric acid, glucuronic acid, glycolic acid, malic acid, hippuric acid, gluconic acid, lactic acid, succinic acid, ascorbic acid, adipic acid, *p*-toluenesulfonic acid, methanesulfonic acid, benzenesulfonic acid, oxalic acid, 2-hydroxyethanesulfonic acid, ethanesulfonic acid, gentisic acid, or benzoic acid.
In a certain preferred embodiment, the solvent comprises one or more of water, ethyl acetate, methyl *tert*-butyl ether, acetone, *n*-heptane, and isopropanol; preferably one or more of water, ethyl acetate, methyl *tert*-butyl ether, acetone, and *n*-heptane.
In a certain preferred embodiment, the compound of formula I and the acid have a molar ratio of 1:(1 $\pm$ 0.5); preferably, 1:(1 $\pm$ 0.2) or 1:(1 $\pm$ 0.1), for example, 1:1.
In a certain preferred embodiment, the acid is fumaric acid, citric acid, methanesulfonic acid, ethanesulfonic acid, maleic acid, L-tartaric acid, glycolic acid, L-malic acid, hippuric acid, succinic acid, ascorbic acid, adipic acid, *p*-toluenesulfonic acid, benzenesulfonic acid, oxalic acid, or 2-hydroxyethanesulfonic acid.
The present disclosure provides a crystal form A of a fumarate salt of the compound of formula I, which has an X-ray powder diffraction pattern expressed by 2$\theta$ angles comprising diffraction peaks at: 6.86 $\pm$ 0.2°, 7.72 $\pm$ 0.2°, 16.47 $\pm$ 0.2°, 19.20 $\pm$ 0.2°, 19.63 $\pm$ 0.2°, 22.47 $\pm$ 0.2°, and 23.26 $\pm$ 0.2°;

further, the crystal form A of the fumarate salt has an X-ray powder diffraction pattern expressed by 2$\theta$ angles comprising diffraction peaks at: 6.86 $\pm$ 0.2°, 7.72 $\pm$ 0.2°, 9.57 $\pm$ 0.2°, 16.47 $\pm$ 0.2°, 19.20 $\pm$ 0.2°, 19.63 $\pm$ 0.2°, 22.47 $\pm$ 0.2°, 23.26 $\pm$ 0.2°, and 23.95 $\pm$ 0.2°;
further, the crystal form A of the fumarate salt has an X-ray powder diffraction pattern expressed by 2$\theta$ angles, which can comprise diffraction peaks at: 6.86 $\pm$ 0.2°, 7.72 $\pm$ 0.2°, 9.57 $\pm$ 0.2°, 13.72 $\pm$ 0.2°, 15.45 $\pm$ 0.2°, 15.87 $\pm$ 0.2°, 16.47 $\pm$ 0.2°, 17.13 $\pm$ 0.2°, 17.47 $\pm$ 0.2°, 18.19 $\pm$ 0.2°, 18.72 $\pm$ 0.2°, 19.20 $\pm$ 0.2°, 19.63 $\pm$ 0.2°, 19.97 $\pm$ 0.2°, 22.47 $\pm$ 0.2°, 23.26 $\pm$ 0.2°, 23.95 $\pm$ 0.2°, 25.93 $\pm$ 0.2°, and 31.16 $\pm$ 0.2°;
further, the crystal form A of the fumarate salt has an X-ray powder diffraction pattern expressed by 2$\theta$ angles, which can comprise diffraction peaks at: 6.86 $\pm$ 0.2°, 7.72 $\pm$ 0.2°, 9.57 $\pm$ 0.2°, 12.90 $\pm$ 0.2°, 13.17 $\pm$ 0.2°, 13.72 $\pm$ 0.2°, 15.45 $\pm$ 0.2°, 15.87 $\pm$ 0.2°, 16.47 $\pm$ 0.2°, 17.13 $\pm$ 0.2°, 17.47 $\pm$ 0.2°, 18.19 $\pm$ 0.2°, 18.72 $\pm$ 0.2°, 19.20 $\pm$ 0.2°, 19.63 $\pm$ 0.2°, 19.97 $\pm$ 0.2°, 20.89 $\pm$ 0.2°, 22.47 $\pm$ 0.2°, 23.26 $\pm$ 0.2°, 23.95 $\pm$ 0.2°, 25.93 $\pm$ 0.2°, 26.28 $\pm$ 0.2°, 27.23 $\pm$ 0.2°, 29.54 $\pm$ 0.2°, 29.82 $\pm$ 0.2°, and 31.16 $\pm$ 0.2°;
even further, the crystal form A of the fumarate salt has an X-ray powder diffraction pattern expressed by 2$\theta$ angles, and diffraction peaks and relative intensities of the X-ray powder diffraction pattern can further be shown in Table 11; the X-ray powder diffraction pattern of the crystal form A of the fumarate salt of the compound of formula I expressed by 2$\theta$ angles can further be basically shown in FIG. 11.

In a certain preferred embodiment, the compound of formula I and fumaric acid have a molar ratio of 1:1.

In a certain preferred embodiment, the crystal form A of the fumarate salt has a thermogravimetric analysis (TGA) pattern with a weight loss of 0% to 5% (such as 3.35%) when heated from an onset to 150 ± 5°C (the percentage of the weight loss is the percentage of the weight reduction of the sample relative to the weight of the sample prior to this weight loss).

In a certain preferred embodiment, the crystal form A of the fumarate salt has a differential scanning calorimetry (DSC) pattern, and the differential scanning calorimetry pattern can have a major endothermic peak at 214.7 ± 3°C.

In a certain preferred embodiment, the DSC pattern of the crystal form A of the fumarate salt can further be basically shown in FIG. 12.

In a certain preferred embodiment, the TGA pattern of the crystal form A of the fumarate salt can further be basically shown in FIG. 13.

In a certain preferred embodiment, the crystal form A of the fumarate salt is a hydrate of the fumarate salt of the compound of formula I, wherein the compound of formula I and water have a molar ratio of 1:(0.5 to 1.5), preferably 1:(1 to 1.5); such as 1:0.5, 1:0.6, 1:0.7, 1:0.8, 1:0.9, 1:1.0, 1:1, 1:1.1, 1:1.2, 1:1.3, 1:1.4, or 1:1.5; most preferably 1:1 or 1:1.5.

The present disclosure provides a crystal form B of a fumarate salt of the compound of formula I, which has an X-ray powder diffraction pattern expressed by 2θ angles comprising diffraction peaks at: 4.78 ± 0.2°, 8.02 ± 0.2°, 9.68 ± 0.2°, 16.57 ± 0.2°, 17.93 ± 0.2°, 18.57 ± 0.2°, and 28.81 ± 0.2°;
further, the crystal form B of the fumarate salt has an X-ray powder diffraction pattern expressed by 2θ angles comprising diffraction peaks at: 4.78 ± 0.2°, 8.02 ± 0.2°, 9.68 ± 0.2°, 11.93 ± 0.2°, 16.57 ± 0.2°, 17.93 ± 0.2°, 18.57 ± 0.2°, 19.41 ± 0.2°, 19.95 ± 0.2°, 21.10 ± 0.2°, and 28.81 ± 0.2°.

Further, the crystal form B of the fumarate salt has an X-ray powder diffraction pattern expressed by 2θ angles comprising diffraction peaks at: 4.78 ± 0.2°, 5.98 ± 0.2°, 8.02 ± 0.2°, 9.68 ± 0.2°, 11.93 ± 0.2°, 12.44 ± 0.2°, 12.78 ± 0.2°, 14.01 ± 0.2°, 15.38 ± 0.2°, 15.80 ± 0.2°, 16.57 ± 0.2°, 17.36 ± 0.2°, 17.93 ± 0.2°, 18.57 ± 0.2°, 19.41 ± 0.2°, 19.95 ± 0.2°, 21.10 ± 0.2°, 23.97 ± 0.2°, 24.76 ± 0.2°, 25.74 ± 0.2°, 26.56 ± 0.2°, 28.81 ± 0.2°, and 29.48 ± 0.2°;

further, the crystal form B of the fumarate salt has an X-ray powder diffraction pattern expressed by 2θ angles comprising diffraction peaks at: 4.78 ± 0.2°, 5.98 ± 0.2°, 8.02 ± 0.2°, 9.68 ± 0.2°, 11.55 ± 0.2°, 11.93 ± 0.2°, 12.44 ± 0.2°, 12.78 ± 0.2°, 14.01 ± 0.2°, 15.24 ± 0.2°, 15.38 ± 0.2°, 15.80 ± 0.2°, 16.57 ± 0.2°, 17.36 ± 0.2°, 17.93 ± 0.2°, 18.57 ± 0.2°, 19.41 ± 0.2°, 19.95 ± 0.2°, 21.10 ± 0.2°, 22.15 ± 0.2°, 22.53 ± 0.2°, 22.89 ± 0.2°, 23.97 ± 0.2°, 24.76 ± 0.2°, 25.18 ± 0.2°, 25.74 ± 0.2°, 26.56 ± 0.2°, 28.81 ± 0.2°, and 29.48 ± 0.2°;

even further, the crystal form B of the fumarate salt has an X-ray powder diffraction pattern expressed by 2θ angles, and diffraction peaks and relative intensities of the X-ray powder diffraction pattern can further be shown in Table 12; the X-ray powder diffraction pattern of the crystal form B of the fumarate salt of the compound of formula I expressed by 2θ angles can further be basically shown in FIG. 16.

In a certain preferred embodiment, the compound of formula I and fumaric acid have a molar ratio of 1:1.

In a certain preferred embodiment, the crystal form B of the fumarate salt has a thermogravimetric analysis (TGA) pattern with a weight loss of 4% to 6% (such as 5.64%) when heated from an onset to 150 ± 5°C and a weight loss of 5% to 7% (such as 6.76%) when heated from 150 ± 5°C to 250 ± 5°C (the percentage of the weight loss is the percentage of the weight reduction of the sample relative to the weight of the sample prior to this weight loss).

In a certain preferred embodiment, the crystal form B of the fumarate salt has a differential scanning calorimetry (DSC) pattern, and the differential scanning calorimetry pattern can have a major endothermic peak at 154.4 ± 3°C and an exothermic peak at 191.9 ± 3°C.

In a certain preferred embodiment, the DSC pattern of the crystal form B of the fumarate salt can further be basically shown in FIG. 17.

In a certain preferred embodiment, the TGA pattern of the crystal form B of the fumarate salt can further be basically shown in FIG. 18.

The present disclosure provides a crystal form C of a fumarate salt of the compound of formula I, which has an X-ray powder diffraction pattern expressed by 2θ angles comprising diffraction peaks at: 6.85 ± 0.2°, 8.72 ± 0.2°, 13.13 ± 0.2°, 14.09 ± 0.2°, and 17.27 ± 0.2°;
further, the crystal form C of the fumarate salt has an X-ray powder diffraction pattern expressed by 2θ angles comprising diffraction peaks at: 6.85 ± 0.2°, 8.72 ± 0.2°, 13.13 ± 0.2°, 14.09 ± 0.2°, 17.27 ± 0.2°, 17.90 ± 0.2°, 20.60 ± 0.2°, 21.42 ± 0.2°, 23.16 ± 0.2°, and 24.04 ± 0.2°.

Further, the crystal form C of the fumarate salt has an X-ray powder diffraction pattern expressed by 2θ angles comprising diffraction peaks at: 6.85 ± 0.2°, 8.72 + 0.2°, 9.90 ± 0.2°, 12.17 ± 0.2°, 13.13 ± 0.2°, 14.09 ± 0.2°, 14.29 ± 0.2°, 16.33 ± 0.2°, 17.27 ± 0.2°, 17.90 ± 0.2°, 20.60 ± 0.2°, 21.42 ± 0.2°, 23.16 ± 0.2°, and 24.04 ± 0.2°;

the crystal form C of the fumarate salt has an X-ray powder diffraction pattern expressed by 2θ angles, and diffraction peaks and relative intensities of the X-ray powder diffraction pattern can further be shown in Table 13;

the X-ray powder diffraction pattern of the crystal form C of the fumarate salt of the compound of formula I expressed by 2θ angles can further be basically shown in FIG. 19.

In a certain preferred embodiment, the compound of formula I and fumaric acid have a molar ratio of 1:1.
In a certain preferred embodiment, the crystal form C of the fumarate salt has a thermogravimetric analysis (TGA) pattern with a weight loss of 1% to 3% (such as 1.69%) when heated from an onset to 150 ± 5°C and a weight loss of 5% to 7% (such as 6.21%) when heated from 150 ± 5°C to 250 ± 5°C (the percentage of the weight loss is the percentage of the weight reduction of the sample relative to the weight of the sample prior to this weight loss).
In a certain preferred embodiment, the crystal form C of the fumarate salt has a differential scanning calorimetry (DSC) pattern, and the differential scanning calorimetry pattern can have major endothermic peaks at 94.4 ± 3°C, 145.0 ± 3°C, and 161.2 ± 3°C and an exothermic peak at 190.4 ± 3°C.
In a certain preferred embodiment, the DSC pattern of the crystal form C of the fumarate salt can further be basically shown in FIG. 20.
In a certain preferred embodiment, the TGA pattern of the crystal form C of the fumarate salt can further be basically shown in FIG. 21.
The present disclosure provides a crystal form A of a citrate salt of the compound of formula I, which has an X-ray powder diffraction pattern expressed by 2θ angles comprising diffraction peaks at: 6.27 ± 0.2°, 10.79 ± 0.2°, 12.21 ± 0.2°, 12.58 ± 0.2°, 16.38 ± 0.2°, and 25.33 ± 0.2°.
Further, the crystal form A of the citrate salt has an X-ray powder diffraction pattern expressed by 2θ angles comprising diffraction peaks at: 6.27 ± 0.2°, 10.79 ± 0.2°, 12.21 ± 0.2°, 12.58 ± 0.2°, 16.38 ± 0.2°, 18.33 ± 0.2°, and 25.33 ± 0.2°;

even further, the crystal form A of the citrate salt of the compound of formula I has an X-ray powder diffraction pattern expressed by 2θ angles, and diffraction peaks and relative intensities of the X-ray powder diffraction pattern can further be shown in Table 14;
the X-ray powder diffraction pattern of the crystal form A of the citrate salt of the compound of formula I expressed by 2θ angles can further be basically shown in FIG. 22.

In a certain preferred embodiment, the compound of formula I and citric acid have a molar ratio of 1:1.
In a certain preferred embodiment, the crystal form A of the citrate salt has a thermogravimetric analysis (TGA) pattern with a weight loss of 5% to 7% (such as 5.72%) when heated from an onset to 150 ± 5°C and a weight loss of 18% to 20% (such as 19.25%) when heated from 150 ± 5°C to 230 ± 5°C (the percentage of the weight loss is the percentage of the weight reduction of the sample relative to the weight of the sample prior to this weight loss).
In a certain preferred embodiment, the crystal form A of the citrate salt has a differential scanning calorimetry (DSC) pattern, and the differential scanning calorimetry pattern can have major endothermic peaks at 99.1 ± 3°C and 137.9 ± 3°C.
In a certain preferred embodiment, the DSC pattern of the crystal form A of the citrate salt can further be basically shown in FIG. 23.
In a certain preferred embodiment, the TGA pattern of the crystal form A of the citrate salt can further be basically shown in FIG. 24.
The present disclosure provides a crystal form B of a citrate salt of the compound of formula I, which has an X-ray powder diffraction pattern expressed by 2θ angles comprising diffraction peaks at: 11.93 ± 0.2°, 15.45 ± 0.2°, 16.45 ± 0.2°, 17.60 ± 0.2°, 19.86 ± 0.2°, and 21.18 ± 0.2°, or comprising diffraction peaks at: 9.91 ± 0.2°, 12.79 ± 0.2°, 16.45 ± 0.2°, 17.60 ± 0.2°, and 20.88 ± 0.2°;
further, the crystal form B of the citrate salt has an X-ray powder diffraction pattern expressed by 2θ angles comprising diffraction peaks at: 11.93 ± 0.2°, 12.79 ± 0.2°, 15.45 ± 0.2°, 16.45 ± 0.2°, 17.60 ± 0.2°, 19.86 ± 0.2°, 20.88 ± 0.2°, 21.18 ± 0.2°, 23.55 ± 0.2°, and 25.19 ± 0.2°, or comprising diffraction peaks at: 9.91 ± 0.2°, 12.79 ± 0.2°, 16.45 ± 0.2°, 17.60 ± 0.2°, 20.88 ± 0.2°, 24.34 ± 0.2°, and 24.70 ± 0.2°.
Further, the crystal form B of the citrate salt has an X-ray powder diffraction pattern expressed by 2θ angles, which can comprise diffraction peaks at one or more of the following 2θ angles: 9.90 ± 0.2°, 11.93 ± 0.2°, 12.79 ± 0.2°, 15.45 ± 0.2°, 16.45 ± 0.2°, 17.60 ± 0.2°, 18.25 ± 0.2°, 19.34 ± 0.2°, 19.86 ± 0.2°, 20.88 ± 0.2°, 21.18 ± 0.2°, 23.55 ± 0.2°, 25.19 ± 0.2°, 27.72 ± 0.2°, 28.47 ± 0.2°, 29.97 ± 0.2°, 30.65 ± 0.2°, and 31.19 ± 0.2°;

further, the crystal form B of the citrate salt has an X-ray powder diffraction pattern expressed by 2θ angles, which can comprise diffraction peaks at one or more of the following 2θ angles: 9.90 ± 0.2°, 11.93 ± 0.2°, 12.79 ± 0.2°, 15.45 ± 0.2°, 16.45 ± 0.2°, 16.72 ± 0.2°, 17.60 ± 0.2°, 18.25 ± 0.2°, 19.34 ± 0.2°, 19.86 ± 0.2°, 20.88 ± 0.2°, 21.18 ± 0.2°, 23.22 ± 0.2°, 23.55 ± 0.2°, 24.35 ± 0.2°, 24.68 ± 0.2°, 25.19 ± 0.2°, 27.72 ± 0.2°, 28.47 ± 0.2°, 29.97 ± 0.2°, 30.65 ± 0.2°, and 31.19 ± 0.2°;

even further, the crystal form B of the citrate salt has an X-ray powder diffraction pattern expressed by 2θ angles, which can comprise diffraction peaks at one or more of the following 2θ angles: 9.90 ± 0.2°, 10.38 ± 0.2°, 11.78 ± 0.2°, 11.93 ± 0.2°, 12.79 ± 0.2°, 15.19 ± 0.2°, 15.45 ± 0.2°, 16.45 ± 0.2°, 16.72 ± 0.2°, 17.60 ± 0.2°, 18.25 ± 0.2°, 19.34 ± 0.2°, 19.86 ± 0.2°, 20.88 ± 0.2°, 21.18 ± 0.2°, 23.22 ± 0.2°, 23.55 ± 0.2°, 24.35 ± 0.2°, 24.68 ± 0.2°, 25.19 ± 0.2°, 25.75 ± 0.2°, 27.72 ± 0.2°, 28.47 ± 0.2°, 29.97 ± 0.2°, 30.65 ± 0.2°, and 31.19 ± 0.2°;

the crystal form B of the citrate salt of the compound of formula I has an X-ray powder diffraction pattern expressed by 2θ angles, and diffraction peaks and relative intensities of the X-ray powder diffraction pattern can further be shown in Table 15;

the X-ray powder diffraction pattern of the crystal form B of the citrate salt expressed by 2θ angles can further be basically shown in FIG. 25.

In a certain preferred embodiment, the compound of formula I and citric acid have a molar ratio of 1:1.

In a certain preferred embodiment, the crystal form B of the citrate salt has a thermogravimetric analysis (TGA) pattern with a weight loss of 0% to 2% (such as 1.26%) when heated from an onset to 150 ± 5°C (the percentage of the weight loss is the percentage of the weight reduction of the sample relative to the weight of the sample prior to this weight loss).

In a certain preferred embodiment, the crystal form B of the citrate salt has a differential scanning calorimetry (DSC) pattern, and the differential scanning calorimetry pattern can have a major endothermic peak at 195.0 ± 3°C.

In a certain preferred embodiment, the DSC pattern of the crystal form B of the citrate salt can further be basically shown in FIG. 27.

In a certain preferred embodiment, the TGA pattern of the crystal form B of the citrate salt can further be basically shown in FIG. 28.

The present disclosure provides a crystal form A of a methanesulfonate salt of the compound of formula I, which has an X-ray powder diffraction pattern expressed by 2θ angles comprising diffraction peaks at: 7.28 ± 0.2°, 10.86 ± 0.2°, 12.71 ± 0.2°, 14.20 ± 0.2°, 14.58 ± 0.2°, 16.67 ± 0.2°, 18.70 ± 0.2°, and 19.96 ± 0.2°;

further, the crystal form A of the methanesulfonate salt has an X-ray powder diffraction pattern expressed by 2θ angles comprising diffraction peaks at: 7.28 ± 0.2°, 10.86 ± 0.2°, 12.71 ± 0.2°, 14.20 ± 0.2°, 14.58 ± 0.2°, 16.67 ± 0.2°, 18.70 ± 0.2°, 19.96 ± 0.2°, 21.57 ± 0.2°, and 21.93 ± 0.2°;

further, the crystal form A of the methanesulfonate salt has an X-ray powder diffraction pattern expressed by 2θ angles, which can comprise diffraction peaks at: 7.28 ± 0.2°, 10.86 ± 0.2°, 12.71 ± 0.2°, 14.20 ± 0.2°, 14.58 ± 0.2°, 16.67 ± 0.2°, 18.70 ± 0.2°, 19.96 ± 0.2°, 21.57 ± 0.2°, 21.93 ± 0.2°, 22.86 ± 0.2°, 23.39 ± 0.2°, 23.77 ± 0.2°, 24.50 ± 0.2°, 24.84 ± 0.2°, and 25.59 ± 0.2°;

further, the crystal form A of the methanesulfonate salt has an X-ray powder diffraction pattern expressed by 2θ angles, which can comprise diffraction peaks at: 7.28 ± 0.2°, 9.84 ± 0.2°, 10.86 ± 0.2°, 12.71 ± 0.2°, 14.20 ± 0.2°, 14.58 ± 0.2°, 16.67 ± 0.2°, 18.70 ± 0.2°, 18.92 ± 0.2°, 19.96 ± 0.2°, 20.38 ± 0.2°, 21.31 ± 0.2°, 21.57 ± 0.2°, 21.93 ± 0.2°, 22.86 ± 0.2°, 23.39 ± 0.2°, 23.77 ± 0.2°, 24.50 ± 0.2°, 24.84 ± 0.2°, and 25.59 ± 0.2°;

the crystal form A of the methanesulfonate salt has an X-ray powder diffraction pattern expressed by 2θ angles, and diffraction peaks and relative intensities of the X-ray powder diffraction pattern can further be shown in Table 16;

the X-ray powder diffraction pattern of the crystal form A of the methanesulfonate salt expressed by 2θ angles can further be basically shown in FIG. 30.

In a certain preferred embodiment, the compound of formula I and methanesulfonic acid have a molar ratio of 1:1.

In a certain preferred embodiment, the crystal form A of the methanesulfonate salt has a thermogravimetric analysis (TGA) pattern with a weight loss of 0% to 5% (such as 2.14%) when heated from an onset to 150 ± 5°C (the percentage of the weight loss is the percentage of the weight reduction of the sample relative to the weight of the sample prior to this weight loss).

In a certain preferred embodiment, the crystal form A of the methanesulfonate salt has a differential scanning calorimetry (DSC) pattern with a major endothermic peak at 206.0 ± 3°C.

In a certain preferred embodiment, the DSC pattern of the crystal form A of the methanesulfonate salt can further be basically shown in FIG. 31.

In a certain preferred embodiment, the TGA pattern of the crystal form A of the methanesulfonate salt can further be basically shown in FIG. 32.

The present disclosure provides a crystal form A of an ethanesulfonate salt of the compound of formula I, which has an X-ray powder diffraction pattern expressed by 2θ angles comprising diffraction peaks at: 7.13 ± 0.2°, 10.80 ± 0.2°, 12.60 ± 0.2°, 14.26 ± 0.2°, 16.43 ± 0.2°, 18.28 ± 0.2°, 19.95 ± 0.2°, 21.45 ± 0.2°, and 23.26 ± 0.2°;

further, the crystal form A of the ethanesulfonate salt has an X-ray powder diffraction pattern expressed by 2θ angles

comprising diffraction peaks at: 7.13 ± 0.2°, 9.63 ± 0.2°, 10.80 ± 0.2°, 12.60 ± 0.2°, 14.03 ± 0.2°, 14.26 ± 0.2°, 16.43 ± 0.2°, 18.28 ± 0.2°, 18.73 ± 0.2°, 18.95 ± 0.2°, 19.96 ± 0.2°, 20.98 ± 0.2°, 21.45 ± 0.2°, 22.46 ± 0.2°, 22.95 ± 0.2°, 23.26 ± 0.2°, 24.40 ± 0.2°, 24.69 ± 0.2°, and 25.36 ± 0.2°;

the crystal form A of the ethanesulfonate salt has an X-ray powder diffraction pattern expressed by 2θ angles, and diffraction peaks and relative intensities of the X-ray powder diffraction pattern can further be shown in Table 17;

the X-ray powder diffraction pattern of the crystal form A of the ethanesulfonate salt of the compound of formula I expressed by 2θ angles can further be basically shown in FIG. 35.

In a certain preferred embodiment, the compound of formula I and ethanesulfonic acid have a molar ratio of 1:1.

In a certain preferred embodiment, the crystal form A of the ethanesulfonate salt of the compound of formula I has a thermogravimetric analysis (TGA) pattern with a weight loss of 0% to 5% (such as 0.87%) when heated from an onset to 150 ± 5°C (the percentage of the weight loss is the percentage of the weight reduction of the sample relative to the weight of the sample prior to this weight loss).

In a certain preferred embodiment, the crystal form A of the ethanesulfonate salt of the compound of formula I has a differential scanning calorimetry (DSC) pattern, and the differential scanning calorimetry pattern can have a major endothermic peak at 218.7 ± 3°C.

In a certain preferred embodiment, the DSC pattern of the crystal form A of the ethanesulfonate salt of the compound of formula I can further be basically shown in FIG. 37.

In a certain preferred embodiment, the TGA pattern of the crystal form A of the ethanesulfonate salt of the compound of formula I can further be basically shown in FIG. 38.

The present disclosure provides a crystal form B of an ethanesulfonate salt of the compound of formula I, which has an X-ray powder diffraction pattern expressed by 2θ angles comprising diffraction peaks at: 6.92 ± 0.2°, 9.86 ± 0.2°, 19.20 ± 0.2°, 20.10 ± 0.2°, 21.38 ± 0.2°, 25.27 ± 0.2°, and 29.85 ± 0.2°;

further, the crystal form B of the ethanesulfonate salt has an X-ray powder diffraction pattern expressed by 2θ angles comprising diffraction peaks at: 6.92 ± 0.2°, 8.71 ± 0.2°, 9.86 ± 0.2°, 12.73 ± 0.2°, 15.01 ± 0.2°, 16.84 ± 0.2°, 19.20 ± 0.2°, 20.10 ± 0.2°, 21.38 ± 0.2°, 23.30 ± 0.2°, 25.27 ± 0.2°, and 29.85 ± 0.2°;

the crystal form B of the ethanesulfonate salt has an X-ray powder diffraction pattern expressed by 2θ angles, and diffraction peaks and relative intensities of the X-ray powder diffraction pattern can further be shown in Table 18;

the X-ray powder diffraction pattern of the crystal form B of the ethanesulfonate salt expressed by 2θ angles can further be basically shown in FIG. 40.

In a certain preferred embodiment, the compound of formula I and ethanesulfonic acid have a molar ratio of 1:1.

In a certain preferred embodiment, the crystal form B of the ethanesulfonate salt has a thermogravimetric analysis (TGA) pattern with a weight loss of 1% to 3% (such as 2.17%) when heated from an onset to 150 ± 5°C (the percentage of the weight loss is the percentage of the weight reduction of the sample relative to the weight of the sample prior to this weight loss).

In a certain preferred embodiment, the crystal form B of the ethanesulfonate salt has a differential scanning calorimetry (DSC) pattern, and the differential scanning calorimetry pattern can have major endothermic peaks at 156.8 ± 3°C and 211.9 ± 3°C.

In a certain preferred embodiment, the DSC pattern of the crystal form B of the ethanesulfonate salt can further be basically shown in FIG. 41.

In a certain preferred embodiment, the TGA pattern of the crystal form B of the ethanesulfonate salt can further be basically shown in FIG. 42.

The present disclosure provides a crystal form A of a maleate salt of the compound of formula I, which has an X-ray powder diffraction pattern expressed by 2θ angles comprising diffraction peaks at: 4.46 ± 0.2°, 5.61 ± 0.2°, 11.23 ± 0.2°, 16.86 ± 0.2°, 18.30 ± 0.2°, 19.20 ± 0.2°, and 19.91 ± 0.2°;

further, the crystal form A of the maleate salt has an X-ray powder diffraction pattern expressed by 2θ angles comprising diffraction peaks at: 4.46 ± 0.2°, 5.61 ± 0.2°, 11.23 ± 0.2°, 15.38 ± 0.2°, 16.86 ± 0.2°, 18.30 ± 0.2°, 19.20 ± 0.2°, 19.91 ± 0.2°, 20.42 ± 0.2°, and 23.00 ± 0.2°.

Further, the crystal form A of the maleate salt has an X-ray powder diffraction pattern expressed by 2θ angles comprising diffraction peaks at: 4.46 ± 0.2°, 5.61 ± 0.2°, 11.23 ± 0.2°, 11.86 ± 0.2°, 15.38 ± 0.2°, 16.86 ± 0.2°, 18.30 ± 0.2°, 19.20 ± 0.2°, 19.91 ± 0.2°, 20.42 ± 0.2°, 23.00 ± 0.2°, and 24.26 ± 0.2°;

even further, the crystal form A of the maleate salt has an X-ray powder diffraction pattern expressed by 2θ angles, and diffraction peaks and relative intensities of the X-ray powder diffraction pattern can further be shown in Table 19;

the X-ray powder diffraction pattern of the crystal form A of the maleate salt expressed by 2θ angles can further be basically shown in FIG. 43.

In a certain preferred embodiment, the compound of formula I and maleic acid have a molar ratio of 1:1.

In a certain preferred embodiment, the crystal form A of the maleate salt has a thermogravimetric analysis (TGA) pattern with a weight loss of 2% to 4% (such as 3.14%) when heated from an onset to 100 ± 5°C and a weight loss of 14% to 16% (such as 14.49%) when heated from 100 ± 5°C to 225 ± 5°C (the percentage of the weight loss is the percentage of the weight reduction of the sample relative to the weight of the sample prior to this weight loss).

In a certain preferred embodiment, the crystal form A of the maleate salt has a differential scanning calorimetry (DSC) pattern, and the differential scanning calorimetry pattern can have major endothermic peaks at 160.4 ± 3°C and 219.1 ± 3°C.

In a certain preferred embodiment, the DSC pattern of the crystal form A of the maleate salt can further be basically shown in FIG. 44.

In a certain preferred embodiment, the TGA pattern of the crystal form A of the maleate salt can further be basically shown in FIG. 45.

The present disclosure provides a crystal form B of a maleate salt of the compound of formula I, which has an X-ray powder diffraction pattern expressed by 2θ angles comprising diffraction peaks at: 4.99 ± 0.2°, 10.46 ± 0.2°, 11.21 ± 0.2°, 16.82 ± 0.2°, 18.30 ± 0.2°, 18.92 ± 0.2°, and 19.50 ± 0.2°;

further, the crystal form B of the maleate salt has an X-ray powder diffraction pattern expressed by 2θ angles comprising diffraction peaks at: 4.99 ± 0.2°, 6.99 ± 0.2°, 10.46 ± 0.2°, 11.21 ± 0.2°, 15.02 ± 0.2°, 15.60 ± 0.2°, 16.82 ± 0.2°, 18.30 ± 0.2°, 18.92 ± 0.2°, and 19.50 ± 0.2°.

The crystal form B of the maleate salt has an X-ray powder diffraction pattern expressed by 2θ angles, and diffraction peaks and relative intensities of the X-ray powder diffraction pattern can further be shown in Table 20;

the X-ray powder diffraction pattern of the crystal form B of the maleate salt expressed by 2θ angles can further be basically shown in FIG. 46.

In a certain preferred embodiment, the compound of formula I and maleic acid have a molar ratio of 1:1.

In a certain preferred embodiment, the crystal form B of the maleate salt has a thermogravimetric analysis (TGA) pattern with a weight loss of 2% to 4% (such as 2.84%) when heated from an onset to 100 ± 5°C and a weight loss of 11% to 13% (such as 12.43%) when heated from 100 ± 5°C to 225 ± 5°C (the percentage of the weight loss is the percentage of the weight reduction of the sample relative to the weight of the sample prior to this weight loss).

In a certain preferred embodiment, the crystal form B of the maleate salt has a differential scanning calorimetry (DSC) pattern, and the differential scanning calorimetry pattern can have major endothermic peaks at 152.9 ± 3°C and 220.7 ± 3°C.

In a certain preferred embodiment, the DSC pattern of the crystal form B of the maleate salt can further be basically shown in FIG. 47.

In a certain preferred embodiment, the TGA pattern of the crystal form B of the maleate salt can further be basically shown in FIG. 48.

The present disclosure provides a crystal form A of an L-tartrate salt of the compound of formula I, which has an X-ray powder diffraction pattern expressed by 2θ angles comprising diffraction peaks at: 9.28 ± 0.2°, 11.38 ± 0.2°, 15.62 ± 0.2°, 19.54 ± 0.2°, 19.89 ± 0.2°, and 25.21 ± 0.2°.

Further, the crystal form A of the L-tartrate salt has an X-ray powder diffraction pattern expressed by 2θ angles comprising diffraction peaks at: 9.28 ± 0.2°, 11.38 ± 0.2°, 12.97 ± 0.2°, 15.62 ± 0.2°, 16.95 ± 0.2°, 18.07 ± 0.2°, 19.54 ± 0.2°, 19.89 ± 0.2°, 22.94 ± 0.2°, 23.51 ± 0.2°, 25.21 ± 0.2°, 26.92 ± 0.2°, and 28.46 ± 0.2°;

the crystal form A of the L-tartrate salt has an X-ray powder diffraction pattern expressed by 2θ angles, and diffraction peaks and relative intensities of the X-ray powder diffraction pattern can further be shown in Table 21;

the X-ray powder diffraction pattern of the crystal form A of the L-tartrate salt expressed by 2θ angles can further be basically shown in FIG. 49.

In a certain preferred embodiment, the compound of formula I and L-tartaric acid have a molar ratio of 1:1.

In a certain preferred embodiment, the crystal form A of the L-tartrate salt has a thermogravimetric analysis (TGA) pattern with a weight loss of 2% to 4% (such as 3.18%) when heated from an onset to 150 ± 5°C (the percentage of the weight loss is the percentage of the weight reduction of the sample relative to the weight of the sample prior to this weight loss).

In a certain preferred embodiment, the crystal form A of the L-tartrate salt has a differential scanning calorimetry (DSC) pattern, and the differential scanning calorimetry pattern can have major endothermic peaks at 82.2 ± 3°C and 213.2 ± 3°C.

In a certain preferred embodiment, the DSC pattern of the crystal form A of the L-tartrate salt can further be basically shown in FIG. 50.

In a certain preferred embodiment, the TGA pattern of the crystal form A of the L-tartrate salt can further be basically shown in FIG. 51.

The present disclosure provides a crystal form B of an L-tartrate salt of the compound of formula I, which has an X-ray powder diffraction pattern expressed by 2θ angles comprising diffraction peaks at: 4.22 ± 0.2°, 5.17 ± 0.2°, 5.77 ± 0.2°, 8.65 ± 0.2°, 11.51 ± 0.2°, 14.08 ± 0.2°, 16.59 ± 0.2°, 17.29 ± 0.2°, and 23.11 ± 0.2°;

further, the crystal form B of the L-tartrate salt has an X-ray powder diffraction pattern expressed by 2θ angles comprising diffraction peaks at: 4.22 ± 0.2°, 5.17 ± 0.2°, 5.77 ± 0.2°, 8.65 ± 0.2°, 10.09 ± 0.2°, 11.51 ± 0.2°, 14.08 ± 0.2°, 16.59 ± 0.2°, 17.29 ± 0.2°, 18.20 ± 0.2°, 18.64 ± 0.2°, 19.75 ± 0.2°, 20.22 ± 0.2°, 20.74 ± 0.2°, 21.95 ± 0.2°, and 23.11 ± 0.2°;
further, the crystal form B of the L-tartrate salt has an X-ray powder diffraction pattern expressed by 2θ angles comprising diffraction peaks at: 4.22 ± 0.2°, 5.17 ± 0.2°, 5.77 ± 0.2°, 8.65 ± 0.2°, 10.09 ± 0.2°, 11.51 ± 0.2°, 12.58 ± 0.2°, 14.08 ± 0.2°, 14.91 ± 0.2°, 15.59 ± 0.2°, 16.41 ± 0.2°, 16.59 ± 0.2°, 17.29 ± 0.2°, 18.20 ± 0.2°, 18.64 ± 0.2°, 19.75 ± 0.2°, 20.22 ± 0.2°, 20.74 ± 0.2°, 21.95 ± 0.2°, 23.11 ± 0.2°, 24.84 ± 0.2°, 25.27 ± 0.2°, and 25.56 ± 0.2°;
even further, the crystal form B of the L-tartrate salt has an X-ray powder diffraction pattern expressed by 2θ angles, and diffraction peaks and relative intensities of the X-ray powder diffraction pattern can further be shown in Table 22;
the X-ray powder diffraction pattern of the crystal form B of the L-tartrate salt expressed by 2θ angles can further be basically shown in FIG. 52.

In a certain preferred embodiment, the compound of formula I and L-tartaric acid have a molar ratio of 1:1.
In a certain preferred embodiment, the crystal form B of the L-tartrate salt has a thermogravimetric analysis (TGA) pattern with a weight loss of 5% to 7% (such as 5.57%) when heated from an onset to 180 ± 5°C (the percentage of the weight loss is the percentage of the weight reduction of the sample relative to the weight of the sample prior to this weight loss).
In a certain preferred embodiment, the crystal form B of the L-tartrate salt has a differential scanning calorimetry (DSC) pattern, and the differential scanning calorimetry pattern can have major endothermic peaks at 114.5 ± 3°C, 160.5 ± 3°C, 193.2 ± 3°C, and 219.5 ± 3°C.
In a certain preferred embodiment, the DSC pattern of the crystal form B of the L-tartrate salt can further be basically shown in FIG. 53.
In a certain preferred embodiment, the TGA pattern of the crystal form B of the L-tartrate salt can further be basically shown in FIG. 54.
The present disclosure provides a crystal form C of an L-tartrate salt of the compound of formula I, which has an X-ray powder diffraction pattern expressed by 2θ angles comprising diffraction peaks at: 5.77 ± 0.2°, 11.54 ± 0.2°, 16.50 ± 0.2°, 17.33 ± 0.2°, 18.46 ± 0.2°, 19.80 ± 0.2°, and 24.85 ± 0.2°;
further, the crystal form C of the L-tartrate salt has an X-ray powder diffraction pattern expressed by 2θ angles comprising diffraction peaks at: 4.60 ± 0.2°, 5.77 ± 0.2°, 11.54 ± 0.2°, 14.06 ± 0.2°, 16.50 ± 0.2°, 17.33 ± 0.2°, 18.46 ± 0.2°, 19.80 ± 0.2°, 21.91 ± 0.2°, 23.16 ± 0.2°, 24.85 ± 0.2°, and 25.27 ± 0.2°.
Further, the crystal form C of the L-tartrate salt has an X-ray powder diffraction pattern expressed by 2θ angles comprising diffraction peaks at: 4.60 ± 0.2°, 5.77 ± 0.2°, 9.20 ± 0.2°, 11.54 ± 0.2°, 12.07 ± 0.2°, 12.56 ± 0.2°, 14.06 ± 0.2°, 14.68 ± 0.2°, 16.50 ± 0.2°, 17.33 ± 0.2°, 18.46 ± 0.2°, 19.10 ± 0.2°, 19.80 ± 0.2°, 20.56 ± 0.2°, 21.91 ± 0.2°, 23.16 ± 0.2°, 24.16 ± 0.2°, 24.85 ± 0.2°, and 25.27 ± 0.2°;

even further, the crystal form C of the L-tartrate salt has an X-ray powder diffraction pattern expressed by 2θ angles, and diffraction peaks and relative intensities of the X-ray powder diffraction pattern can further be shown in Table 23;
the X-ray powder diffraction pattern of the crystal form C of the L-tartrate salt expressed by 2θ angles can further be basically shown in FIG. 55.

In a certain preferred embodiment, the compound of formula I and L-tartaric acid have a molar ratio of 1:0.5.
In a certain preferred embodiment, the crystal form C of the L-tartrate salt has a thermogravimetric analysis (TGA) pattern with a weight loss of 2% to 4% (such as 3.16%) when heated from an onset to 150 ± 5°C (the percentage of the weight loss is the percentage of the weight reduction of the sample relative to the weight of the sample prior to this weight loss).
In a certain preferred embodiment, the crystal form C of the L-tartrate salt has a differential scanning calorimetry (DSC) pattern, and the differential scanning calorimetry pattern can have major endothermic peaks at 128.5 ± 3°C, 157.1 ± 3°C, and 220.5 ± 3°C.
In a certain preferred embodiment, the DSC pattern of the crystal form C of the L-tartrate salt can further be basically shown in FIG. 56.
In a certain preferred embodiment, the TGA pattern of the crystal form C of the L-tartrate salt can further be basically shown in FIG. 57.
The present disclosure provides a crystal form D of an L-tartrate salt of the compound of formula I, which has an X-ray powder diffraction pattern expressed by 2θ angles comprising diffraction peaks at: 5.80 ± 0.2°, 11.56 ± 0.2°, 15.74 ± 0.2°, 16.50 ± 0.2°, 17.38 ± 0.2°, 19.63 ± 0.2°, 23.23 ± 0.2°, 23.64 ± 0.2°, 24.86 ± 0.2°, and 25.28 ± 0.2°;

further, the crystal form D of the L-tartrate salt has an X-ray powder diffraction pattern expressed by 2θ angles comprising diffraction peaks at: 5.80 ± 0.2°, 11.56 ± 0.2°, 15.74 ± 0.2°, 16.50 ± 0.2°, 17.38 ± 0.2°, 18.24 ± 0.2°, 19.35 ± 0.2°, 19.63 ± 0.2°, 20.02 ± 0.2°, 23.23 ± 0.2°, 23.64 ± 0.2°, 24.86 ± 0.2°, and 25.28 ± 0.2°;
further, the crystal form D of the L-tartrate salt has an X-ray powder diffraction pattern expressed by 2θ angles comprising diffraction peaks at: 4.24 ± 0.2°, 5.80 ± 0.2°, 9.45 ± 0.2°, 11.56 ± 0.2°, 13.07 ± 0.2°, 15.74 ± 0.2°, 16.50 ± 0.2°, 17.38 ± 0.2°, 18.24 ± 0.2°, 19.35 ± 0.2°, 19.63 ± 0.2°, 20.02 ± 0.2°, 20.48 ± 0.2°, 20.70 ± 0.2°, 21.93 ± 0.2°, 23.23 ± 0.2°, 23.64 ± 0.2°, 24.86 ± 0.2°, and 25.28 ± 0.2°;
even further, the crystal form D of the L-tartrate salt has an X-ray powder diffraction pattern expressed by 2θ angles, and diffraction peaks and relative intensities of the X-ray powder diffraction pattern can further be shown in Table 24; the X-ray powder diffraction pattern of the crystal form D of the L-tartrate salt expressed by 2θ angles can further be basically shown in FIG. 58.

In a certain preferred embodiment, the compound of formula I and L-tartaric acid have a molar ratio of 1:1.
In a certain preferred embodiment, the crystal form D of the L-tartrate salt has a thermogravimetric analysis (TGA) pattern with a weight loss of 2% to 4% (such as 2.72%) when heated from an onset to 150 ± 5°C (the percentage of the weight loss is the percentage of the weight reduction of the sample relative to the weight of the sample prior to this weight loss).
In a certain preferred embodiment, the crystal form D of the L-tartrate salt has a differential scanning calorimetry (DSC) pattern, and the differential scanning calorimetry pattern can have major endothermic peaks at 75.0± 3°C, 111.6 ± 3°C, 161.9 ± 3°C, 200.9 ± 3°C, and 218.8 ± 3°C.
In a certain preferred embodiment, the DSC pattern of the crystal form D of the L-tartrate salt can further be basically shown in FIG. 59.
In a certain preferred embodiment, the TGA pattern of the crystal form D of the L-tartrate salt can further be basically shown in FIG. 60.
The present disclosure provides a crystal form A of a glycolate salt of the compound of formula I, which has an X-ray powder diffraction pattern expressed by 2θ angles comprising diffraction peaks at: 5.90 ± 0.2°, 7.21 ± 0.2°, 7.80 ± 0.2°, 11.42 ± 0.2°, 11.79 ± 0.2°, 17.72 ± 0.2°, 19.80 ± 0.2°, 23.10 ± 0.2°, and 23.71 ± 0.2°;
further, the crystal form A of the glycolate salt has an X-ray powder diffraction pattern expressed by 2θ angles comprising diffraction peaks at: 5.90 ± 0.2°, 7.21 ± 0.2°, 7.80 ± 0.2°, 11.42 ± 0.2°, 11.79 ± 0.2°, 13.24 ± 0.2°, 14.74 ± 0.2°, 16.45 ± 0.2°, 17.72 ± 0.2°, 18.65 ± 0.2°, 19.80 ± 0.2°, 20.54 ± 0.2°, 21.96 ± 0.2°, 23.10 ± 0.2°, and 23.71 ± 0.2°.
Further, the crystal form A of the glycolate salt has an X-ray powder diffraction pattern expressed by 2θ angles comprising diffraction peaks at: 5.90 ± 0.2°, 7.21 ± 0.2°, 7.80 ± 0.2°, 8.83 ± 0.2°, 9.34 ± 0.2°, 11.03 ± 0.2°, 11.42 ± 0.2°, 11.79 ± 0.2°, 12.18 ± 0.2°, 13.24 ± 0.2°, 13.49 ± 0.2°, 14.43 ± 0.2°, 14.74 ± 0.2°, 15.60 ± 0.2°, 15.86 ± 0.2°, 16.16 ± 0.2°, 16.45 ± 0.2°, 16.98 ± 0.2°, 17.72 ± 0.2°, 18.65 ± 0.2°, 19.80 ± 0.2°, 20.54 ± 0.2°, 21.96 ± 0.2°, 23.10 ± 0.2°, and 23.71 ± 0.2°;

even further, the crystal form A of the glycolate salt has an X-ray powder diffraction pattern expressed by 2θ angles, and diffraction peaks and relative intensities of the X-ray powder diffraction pattern can further be shown in Table 25; the X-ray powder diffraction pattern of the crystal form A of the glycolate salt expressed by 2θ angles can further be basically shown in FIG. 61.

In a certain preferred embodiment, the compound of formula I and glycolic acid have a molar ratio of 1:1.
In a certain preferred embodiment, the crystal form A of the glycolate salt has a thermogravimetric analysis (TGA) pattern with a weight loss of 2% to 4% (such as 3.40%) when heated from an onset to 150 ± 5°C (the percentage of the weight loss is the percentage of the weight reduction of the sample relative to the weight of the sample prior to this weight loss).
In a certain preferred embodiment, the crystal form A of the glycolate salt has a differential scanning calorimetry (DSC) pattern, and the differential scanning calorimetry pattern can have a major endothermic peak at 110.1 ± 3°C.
In a certain preferred embodiment, the DSC pattern of the crystal form A of the glycolate salt can further be basically shown in FIG. 62.
In a certain preferred embodiment, the TGA pattern of the crystal form A of the glycolate salt can further be basically shown in FIG. 63.
The present disclosure provides a crystal form A of an L-malate salt of the compound of formula I, which has an X-ray powder diffraction pattern expressed by 2θ angles comprising diffraction peaks at: 5.92 ± 0.2°, 11.82 ± 0.2°, 16.56 ± 0.2°, 17.68 ± 0.2°, 21.97 ± 0.2°, and 23.75 ± 0.2°;

further, the crystal form A of the L-malate salt has an X-ray powder diffraction pattern expressed by 2θ angles comprising diffraction peaks at: 5.92 ± 0.2°, 7.54 ± 0.2°, 8.32 ± 0.2°, 8.76 ± 0.2°, 11.82 ± 0.2°, 16.56 ± 0.2°, 17.68 ± 0.2°, 18.71 ± 0.2°, 19.69 ± 0.2°, 21.97 ± 0.2°, and 23.75 ± 0.2°.

Further, the crystal form A of the L-malate salt has an X-ray powder diffraction pattern expressed by 2θ angles comprising diffraction peaks at: 5.92 ± 0.2°, 7.54 ± 0.2°, 8.32 ± 0.2°, 8.76 ± 0.2°, 10.35 ± 0.2°, 11.82 ± 0.2°, 12.40 ± 0.2°, 14.10 ± 0.2°, 16.56 ± 0.2°, 17.68 ± 0.2°, 18.71 ± 0.2°, 19.69 ± 0.2°, 20.81 ± 0.2°, 21.97 ± 0.2°, and 23.75 ± 0.2°;

even further, the crystal form A of the L-malate salt has an X-ray powder diffraction pattern expressed by 2θ angles, and diffraction peaks and relative intensities of the X-ray powder diffraction pattern can further be shown in Table 26; the X-ray powder diffraction pattern of the crystal form A of the L-malate salt expressed by 2θ angles can further be basically shown in FIG. 64.

In a certain preferred embodiment, the compound of formula I and L-malic acid have a molar ratio of 1:1.

In a certain preferred embodiment, the crystal form A of the L-malate salt has a thermogravimetric analysis (TGA) pattern with a weight loss of 2% to 4% (such as 2.88%) when heated from an onset to 50 ± 5°C, a weight loss of 1% to 3% (such as 2.46%) when heated from 50 ± 5°C to 100 ± 5°C, and a weight loss of 0% to 2% (such as 1.31%) when heated from 100 ± 5°C to 150 ± 5°C (the percentage of the weight loss is the percentage of the weight reduction of the sample relative to the weight of the sample prior to this weight loss).

In a certain preferred embodiment, the crystal form A of the L-malate salt has a differential scanning calorimetry (DSC) pattern, and the differential scanning calorimetry pattern can have major endothermic peaks at 72.9 ± 3°C, 122.2 ± 3°C, 142.1 ± 3°C, and 222.4 ± 3°C and an exothermic peak at 195.9 ± 3°C.

In a certain preferred embodiment, the DSC pattern of the crystal form A of the L-malate salt can further be basically shown in FIG. 65.

In a certain preferred embodiment, the TGA pattern of the crystal form A of the L-malate salt can further be basically shown in FIG. 66.

The present disclosure provides a crystal form B of an L-malate salt of the compound of formula I, which has an X-ray powder diffraction pattern expressed by 2θ angles comprising diffraction peaks at: 6.69 ± 0.2°, 8.99 ± 0.2°, 9.30 ± 0.2°, 13.38 ± 0.2°, 17.00 ± 0.2°, 18.71 ± 0.2°, 21.18 ± 0.2°, and 26.89 ± 0.2°.

Further, the crystal form B of the L-malate salt has an X-ray powder diffraction pattern expressed by 2θ angles comprising diffraction peaks at: 6.69 ± 0.2°, 8.99 ± 0.2°, 9.30 ± 0.2°, 10.69 ± 0.2°, 10.97 ± 0.2°, 11.29 ± 0.2°, 12.77 ± 0.2°, 13.02 ± 0.2°, 13.38 ± 0.2°, 14.64 ± 0.2°, 14.89 ± 0.2°, 15.89 ± 0.2°, 17.00 ± 0.2°, 17.55 ± 0.2°, 18.27 ± 0.2°, 18.71 ± 0.2°, 20.70 ± 0.2°, 21.18 ± 0.2°, 21.71 ± 0.2°, and 26.89 ± 0.2°;

even further, the crystal form B of the L-malate salt has an X-ray powder diffraction pattern expressed by 2θ angles, and diffraction peaks and relative intensities of the X-ray powder diffraction pattern can further be shown in Table 27; the X-ray powder diffraction pattern of the crystal form B of the L-malate salt expressed by 2θ angles can further be basically shown in FIG. 67.

In a certain preferred embodiment, the compound of formula I and L-malic acid have a molar ratio of 1:1.

In a certain preferred embodiment, the crystal form B of the L-malate salt has a thermogravimetric analysis (TGA) pattern with a weight loss of 2% to 4% (such as 3.40%) when heated from an onset to 150 ± 5°C (the percentage of the weight loss is the percentage of the weight reduction of the sample relative to the weight of the sample prior to this weight loss).

In a certain preferred embodiment, the crystal form B of the L-malate salt has a differential scanning calorimetry (DSC) pattern, and the differential scanning calorimetry pattern can have a major endothermic peak at 134.8 ± 3°C.

In a certain preferred embodiment, the DSC pattern of the crystal form B of the L-malate salt can further be basically shown in FIG. 68.

In a certain preferred embodiment, the TGA pattern of the crystal form B of the L-malate salt can further be basically shown in FIG. 69.

The present disclosure provides a crystal form A of a hippurate salt of the compound of formula I, which has an X-ray powder diffraction pattern expressed by 2θ angles comprising diffraction peaks at: 9.10 ± 0.2°, 10.41 ± 0.2°, 12.69 ± 0.2°, 15.00 ± 0.2°, 15.22 ± 0.2°, 16.93 ± 0.2°, 18.00 ± 0.2°, 18.71 ± 0.2°, 24.53 ± 0.2°, and 26.00 ± 0.2°;
further, the crystal form A of the hippurate salt has an X-ray powder diffraction pattern expressed by 2θ angles comprising diffraction peaks at: 9.10 ± 0.2°, 10.41 ± 0.2°, 12.69 ± 0.2°, 12.91 ± 0.2°, 15.00 ± 0.2°, 15.22 ± 0.2°, 16.02 ± 0.2°, 16.57 ± 0.2°, 16.93 ± 0.2°, 17.69 ± 0.2°, 18.00 ± 0.2°, 18.71 ± 0.2°, 20.65 ± 0.2°, 21.27 ± 0.2°, 22.33 ± 0.2°, 24.53 ± 0.2°, and 26.00 ± 0.2°.

Further, the crystal form A of the hippurate salt has an X-ray powder diffraction pattern expressed by 2θ angles comprising diffraction peaks at: 8.78 ± 0.2°, 9.10 ± 0.2°, 10.41 ± 0.2°, 12.69 ± 0.2°, 12.91 ± 0.2°, 15.00 ± 0.2°, 15.22 ±

0.2°, 16.02 ± 0.2°, 16.57 ± 0.2°, 16.93 ± 0.2°, 17.69 ± 0.2°, 18.00 ± 0.2°, 18.71 ± 0.2°, 20.40 ± 0.2°, 20.65 ± 0.2°, 20.89 ± 0.2°, 21.27 ± 0.2°, 22.33 ± 0.2°, 24.53 ± 0.2°, 24.87 ± 0.2°, 25.50 ± 0.2°, 26.00 ± 0.2°, 26.14 ± 0.2°, 27.64 ± 0.2°, 28.43 ± 0.2°, 29.31 ± 0.2°, 29.89 ± 0.2°, 31.11 ± 0.2°, and 32.36 ± 0.2°.

Even further, the crystal form A of the hippurate salt has an X-ray powder diffraction pattern expressed by 2θ angles, and diffraction peaks and relative intensities of the X-ray powder diffraction pattern can further be shown in Table 28;

the X-ray powder diffraction pattern of the crystal form A of the hippurate salt expressed by 2θ angles can further be basically shown in FIG. 70.

In a certain preferred embodiment, the compound of formula I and hippuric acid have a molar ratio of 1:1.

In a certain preferred embodiment, the crystal form A of the hippurate salt has a thermogravimetric analysis (TGA) pattern with a weight loss of 2% to 4% (such as 3.04%) when heated from an onset to 150 ± 5°C (the percentage of the weight loss is the percentage of the weight reduction of the sample relative to the weight of the sample prior to this weight loss).

**In** a certain preferred embodiment, the crystal form A of the hippurate salt has a differential scanning calorimetry (DSC) pattern, and the differential scanning calorimetry pattern can have major endothermic peaks at 62.4 ± 3°C, 120.1 ± 3°C, and 207.0 ± 3°C.

**In** a certain preferred embodiment, the DSC pattern of the crystal form A of the hippurate salt can further be basically shown in FIG. 71.

**In** a certain preferred embodiment, the TGA pattern of the crystal form A of the hippurate salt can further be basically shown in FIG. 72.

The present disclosure provides a crystal form A of a succinate salt of the compound of formula I, which has an X-ray powder diffraction pattern expressed by 2θ angles comprising diffraction peaks at: 4.72 ± 0.2°, 5.83 ± 0.2°, 7.87 ± 0.2°, 9.34 ± 0.2°, 11.71 ± 0.2°, 12.39 ± 0.2°, 16.67 ± 0.2°, 17.58 ± 0.2°, 19.99 ± 0.2°, 21.89 ± 0.2°, 23.57 ± 0.2°, and 25.40 ± 0.2°.

Further, the crystal form A of the succinate salt has an X-ray powder diffraction pattern expressed by 2θ angles, and diffraction peaks and relative intensities of the X-ray powder diffraction pattern can further be shown in Table 29.

The X-ray powder diffraction pattern of the crystal form A of the succinate salt expressed by 2θ angles can further be basically shown in FIG. 128.

In a certain preferred embodiment, the compound of formula I and succinic acid have a molar ratio of 1:1.

In a certain preferred embodiment, the crystal form A of the succinate salt has a thermogravimetric analysis (TGA) pattern with a weight loss of 3% to 5% (such as 3.94%) when heated from an onset to 150 ± 5°C (the percentage of the weight loss is the percentage of the weight reduction of the sample relative to the weight of the sample prior to this weight loss).

In a certain preferred embodiment, the crystal form A of the succinate salt has a differential scanning calorimetry (DSC) pattern, and the differential scanning calorimetry pattern can have major endothermic peaks at 70.0 ± 3°C, 124.1 ± 3°C, and 221.0 ± 3°C.

In a certain preferred embodiment, the DSC pattern of the crystal form A of the succinate salt can further be basically shown in FIG. 73.

In a certain preferred embodiment, the TGA pattern of the crystal form A of the succinate salt can further be basically shown in FIG. 74.

The present disclosure provides a crystal form B of a succinate salt of the compound of formula I, which has an X-ray powder diffraction pattern expressed by 2θ angles comprising diffraction peaks at: 5.70 ± 0.2°, 9.24 ± 0.2°, 11.42 ± 0.2°, 13.55 ± 0.2°, 17.17 ± 0.2°, and 22.95 ± 0.2°;

further, the crystal form B of the succinate salt has an X-ray powder diffraction pattern expressed by 2θ angles comprising diffraction peaks at: 5.70 ± 0.2°, 9.24 ± 0.2°, 11.42 ± 0.2°, 13.55 ± 0.2°, 15.10 ± 0.2°, 17.17 ± 0.2°, 18.67 ± 0.2°, 19.13 ± 0.2°, 19.99 ± 0.2°, 20.59 ± 0.2°, 22.95 ± 0.2°, 23.25 ± 0.2°, 24.10 ± 0.2°, 25.13 ± 0.2°, 25.48 ± 0.2°, and 26.12 ± 0.2°;

further, the crystal form B of the succinate salt has an X-ray powder diffraction pattern expressed by 2θ angles comprising diffraction peaks at: 5.70 ± 0.2°, 6.75 ± 0.2°, 9.24 ± 0.2°, 11.42 ± 0.2°, 12.31 ± 0.2°, 13.13 ± 0.2°, 13.55 ± 0.2°, 15.10 ± 0.2°, 15.80 ± 0.2°, 16.35 ± 0.2°, 17.17 ± 0.2°, 18.67 ± 0.2°, 19.13 ± 0.2°, 19.50 ± 0.2°, 19.99 ± 0.2°, 20.59 ± 0.2°, 21.60 ± 0.2°, 21.93 ± 0.2°, 22.58 ± 0.2°, 22.95 ± 0.2°, 23.25 ± 0.2°, 24.10 ± 0.2°, 25.13 ± 0.2°, 25.48 ± 0.2°, 25.79 ± 0.2°, 26.12 ± 0.2°, 28.98 ± 0.2°, and 31.50 ± 0.2°;

further, the crystal form B of the succinate salt has an X-ray powder diffraction pattern expressed by 2θ angles, and diffraction peaks and relative intensities of the X-ray powder diffraction pattern can further be shown in Table 30;

the X-ray powder diffraction pattern of the crystal form B of the succinate salt expressed by 2θ angles can further be basically shown in FIG. 75.

In a certain preferred embodiment, the compound of formula I and succinic acid have a molar ratio of 1:1.

In a certain preferred embodiment, the crystal form B of the succinate salt has a thermogravimetric analysis (TGA) pattern with a weight loss of 3% to 5% (such as 4.44%) when heated from an onset to 150 ± 5°C (the percentage of the weight loss is the percentage of the weight reduction of the sample relative to the weight of the sample prior to this weight loss).

In a certain preferred embodiment, the crystal form B of the succinate salt has a differential scanning calorimetry (DSC) pattern, and the differential scanning calorimetry pattern can have major endothermic peaks at 105.1 ± 3°C and 223.3 ± 3°C.

In a certain preferred embodiment, the DSC pattern of the crystal form B of the succinate salt can further be basically shown in FIG. 76.

In a certain preferred embodiment, the TGA pattern of the crystal form B of the succinate salt can further be basically shown in FIG. 77.

The present disclosure provides a crystal form A of an ascorbate salt of the compound of formula I, which has an X-ray powder diffraction pattern expressed by 2θ angles comprising diffraction peaks at: 5.72 ± 0.2°, 9.20 ± 0.2°, 11.30 ± 0.2°, 15.55 ± 0.2°, 16.71 ± 0.2°, 17.58 ± 0.2°, 18.83 ± 0.2°, and 25.94 ± 0.2°;

further, the crystal form A of the ascorbate salt has an X-ray powder diffraction pattern expressed by 2θ angles comprising diffraction peaks at: 5.72 ± 0.2°, 9.20 ± 0.2°, 11.30 ± 0.2°, 14.38 ± 0.2°, 15.55 ± 0.2°, 16.71 ± 0.2°, 17.58 ± 0.2°, 18.83 ± 0.2°, 20.30 ± 0.2°, 22.33 ± 0.2°, and 25.94 ± 0.2°.

Further, the crystal form A of the ascorbate salt has an X-ray powder diffraction pattern expressed by 2θ angles, and diffraction peaks and relative intensities of the X-ray powder diffraction pattern can further be shown in Table 31.

The X-ray powder diffraction pattern of the crystal form A of the ascorbate salt expressed by 2θ angles can further be shown in FIG. 129.

In a certain preferred embodiment, the compound of formula I and ascorbic acid have a molar ratio of 1:0.5.

In a certain preferred embodiment, the crystal form A of the ascorbate salt has a thermogravimetric analysis (TGA) pattern with a weight loss of 3% to 5% (such as 4.36%) when heated from an onset to 150 ± 5°C (the percentage of the weight loss is the percentage of the weight reduction of the sample relative to the weight of the sample prior to this weight loss).

In a certain preferred embodiment, the crystal form A of the ascorbate salt has a differential scanning calorimetry (DSC) pattern, and the differential scanning calorimetry pattern can have a major endothermic peak at 185.7 ± 3°C and one exothermic peak at 195.8 ± 3°C.

In a certain preferred embodiment, the DSC pattern of the crystal form A of the ascorbate salt can further be basically shown in FIG. 78.

In a certain preferred embodiment, the TGA pattern of the crystal form A of the ascorbate salt can further be basically shown in FIG. 79.

The present disclosure provides a crystal form B of an ascorbate salt of the compound of formula I, which has an X-ray powder diffraction pattern expressed by 2θ angles comprising diffraction peaks at: 5.50 ± 0.2°, 11.00 ± 0.2°, 16.63 ± 0.2°, 17.47 ± 0.2°, 19.82 ± 0.2°, 28.08 ± 0.2°, and 30.05 ± 0.2°;

further, the crystal form B of the ascorbate salt has an X-ray powder diffraction pattern expressed by 2θ angles comprising diffraction peaks at: 4.47 ± 0.2°, 5.50 ± 0.2°, 9.11 ± 0.2°, 11.00 ± 0.2°, 15.27 ± 0.2°, 16.09 ± 0.2°, 16.63 ± 0.2°, 17.47 ± 0.2°, 19.82 ± 0.2°, 25.30 ± 0.2°, 28.08 ± 0.2°, and 30.05 ± 0.2°.

Further, the crystal form B of the ascorbate salt has an X-ray powder diffraction pattern expressed by 2θ angles comprising diffraction peaks at: 4.47 ± 0.2°, 5.50 ± 0.2°, 9.11 ± 0.2°, 11.00 ± 0.2°, 13.23 ± 0.2°, 15.27 ± 0.2°, 16.09 ± 0.2°, 16.63 ± 0.2°, 17.47 ± 0.2°, 19.82 ± 0.2°, 21.09 ± 0.2°, 21.53 ± 0.2°, 25.30 ± 0.2°, 25.92 ± 0.2°, 26.81 ± 0.2°, 28.08 ± 0.2°, and 30.05 ± 0.2°;

even further, the crystal form B of the ascorbate salt has an X-ray powder diffraction pattern expressed by 2θ angles, and diffraction peaks and relative intensities of the X-ray powder diffraction pattern can be shown in Table 32;
the X-ray powder diffraction pattern of the crystal form B of the ascorbate salt expressed by 2θ angles can further be basically shown in FIG. 80.

In a certain preferred embodiment, the compound of formula I and ascorbic acid have a molar ratio of 1:1.

In a certain preferred embodiment, the crystal form B of the ascorbate salt has a thermogravimetric analysis (TGA) pattern with a weight loss of 0% to 2% (such as 1.33%) when heated from an onset to 150 ± 5°C (the percentage of the weight loss is the percentage of the weight reduction of the sample relative to the weight of the sample prior to this weight loss).

In a certain preferred embodiment, the crystal form B of the ascorbate salt has a differential scanning calorimetry (DSC) pattern, and the differential scanning calorimetry pattern can have a major endothermic peak at 153.6 ± 3°C and one exothermic peak at 190.3 ± 3°C.

In a certain preferred embodiment, the DSC pattern of the crystal form B of the ascorbate salt can further be basically shown in FIG. 81.

In a certain preferred embodiment, the TGA pattern of the crystal form B of the ascorbate salt can further be basically shown in FIG. 82.

The present disclosure provides a crystal form A of an adipate salt of a compound of formula I, which has an X-ray powder diffraction pattern expressed by 2θ angles comprising diffraction peaks at: 8.47 ± 0.2°, 11.41 ± 0.2°, 15.57 ± 0.2°, 16.29 ± 0.2°, 17.52 ± 0.2°, 18.23 ± 0.2°, and 19.84 ± 0.2°;

further, the crystal form A of the adipate salt has an X-ray powder diffraction pattern expressed by 2θ angles comprising diffraction peaks at: 8.47 ± 0.2°, 8.99 ± 0.2°, 11.41 ± 0.2°, 12.40 ± 0.2°, 13.60 ± 0.2°, 15.57 ± 0.2°, 16.29 ± 0.2°, 17.52 ± 0.2°, 18.23 ± 0.2°, 19.84 ± 0.2°, 21.29 ± 0.2°, 23.81 ± 0.2°, 24.62 ± 0.2°, 26.55 ± 0.2°, 27.79 ± 0.2°, and 29.82 ± 0.2°.

Further, the crystal form A of the adipate salt has an X-ray powder diffraction pattern expressed by 2θ angles, and diffraction peaks and relative intensities of the X-ray powder diffraction pattern can further be shown in Table 33.

The X-ray powder diffraction pattern of the crystal form A of the adipate salt expressed by 2θ angles can further be shown in FIG. 130.

**In** a certain preferred embodiment, the compound of formula I and adipic acid have a molar ratio of 1:1.

**In** a certain preferred embodiment, the crystal form A of the adipate salt has a thermogravimetric analysis (TGA) pattern with a weight loss of 6% to 8% (such as 6.60%) when heated from an onset to 150 ± 5°C (the percentage of the weight loss is the percentage of the weight reduction of the sample relative to the weight of the sample prior to this weight loss).

In a certain preferred embodiment, the crystal form A of the adipate salt has a differential scanning calorimetry (DSC) pattern, and the differential scanning calorimetry pattern can have a major endothermic peak at 88.3 ± 3°C.

In a certain preferred embodiment, the DSC pattern of the crystal form A of the adipate salt can further be basically shown in FIG. 83.

In a certain preferred embodiment, the TGA pattern of the crystal form A of the adipate salt can further be basically shown in FIG. 84.

The present disclosure provides a crystal form A of a p-toluenesulfonate salt of the compound of formula I, which has an X-ray powder diffraction pattern expressed by 2θ angles comprising diffraction peaks at: 8.91 ± 0.2°, 9.43 ± 0.2°, 12.31 ± 0.2°, 15.93 ± 0.2°, 16.43 ± 0.2°, 20.69 ± 0.2°, 20.95 ± 0.2°, 24.76 ± 0.2°, and 26.36 ± 0.2°;

further, the crystal form A of the p-toluenesulfonate salt has an X-ray powder diffraction pattern expressed by 2θ angles comprising diffraction peaks at: 8.91 ± 0.2°, 9.43 ± 0.2°, 12.31 ± 0.2°, 15.93 ± 0.2°, 16.43 ± 0.2°, 17.26 ± 0.2°, 17.91 ± 0.2°, 18.31 ± 0.2°, 20.69 ± 0.2°, 20.95 ± 0.2°, 23.11 ± 0.2°, 23.29 ± 0.2°, 24.76 ± 0.2°, and 26.36 ± 0.2°.

Further, the crystal form A of the p-toluenesulfonate salt has an X-ray powder diffraction pattern expressed by 2θ angles comprising diffraction peaks at: 8.91 ± 0.2°, 9.43 ± 0.2°, 11.51 ± 0.2°, 12.31 ± 0.2°, 13.70 ± 0.2°, 15.93 ± 0.2°, 16.43 ± 0.2°, 17.26 ± 0.2°, 17.91 ± 0.2°, 18.31 ± 0.2°, 19.49 ± 0.2°, 20.69 ± 0.2°, 20.95 ± 0.2°, 21.19 ± 0.2°, 22.77 ± 0.2°, 23.11 ± 0.2°, 23.29 ± 0.2°, 24.34 ± 0.2°, 24.76 ± 0.2°, 25.55 ± 0.2°, 26.36 ± 0.2°, 26.83 ± 0.2°, 27.69 ± 0.2°, and 29.25 ± 0.2°;

even further, the crystal form A of the p-toluenesulfonate salt has an X-ray powder diffraction pattern expressed by 2θ angles, and diffraction peaks and relative intensities of the X-ray powder diffraction pattern can further be shown in Table 34;

the X-ray powder diffraction pattern of the crystal form A of the p-toluenesulfonate salt expressed by 2θ angles can further be basically shown in FIG. 85.

In a certain preferred embodiment, the compound of formula I and p-toluenesulfonic acid have a molar ratio of 1:1.

In a certain preferred embodiment, the crystal form A of the p-toluenesulfonate salt has a thermogravimetric analysis (TGA) pattern with a weight loss of 0% to 2% (such as 0.76%) when heated from an onset to 100 ± 5°C and a weight loss of 2% to 4% (such as 3.07%) when heated from 100°C to 170°C (the percentage of the weight loss is the percentage of the weight reduction of the sample relative to the weight of the sample prior to this weight loss).

In a certain preferred embodiment, the crystal form A of the p-toluenesulfonate salt has a differential scanning calorimetry (DSC) pattern, and the differential scanning calorimetry pattern can have a major endothermic peak at 150.9 ± 3°C.

In a certain preferred embodiment, the DSC pattern of the crystal form A of the *p*-toluenesulfonate salt can further be basically shown in FIG. 86.

In a certain preferred embodiment, the TGA pattern of the crystal form A of the *p*-toluenesulfonate salt can further be basically shown in FIG. 87.

The present disclosure provides a crystal form A of a benzenesulfonate salt of the compound of formula I, which has an X-ray powder diffraction pattern expressed by 2θ angles comprising diffraction peaks at: 10.81 ± 0.2°, 11.38 ± 0.2°,

16.25 ± 0.2°, 17.41 ± 0.2°, 18.35 ± 0.2°, 19.90 ± 0.2°, and 21.31 ± 0.2°;

further, the crystal form A of the benzenesulfonate salt has an X-ray powder diffraction pattern expressed by 2θ angles comprising diffraction peaks at: 6.54 ± 0.2°, 8.76 ± 0.2°, 10.81 ± 0.2°, 11.38 ± 0.2°, 11.91 ± 0.2°, 13.32 ± 0.2°, 14.01 ± 0.2°, 15.40 ± 0.2°, 16.25 ± 0.2°, 17.41 ± 0.2°, 18.35 ± 0.2°, 19.38 ± 0.2°, 19.90 ± 0.2°, 20.67 ± 0.2°, 21.31 ± 0.2°, 22.89 ± 0.2°, 25.86 ± 0.2°, 27.75 ± 0.2°, and 32.21 ± 0.2°;
further, the crystal form A of the benzenesulfonate salt has an X-ray powder diffraction pattern expressed by 2θ angles, and diffraction peaks and relative intensities of the X-ray powder diffraction pattern can further be shown in Table 35.

The X-ray powder diffraction pattern of the crystal form A of the benzenesulfonate salt expressed by 2θ angles can further be basically shown in FIG. 131.
In a certain preferred embodiment, the compound of formula I and benzenesulfonic acid have a molar ratio of 1:1.
In a certain preferred embodiment, the crystal form A of the benzenesulfonate salt has a thermogravimetric analysis (TGA) pattern with a weight loss of 2% to 4% (such as 2.55%) when heated from an onset to 80 ± 5°C and a weight loss of 5% to 7% (such as 6.23%) when heated from 80°C to 150°C (the percentage of the weight loss is the percentage of the weight reduction of the sample relative to the weight of the sample prior to this weight loss).
In a certain preferred embodiment, the crystal form A of the benzenesulfonate salt has a differential scanning calorimetry (DSC) pattern, and the differential scanning calorimetry pattern can have a major endothermic peak at 111.7 ± 3°C.
In a certain preferred embodiment, the DSC pattern of the crystal form A of the benzenesulfonate salt can further be basically shown in FIG. 88.
In a certain preferred embodiment, the TGA pattern of the crystal form A of the benzenesulfonate salt can further be basically shown in FIG. 89.
The present disclosure provides a crystal form A of an oxalate salt of the compound of formula I, which has an X-ray powder diffraction pattern expressed by 2θ angles comprising diffraction peaks at: 6.15 ± 0.2°, 10.44 ± 0.2°, 13.94 ± 0.2°, 16.74 ± 0.2°, 17.49 ± 0.2°, and 24.50 ± 0.2°;

further, the crystal form A of the oxalate salt has an X-ray powder diffraction pattern expressed by 2θ angles comprising diffraction peaks at: 6.15 ± 0.2°, 7.02 ± 0.2°, 10.44 ± 0.2°, 13.94 ± 0.2°, 16.74 ± 0.2°, 17.49 ± 0.2°, 20.07 ± 0.2°, 24.50 ± 0.2°, 25.79 ± 0.2°, and 26.53 ± 0.2°;
further, the crystal form A of the oxalate salt has an X-ray powder diffraction pattern expressed by 2θ angles comprising diffraction peaks at: 6.15 ± 0.2°, 7.02 ± 0.2°, 9.18 ± 0.2°, 10.44 ± 0.2°, 13.94 ± 0.2°, 15.12 ± 0.2°, 15.54 ± 0.2°, 16.74 ± 0.2°, 17.49 ± 0.2°, 18.16 ± 0.2°, 19.51 ± 0.2°, 20.07 ± 0.2°, 22.64 ± 0.2°, 23.59 ± 0.2°, 24.50 ± 0.2°, 25.22 ± 0.2°, 25.79 ± 0.2°, 26.53 ± 0.2°, 27.90 ± 0.2°, and 28.47 ± 0.2°.

Further, the crystal form A of the oxalate salt has an X-ray powder diffraction pattern expressed by 2θ angles, and diffraction peaks and relative intensities of the X-ray powder diffraction pattern can further be shown in Table 36;
the X-ray powder diffraction pattern of the crystal form A of the oxalate salt of the compound of formula I expressed by 2θ angles can further be basically shown in FIG. 90.
In a certain preferred embodiment, the compound of formula I and oxalic acid have a molar ratio of 1:1.
In a certain preferred embodiment, the crystal form A of the oxalate salt has a thermogravimetric analysis (TGA) pattern with a weight loss of 2% to 7% (such as 5.88%) when heated from an onset to 100 ± 5°C (the percentage of the weight loss is the percentage of the weight reduction of the sample relative to the weight of the sample prior to this weight loss).
In a certain preferred embodiment, the crystal form A of the oxalate salt has a differential scanning calorimetry (DSC) pattern, and the differential scanning calorimetry pattern can have major endothermic peaks at 103.4 ± 3°C and 141.6 ± 3°C.
In a certain preferred embodiment, the DSC pattern of the crystal form A of the oxalate salt can further be basically shown in FIG. 91.
In a certain preferred embodiment, the TGA pattern of the crystal form A of the oxalate salt can further be basically shown in FIG. 92.
The present disclosure provides a crystal form A of a 2-hydroxyethanesulfonate salt of the compound of formula I, which has an X-ray powder diffraction pattern expressed by 2θ angles comprising diffraction peaks at: 6.46 ± 0.2°, 9.89 ± 0.2°, 10.95 ± 0.2°, 13.14 ± 0.2°, 18.38 ± 0.2°, 18.81 ± 0.2°, and 21.70 ± 0.2°.
Further, the crystal form A of the 2-hydroxyethanesulfonate salt of the compound of formula I has an X-ray powder diffraction pattern expressed by 2θ angles comprising diffraction peaks at: 6.46 ± 0.2°, 9.89 ± 0.2°, 10.95 ± 0.2°, 13.14 ± 0.2°, 13.60 ± 0.2°, 15.69 ± 0.2°, 18.38 ± 0.2°, 18.81 ± 0.2°, 20.44 ± 0.2°, and 21.70 ± 0.2°;

further, the crystal form A of the 2-hydroxyethanesulfonate salt of the compound of formula I has an X-ray powder diffraction pattern expressed by 2θ angles comprising diffraction peaks at: 6.46 ± 0.2°, 9.89 ± 0.2°, 10.95 ± 0.2°, 13.14 ± 0.2°, 13.60 ± 0.2°, 15.69 ± 0.2°, 18.38 ± 0.2°, 18.81 ± 0.2°, 20.44 ± 0.2°, 21.70 ± 0.2°, 23.25 ± 0.2°, 24.04 ± 0.2°, 24.82 ± 0.2°, 26.03 ± 0.2°, 26.35 ± 0.2°, and 26.70 ± 0.2°;

further, the crystal form A of the 2-hydroxyethanesulfonate salt has an X-ray powder diffraction pattern expressed by 2θ angles, and diffraction peaks and relative intensities of the X-ray powder diffraction pattern can further be shown in Table 37;

the X-ray powder diffraction pattern of the crystal form A of the 2-hydroxyethanesulfonate salt of the compound of formula I expressed by 2θ angles can further be basically shown in FIG. 93.

In a certain preferred embodiment, the compound of formula I and 2-hydroxyethanesulfonic acid have a molar ratio of 1:1.

In a certain preferred embodiment, the crystal form A of the 2-hydroxyethanesulfonate salt has a thermogravimetric analysis (TGA) pattern with a weight loss of 0% to 3% (such as 1.61%) when heated from an onset to 150 ± 5°C (the percentage of the weight loss is the percentage of the weight reduction of the sample relative to the weight of the sample prior to this weight loss).

In a certain preferred embodiment, the crystal form A of the 2-hydroxyethanesulfonate salt has a differential scanning calorimetry (DSC) pattern, and the differential scanning calorimetry pattern can have a major endothermic peak at 203.5 ± 3°C.

In a certain preferred embodiment, the DSC pattern of the crystal form A of the 2-hydroxyethanesulfonate salt can further be basically shown in FIG. 94.

In a certain preferred embodiment, the TGA pattern of the crystal form A of the 2-hydroxyethanesulfonate salt can further be basically shown in FIG. 95.

The present disclosure further provides a preparation method for the crystal form A of the compound of formula I, comprising the following steps: crystallizing the compound of formula I in a solvent M, and separating solids;
the solvent M is an alkane solvent.

The alkane solvent is preferably n-hexane.

The crystallization is carried out at room temperature, preferably 40 to 60°C, such as 50°C.

The solvent M and the compound of formula I preferably have a mass-to-volume ratio of 30 to 50 mg/mL, such as 40 mg/mL.

The present disclosure further provides a crystal form A of a compound of formula I prepared by the above preparation method for the crystal form A of the compound of formula I.

The present disclosure further provides a preparation method for the crystal form of the pharmaceutically acceptable salt of the compound of formula I, comprising the following steps: reacting the compound of formula I with an acid in a solvent N, and separating solids;

the crystal form of the pharmaceutically acceptable salt of the compound of formula I is the crystal form A of the fumarate salt of the compound of formula I, the crystal form B of the fumarate salt of the compound of formula I, the crystal form C of the fumarate salt the compound of formula I, the crystal form A of the citrate salt of the compound of formula I, the crystal form B of the citrate salt of the compound of formula I, the crystal form A of the methanesulfonate salt of the compound of formula I, the crystal form A of the ethanesulfonate salt of the compound of formula I, the crystal form B of the ethanesulfonate salt of the compound of formula I, the crystal form A of the maleate salt of the compound of formula I, the crystal form B of the maleate salt of the compound of formula I, the crystal form A of the L-tartrate salt of the compound of formula I, the crystal form B of the L-tartrate salt of the compound of formula I, the crystal form C of the L-tartrate salt of the compound of formula I, the crystal form D of the L-tartrate salt of the compound of formula I, the crystal form A of the glycolate salt of the compound of formula I, the crystal form A of the L-malate salt of the compound of formula I, the crystal form B of the L-malate salt of the compound of formula I, the crystal form A of the hippurate salt of the compound of formula I, the crystal form A of the succinate salt of the compound of formula I, the crystal form B of the succinate salt of the compound of formula I, the crystal form A of the ascorbate salt of the compound of formula I, the crystal form B of the ascorbate salt of the compound of formula I, the crystal form A of the adipate salt of the compound of formula I, the crystal form A of the p-toluenesulfonate salt of the compound of formula I, the crystal form A of the benzenesulfonate salt of the compound of formula I, the crystal form A of the oxalate salt of the compound of formula I, or the crystal form A of the 2-hydroxyethanesulfonate salt of the compound of formula I;

the solvent N is one or more of an alcohol solvent, an alkane solvent, an ester solvent, and water;

the acid is fumaric acid, citric acid, methanesulfonic acid, ethanesulfonic acid, maleic acid, L-tartaric acid, glycolic acid, L-malic acid, hippuric acid, succinic acid, ascorbic acid, adipic acid, *p*-toluenesulfonic acid, benzenesulfonic acid, 2-hydroxyethanesulfonic acid, oxalic acid, or 2-hydroxyethanesulfonic acid.

The solvent N is preferably a mixture of isopropanol and water, n-hexane, or ethyl acetate; more preferably, the isopropanol and water have a volume ratio of 19: 1 ± 5.

The solvent N and the compound of formula I preferably have a mass-to-volume ratio of 30 to 50 mg/mL, such as 40 mg/mL.

The compound of formula I and the acid have a molar ratio of 1:(1 ± 0.5), such as 1:1.

The reaction can be carried out by crystallization with stirring.

The crystallization is carried out at room temperature, preferably 10 to 30°C.

The present disclosure further provides a preparation method for the crystal form A of the fumarate salt of the compound of formula I, comprising the following steps: reacting the pyridopyrimidinone compound of formula I in a solvent A with fumaric acid and separating solids;

the solvent A is a mixture of an alcohol solvent and water.

The solvent A is preferably a mixture of isopropanol and water, more preferably a mixture of isopropanol and water in a volume ratio of 19:1 ± 5.

The reaction is preferably carried out at a temperature of 10 to 30°C.

The solvent A and the pyridopyrimidinone compound of formula I preferably have a mass-to-volume ratio of 30 to 50 mg/mL, such as 40 mg/mL.

The reaction can be carried out by crystallization with stirring.

The present disclosure further provides a crystal form A of a fumarate salt of a compound of formula I prepared by the above preparation method for the crystal form A of the fumarate salt of the compound of formula I.

The present disclosure further provides a pharmaceutical composition I, comprising a substance A and a pharmaceutically acceptable carrier, wherein the substance A is one or more of the crystal form A of the compound of formula I, the crystal form B of the compound of formula I, the crystal form A of the fumarate salt of the compound of formula I, the crystal form B of the fumarate salt of the compound of formula I, the crystal form C of the fumarate salt the compound of formula I, the crystal form A of the citrate salt of the compound of formula I, the crystal form B of the citrate salt of the compound of formula I, the crystal form A of the methanesulfonate salt of the compound of formula I, the crystal form A of the ethanesulfonate salt of the compound of formula I, the crystal form B of the ethanesulfonate salt of the compound of formula I, the crystal form A of the maleate salt of the compound of formula I, the crystal form B of the maleate salt of the compound of formula I, the crystal form A of the L-tartrate salt of the compound of formula I, the crystal form B of the L-tartrate salt of the compound of formula I, the crystal form C of the L-tartrate salt of the compound of formula I, the crystal form D of the L-tartrate salt of the compound of formula I, the crystal form A of the glycolate salt of the compound of formula I, the crystal form A of the L-malate salt of the compound of formula I, the crystal form B of the L-malate salt of the compound of formula I, the crystal form A of the hippurate salt of the compound of formula I, the crystal form A of the succinate salt of the compound of formula I, the crystal form B of the succinate salt of the compound of formula I, the crystal form A of the ascorbate salt of the compound of formula I, the crystal form B of the ascorbate salt of the compound of formula I, the crystal form A of the adipate salt of the compound of formula I, the crystal form A of the p-toluenesulfonate salt of the compound of formula I, the crystal form A of the benzenesulfonate salt of the compound of formula I, the crystal form A of the oxalate salt of the compound of formula I, the crystal form A of the 2-hydroxyethanesulfonate salt of the compound of formula I, and the pharmaceutically acceptable salt of the compound of formula I.

The present disclosure further provides a pharmaceutical composition II, comprising the substance A and at least one other pharmacologically active inhibitor, and the substance A is defined as above.

Preferably, the other pharmacologically activity inhibitor is an inhibitor of MEK, an inhibitor of EGFR, or an inhibitor of KRAS;

more preferably, the inhibitor of MEK is trametinib;
more preferably, the inhibitor of EGFR is osimertinib or gefitinib, preferably osimertinib;
more preferably, the inhibitor of KRAS is MRTX-849, AMG-510, or JDQ443.

The MRTX849 has a chemical structure of

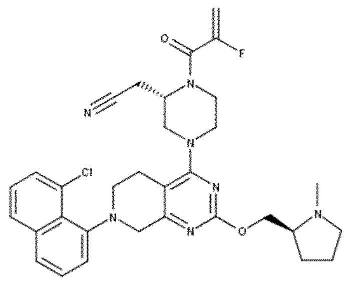

.

The AMG-510 has a chemical structure of

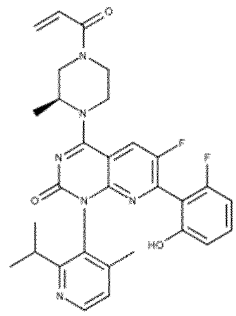

The JDQ443 has a chemical structure of

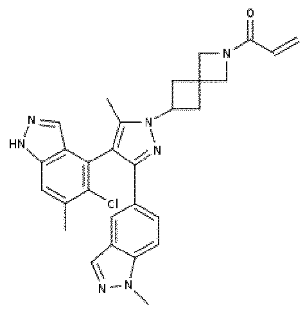

Preferably, the other pharmacologically active inhibitor is an inhibitor of MEK and/or a mutant thereof. More preferably, the other pharmacologically active inhibitor is trametinib.

Preferably, the other pharmacologically active inhibitor is an inhibitor of EGFR and/or a mutant thereof. More preferably, the other pharmacologically active inhibitor is osimertinib or gefitinib, preferably osimertinib.

The present disclosure further provides a use of the substance **A,** the pharmaceutical composition I, or the pharmaceutical composition II, wherein the use is selected from:

(1) inhibiting an interaction between SOS1 and RAS family proteins;
(2) preventing and/or treating a disease related to SOS1 and RAS family proteins;
(3) preparing a drug for inhibiting an interaction between SOS1 and RAS family proteins;
(4) preparing a drug for preventing and/or treating a disease related to (or mediated by) SOS1 and RAS family proteins, and the substance A is defined as above.

The disease related to (or mediated by) SOS1 and RAS family proteins includes, but is not limited to: cancer and RASopathies. The RASopathies are preferably Noonan syndrome, cardiofaciocutaneous syndrome, hereditary gingival fibromatosis type 1, neurofibromatosis type 1 (NF1), capillary malformation-arteriovenous malformation syndrome, Costello syndrome, or Legius syndrome, more preferably neurofibromatosis type 1;
the cancer is preferably selected from melanoma, skin cancer, liver cancer, kidney cancer, lung cancer, nasopharyngeal cancer, gastric cancer, esophageal cancer, colorectal cancer, gallbladder carcinoma, cholangiocarcinoma, choriocarcinoma, pancreatic cancer, polycythemia vera, pediatric tumor, cervical cancer, ovarian cancer, breast cancer, bladder cancer, urothelial carcinoma, ureteral tumor, prostate cancer, seminoma, testicular cancer, leukemia, head and neck tumor, endometrial cancer, thyroid cancer, lymphoma, sarcoma, osteoma, neuroblastoma, neuroblastoma, brain tumor,

myeloma, astrocytoma, glioblastoma, and glioma; more preferably selected from colorectal cancer, lung cancer, and pancreatic cancer.

The lung cancer is preferably non-small cell lung cancer, further preferably metastatic non-small cell lung cancer; the leukemia is preferably chronic lymphocytic leukemia or acute myeloid leukemia; the lymphoma is preferably diffuse large B-cell lymphoma; the myeloma is preferably multiple myeloma; the osteoma is preferably osteochondroma; the liver cancer is preferably hepatocellular carcinoma; the head and neck tumor is preferably head and neck squamous cell carcinoma; the sarcoma is preferably osteosarcoma; and the colorectal cancer is preferably colon cancer or rectal cancer.

Preferably, the use comprises a use of the substance **A** in combination with an inhibitor of MEK (such as trametinib).

Preferably, the drug comprises the substance **A** and an inhibitor of MEK (such as trametinib).

Preferably, the use comprises a use of the substance **A in** combination with an inhibitor of EGFR (such as osimertinib and gefitinib).

Preferably, the drug comprises the substance **A** and an inhibitor of EGFR (such as osimertinib and gefitinib).

Preferably, the use comprises a use of the substance **A in** combination with an inhibitor of KRAS (such as MRTX-849, AMG-510, and JDQ443).

Preferably, the drug comprises the substance **A** and an inhibitor of KRAS (such as MRTX-849, AMG-510, and JDQ443).

Preferably, the use comprises a use of the substance **A** in combination with trametinib or osimertinib.

Preferably, the drug comprises the substance **A** and trametinib or osimertinib.

The RAS family protein can be KRAS, such as KRAS G12C, KRAS G12D, and KRAS G12V.

The present disclosure provides a method for inhibiting SOS1 and RAS family proteins, or preventing and/or treating a disease related to (or mediated by) SOS1 and RAS family proteins, comprising the steps of: administering to a subject in need thereof a therapeutically effective amount of the substance **A.** Preferably, the method comprises using the substance **A** in combination with an inhibitor of MEK, an inhibitor of EGFR, or an inhibitor of KRAS; more preferably, the method comprises using the substance **A** in combination with trametinib or osimertinib.

The present disclosure further provides a quality detection method for a substance **B,** comprising the following steps: eluting a test substance in a silica gel chromatographic column using high-performance liquid chromatography; the test substance comprises the substance **B;** wherein the substance **B** is one or more of the compound of formula I, the crystal form A of the compound of formula I, the crystal form B of the compound of formula I, the crystal form A of the fumarate salt of the compound of formula I, the crystal form B of the fumarate salt of the compound of formula I, the crystal form C of the fumarate salt the compound of formula I, the crystal form A of the citrate salt of the compound of formula I, the crystal form B of the citrate salt of the compound of formula I, the crystal form A of the methanesulfonate salt of the compound of formula I, the crystal form A of the ethanesulfonate salt of the compound of formula I, the crystal form B of the ethanesulfonate salt of the compound of formula I, the crystal form A of the maleate salt of the compound of formula I, the crystal form B of the maleate salt of the compound of formula I, the crystal form A of the L-tartrate salt of the compound of formula I, the crystal form B of the L-tartrate salt of the compound of formula I, the crystal form C of the L-tartrate salt of the compound of formula I, the crystal form D of the L-tartrate salt of the compound of formula I, the crystal form A of the glycolate salt of the compound of formula I, the crystal form A of the L-malate salt of the compound of formula I, the crystal form B of the L-malate salt of the compound of formula I, the crystal form A of the hippurate salt of the compound of formula I, the crystal form A of the succinate salt of the compound of formula I, the crystal form B of the succinate salt of the compound of formula I, the crystal form A of the ascorbate salt of the compound of formula I, the crystal form B of the ascorbate salt of the compound of formula I, the crystal form A of the adipate salt of the compound of formula I, the crystal form A of the*p*-toluenesulfonate salt of the compound of formula I, the crystal form A of the benzenesulfonate salt of the compound of formula I, the crystal form A of the oxalate salt of the compound of formula I, the crystal form A of the 2-hydroxyethanesulfonate salt of the compound of formula I, and the pharmaceutically acceptable salt of the compound of formula I;
the mobile phase used for eluting is a mobile phase A and a mobile phase B;
the mobile phase A is an aqueous solution of 0.1 $\pm$ 0.05% ammonia;
the mobile phase B is acetonitrile;
in the elution procedure, the mobile phase A and the mobile phase B have a volume ratio of 1: 1.5 to 9;
preferably, the elution procedure used for eluting is:

| Time (min) | Mobile phase B (%) |
|---|---|
| 0.0 | 15 |
| 8.0 | 90 |
| 10.0 | 90 |
| 10.1 | 15 |

(continued)

| Time (min) | Mobile phase B (%) |
|---|---|
| 13.0 | 15 |

;

preferably, the quality detection refers to a purity test, a dynamic solubility test, and a stability test;
preferably, the detection method satisfies one or more of the following conditions:

(1) the injection volume for eluting is 5 ± 1 μL;
(2) the flow rate for eluting is 1.0 ± 0.2 mL/min;
(3) the wavelength of the UV detector for eluting is 228 ± 5 nm;
(4) the model of the chromatographic column is Waters XBridge C18, 4.6 mm/150 mm/3 μm;
(5) the column temperature of the chromatographic column is room temperature, preferably 30°C;
(6) the diluent of the test substance is ACN/$H_2O$ (1:1 ± 0.5).

On the basis of common knowledge in the art, each of the above preferred conditions can be arbitrarily combined to obtain each preferred example of the present disclosure.
The reagents and raw materials used in the present disclosure are all commercially available.
In the present disclosure, the X-ray powder diffraction patterns are all measured by using the Cu Kα spectral line.
When referring to XRPD peak positions, the 2θ angles can optionally be retained with or without rounding to 2 or 4 significant digits after the decimal point. For example, when the 2θ angle is 4.8473, the 2θ angle can be 4.84 or 4.85 after retaining 2 significant digits after the decimal point, and it should be understood that the preservation of such a significant digit has the same meaning.
The positive progressive effect of the present disclosure lies in that: after extensive and in-depth research, the present inventor surprisingly develops a crystal form, a salt, and a crystal form of the salt of a compound of formula I (such as substance **A)** as described herein, a preparation method therefor, and a use thereof. The compound of formula I (including a crystal form, a salt, and a crystal form of the salt thereof) shows a significant inhibitory effect on KRAS G12C::SOS1 binding, a significant inhibitory effect on KRAS G12C-SOS1, a significant inhibitory effect on the ERK phosphorylation level in DLD-1 cells, and a strong inhibitory effect on the 3D proliferation of H358 cells; the compound of the present disclosure exhibits good hepatic metabolic stability, slow metabolism in the human body, and high exposure; no inhibitory effect on CYP3A4 enzymes, low risk of potential drug-drug interactions, excellent pharmacokinetic properties, high safety and druggability, and is more suitable for combination medication. Experiments indicate that the compound of the present disclosure, alone or in combination with trametinib, has a significant inhibitory effect on Mia Paca-2 tumor growth, and the combined use is more effective than the single use.
The crystal form, the salt, and the crystal form of the salt of the compound of formula I (such as substance **A)** described herein have desirable physicochemical properties, such as high stability, solubility and bioavailability, and low hygroscopicity, and are suitable for preparing a desirable pharmaceutical preparation.
The present disclosure further provides a method for preparing the crystal form, the salt, and the crystal form of the salt of the compound of formula I (such as substance **A).** The method is simple to operate, high in yield and high in purity, and can be used in industrial production of medicines.

BRIEF DESCRIPTION OF THE DRAWINGS

FIG. 1 is an XRPD pattern of the compound of formula I in Example 1.
FIG. 2 is a DSC pattern of the compound of formula I in Example 1.
FIG. 3 is a TGA pattern of the compound of formula I in Example 1.
FIG. 4 is an XRPD pattern of the crystal form A of the compound of formula I in Example 2.
FIG. 5 is a DSC pattern of the crystal form A of the compound of formula I in Example 2.
FIG. 6 is a TGA pattern of the crystal form A of the compound of formula I in Example 2.
FIG. 7 is a PLM pattern of the crystal form A of the compound of formula I in Example 2.
FIG. 8 is an XRPD overlay pattern of samples before and after drying of the crystal form A of the compound of formula I in Example 2.
FIG. 9 is a VT-XRPD pattern of the crystal form A of the compound of formula I in Example 2.

FIG. 10 is an XRPD pattern of the crystal form B of the compound of formula I in Example 3.
FIG. 11 is an XRPD pattern of the crystal form A of the fumarate salt of the compound of formula I in Example 4.
FIG. 12 is a DSC pattern of the crystal form A of the fumarate salt of the compound of formula I in Example 4.
FIG. 13 is a TGA pattern of the crystal form A of the fumarate salt of the compound of formula I in Example 4.
FIG. 14 is a PLM pattern of the crystal form A of the fumarate salt of the compound of formula I in Example 4.
FIG. 15 is a VT-XRPD pattern of the crystal form A of the fumarate salt of the compound of formula I in Example 4.
FIG. 16 is an XRPD pattern of the crystal form B of the fumarate salt of the compound of formula I in Example 5.
FIG. 17 is a DSC pattern of the crystal form B of the fumarate salt of the compound of formula I in Example 5.
FIG. 18 is a TGA pattern of the crystal form B of the fumarate salt of the compound of formula I in Example 5.
FIG. 19 is an XRPD pattern of the crystal form C of the fumarate salt of the compound of formula I in Example 6.
FIG. 20 is a DSC pattern of the crystal form C of the fumarate salt of the compound of formula I in Example 6.
FIG. 21 is a TGA pattern of the crystal form C of the fumarate salt of the compound of formula I in Example 6.
FIG. 22 is an XRPD pattern of the crystal form A of the citrate salt of the compound of formula I in Example 7.
FIG. 23 is a DSC pattern of the crystal form A of the citrate salt of the compound of formula I in Example 7.
FIG. 24 is a TGA pattern of the crystal form A of the citrate salt of the compound of formula I in Example 7.
FIG. 25 is an XRPD pattern of the crystal form B of the citrate salt of the compound of formula I in Example 8.
FIG. 26 is a VT-XRPD pattern of the crystal form B of the citrate salt of the compound of formula I in Example 8.
FIG. 27 is a DSC pattern of the crystal form B of the citrate salt of the compound of formula I in Example 8.
FIG. 28 is a TGA pattern of the crystal form B of the citrate salt of the compound of formula I in Example 8.
FIG. 29 is a PLM pattern of the crystal form B of the citrate salt of the compound of formula I in Example 8.
FIG. 30 is an XRPD pattern of the crystal form A of the methanesulfonate salt of the compound of formula I in Example 9.
FIG. 31 is a DSC pattern of the crystal form A of the methanesulfonate salt of the compound of formula I in Example 9.
FIG. 32 is a TGA pattern of the crystal form A of the methanesulfonate salt of the compound of formula I in Example 9.
FIG. 33 is a PLM pattern of the crystal form A of the methanesulfonate salt of the compound of formula I in Example 9.
FIG. 34 is a VT-XRPD pattern of the crystal form A of the methanesulfonate salt of the compound of formula I in Example 9.
FIG. 35 is an XRPD pattern of the crystal form A of the ethanesulfonate salt of the compound of formula I in Example 10.
FIG. 36 is a VT-XRPD pattern of the crystal form A of the ethanesulfonate salt of the compound of formula I in Example 10.
FIG. 37 is a DSC pattern of the crystal form A of the ethanesulfonate salt of the compound of formula I in Example 10.
FIG. 38 is a TGA pattern of the crystal form A of the ethanesulfonate salt of the compound of formula I in Example 10.
FIG. 39 is a PLM pattern of the crystal form A of the ethanesulfonate salt of the compound of formula I in Example 10.
FIG. 40 is an XRPD pattern of the crystal form B of the ethanesulfonate salt of the compound of formula I in Example 11.
FIG. 41 is a DSC pattern of the crystal form B of the ethanesulfonate salt of the compound of formula I in Example 11.
FIG. 42 is a TGA pattern of the crystal form B of the ethanesulfonate salt of the compound of formula I in Example 11.
FIG. 43 is an XRPD pattern of the crystal form A of the maleate salt of the compound of formula I in Example 12.
FIG. 44 is a DSC pattern of the crystal form A of the maleate salt of the compound of formula I in Example 12.
FIG. 45 is a TGA pattern of the crystal form A of the maleate salt of the compound of formula I in Example 12.
FIG. 46 is an XRPD pattern of the crystal form B of the maleate salt of the compound of formula I in Example 13.
FIG. 47 is a DSC pattern of the crystal form B of the maleate salt of the compound of formula I in Example 13.
FIG. 48 is a TGA pattern of the crystal form B of the maleate salt of the compound of formula I in Example 13.
FIG. 49 is an XRPD pattern of the crystal form A of the L-tartrate salt of the compound of formula I in Example 14.
FIG. 50 is a DSC pattern of the crystal form A of the L-tartrate salt of the compound of formula I in Example 14.
FIG. 51 is a TGA pattern of the crystal form A of the L-tartrate salt of the compound of formula I in Example 14.
FIG. 52 is an XRPD pattern of the crystal form B of the L-tartrate salt of the compound of formula I in Example 15.
FIG. 53 is a DSC pattern of the crystal form B of the L-tartrate salt of the compound of formula I in Example 15.
FIG. 54 is a TGA pattern of the crystal form B of the L-tartrate salt of the compound of formula I in Example 15.
FIG. 55 is an XRPD pattern of the crystal form C of the L-tartrate salt of the compound of formula I in Example 16.
FIG. 56 is a DSC pattern of the crystal form C of the L-tartrate salt of the compound of formula I in Example 16.
FIG. 57 is a TGA pattern of the crystal form C of the L-tartrate salt of the compound of formula I in Example 16.
FIG. 58 is an XRPD pattern of the crystal form D of the L-tartrate salt of the compound of formula I in Example 17.
FIG. 59 is a DSC pattern of the crystal form D of the L-tartrate salt of the compound of formula I in Example 17.
FIG. 60 is a TGA pattern of the crystal form D of the L-tartrate salt of the compound of formula I in Example 17.
FIG. 61 is an XRPD pattern of the crystal form A of the glycolate salt of the compound of formula I in Example 18.
FIG. 62 is a DSC pattern of the crystal form A of the glycolate salt of the compound of formula I in Example 18.
FIG. 63 is a TGA pattern of the crystal form A of the glycolate salt of the compound of formula I in Example 18.
FIG. 64 is an XRPD pattern of the crystal form A of the L-malate salt of the compound of formula I in Example 19.

FIG. 65 is a DSC pattern of the crystal form A of the L-malate salt of the compound of formula I in Example 19.
FIG. 66 is a TGA pattern of the crystal form A of the L-malate salt of the compound of formula I in Example 19.
FIG. 67 is an XRPD pattern of the crystal form B of the L-malate salt of the compound of formula I in Example 20.
FIG. 68 is a DSC pattern of the crystal form B of the L-malate salt of the compound of formula I in Example 20.
FIG. 69 is a TGA pattern of the crystal form B of the L-malate salt of the compound of formula I in Example 20.
FIG. 70 is an XRPD pattern of the crystal form A of the hippurate salt of the compound of formula I in Example 21.
FIG. 71 is a DSC pattern of the crystal form A of the hippurate salt of the compound of formula I in Example 21.
FIG. 72 is a TGA pattern of the crystal form A of the hippurate salt of the compound of formula I in Example 21.
FIG. 73 is a DSC pattern of the crystal form A of the succinate salt of the compound of formula I in Example 22.
FIG. 74 is a TGA pattern of the crystal form A of the succinate salt of the compound of formula I in Example 22.
FIG. 75 is an XRPD pattern of the crystal form B of the succinate salt of the compound of formula I in Example 23.
FIG. 76 is a DSC pattern of the crystal form B of the succinate salt of the compound of formula I in Example 23.
FIG. 77 is a TGA pattern of the crystal form B of the succinate salt of the compound of formula I in Example 23.
FIG. 78 is a DSC pattern of the crystal form A of the ascorbate salt of the compound of formula I in Example 24.
FIG. 79 is a TGA pattern of the crystal form A of the ascorbate salt of the compound of formula I in Example 24.
FIG. 80 is an XRPD pattern of the crystal form B of the ascorbate salt of the compound of formula I in Example 25.
FIG. 81 is a DSC pattern of the crystal form B of the ascorbate salt of the compound of formula I in Example 25.
FIG. 82 is a TGA pattern of the crystal form B of the ascorbate salt of the compound of formula I in Example 25.
FIG. 83 is a DSC pattern of the crystal form A of the adipate salt of the compound of formula I in Example 26.
FIG. 84 is a TGA pattern of the crystal form A of the adipate salt of the compound of formula I in Example 26.
FIG. 85 is an XRPD pattern of the crystal form A of the *p*-toluenesulfonate salt of the compound of formula I in Example 27.
FIG. 86 is a DSC pattern of the crystal form A of the p-toluenesulfonate salt of the compound of formula I in Example 27.
FIG. 87 is a TGA pattern of the crystal form A of the p-toluenesulfonate salt of the compound of formula I in Example 27.
FIG. 88 is a DSC pattern of the crystal form A of the benzenesulfonate salt of the compound of formula I in Example 28.
FIG. 89 is a TGA pattern of the crystal form A of the benzenesulfonate salt of the compound of formula I in Example 28.
FIG. 90 is an XRPD pattern of the crystal form A of the oxalate salt of the compound of formula I in Example 29.
FIG. 91 is a DSC pattern of the crystal form A of the oxalate salt of the compound of formula I in Example 29.
FIG. 92 is a TGA pattern of the crystal form A of the oxalate salt of the compound of formula I in Example 29.
FIG. 93 is an XRPD pattern of the crystal form A of the 2-hydroxyethanesulfonate salt of the compound of formula I in Example 30.
FIG. 94 is a DSC pattern of the crystal form A of the 2-hydroxyethanesulfonate salt of the compound of formula I in Example 30.
FIG. 95 is a TGA pattern of the crystal form A of the 2-hydroxyethanesulfonate salt of the compound of formula I in Example 30.
FIG. 96 is an XRPD overlay pattern of the solubility sample of the crystal form A of the compound of formula I in Example 2 in $H_2O$.
FIG. 97 is an XRPD overlay pattern of the solubility sample of the crystal form A of the compound of formula I in Example 2 in SGF.
FIG. 98 is an XRPD overlay pattern of the solubility sample of the crystal form A of the compound of formula I in Example 2 in FaSSIF.
FIG. 99 is an XRPD overlay pattern of the solubility sample of the crystal form A of the compound of formula I in Example 2 in FeSSIF.
FIG. 100 is an XRPD overlay pattern of the solubility sample of the crystal form A of the fumarate salt of the compound of formula I in Example 4 in $H_2O$.
FIG. 101 is an XRPD overlay pattern of the solubility sample of the crystal form A of the fumarate salt of the compound of formula I in Example 4 in SGF.
FIG. 102 is an XRPD overlay pattern of the solubility sample of the crystal form A of the fumarate salt of the compound of formula I in Example 4 in FaSSIF.
FIG. 103 is an XRPD overlay pattern of the solubility sample of the crystal form A of the fumarate salt of the compound of formula I in Example 4 in FeSSIF.
FIG. 104 is an XRPD overlay pattern of the solubility sample of the crystal form B of the citrate salt of the compound of formula I in Example 8 in $H_2O$.
FIG. 105 is an XRPD overlay pattern of the solubility sample of the crystal form B of the citrate salt of the compound of formula I in Example 8 in SGF.
FIG. 106 is an XRPD overlay pattern of the solubility sample of the crystal form B of the citrate salt of the compound of formula I in Example 8 in FaSSIF.
FIG. 107 is an XRPD overlay pattern of the solubility sample of the crystal form B of the citrate salt of the compound of

formula I in Example 8 in FeSSIF.

FIG. 108 is an XRPD overlay pattern of the solubility sample of the crystal form A of the methanesulfonate salt of the compound of formula I in Example 9 in FaSSIF.

FIG. 109 is an XRPD overlay pattern of the solubility sample of the crystal form A of the methanesulfonate salt of the compound of formula I in Example 9 in FeSSIF.

FIG. 110 is an XRPD overlay pattern of the solubility sample of the crystal form A of the ethanesulfonate salt of the compound of formula I in Example 10 in $H_2O$.

FIG. 111 is an XRPD overlay pattern of the solubility sample of the crystal form A of the ethanesulfonate salt of the compound of formula I in Example 10 in FaSSIF.

FIG. 112 is an XRPD overlay pattern of the solubility sample of the crystal form A of the ethanesulfonate salt of the compound of formula I in Example 10 in FeSSIF.

FIG. 113 is a DVS pattern of the crystal form A of the compound of formula I in Example 2.

FIG. 114 is an XRPD overlay pattern of the crystal form A of the compound of formula I in Example 2 before and after DVS test.

FIG. 115 is a DVS pattern of the crystal form A of the fumarate salt of the compound of formula I in Example 4.

FIG. 116 is an XRPD overlay pattern of the crystal form A of the fumarate salt of the compound of formula I in Example 4 before and after DVS test.

FIG. 117 is a DVS pattern of the crystal form B of the citrate salt of the compound of formula I in Example 8.

FIG. 118 is an XRPD overlay pattern of the crystal form B of the citrate salt of the compound of formula I in Example 8 before and after DVS test.

FIG. 119 is a DVS pattern of the crystal form A of the methanesulfonate salt of the compound of formula I in Example 9.

FIG. 120 is an XRPD overlay pattern of the crystal form A of the methanesulfonate salt of the compound of formula I in Example 9 before and after DVS test.

FIG. 121 is a DVS pattern of the crystal form A of the ethanesulfonate salt of the compound of formula I in Example 10.

FIG. 122 is an XRPD overlay pattern of the crystal form A of the ethanesulfonate salt of the compound of formula I in Example 10 before and after DVS test.

FIG. 123 is an XRPD overlay pattern of the stability evaluation sample of the crystal form A of the compound of formula I in Example 2.

FIG. 124 is an XRPD overlay pattern of the stability evaluation sample of the crystal form A of the fumarate salt of the compound of formula I in Example 4.

FIG. 125 is an XRPD overlay pattern of the stability evaluation sample of the crystal form B of the citrate salt of the compound of formula I in Example 8.

FIG. 126 is an XRPD overlay pattern of the stability evaluation sample of the crystal form A of the methanesulfonate salt of the compound of formula I in Example 9.

FIG. 127 is an XRPD overlay pattern of the stability evaluation sample of the crystal form A of the ethanesulfonate salt of the compound of formula I in Example 10.

FIG. 128 is an XRPD pattern of the crystal form A of the succinate salt of the compound of formula I in Example 22.

FIG. 129 is an XRPD pattern of the crystal form A of the ascorbate salt of the compound of formula I in Example 24.

FIG. 130 is an XRPD pattern of the crystal form A of the adipate salt of the compound of formula I in Example 26.

FIG. 131 is an XRPD pattern of the crystal form A of the benzenesulfonate salt of the compound of formula I in Example 28.

## DETAILED DESCRIPTION OF THE PREFERRED EMBODIMENT

The present disclosure will be described in detail below by way of examples, but the scope of the present disclosure is not limited thereto. Experimental methods that do not indicate specific conditions in the following examples should be selected according to conventional methods and conditions, or according to product specifications.

The instruments used in the following examples are shown in Table 1.

Table 1

| Instrument | Model | Manufacturer |
|---|---|---|
| X-ray powder diffractometer | Empyrean/X’ Pert3 | PANalytacal |
| Differential scanning calorimeter | TA 2500 | TA |
| Thermogravimetric analyzer | TA 5500 | TA |
| Dynamic vapor sorption analyzer | Intrinsic | SMS |

(continued)

| Instrument | Model | Manufacturer |
|---|---|---|
| Polarized light microscopy | Axio Scope.A1 | Zeiss |
| Nuclear magnetic resonance spectrometer | Bruker 400M | Bruker |
| High-performance liquid chromatograph | 1260 | Agilent |
| Ion chromatograph | ICS-1100 | ThermoFisher |

**X-ray powder diffraction (XRPD) analysis**

The XRPD pattern was collected on an X-ray powder diffraction analyzer produced by PANalytacal, and the scanning parameters are shown in Table 2.

Table 2

| Parameter | VT-XRPD | XRPD |
|---|---|---|
| Model | Empyrean | Empyrean/X' Pert3 |
| X-ray | Cu, Kα,<br>Kα1 (Å): 1.540598,<br>Kα2 (Å): 1.544426<br>Intensity ratio of Kα2/Kα1: 0.50 | Cu, Kα,<br>Kα1 (Å): 1.540598,<br>Kα2 (Å): 1.544426<br>Intensity ratio of Kα2/Kα1: 0.50 |
| X-ray tube setting | 45 kV, 40 mA | 45 kV, 40 mA |
| Divergence slit | 1/8° | 1/8° |
| Scan mode | Continuous | Continuous |
| Scan range (°2θ) | 3-40 | 3-40 |
| Scan time per step (s) | 33.0 | 46.7 |
| Scan step size (°2θ) | 0.0167 | 0.0263 |
| Test time | 10 minutes and 13 seconds | 5 minutes |

**Thermogravimetric analysis (TGA) and differential scanning calorimetry** (DSC)

The TGA and DSC patterns were collected on a TA 5500 thermogravimetric analyzer and a TA 2500 differential scanning calorimeter, respectively, and the test parameters are listed in Table 3.

Table 3

| Parameter | TGA | DSC |
|---|---|---|
| Method | Linear heating | Linear heating |
| Sample tray | Aluminum pan, open | Aluminum pan, with lid/without lid |
| Temperature range | Room temperature-set endpoint temperature | 25°C-set endpoint temperature |
| Purge rate (°C/min) | 10 | 10 |
| Protective gas | Nitrogen | Nitrogen |

**Dynamic vapor sorption (DVS)**

The dynamic vapor sorption (DVS) curve was collected on DVS IntrInsic from SMS (surface measurement systems). The relative humidity at 25°C was corrected using the deliquescence points of LiCl, $Mg(NO_3)_2$, and KCl. DVS test parameters are listed in Table 4.

Table 4

| Parameter | Set value |
|---|---|
| Temperature | 25°C |
| Sample weight | 10-20 mg |
| Protective gas and flow rate | $N_2$, 200 mL/min |
| dm/dt | 0.002%/min |
| Minimum dm/dt equilibration time | 10 min |
| Maximum equilibrium time | 180 min |
| RH range | 0%RH-95%RH |
| RH gradient | 10%RH (0%RH - 90%RH & 90%RH - 0%RH)<br>5%RH (90%RH - 95%RH & 95%RH- 90%RH) |

**Polarized light microscopy (PLM)**

Polarized light microscope photographs were taken at room temperature using a Zeiss Axio Scope. A1 microscope.

**Liquid nuclear magnetic resonance ($^1$H NMR)**

Liquid NMR spectra were collected on a Bruker 400M NMR instrument, using DMSO-$d_6$, $CDCl_3$, or $CD_3OD$ as solvents.

**High-performance liquid chromatography and ion chromatography (HPLC/IC)**

The purity test, dynamic solubility test, and stability test in the assay were measured with an Agilent 1260 high-performance liquid chromatograph, and the salt formation molar ratio of ions was tested by ion chromatography, and the analytical conditions are shown in Table 5 and Table 6.

Table 5

| Liquid chromatograph | Agilent 1260/1290 detectors | |
|---|---|---|
| Chromatographic column | Waters XBridge C18, 4.6 mm/150 mm/3 μm | |
| Mobile phase | A: 0.1% aqueous solution of ammonia | |
| | B: Acetonitrile | |
| | **Time (min)** | **%B** |
| | 0.0 | 15 |
| | 8.0 | 90 |
| | 10.0 | 90 |
| | 10.1 | 15 |
| | 13.0 | 15 |
| Run time | 13 min | |
| Flow rate for mobile phase | 1.0 mL/min | |
| Injection volume | 5 μL | |
| Detection wavelength | UV at 228 nm | |
| Column temperature | 30°C | |
| Injector temperature | RT | |

(continued)

| Liquid chromatograph | Agilent 1260/1290 detectors |
|---|---|
| Diluent | ACN/$H_2O$ (1:1) |

Table 6

| Ion chromatograph | ThermoFisher ICS-1100 |
|---|---|
| Chromatographic column | IonPac AS18 Analytical Column, 250 * 4 mm |
| Mobile phase | 25 mM NaOH |
| Injection volume | 25 μL |
| Flow rate | 1.0 mL/min |
| Temperature | 35°C |
| Column temperature | 35°C |
| Current | 80 mA |
| Run time | $C_2O_4^{2-}$: 16 min |

**Chinese-English reference table for solvents**

The abbreviations of solvents are listed against the corresponding Chinese names in Table 7.

Table 7

| English | Chinese | English | Chinese |
|---|---|---|---|
| MeOH | Methanol | 1,4-Dioxane | 1,4-Dioxane |
| EtOH | Ethanol | ACN | Acetonitrile |
| IPA | Isopropanol | DCM | Dichloromethane: |
| Acetone | Acetone | $CHCl_3$ | Chloroform |
| MIBK | Methyl isobutyl ketone | Toluene | Toluene |
| EtOAc | Ethyl acetate | n-Heptane | *n*-Heptane |
| IPAc | Isopropyl acetate | DMSO | Dimethyl sulfoxide |
| MTBE | Methyl tert-butyl ether | DMF | *N,N*-Dimethylformamide |
| THF | Tetrahydrofuran | $H_2O$ | Water |
| 2-MeTHF | 2-Methyltetrahydrofuran | *n*-Pentane | *n*-Pentane |
| CPME | Cyclopentyl methyl ether | Cyclohexane | Cyclohexane |
| Chlorobenzene | Chlorobenzene | MEK | 2-Butanone |
| Anisole | Anisole | Cumene | Cumene |
| n-Hexane | n-Hexane | -- | -- |

**Example 1: Preparation of compound of formula I**

The synthetic route (see patent CN202210117751.6) is as follows:

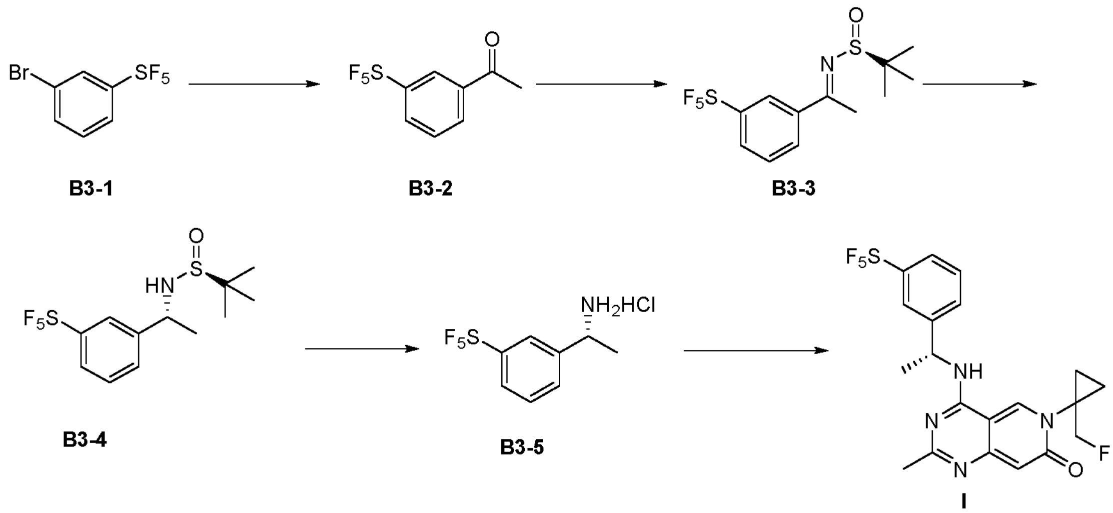

B3-1 B3-2 B3-3

B3-4 B3-5 I

Step 1: Synthesis of 1-(3-(pentafluorosulfanyl)phenyl)ethan-1-one (B3-2)

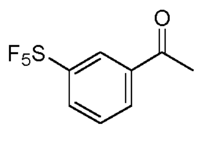

B3-2

To dioxane (100 mL) was added compound 3-bromo-(pentafluorosulfanyl)benzene (3.00 g, 10.6 mmol) at room temperature, the reaction mixture was added with bis(triphenylphosphine)palladium(II) chloride (744 mg, 1.06 mmol) and tributyl(1-ethoxyvinyl)tin (4.20 g, 11.7 mmol), heated to 90°C under nitrogen atmosphere, and stirred for 14 hours. The reaction mixture was cooled to room temperature, added with 2 N hydrochloric acid (100 mL), and stirred for 4 hours. The reaction mixture was extracted with ethyl acetate (200 mL × 3), and the phases were separated. The organic phases were combined, dried over anhydrous sodium sulfate, filtered, concentrated, and the residue was purified by silica gel column chromatography (petroleum ether: ethyl acetate (V/V) = 8:1) to obtain 1-(3-(pentafluorosulfanyl)phenyl)ethan-1-one (B3-2, yellow liquid, 2.4 g, yield: 89%).

LC-MS, M/Z (ESI): 247.0 $[M+H]^+$.

Step 2: Synthesis of (S,E)-2-methyl-N-(1-(3-(pentafluorosulfanyl)phenyl)ethylidene)propane-2-sulfinamide (B3-3)

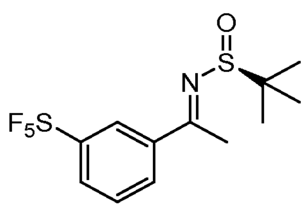

B3-3

To THF (150 mL) was added compound 1-(3-(pentafluorosulfanyl)phenyl)ethan-1-one (1.0 g, 4.06 mmol) at room temperature, the reaction mixture was added with *(S)-tert*-butylsulfinamide (492 mg, 4.06 mmol) and tetraethyl titanate (1.14 g, 5.0 mmol), heated to 70°C and stirred for 16 hours. The reaction mixture was cooled to room temperature, diluted with water (100 mL), extracted with ethyl acetate (100 mL × 3), and the phases were separated. The organic phases were combined, dried over anhydrous sodium sulfate, filtered, concentrated, and the residue was purified by silica gel column chromatography (petroleum ether: ethyl acetate (V/V) = 4:1) to obtain (S,E)-2-methyl-N-(1-(3-(pentafluorosulfanyl)phenyl)ethylidene)propane-2-sulfinamide (B3-3, white solid, 1.42 g, yield: 100%).

LC-MS, M/Z (ESI): 350.2 $[M+H]^+$.

Step 3: Synthesis of (S)-2-methyl-N-((R)-1-(3-(pentafluorosulfanyl)phenyl)ethyl)propane-2-sulfinamide (B3-4)

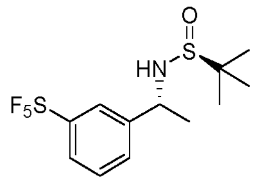

**B3-4**

To methanol (30 mL) was added raw material (S,E)-2-methyl-N-(1-(3-(pentafluorosulfanyl)phenyl)ethylidene) propane-2-sulfinamide (1.5 g, 4.3 mmol) at room temperature, and the reaction mixture was cooled to 0°C. $NaBH_4$ (744 mg, 20.1 mmol) was added to methanol in batches, and the reaction mixture was warmed to room temperature and stirred for 3 hours. The reaction mixture was concentrated and purified by preparative thin-layer chromatography to obtain (*S*)-2-methyl-*N*-((*R*)-1-(3-(pentafluorosulfanyl)phenyl)ethyl)propane-2-sulfinamide (B3-4, white solid, 600 mg, yield: 40.0%).

LC-MS, M/Z (ESI): 352.1 $[M+H]^+$.

Step 4: Synthesis of (*R*)-1-(3-(pentafluorosulfanyl)phenyl)ethan-1-amine hydrochloride (B3-5)

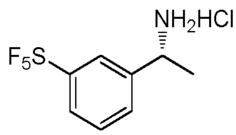

**B3-5**

To a 4 mol/L solution of hydrochloric acid in dioxane (10 mL) was added raw material (*R*)-2-methyl-*N*-((*R*)-1-(3-(pentafluorosulfanyl)phenyl)ethyl)propane-2-sulfinamide (600 mg, 1.70 mmol) at room temperature, and the reaction mixture was stirred for 4 hours. The reaction mixture was concentrated, added with methyl tert-butyl ether (20 mL), stirred for 1 hour, and filtered to obtain (*R*)-1-(3-(pentafluorosulfanyl)phenyl)ethan-1-amine hydrochloride (B3-5, white solid, 350 mg, yield: 72.7%).

LC-MS, M/Z (ESI): 248.2 $[M+H]^+$.

Step 5: Synthesis of (*R*)-6-(1-(fluoromethyl)cyclopropyl)-2-methyl-4-((1-(3-(pentafluorosulfanyl)phenyl)ethyl)amino) pyrido[4,3-*d*]pyrimidin-7(6*H*)-one

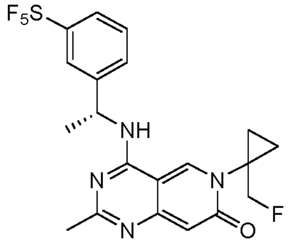

**I**

To acetonitrile (20 mL) was added raw material 6-(1-(fluoromethyl)cyclopropyl)-4-hydroxy-2-methylpyrido[4,3-*d*] pyrimidin-7(6*H*)-one (**A1**) (200 mg, 0.80 mmol) at room temperature, the reaction mixture was added with potassium phosphate (678 mg, 3.20 mmol) and phosphonitrilic chloride trimer (416 mg, 1.20 mmol), and stirred at room temperature for 16 hours. To DCM (10 mL) was added raw material *(R)-1-(3*-(pentafluorosulfanyl)phenyl)ethan-1-amine hydrochloride

(160 mg, 0.56 mmol), the reaction mixture was added with DIPEA (2 mL), and stirred for 0.5 h. The above system was added with the reaction mixture, and stirred at room temperature for 6 hours. The reaction mixture was concentrated and subjected to acidic preparation condition B (Welch, Ultimate C18 column, 10 μm, 21.2 mm × 250 mm. Mobile phase A was a 1‰ solution of formic acid in pure water, and mobile phase B was an acetonitrile solution. Gradient conditions: mobile phase A was kept at 90% (0 to 3 min), then reduced from 90% to 5% by gradient elution (3 to 18 min), and kept at 5% (18 to 22 min)) to obtain (*R*)-6-(1-(fluoromethyl)cyclopropyl)-2-methyl-4-((1-(3-(pentafluorosulfanyl)phenyl)ethyl)amino)pyrido [4,3-*d*]pyrimidin-7(6*H*)-one (*i.e.,* the compound of formula I, white solid, 44 mg, yield: 16.4%).

The XRPD, as shown in FIG. 1, indicates that the resulting sample of the compound of formula I is substantially amorphous.

In FIG. 2, the DSC pattern shows that the compound of formula I has major endothermic signals at 75.7°C, 95.2°C, 142.2°C, and 180.4°C.

In FIG. 3, the TGA pattern shows a weight loss of the compound of formula I by 6.26% when heated from an onset to 180°C.

The $^1$H NMR spectrum data of the compound of formula I is: $^1$H NMR (400 MHz, DMSO-$d_6$) δ 9.18 (s,1H), 8.87 (d, 1H),7.96 (s, 1H),7.78 (d, 1H), 7.71 (d, 1H),7.60 (t, 1H),6.08 (s, 1H), 5.60 (q, 1H), 4.68-4.56 (m, 2H), 2.21 (s, 3H), 1.61 (d, 3H), 1.32-1.28 (m, 4H).

LC-MS, M/Z (ESI): 479.4 $[M+H]^+$.

**Example 2: Preparation of crystal form A of compound of formula I**

The preparation steps are as follows: 300.3 mg of the compound of formula I was weighed into a 20 mL glass vial, and 7.5 mL of n-heptane was added to form a suspension. The suspension was then placed at 50°C for 5 days under magnetic stirring. The solid was separated and vacuum dried at room temperature overnight to obtain a solid sample.

The resulting sample was identified by X-ray powder diffraction pattern as a crystal form A of the compound of formula I. The crystal form A of the compound of formula I has an X-ray powder diffraction pattern expressed by 2θ angles, and diffraction peaks and relative intensities of the X-ray powder diffraction pattern can further be shown in Table 8.

Table 8

| Diffraction peak number | 2θ angle (°) | Relative intensity (%) |
|---|---|---|
| 1 | 6.0228 | 3.59 |
| 2 | 7.7830 | 3.30 |
| 3 | 10.3092 | 11.15 |
| 4 | 11.4209 | 6.30 |
| 5 | 12.0638 | 100.00 |
| 6 | 12.4274 | 23.84 |
| 7 | 14.6867 | 28.86 |
| 8 | 15.1087 | 11.55 |
| 9 | 16.6189 | 9.65 |
| 10 | 17.7871 | 17.72 |
| 11 | 18.1363 | 28.30 |
| 12 | 18.4865 | 27.27 |
| 13 | 19.1245 | 26.11 |
| 14 | 19.9890 | 17.55 |
| 15 | 20.2538 | 26.72 |
| 16 | 21.7638 | 17.22 |
| 17 | 22.0984 | 25.18 |
| 18 | 23.4584 | 2.72 |
| 19 | 24.7554 | 13.48 |
| 20 | 26.1969 | 4.94 |

(continued)

| Diffraction peak number | 2θ angle (°) | Relative intensity (%) |
|---|---|---|
| 21 | 29.0571 | 1.31 |
| 22 | 30.4824 | 1.85 |
| 23 | 31.0426 | 1.54 |
| 24 | 32.4179 | 1.62 |
| 25 | 35.9264 | 1.20 |

The XRPD pattern of a sample of the crystal form A of the compound of formula I is shown in FIG. 4.

In FIG. 5, the DSC pattern shows that the crystal form A of the compound of formula I has major endothermic signals at 73.3°C and 178.0°C.

In FIG. 6, the TGA pattern shows a weight loss of the crystal form A of the compound of formula I by 3.24% when heated from an onset to 120 ± 5°C.

The $^1H$ NMR spectrum data of the crystal form A of the compound of formula I is: $^1H$ NMR (400 MHz, DMSO-$d_6$) δ 9.12 (1H, s), 8.89 (1H, d), 7.91 (1H, t), 7.83-7.76 (1H, m), 7.72 (1H, d), 7.60 (1H, t), 6.09 (1H, s), 5.60 (1H, m), 4.62 (2H, d), 2.21 (3H, s), 1.61 (3H, d), 1.37-1.24 (4H, m).

In FIG. 7, the crystal form Ais observed to be aggregated into small particles as shown in PLM.

In order to evaluate the influence of the drying conditions on the crystal form A of the compound of formula I, the changes of the sample before and after vacuum drying of the crystal form A of the compound of formula I under the condition of 60°C/80°C for 4 hours were respectively investigated. The XRPD, $^1H$ NMR, and HPLC purity of the dried sample were tested. After the dried sample was subjected to XRPD testing after exposure to room temperature and humidity conditions (19°C/27% RH) for 10-15 min, the results (FIG. 8) showed that it was still the crystal form A of the compound of formula I. The $^1H$ NMR results indicated that no solvent residue of n-heptane was observed in the samples after drying. The HPLC results indicated (Table 9) that no significant change in purity was observed in the samples before and after drying.

Table 9

| # Peak | Relative retention time | Area (%) | | |
|---|---|---|---|---|
| | | Onset of crystal form A of compound of formula I | After vacuum drying at 60°C | After vacuum drying at 80°C |
| 1 | 0.60 | 0.11 | 0.10 | 0.10 |
| 2 | 0.85 | 0.43 | 0.43 | 0.43 |
| 3 | 0.94 | 0.49 | 0.49 | 0.48 |
| 4 | 1.00 | 97.07 | 97.11 | 97.14 |
| 5 | 1.18 | 0.19 | 0.19 | 0.19 |
| 6 | 1.20 | 0.62 | 0.63 | 0.62 |
| 7 | 1.27 | 0.21 | 0.21 | 0.21 |
| 8 | 1.29 | 0.36 | 0.34 | 0.35 |
| 9 | 1.34 | 0.36 | 0.35 | 0.35 |
| 10 | 1.43 | 0.15 | 0.16 | 0.13 |

**Example 3: Preparation of crystal form B of compound of formula I**

The crystal form A of the compound of formula I of Example 2 was identified using variable temperature XRPD (VT-XRPD). The VT-XRPD results showed (FIG. 9) that the crystal form A of the compound of formula I transformed into a new crystal form B after being purged with $N_2$ for 20 minutes. No crystal change was observed after heating to 100°C under the protection of $N_2$ and cooling to 30°C. When the sample was exposed to air, it was observed that the sample transcrystallized back to the crystal form A of the compound of formula I. In conjunction with TGA, DSC, and $^1H$ NMR results for crystal form A, it is speculated that crystal form A is a hydrate, possibly a hydrate containing 0.5-1.5 water

molecules, such as a monohydrate or sesquihydrate. The crystal form A is dehydrated under $N_2$ purging or heating conditions to form an anhydrous crystal form B, and after being exposed to air, the sample absorbs water and then is transcrystallized back to the crystal form A.

The X-ray powder diffraction pattern shows that the obtained sample is the crystal form B of the compound of formula I. The crystal form B of the compound of formula I has an X-ray powder diffraction pattern expressed by 2θ angles, and its diffraction peaks and relative intensities can be further shown in Table 10.

Table 10

| Diffraction peak number | 2θ angle (°) | Relative intensity (%) |
|---|---|---|
| 1 | 8.2865 | 2.76 |
| 2 | 9.2885 | 2.12 |
| 3 | 10.0872 | 6.27 |
| 4 | 10.3092 | 10.73 |
| 5 | 11.5894 | 7.31 |
| 6 | 12.3098 | 30.33 |
| 7 | 12.8969 | 100.00 |
| 8 | 14.4978 | 66.58 |
| 9 | 15.3275 | 14.46 |
| 10 | 16.3902 | 9.76 |
| 11 | 16.8991 | 19.27 |
| 12 | 17.4565 | 24.35 |
| 13 | 18.1094 | 94.08 |
| 14 | 18.2100 | 91.83 |
| 15 | 18.7070 | 58.39 |
| 16 | 19.8490 | 12.99 |
| 17 | 20.3335 | 65.71 |
| 18 | 21.0502 | 10.48 |
| 19 | 21.6664 | 30.40 |
| 20 | 22.0197 | 13.74 |
| 21 | 22.5234 | 14.20 |
| 22 | 23.2319 | 17.04 |
| 23 | 24.3595 | 23.56 |
| 24 | 24.6980 | 15.70 |
| 25 | 25.4100 | 7.92 |
| 26 | 25.7299 | 5.40 |
| 27 | 25.9974 | 4.74 |
| 28 | 26.6367 | 6.53 |
| 29 | 27.3167 | 6.58 |
| 30 | 28.6459 | 6.47 |
| 31 | 29.1507 | 2.52 |
| 32 | 30.7774 | 6.28 |
| 33 | 31.6249 | 2.31 |
| 34 | 32.8463 | 1.84 |

(continued)

| Diffraction peak number | 2θ angle (°) | Relative intensity (%) |
|---|---|---|
| 35 | 34.6151 | 1.98 |
| 36 | 35.8761 | 2.23 |
| 37 | 37.5599 | 1.89 |

The X-ray powder diffraction pattern of the crystal form B of the compound of formula I is shown in FIG. 10.

**Example 4: Preparation of crystal form A of fumarate salt of compound of formula I**

300.1 mg of the compound of formula I and 72.5 mg of fumaric acid (feeding molar ratio of 1:1) were weighed into a 20 mL glass vial, then 7.5 mL of IPA/$H_2O$ (19:1, v/v) was added thereto and stirred at room temperature for 4 days, and the solid was separated and vacuum dried at room temperature overnight to obtain a total of 280 mg of sample.

The X-ray powder diffraction pattern shows that the obtained sample is the crystal form A of the fumarate salt of the compound of formula I. The crystal form A of the fumarate salt of the compound of formula I has an X-ray powder diffraction pattern expressed by 2θ angles, and diffraction peaks and relative intensities of the X-ray powder diffraction pattern can be shown in Table 11.

Table 11

| Diffraction peak number | 2θ angle (°) | Relative intensity (%) |
|---|---|---|
| 1 | 6.8635 | 16.34 |
| 2 | 7.7252 | 48.44 |
| 3 | 9.5770 | 15.24 |
| 4 | 11.9972 | 1.28 |
| 5 | 12.9002 | 5.22 |
| 6 | 13.1749 | 3.47 |
| 7 | 13.7294 | 12.06 |
| 8 | 14.7803 | 2.02 |
| 9 | 15.4533 | 5.22 |
| 10 | 15.8774 | 6.84 |
| 11 | 16.4762 | 36.15 |
| 12 | 16.7209 | 2.77 |
| 13 | 17.1307 | 11.61 |
| 14 | 17.4731 | 6.35 |
| 15 | 18.1952 | 10.02 |
| 16 | 18.7222 | 9.03 |
| 17 | 19.2097 | 18.81 |
| 18 | 19.6315 | 20.13 |
| 19 | 19.9729 | 5.41 |
| 20 | 20.6384 | 1.38 |
| 21 | 20.8956 | 3.60 |
| 22 | 22.4788 | 87.04 |
| 23 | 22.7668 | 13.84 |
| 24 | 23.2638 | 100.00 |
| 25 | 23.5487 | 6.31 |

(continued)

| Diffraction peak number | 2θ angle (°) | Relative intensity (%) |
|---|---|---|
| 26 | 23.9591 | 18.73 |
| 27 | 25.5844 | 2.38 |
| 28 | 25.9303 | 14.48 |
| 29 | 26.2872 | 5.24 |
| 30 | 26.5499 | 1.63 |
| 31 | 27.2374 | 3.53 |
| 32 | 27.9091 | 1.38 |
| 33 | 28.9544 | 1.68 |
| 34 | 29.5480 | 4.81 |
| 35 | 29.8239 | 6.24 |
| 36 | 30.5191 | 1.77 |
| 37 | 31.1617 | 15.39 |
| 38 | 32.6027 | 1.68 |
| 39 | 33.6475 | 1.74 |
| 40 | 34.9521 | 1.90 |
| 41 | 35.7055 | 3.05 |
| 42 | 37.0550 | 2.07 |
| 43 | 39.2410 | 1.65 |

The X-ray powder diffraction pattern of the crystal form A of the fumarate salt is shown in FIG. 11.

In FIG. 12, the DSC pattern shows that the sample has one endothermic peak at 214.7°C.

In FIG. 13, the TGA pattern shows that the sample has a weight loss of 3.35% when heated to 150°C.

The $^1$H NMR spectrum data of the crystal form A of the fumarate salt of the compound of formula I is: $^1$H NMR (400 MHz, DMSO-*d*$_6$) δ 9.12 (1H, s), 8.89 (1H, s), 7.96 (1H, t), 7.79 (1H, dd), 7.71 (1H, d), 7.60 (1H, t), 6.62 (2H, s), 6.09 (1H, s), 5.60 (1H, m), 4.62 (2H, d), 2.21 (3H, s), 1.61 (3H, d), 1.37-1.23 (4H, m). In the sample of the crystal form A of the fumarate salt, the molar ratio of fumaric acid to the compound of formula I is 1.0:1.

In FIG. 14, PLM shows that the crystal form A of the fumarate salt is a needle-like small particle and aggregation of the samples is observed.

VT-XRPD was used to identify the crystal form A of the fumarate salt. The results showed that (see FIG. 15): after purging the crystal form A of the fumarate salt under $N_2$ for 20 minutes, a peak shift was observed. No change in the crystal form was observed when the sample was heated to 150°C under the protection of $N_2$ and then cooled to 30°C. When the sample was exposed to air, it was observed that the sample transcrystallized back to the crystal form A of the fumarate salt. In conjunction with TGA, DSC, and $^1$H NMR results for the crystal form A of the fumarate salt, it is speculated that the crystal form A of the fumarate salt is a hydrate, possibly a hydrate containing 0.5-1.5 water molecules, such as a monohydrate or sesquihydrate.

**Example 5: Preparation of crystal form B of fumarate salt of compound of formula I**

The compound of formula I and an equimolar amount of fumaric acid were suspended and stirred in EtOAc at room temperature for 3 days, and then the solid was separated by centrifugation and vacuum dried at room temperature overnight.

The X-ray powder diffraction pattern shows that the obtained sample is the crystal form B of the fumarate salt of the compound of formula I. The crystal form B of the fumarate salt of the compound of formula I has an X-ray powder diffraction pattern expressed by 2θ angles, and diffraction peaks and relative intensities of the X-ray powder diffraction pattern can further be shown in Table 12.

Table 12

| Diffraction peak number | 2θ angle (°) | Relative intensity (%) |
|---|---|---|
| 1 | 3.1306 | 30.47 |
| 2 | 4.7842 | 88.15 |
| 3 | 5.9872 | 23.25 |
| 4 | 8.0240 | 35.07 |
| 5 | 9.6881 | 59.76 |
| 6 | 11.5567 | 18.92 |
| 7 | 11.9324 | 40.62 |
| 8 | 12.4487 | 19.30 |
| 9 | 12.7894 | 19.80 |
| 10 | 14.0148 | 14.98 |
| 11 | 15.2481 | 23.64 |
| 12 | 15.3870 | 25.91 |
| 13 | 15.8013 | 17.28 |
| 14 | 16.5798 | 49.63 |
| 15 | 17.3697 | 19.13 |
| 16 | 17.9353 | 35.64 |
| 17 | 18.5729 | 39.25 |
| 18 | 19.4112 | 21.04 |
| 19 | 19.9508 | 16.56 |
| 20 | 21.1098 | 24.00 |
| 21 | 22.1541 | 16.63 |
| 22 | 22.5310 | 20.66 |
| 23 | 22.8977 | 18.36 |
| 24 | 23.9784 | 40.14 |
| 25 | 24.7603 | 18.73 |
| 26 | 25.1801 | 12.42 |
| 27 | 25.7408 | 8.11 |
| 28 | 26.5670 | 9.32 |
| 29 | 27.0251 | 4.34 |
| 30 | 28.8193 | 100.00 |
| 31 | 29.4817 | 22.24 |
| 32 | 33.3756 | 2.57 |

The X-ray powder diffraction pattern of the crystal form B of the fumarate salt is shown in FIG. 16.

In FIG. 17, the DSC pattern shows that the sample has one endothermic peak at 154.4°C and one exothermic peak at 191.9°C.

In FIG. 18, the TGA pattern shows that the sample has a weight loss of 5.64% when heated to 150°C, and a weight loss of 6.76% when heated from 150°C to 250°C.

The $^1H$ NMR spectrum data of the crystal form B of the fumarate salt of the compound of formula I is: $^1H$ NMR (400 MHz, DMSO-$d_6$) δ 9.12 (1H, s), 8.90 (1H, d), 7.96 (1H, t), 7.80 (1H, dd), 7.72 (1H, d), 7.60 (1H, t), 6.62 (2H, s), 6.09 (1H, s), 5.60 (1H, m), 4.61 (2H, d), 2.22 (3H, s), 1.61 (3H, d), 1.35-1.26 (4H, m). The $^1H$ NMR results show that the molar ratio of fumaric acid to the compound of formula I is 1:1 in the sample of the crystal form B of the fumarate salt.

**Example 6: Preparation of crystal form C of fumarate salt of compound of formula I**

The compound of formula I and an equimolar amount of fumaric acid were suspended and stirred in MTBE at room temperature for 3 days, and then the solid was separated by centrifugation and vacuum dried at room temperature overnight.

The X-ray powder diffraction pattern shows that the obtained sample is the crystal form C of the fumarate salt of the compound of formula I. The crystal form C of the fumarate salt of the compound of formula I has an X-ray powder diffraction pattern expressed by 2θ angles, and diffraction peaks and relative intensities of the X-ray powder diffraction pattern can further be shown in Table 13.

Table 13

| Diffraction peak number | 2θ angle (°) | Relative intensity (%) |
|---|---|---|
| 1 | 6.8589 | 65.48 |
| 2 | 8.7271 | 75.22 |
| 3 | 9.9037 | 12.52 |
| 4 | 12.1726 | 13.47 |
| 5 | 13.1356 | 42.02 |
| 6 | 14.0951 | 100.00 |
| 7 | 14.2993 | 91.55 |
| 8 | 16.3367 | 18.89 |
| 9 | 17.2742 | 74.17 |
| 10 | 17.9016 | 47.93 |
| 11 | 20.6039 | 39.40 |
| 12 | 21.4276 | 39.71 |
| 13 | 23.1627 | 45.22 |
| 14 | 24.0403 | 36.91 |
| 15 | 25.4177 | 11.45 |
| 16 | 29.8822 | 8.71 |

The X-ray powder diffraction pattern of the crystal form C of the fumarate salt is shown in FIG. 19.

In FIG. 20, the DSC pattern shows that the sample has three endothermic peaks at 94.4°C, 145.0°C, and 161.2°C, and one exothermic peak at 190.4°C.

In FIG. 21, the TGA pattern shows that the sample has a weight loss of 1.69% when heated to 150°C, and a weight loss of 6.21% when heated from 150°C to 250°C.

The $^1H$ NMR spectrum data of the crystal form C of the fumarate salt is: $^1H$ NMR (400 MHz, DMSO-$d_6$) δ 9.12 (1H, s), 8.90 (1H, d), 7.96 (1H, t), 7.79 (1H, dd), 7.72 (1H, d), 7.60 (1H, t), 6.63 (2H, s), 6.09 (1H, s), 5.60 (1H, m), 4.59 (2H, d), 2.22 (3H, s), 1.61 (3H, d), 1.35-1.24 (4H, m). In the sample of the crystal form C of the fumarate salt, the molar ratio of fumaric acid to the compound of formula I is 1:1.

**Example 7: Preparation of crystal form A of citrate salt of compound of formula I**

The compound of formula I and an equimolar amount of anhydrous citric acid were suspended and stirred in acetone/n-heptane (1:3, v/v) at room temperature for 3 days, and then the solid was separated by centrifugation and vacuum dried at room temperature overnight.

The X-ray powder diffraction pattern shows that the obtained sample is the crystal form A of the citrate salt of the compound of formula I. The crystal form A of the citrate salt of the compound of formula I has an X-ray powder diffraction pattern expressed by 2θ angles, and diffraction peaks and relative intensities of the X-ray powder diffraction pattern can further be shown in Table 14.

Table 14

| Diffraction peak number | 2θ angle (°) | Relative intensity (%) |
|---|---|---|
| 1 | 6.2756 | 100.00 |
| 2 | 10.7946 | 4.33 |
| 3 | 12.2105 | 7.49 |
| 4 | 12.5828 | 22.11 |
| 5 | 16.3816 | 10.38 |
| 6 | 18.3385 | 2.67 |
| 7 | 25.3323 | 20.65 |
| 8 | 29.0006 | 0.90 |

The X-ray powder diffraction pattern of the crystal form A of the citrate salt is shown in FIG. 22.

In FIG. 23, the DSC pattern shows that the sample has two endothermic peaks at 99.1°C and 137.9°C.

In FIG. 24, the TGA pattern shows that the sample has a weight loss of 5.72% when heated to 150°C, and a weight loss of 19.25% when heated from 150°C to 230°C.

The $^1$H NMR spectrum data of the crystal form A of the citrate salt is: $^1$H NMR (400 MHz, DMSO-$d_6$) δ 9.15 (1H, s), 7.97 (1H, t), 7.80 (1H, dd), 7.72 (1H, d), 7.61 (1H, t), 6.09 (1H, s), 5.63 (1H, m), 4.59 (2H, d), 2.74 (2H, d), 2.63 (2H, d), 2.24 (3H, s), 1.62 (3H, d), 1.35-1.26 (4H, m). The $^1$H NMR results show that the molar ratio of citric acid to the compound of formula I is 1: 1 in the sample of the crystal form A of the citrate salt.

**Example 8: Preparation of crystal form B of citrate salt of compound of formula I**

300.0 mg of the compound of formula I and 120.7 mg of anhydrous citric acid (feeding molar ratio of 1:1) were weighed into a 60 mL glass vial, and 7.5 mL of IPA/$H_2O$ (19:1, v/v) was added and stirred at room temperature. After 10 min, the sample was observed to be almost dissolved and clear, and after that, 15 mL of *n*-heptane was added to the vial, and the mixture was subjected to temperature cycling and stirred for 3 days. The solid was separated and vacuum dried at room temperature overnight, and a total of 303 mg of sample was collected.

The X-ray powder diffraction pattern shows that the obtained sample is the crystal form B of the citrate salt of the compound of formula I. The crystal form B of the citrate salt of the compound of formula I has an X-ray powder diffraction pattern expressed by 2θ angles, and diffraction peaks and relative intensities of the X-ray powder diffraction pattern can further be shown in Table 15.

Table 15

| Diffraction peak number | 2θ angle (°) | Relative intensity (%) |
|---|---|---|
| 1 | 8.1218 | 1.02 |
| 2 | 9.9016 | 14.74 |
| 3 | 10.0614 | 8.51 |
| 4 | 10.3896 | 3.73 |
| 5 | 11.7789 | 14.10 |
| 6 | 11.9292 | 17.11 |
| 7 | 12.7906 | 11.73 |
| 8 | 15.1889 | 6.82 |
| 9 | 15.4521 | 27.86 |
| 10 | 16.4518 | 33.98 |
| 11 | 16.7237 | 12.65 |
| 12 | 17.6030 | 32.39 |
| 13 | 18.2515 | 8.85 |

(continued)

| Diffraction peak number | 2θ angle (°) | Relative intensity (%) |
|---|---|---|
| 14 | 19.3428 | 7.74 |
| 15 | 19.8659 | 100.00 |
| 16 | 20.0922 | 9.69 |
| 17 | 20.8796 | 28.12 |
| 18 | 21.1802 | 51.78 |
| 19 | 21.6692 | 2.76 |
| 20 | 22.3401 | 0.84 |
| 21 | 23.2208 | 6.47 |
| 22 | 23.5512 | 17.52 |
| 23 | 23.6983 | 13.72 |
| 24 | 23.9640 | 3.65 |
| 25 | 24.3547 | 9.13 |
| 26 | 24.6846 | 7.83 |
| 27 | 25.1969 | 30.57 |
| 28 | 25.7570 | 5.66 |
| 29 | 26.0958 | 2.76 |
| 30 | 26.5982 | 2.22 |
| 31 | 27.0313 | 1.92 |
| 32 | 27.7238 | 8.24 |
| 33 | 28.4748 | 9.85 |
| 34 | 29.9717 | 5.53 |
| 35 | 30.6555 | 10.80 |
| 36 | 31.1973 | 5.24 |
| 37 | 33.1554 | 2.03 |
| 38 | 33.8397 | 1.46 |
| 39 | 34.8016 | 2.27 |
| 40 | 35.7488 | 1.00 |
| 41 | 36.3921 | 1.20 |
| 42 | 37.5999 | 0.63 |
| 43 | 38.3018 | 1.16 |

The X-ray powder diffraction pattern of the crystal form B of the citrate salt is shown in FIG. 25.

The crystal form identification of the crystal form B of the citrate salt was carried out through VT-XRPD. The results showed that (FIG. 26): no change in the crystal form was observed when the sample was purged under $N_2$ for 20 minutes, heated to 150°C, and cooled to room temperature.

In FIG. 27, the DSC pattern shows that the sample has one endothermic peak at 195.0°C.

In FIG. 28, the TGA pattern shows that the sample has a weight loss of 1.26% when heated to 150°C.

The $^1$H NMR spectrum data of the crystal form B of the citrate salt is: $^1$H NMR (400 MHz, DMSO-$d_6$) δ 9.15 (1H, s), 7.96 (1H, t), 7.80 (1H, dd), 7.72 (1H, d), 7.60 (1H, t), 6.09 (1H, s), 5.62 (1H, m), 4.61 (2H, d), 2.73 (2H, d), 2.63 (2H, d), 2.24 (3H, s), 1.61 (3H, d), 1.37-1.20 (4H, m). The molar ratio of citric acid to the compound of formula I is 1: 1 in the sample of the crystal form B of the citrate salt.

In FIG. 29, PLM shows that the crystal form B of the citrate salt is in the form of irregular small particles and aggregation of the samples is observed.

**Example 9: Preparation of crystal form A of methanesulfonate salt of compound of formula I**

60.6 mg of methanesulfonic acid (feeding molar ratio of 1:1) was weighed into a 20 mL glass vial, followed by the addition of 7.5 mL of EtOAc for dilution. 300.2 mg of the compound of formula I was weighed into the diluted system. After 10 min, the sample was observed to be almost dissolved and clear, and after that, 3 mL of n-heptane was added to the vial, and the mixture was subjected to temperature cycling and stirred for 3 days. The solid was separated and vacuum dried at room temperature overnight, and a total of 270 mg of sample was collected.

The X-ray powder diffraction pattern shows that the obtained sample is the crystal form A of the methanesulfonate salt of the compound of formula I. The crystal form A of the methanesulfonate salt of the compound of formula I has an X-ray powder diffraction pattern expressed by 2θ angles, and diffraction peaks and relative intensities of the X-ray powder diffraction pattern can further be shown in Table 16.

Table 16

| Diffraction peak number | 2θ angle (°) | Relative intensity (%) |
|---|---|---|
| 1 | 7.2862 | 89.34 |
| 2 | 9.8423 | 7.40 |
| 3 | 10.8616 | 56.83 |
| 4 | 12.7185 | 67.06 |
| 5 | 14.2081 | 47.55 |
| 6 | 14.5823 | 45.24 |
| 7 | 16.6720 | 57.90 |
| 8 | 18.7082 | 58.29 |
| 9 | 18.9279 | 40.54 |
| 10 | 19.9608 | 100.00 |
| 11 | 20.3837 | 14.37 |
| 12 | 21.3164 | 25.10 |
| 13 | 21.5738 | 51.13 |
| 14 | 21.9370 | 81.96 |
| 15 | 22.8600 | 10.38 |
| 16 | 23.3925 | 18.64 |
| 17 | 23.7735 | 15.02 |
| 18 | 24.5069 | 28.44 |
| 19 | 24.8441 | 13.03 |
| 20 | 25.5949 | 15.81 |
| 21 | 26.2468 | 5.61 |
| 22 | 27.3285 | 4.14 |
| 23 | 28.2624 | 5.37 |
| 24 | 29.7468 | 6.93 |
| 25 | 30.8227 | 9.05 |
| 26 | 31.9822 | 3.70 |
| 27 | 34.1619 | 5.00 |
| 28 | 35.6490 | 2.56 |
| 29 | 38.2044 | 4.63 |

The X-ray powder diffraction pattern of the crystal form A of the methanesulfonate salt is shown in FIG. 30.

In FIG. 31, the DSC pattern shows that the sample has one endothermic peak at 206.0°C.

In FIG. 32, the TGA pattern shows that the sample has a weight loss of 2.14% when heated to 150°C.

The $^1H$ NMR spectrum data of the crystal form A of the methanesulfonate salt is: $^1H$ NMR (400 MHz, $CDCl_3$) $\delta$ 13.27 (1H, s), 10.69 (1H, d), 9.32 (1H, s), 7.90 (1H, t), 7.79 (1H, d), 7.63 (1H, dd), 7.43 (1H, s), 6.40 (1H, s), 5.64 (1H, m), 4.44 (2H, t), 2.86 (3H, s), 2.49 (3H, s), 1.86 (3H, d), 1.55 (2H, s), 1.27 (2H, s). The $^1H$ NMR results show that the molar ratio of methanesulfonic acid to the compound of formula I is 1: 1 in the sample of the crystal form A of the methanesulfonate salt.

In FIG. 33, PLM shows that the crystal form A of the methanesulfonate salt is in the form of irregular small particles and aggregation of the samples is observed.

The crystal form identification of the crystal form A of the methanesulfonate salt was carried out through VT-XRPD. The results showed that (FIG. 34): no change in the crystal form was observed when the sample was purged under $N_2$ for 20 minutes, heated to 150°C, and cooled to room temperature.

**Example 10: Preparation of crystal form A of ethanesulfonate salt of compound of formula I**

69.3 mg of ethanesulfonic acid (feeding molar ratio of 1:1) was weighed into a 20 mL glass vial, followed by the addition of 7.5 mL of EtOAc for dilution. 300.0 mg of the compound of formula I was weighed into the diluted system. After stirring at room temperature for 3 days, the solid was separated and vacuum dried at room temperature overnight, and a total of 304 mg of sample was collected.

The X-ray powder diffraction pattern shows that the obtained sample is the crystal form A of the ethanesulfonate salt of the compound of formula I. The crystal form A of the ethanesulfonate salt of the compound of formula I has an X-ray powder diffraction pattern expressed by 2θ angles, and diffraction peaks and relative intensities of the X-ray powder diffraction pattern can further be shown in Table 17.

Table 17

| Diffraction peak number | 2θ angle (°) | Relative intensity (%) |
|---|---|---|
| 1 | 7.1331 | 100.00 |
| 2 | 7.7512 | 2.43 |
| 3 | 9.6314 | 6.73 |
| 4 | 10.8074 | 16.62 |
| 5 | 12.6099 | 15.69 |
| 6 | 13.4343 | 3.51 |
| 7 | 14.0370 | 28.74 |
| 8 | 14.2676 | 55.92 |
| 9 | 16.4327 | 39.29 |
| 10 | 16.8493 | 3.36 |
| 11 | 18.2789 | 23.50 |
| 12 | 18.7338 | 9.33 |
| 13 | 18.9545 | 5.06 |
| 14 | 19.7019 | 7.62 |
| 15 | 19.9629 | 30.11 |
| 16 | 20.9824 | 7.83 |
| 17 | 21.4515 | 83.20 |
| 18 | 22.4679 | 7.89 |
| 19 | 22.9554 | 10.07 |
| 20 | 23.2675 | 31.01 |
| 21 | 24.4085 | 11.46 |
| 22 | 24.6912 | 9.87 |
| 23 | 25.3570 | 7.70 |

(continued)

| Diffraction peak number | 2θ angle (°) | Relative intensity (%) |
|---|---|---|
| 24 | 26.3787 | 3.47 |
| 25 | 26.6481 | 5.75 |
| 26 | 27.9500 | 1.93 |
| 27 | 29.2757 | 2.78 |
| 28 | 30.0459 | 2.37 |
| 29 | 31.3811 | 3.35 |
| 30 | 32.5227 | 1.46 |
| 31 | 33.4655 | 4.69 |
| 32 | 34.1098 | 1.06 |
| 33 | 34.6685 | 2.96 |
| 34 | 35.0228 | 3.01 |
| 35 | 37.2615 | 6.38 |
| 36 | 38.2123 | 1.67 |
| 37 | 39.2030 | 0.70 |

The X-ray powder diffraction pattern of the crystal form A of the ethanesulfonate salt is shown in FIG. 35.

The crystal form identification of the crystal form A of the ethanesulfonate salt was carried out through VT-XRPD. The results showed that (FIG. 36): no change in the crystal form was observed when the sample was purged under $N_2$ for 20 minutes, heated to 150°C, and cooled to room temperature.

In FIG. 37, the DSC pattern shows that the sample has one endothermic peak at 218.7°C.

In FIG. 38, the TGA pattern shows that the sample has a weight loss of 0.87% when heated to 150°C.

PLM (FIG. 39) shows that the crystal form A of the ethanesulfonate salt is in the form of irregular particles.

The $^1$H NMR spectrum data of the crystal form A of the ethanesulfonate salt is: $^1$H NMR (400 MHz, DMSO-$d_6$) δ 9.38 (1H, s), 8.00 (1H, t), 7.86 (1H, dd), 7.77 (1H, d), 7.65 (1H, t), 6.16 (1H, s), 5.81 (1H, m), 4.59 (2H, d), 2.44 (3H, s), 2.36 (2H, dd), 1.68 (3H, d), 1.43-1.27 (4H, m), 1.05 (3H, t). The molar ratio of ethanesulfonic acid to the compound of formula I is 1:1 in the sample of the crystal form A of the ethanesulfonate salt.

**Example 11: Preparation of crystal form B of ethanesulfonate salt of compound of formula I**

The compound of formula I and an equimolar amount of ethanesulfonic acid were suspended and stirred in MTBE at room temperature for 3 days, and then the solid was separated by centrifugation and vacuum dried at room temperature overnight.

The X-ray powder diffraction pattern shows that the obtained sample is the crystal form B of the ethanesulfonate salt of the compound of formula I. The crystal form B of the ethanesulfonate salt of the compound of formula I has an X-ray powder diffraction pattern expressed by 2θ angles, and diffraction peaks and relative intensities of the X-ray powder diffraction pattern can further be shown in Table 18.

Table 18

| Diffraction peak number | 2θ angle (°) | Relative intensity (%) |
|---|---|---|
| 1 | 3.1872 | 80.75 |
| 2 | 5.9961 | 37.13 |
| 3 | 6.9234 | 80.06 |
| 4 | 8.7147 | 23.19 |
| 5 | 9.8666 | 100.00 |
| 6 | 12.7362 | 13.27 |
| 7 | 15.0169 | 10.98 |

(continued)

| Diffraction peak number | 2θ angle (°) | Relative intensity (%) |
|---|---|---|
| 8 | 16.8410 | 10.47 |
| 9 | 19.2082 | 24.35 |
| 10 | 20.1052 | 60.59 |
| 11 | 21.3893 | 13.13 |
| 12 | 23.3054 | 3.54 |
| 13 | 25.2706 | 16.94 |
| 14 | 29.8583 | 6.01 |

The X-ray powder diffraction pattern of the crystal form B of the ethanesulfonate salt is shown in FIG. 40.

In FIG. 41, the DSC pattern shows that the sample has two endothermic peaks at 156.8°C and 211.9°C and one exothermic peak at 193.0°C.

In FIG. 42, the TGA pattern shows that the sample has a weight loss of 2.17% when heated to 150°C.

The $^1H$ NMR spectrum data of the crystal form B of the ethanesulfonate salt is: $^1H$ NMR (400 MHz, DMSO-$d_6$) δ 9.40 (1H, s), 8.01 (1H, t), 7.87 (1H, dd), 7.77 (1H, d), 7.66 (1H, t), 6.16 (1H, s), 5.82 (1H, m), 4.64 (2H, d), 2.44 (3H, s), 2.36 (3H, dd), 1.68 (3H, d), 1.43-1.27 (4H, m), 1.05 (3H, s). The molar ratio of ethanesulfonic acid to the compound of formula I is 1:1 in the sample of the crystal form B of the ethanesulfonate salt.

**Example 12: Preparation of crystal form A of maleate salt of compound of formula I**

The compound of formula I and an equimolar amount of maleic acid were suspended and stirred in IPA/$H_2O$ (19:1, v/v) at room temperature for 3 days. The resulting clear solution was transferred to 5°C and stirred for 1 day. The solid was separated by centrifugation and vacuum dried at room temperature overnight.

The X-ray powder diffraction pattern shows that the obtained sample is the crystal form A of the maleate salt of the compound of formula I. The crystal form A of the maleate salt of the compound of formula I has an X-ray powder diffraction pattern expressed by 2θ angles, and diffraction peaks and relative intensities of the X-ray powder diffraction pattern can further be shown in Table 19.

Table 19

| Diffraction peak number | 2θ angle (°) | Relative intensity (%) |
|---|---|---|
| 1 | 4.4667 | 54.95 |
| 2 | 5.6155 | 59.30 |
| 3 | 9.0632 | 3.30 |
| 4 | 11.2334 | 100.00 |
| 5 | 11.8651 | 7.21 |
| 6 | 15.3855 | 12.47 |
| 7 | 16.8641 | 28.25 |
| 8 | 18.3097 | 19.29 |
| 9 | 19.2073 | 24.01 |
| 10 | 19.9183 | 5.92 |
| 11 | 20.4217 | 5.60 |
| 12 | 23.0040 | 9.90 |
| 13 | 24.2613 | 7.02 |
| 14 | 25.9499 | 2.87 |
| 15 | 31.3936 | 2.21 |
| 16 | 35.0184 | 0.72 |

The X-ray powder diffraction pattern of the crystal form A of the maleate salt is shown in FIG. 43.
In FIG. 44, the DSC pattern shows that the sample has two endothermic peaks at 160.4°C and 219.1°C.
In FIG. 45, the TGA pattern shows that the sample has a weight loss of 3.14% when heated to 100°C, and a weight loss of 14.49% when heated from 100°C to 225°C.
The $^1H$ NMR spectrum data of the crystal form A of the maleate salt is: $^1H$ NMR (400 MHz, DMSO-$d_6$) δ 9.31 (1H, s), 7.99 (1H, t), 7.84 (1H, dd), 7.75 (1H, d), 7.66 (1H, t), 6.13 (1H, s), 6.09 (2H, s), 5.75 (1H, m), 4.61 (2H, d), 2.44 (3H, s), 1.68 (3H, d), 1.41-1.27 (4H, m). In the sample of the crystal form A of the maleate salt, the peak of maleic acid overlapped with the compound of formula I, and the molar ratio of maleic acid to the compound of formula I is 1:1.

**Example 13: Preparation of crystal form B of maleate salt of compound of formula I**

The compound of formula I and an equimolar amount of maleic acid were suspended and stirred in MTBE for 3 days, and then the solid was separated by centrifugation and vacuum dried at room temperature overnight. The sample of the crystal form B of the maleate salt was obtained.
The X-ray powder diffraction pattern shows that the obtained sample is the crystal form B of the maleate salt of the compound of formula I. The crystal form B of the maleate salt of the compound of formula I has an X-ray powder diffraction pattern expressed by 2θ angles, and diffraction peaks and relative intensities of the X-ray powder diffraction pattern can further be shown in Table 20.

Table 20

| Diffraction peak number | 2θ angle (°) | Relative intensity (%) |
|---|---|---|
| 1 | 3.1784 | 74.33 |
| 2 | 4.9956 | 100.00 |
| 3 | 6.9966 | 18.03 |
| 4 | 10.4659 | 27.05 |
| 5 | 11.2179 | 47.74 |
| 6 | 15.0246 | 6.39 |
| 7 | 15.6048 | 4.21 |
| 8 | 16.8234 | 14.78 |
| 9 | 18.3075 | 14.91 |
| 10 | 18.9268 | 14.55 |
| 11 | 19.5029 | 12.94 |

The X-ray powder diffraction pattern of the crystal form B of the maleate salt is shown in FIG. 46.
In FIG. 47, the DSC pattern shows that the sample has two endothermic peaks at 152.9°C and 220.7°C.
In FIG. 48, the TGA pattern shows that the sample has a weight loss of 2.84% when heated to 100°C, and a weight loss of 12.43% when heated from 100°C to 225°C.
The $^1H$ NMR spectrum data of the crystal form B of the maleate salt is: $^1H$ NMR (400 MHz, DMSO-$d_6$) δ 9.33 (1H, s), 7.99 (1H, t), 7.85 (1H, dd), 7.76 (1H, d), 7.66 (1H, t), 6.14 (1H, s), 6.11 (2H, s), 5.75 (1H, m), 4.61 (2H, d), 2.39 (3H, s), 1.66 (3H, d), 1.40-1.27 (4H, m). In the sample of the crystal form B of the maleate salt, the peak of maleic acid overlapped with the compound of formula I, and the molar ratio of maleic acid to the compound of formula I is 1:1.

**Example 14: Preparation of crystal form A of L-tartrate salt of compound of formula I**

The compound of formula **I** and an equimolar amount of L-tartaric acid were suspended and stirred in IPA/$H_2O$ (19:1, v/v) at room temperature for 3 days, and then the solid was separated by centrifugation and vacuum dried at room temperature overnight.
The X-ray powder diffraction pattern shows that the obtained sample is the crystal form A of the L-tartrate salt of the compound of formula **I.** The crystal form A of the L-tartrate salt of the compound of formula **I** has an X-ray powder diffraction pattern expressed by 2θ angles, and diffraction peaks and relative intensities of the X-ray powder diffraction pattern can further be shown in Table 21.

Table 21

| Diffraction peak number | 2θ angle (°) | Relative intensity (%) |
|---|---|---|
| 1 | 9.2807 | 51.00 |
| 2 | 11.3838 | 27.35 |
| 3 | 12.9746 | 5.62 |
| 4 | 15.6270 | 53.82 |
| 5 | 16.9513 | 6.71 |
| 6 | 18.0771 | 13.13 |
| 7 | 19.5410 | 100.00 |
| 8 | 19.8984 | 93.04 |
| 9 | 22.9497 | 7.07 |
| 10 | 23.5183 | 14.41 |
| 11 | 25.2139 | 32.14 |
| 12 | 26.9208 | 12.77 |
| 13 | 28.4640 | 14.98 |
| 14 | 32.3397 | 5.56 |
| 15 | 34.2393 | 0.99 |

The X-ray powder diffraction pattern of the crystal form A of the L-tartrate salt is shown in FIG. 49.
In FIG. 50, the DSC pattern shows that the sample has two endothermic peaks at 82.2°C and 213.2°C.
In FIG. 51, the TGA pattern shows that the sample has a weight loss of 3.18% when heated to 150°C.
The $^1$H NMR spectrum data of the crystal form A of the L-tartrate salt is: $^1$H NMR (400 MHz, DMSO-$d_6$) δ 9.13 (1H, s), 7.96 (1H, t), 7.85 (1H, dd), 7.76 (1H, d), 7.66 (1H, t), 6.09 (1H, s), 5.61 (1H, t), 4.61 (2H, d), 4.28 (2H, s), 2.22 (3H, s), 1.61 (3H, d), 1.34-1.27 (4H, m). The $^1$H NMR results show that the molar ratio of L-tartaric acid to the compound of formula I is 1: 1 in the sample of the crystal form A of the L-tartrate salt.

**Example 15: Preparation of crystal form B of L-tartrate salt of compound of formula I**

The compound of formula I and an equimolar amount of L-tartaric acid were suspended and stirred in EtOAc at room temperature for 3 days, and then the solid was separated by centrifugation and vacuum dried at room temperature overnight.
The X-ray powder diffraction pattern shows that the obtained sample is the crystal form B of the L-tartrate salt of the compound of formula I. The crystal form B of the L-tartrate salt of the compound of formula I has an X-ray powder diffraction pattern expressed by 2θ angles, and diffraction peaks and relative intensities of the X-ray powder diffraction pattern can further be shown in Table 22.

Table 22

| Diffraction peak number | 2θ angle (°) | Relative intensity (%) |
|---|---|---|
| 1 | 4.2215 | 38.68 |
| 2 | 5.1706 | 40.36 |
| 3 | 5.7735 | 100.00 |
| 4 | 7.4112 | 14.46 |
| 5 | 8.6523 | 32.84 |
| 6 | 10.0921 | 19.37 |
| 7 | 11.5196 | 76.58 |
| 8 | 12.5832 | 11.88 |
| 9 | 14.0808 | 22.24 |

(continued)

| Diffraction peak number | 2θ angle (°) | Relative intensity (%) |
|---|---|---|
| 10 | 14.9152 | 11.32 |
| 11 | 15.2866 | 7.81 |
| 12 | 15.5985 | 12.72 |
| 13 | 16.4184 | 32.78 |
| 14 | 16.5916 | 35.33 |
| 15 | 17.2969 | 67.11 |
| 16 | 18.2001 | 18.09 |
| 17 | 18.6485 | 27.40 |
| 18 | 19.7513 | 19.59 |
| 19 | 20.2201 | 22.35 |
| 20 | 20.7442 | 16.32 |
| 21 | 21.9572 | 12.21 |
| 22 | 23.1125 | 42.52 |
| 23 | 23.5448 | 7.98 |
| 24 | 24.0135 | 5.03 |
| 25 | 24.8468 | 16.48 |
| 26 | 25.2782 | 12.13 |
| 27 | 25.5658 | 9.78 |
| 28 | 27.1150 | 2.89 |
| 29 | 28.4479 | 2.91 |
| 30 | 29.7783 | 2.26 |

The X-ray powder diffraction pattern of the crystal form B of the L-tartrate salt is shown in FIG. 52.

In FIG. 53, the DSC pattern shows that the sample has four endothermic peaks at 114.5°C, 160.5°C, 193.2°C, and 219.5°C.

In FIG. 54, the TGA pattern shows that the sample has a weight loss of 5.57% when heated to 180°C.

The $^1$H NMR spectrum data of the crystal form B of the L-tartrate salt is: $^1$H NMR (400 MHz, DMSO-$d_6$) δ 9.13 (1H, s), 8.92 (1H, s), 7.96 (1H,t), 7.80 (1H, dd), 7.72 (1H, d), 7.66 (1H, t), 6.09 (1H, s), 5.61 (1H, t), 4.63 (2H, d), 4.28 (2H, s), 2.22 (3H, s), 1.61 (3H, d), 1.37-1.25 (4H, m). The $^1$H NMR results show that the molar ratio of L-tartaric acid to the compound of formula I is 1:1 in the sample of the crystal form B of the L-tartrate salt.

**Example 16: Preparation of crystal form C of L-tartrate salt of compound of formula I**

The compound of formula I and an equimolar amount of L-tartaric acid were suspended and stirred in MTBE at room temperature for 3 days, and then the solid was separated by centrifugation and vacuum dried at room temperature overnight.

The X-ray powder diffraction pattern shows that the obtained sample is the crystal form C of the L-tartrate salt of the compound of formula I. The crystal form C of the L-tartrate salt of the compound of formula I has an X-ray powder diffraction pattern expressed by 2θ angles, and diffraction peaks and relative intensities of the X-ray powder diffraction pattern can further be shown in Table 23.

Table 23

| Diffraction peak number | 2θ angle (°) | Relative intensity (%) |
|---|---|---|
| 1 | 4.6038 | 31.81 |
| 2 | 5.7732 | 38.83 |

(continued)

| Diffraction peak number | 2θ angle (°) | Relative intensity (%) |
|---|---|---|
| 3 | 9.2002 | 10.19 |
| 4 | 11.5430 | 30.16 |
| 5 | 12.0755 | 10.27 |
| 6 | 12.5641 | 10.04 |
| 7 | 14.0631 | 15.56 |
| 8 | 14.6835 | 8.49 |
| 9 | 16.5088 | 100.00 |
| 10 | 17.3391 | 28.18 |
| 11 | 18.4699 | 31.10 |
| 12 | 19.1098 | 8.56 |
| 13 | 19.8012 | 20.09 |
| 14 | 20.5691 | 7.70 |
| 15 | 21.9156 | 17.52 |
| 16 | 23.1670 | 16.09 |
| 17 | 24.1677 | 9.73 |
| 18 | 24.8512 | 31.65 |
| 19 | 25.2758 | 21.42 |
| 20 | 27.1192 | 2.21 |
| 21 | 28.4266 | 5.91 |
| 22 | 28.9705 | 3.24 |
| 23 | 29.5693 | 2.56 |
| 24 | 30.9591 | 2.05 |
| 25 | 31.9485 | 1.58 |

The X-ray powder diffraction pattern of the crystal form C of the L-tartrate salt is shown in FIG. 55.

In FIG. 56, the DSC pattern shows that the sample has three endothermic peaks at 128.5°C, 157.1°C, and 220.5°C.

In FIG. 57, the TGA pattern shows that the sample has a weight loss of 3.16% when heated to 150°C.

The $^1H$ NMR data of the crystal form C of the L-tartrate salt is: $^1H$ NMR (400 MHz, DMSO-$d_6$) δ 9.13 (1H, s), 8.93 (1H, d), 7.96 (1H, t), 7.80 (1H, dd), 7.72 (1H, d), 7.60 (1H, t), 6.09 (1H, s), 5.61 (1H, t), 4.63 (2H, d), 4.28 (1H, s), 2.22 (3H, s), 1.61 (3H, d), 1.39-1.20 (4H, m). The molar ratio of L-tartaric acid to the compound of formula I is 0.5:1 in the sample of the crystal form C of the L-tartrate salt.

**Example 17: Preparation of crystal form D of L-tartrate salt of compound of formula I**

The compound of formula I and an equimolar amount of L-tartaric acid were suspended and stirred in acetone/n-heptane (1:3, v/v) at room temperature for 3 days, and then the solid was separated by centrifugation and vacuum dried at room temperature overnight.

The X-ray powder diffraction pattern shows that the obtained sample is the crystal form D of the L-tartrate salt of the compound of formula I. The crystal form D of the L-tartrate salt of the compound of formula I has an X-ray powder diffraction pattern expressed by 2θ angles, and diffraction peaks and relative intensities of the X-ray powder diffraction pattern can further be shown in Table 24.

Table 24

| Diffraction peak number | 2θ angle (°) | Relative intensity (%) |
|---|---|---|
| 1 | 4.2422 | 42.07 |
| 2 | 5.8020 | 53.33 |
| 3 | 8.7267 | 17.54 |
| 4 | 9.4584 | 26.30 |
| 5 | 11.1738 | 21.81 |
| 6 | 11.5661 | 59.25 |
| 7 | 12.5842 | 13.25 |
| 8 | 13.0799 | 19.41 |
| 9 | 14.1192 | 16.86 |
| 10 | 15.7446 | 30.43 |
| 11 | 16.5077 | 44.95 |
| 12 | 17.3896 | 34.63 |
| 13 | 17.6339 | 16.98 |
| 14 | 18.2421 | 26.29 |
| 15 | 19.3518 | 52.51 |
| 16 | 19.6337 | 100.00 |
| 17 | 20.0205 | 46.45 |
| 18 | 20.4894 | 23.24 |
| 19 | 20.7079 | 21.69 |
| 20 | 21.9343 | 15.39 |
| 21 | 23.2390 | 25.50 |
| 22 | 23.6445 | 25.23 |
| 23 | 24.1736 | 8.79 |
| 24 | 24.8661 | 28.61 |
| 25 | 25.2811 | 34.19 |
| 26 | 26.3682 | 9.14 |
| 27 | 27.0293 | 13.90 |
| 28 | 27.4344 | 6.60 |
| 29 | 27.8155 | 6.32 |
| 30 | 28.4773 | 11.93 |
| 31 | 29.3186 | 4.94 |
| 32 | 29.6527 | 4.60 |
| 33 | 30.4539 | 3.66 |
| 34 | 31.7699 | 2.15 |
| 35 | 32.5069 | 5.18 |
| 36 | 34.3724 | 2.69 |
| 37 | 35.2995 | 4.90 |
| 38 | 35.8595 | 3.58 |
| 39 | 36.8085 | 2.50 |

The X-ray powder diffraction pattern of the crystal form D of the L-tartrate salt is shown in FIG. 58.

In FIG. 59, the DSC pattern shows that the sample has five endothermic peaks at 75.0°C, 111.6°C, 161.9°C, 200.9°C, and 218.8°C.

In FIG. 60, the TGA pattern shows that the sample has a weight loss of 2.72% when heated to 150°C.

The $^1$H NMR spectrum data of the crystal form D of the L-tartrate salt is: $^1$H NMR (400 MHz, DMSO-$d_6$) δ 9.13 (1H, s), 8.93 (1H, s), 7.96 (1H, t), 7.80 (1H, d), 7.72 (1H, d), 7.60 (1H, t), 6.09 (1H, s), 5.61 (1H, t), 4.58 (2H, d), 4.29 (2H, s), 2.22 (3H, s), 1.61 (3H, d), 1.35-1.24 (4H, m). The $^1$H NMR results show that the molar ratio of L-tartaric acid to the compound of formula I is 1: 1 in the sample of the crystal form D of the L-tartrate salt.

**Example 18: Preparation of crystal form A of glycolate salt of compound of formula I**

The compound of formula I and an equimolar amount of glycolic acid were suspended and stirred in acetone/n-heptane (1:3, v/v) at room temperature for 3 days, and then the solid was separated by centrifugation and vacuum dried at room temperature overnight.

The X-ray powder diffraction pattern shows that the obtained sample is the crystal form A of the glycolate salt of the compound of formula I. The crystal form A of the glycolate salt of the compound of formula I has an X-ray powder diffraction pattern expressed by 2θ angles, and diffraction peaks and relative intensities of the X-ray powder diffraction pattern can further be shown in Table 25.

Table 25

| Diffraction peak number | 2θ angle (°) | Relative intensity (%) |
|---|---|---|
| 1 | 3.1271 | 72.60 |
| 2 | 5.9057 | 100.00 |
| 3 | 7.2124 | 72.19 |
| 4 | 7.8025 | 86.03 |
| 5 | 8.8362 | 43.52 |
| 6 | 9.3497 | 27.02 |
| 7 | 11.0324 | 28.05 |
| 8 | 11.4234 | 47.43 |
| 9 | 11.7978 | 72.11 |
| 10 | 12.1867 | 20.13 |
| 11 | 13.2497 | 29.84 |
| 12 | 13.4903 | 21.04 |
| 13 | 14.4342 | 23.68 |
| 14 | 14.7436 | 40.29 |
| 15 | 15.6065 | 23.20 |
| 16 | 15.8646 | 24.09 |
| 17 | 16.1643 | 28.68 |
| 18 | 16.4561 | 34.85 |
| 19 | 16.9834 | 19.90 |
| 20 | 17.7296 | 59.46 |
| 21 | 18.6510 | 23.46 |
| 22 | 19.8034 | 42.99 |
| 23 | 20.5404 | 25.02 |
| 24 | 21.9693 | 26.27 |
| 25 | 23.1042 | 36.55 |
| 26 | 23.7184 | 49.07 |

(continued)

| Diffraction peak number | 2θ angle (°) | Relative intensity (%) |
|---|---|---|
| 27 | 26.1802 | 8.05 |
| 28 | 26.7988 | 6.89 |
| 29 | 27.4725 | 7.21 |

The X-ray powder diffraction pattern of the crystal form A of the glycolate salt is shown in FIG. 61.
In FIG. 62, the DSC pattern shows that the sample has one endothermic peak at 110.1°C.
In FIG. 63, the TGA pattern shows that the sample has a weight loss of 3.40% when heated to 150°C.
The $^1H$ NMR spectrum data of the crystal form A of the glycolate salt is: $^1H$ NMR (400 MHz, DMSO-$d_6$) δ 9.12 (1H, s), 8.90 (1H, d), 7.96 (1H, t), 7.79 (1H, dd), 7.72 (1H, d), 7.60 (1H, t), 6.09 (1H, s), 5.60 (1H, t), 4.59 (2H, d), 3.91 (2H, s), 2.21 (3H, s), 1.61 (3H, d), 1.35-1.23 (4H, m).

**Example 19: Preparation of crystal form A of L-malate salt of compound of formula I**

The compound of formula I and an equimolar amount of L-malic acid were suspended and stirred in IPA/$H_2O$ (19:1, v/v) at room temperature for 3 days, and then the solid was separated by centrifugation and vacuum dried at room temperature overnight.
The X-ray powder diffraction pattern shows that the obtained sample is the crystal form A of the L-malate salt of the compound of formula I. The crystal form A of the L-malate salt of the compound of formula I has an X-ray powder diffraction pattern expressed by 2θ angles, and diffraction peaks and relative intensities of the X-ray powder diffraction pattern can further be shown in Table 26.

Table 26

| Diffraction peak number | 2θ angle (°) | Relative intensity (%) |
|---|---|---|
| 1 | 3.1073 | 84.43 |
| 2 | 4.1818 | 70.54 |
| 3 | 5.3014 | 64.57 |
| 4 | 5.9205 | 80.23 |
| 5 | 7.5428 | 49.60 |
| 6 | 8.3245 | 44.27 |
| 7 | 8.7635 | 40.59 |
| 8 | 10.3509 | 26.87 |
| 9 | 11.8207 | 100.00 |
| 10 | 12.4052 | 26.35 |
| 11 | 14.1024 | 33.87 |
| 12 | 16.5624 | 93.55 |
| 13 | 17.6811 | 46.90 |
| 14 | 18.7116 | 34.53 |
| 15 | 19.6954 | 24.29 |
| 16 | 20.8139 | 17.33 |
| 17 | 21.9726 | 63.79 |
| 18 | 23.7515 | 46.99 |
| 19 | 24.6273 | 16.55 |
| 20 | 25.5067 | 11.46 |
| 21 | 26.6295 | 9.10 |

(continued)

| Diffraction peak number | 2θ angle (°) | Relative intensity (%) |
|---|---|---|
| 22 | 27.1413 | 6.62 |
| 23 | 29.4502 | 7.39 |
| 24 | 35.5830 | 2.34 |

The X-ray powder diffraction pattern of the crystal form A of the L-malate salt is shown in FIG. 64.
In FIG. 65, the DSC pattern shows that the sample has four endothermic peaks at 72.9°C, 122.2°C, 142.1°C, and 222.4°C, and one exothermic peak at 195.9°C.
In FIG. 66, the TGA pattern shows that the sample has a weight loss of 2.88% when heated to 50°C, a weight loss of 2.46% when heated from 50°C to 100°C, and a weight loss of 1.31% when heated from 100°C to 150°C.
The $^1H$ NMR spectrum data of the crystal form A of the L-malate salt is: $^1H$ NMR (400 MHz, DMSO-$d_6$) δ 9.13 (1H, s), 8.94 (1H, s), 7.96 (1H, t), 7.79 (1H, dd), 7.72 (1H, d), 7.60 (1H, t), 6.09 (1H, s), 5.61 (1H, t), 4.58 (2H, d), 4.22 (1H, m), 2.63-2.57 (2H, m), 2.22 (3H, s), 1.61 (3H, d), 1.35-1.25 (4H, m). The molar ratio of L-malic acid to the compound of formula I is 1:1 in the sample of the crystal form A of the L-malate salt.

**Example 20: Preparation of crystal form B of L-malate salt of compound of formula I**

The compound of formula I and an equimolar amount of L-malic acid were suspended and stirred in acetone/n-heptane (1:3, v/v) at room temperature for 3 days, and then the solid was separated by centrifugation and vacuum dried at room temperature overnight.
The X-ray powder diffraction pattern shows that the obtained sample is the crystal form B of the L-malate salt of the compound of formula I. The crystal form B of the L-malate salt of the compound of formula I has an X-ray powder diffraction pattern expressed by 2θ angles, and diffraction peaks and relative intensities of the X-ray powder diffraction pattern can further be shown in Table 27.

Table 27

| Diffraction peak number | 2θ angle (°) | Relative intensity (%) |
|---|---|---|
| 1 | 6.6946 | 100.00 |
| 2 | 8.9934 | 7.32 |
| 3 | 9.3090 | 5.00 |
| 4 | 10.6973 | 5.76 |
| 5 | 10.9766 | 9.36 |
| 6 | 11.2990 | 4.77 |
| 7 | 12.7765 | 3.43 |
| 8 | 13.0261 | 7.42 |
| 9 | 13.3853 | 27.84 |
| 10 | 14.6423 | 8.81 |
| 11 | 14.8965 | 7.02 |
| 12 | 15.8948 | 3.96 |
| 13 | 17.0023 | 19.26 |
| 14 | 17.5519 | 2.89 |
| 15 | 18.2765 | 4.87 |
| 16 | 18.7177 | 14.52 |
| 17 | 19.6178 | 1.64 |
| 18 | 20.7001 | 4.16 |
| 19 | 21.1891 | 11.85 |

(continued)

| Diffraction peak number | 2θ angle (°) | Relative intensity (%) |
|---|---|---|
| 20 | 21.7134 | 4.87 |
| 21 | 24.0216 | 2.04 |
| 22 | 24.8829 | 1.93 |
| 23 | 25.6150 | 1.84 |
| 24 | 26.1470 | 1.78 |
| 25 | 26.8997 | 7.51 |
| 26 | 28.6034 | 0.31 |
| 27 | 29.6357 | 1.07 |
| 28 | 33.0994 | 0.92 |
| 29 | 35.0393 | 0.44 |

The X-ray powder diffraction pattern of the crystal form B of the L-malate salt is shown in FIG. 67.

In FIG. 68, the DSC pattern shows that the sample has one endothermic peak at 134.8°C.

In FIG. 69, the TGA pattern shows that the sample has a weight loss of 3.40% when heated to 150°C.

The $^1$H NMR spectrum data of the crystal form B of the L-malate salt is: $^1$H NMR (400 MHz, DMSO-$d_6$) δ 9.14 (1H, s), 8.96 (1H, s), 7.96 (1H, t), 7.79 (1H, dd), 7.72 (1H, d), 7.60 (1H, t), 6.09 (1H, s), 5.61 (1H, t), 4.63 (2H, d), 4.23 (1H, m), 2.64-2.57 (2H, m), 2.23 (3H, s), 1.61 (3H, d), 1.35-1.26 (4H, m). The $^1$H NMR results show that the molar ratio of L-malic acid to the compound of formula I is 1: 1 in the sample of the crystal form B of the L-malate salt.

**Example 21: Preparation of crystal form A of hippurate salt of compound of formula I**

The compound of formula I and an equimolar amount of hippuric acid were suspended and stirred in acetone/n-heptane (1:3, v/v) at room temperature for 3 days, and then the solid was separated by centrifugation and vacuum dried at room temperature overnight.

The X-ray powder diffraction pattern shows that the obtained sample is the crystal form A of the hippurate salt of the compound of formula I. The crystal form A of the hippurate salt of the compound of formula I has an X-ray powder diffraction pattern expressed by 2θ angles, and diffraction peaks and relative intensities of the X-ray powder diffraction pattern can further be shown in Table 28.

Table 28

| Diffraction peak number | 2θ angle (°) | Relative intensity (%) |
|---|---|---|
| 1 | 3.0729 | 26.97 |
| 2 | 8.7876 | 51.26 |
| 3 | 9.1056 | 74.10 |
| 4 | 10.4103 | 73.32 |
| 5 | 12.6933 | 100.00 |
| 6 | 12.9176 | 43.28 |
| 7 | 15.0080 | 57.45 |
| 8 | 15.2256 | 47.92 |
| 9 | 16.0243 | 33.19 |
| 10 | 16.5774 | 24.31 |
| 11 | 16.9340 | 60.96 |
| 12 | 17.6939 | 57.54 |
| 13 | 18.0061 | 83.99 |
| 14 | 18.7195 | 47.71 |

(continued)

| Diffraction peak number | 2θ angle (°) | Relative intensity (%) |
|---|---|---|
| 15 | 20.4015 | 21.63 |
| 16 | 20.6500 | 44.41 |
| 17 | 20.8900 | 39.95 |
| 18 | 21.2771 | 19.32 |
| 19 | 22.3325 | 16.90 |
| 20 | 23.4499 | 2.64 |
| 21 | 24.5380 | 38.84 |
| 22 | 24.8788 | 14.80 |
| 23 | 25.5077 | 11.39 |
| 24 | 26.0053 | 42.91 |
| 25 | 26.1476 | 40.31 |
| 26 | 27.6400 | 14.93 |
| 27 | 28.4368 | 9.35 |
| 28 | 29.3126 | 7.26 |
| 29 | 29.8946 | 13.69 |
| 30 | 31.1147 | 7.88 |
| 31 | 32.3693 | 4.21 |
| 32 | 33.5114 | 2.00 |
| 33 | 34.8745 | 1.39 |
| 34 | 36.6788 | 4.85 |
| 35 | 38.9536 | 3.25 |

The X-ray powder diffraction pattern of the crystal form A of the hippurate salt is shown in FIG. 70.

In FIG. 71, the DSC pattern shows that the sample has three endothermic peaks at 62.4°C, 120.1°C, and 207.0°C.

In FIG. 72, the TGA pattern shows that the sample has a weight loss of 3.04% when heated to 150°C.

The $^1$H NMR spectrum data of the crystal form A of the hippurate salt is: $^1$H NMR (400 MHz, DMSO-$d_6$) δ 9.12 (1H, s), 8.93-8.80 (2H, m), 7.96 (1H, t), 7.90-7.85 (2H, m), 7.79 (1H, dd), 7.72 (1H, d), 7.60 (1H, t), 7.57-7.53 (1H, m), 7.52-7.46 (2H, m), 6.09 (1H, s), 5.61 (1H, t), 4.63 (2H, d). 3.92 (2H, d), 2.21 (3H, s), 1.61 (3H, d), 1.37-1.22 (4H, m). In the sample of the crystal form A of the hippurate salt, the molar ratio of hippuric acid to the compound of formula I is 1:1.

**Example 22: Preparation of crystal form A of succinate salt of compound of formula I**

The compound of formula I and an equimolar amount of succinic acid were suspended and stirred in MTBE at room temperature for 3 days, and then the solid was separated by centrifugation and vacuum dried at room temperature overnight.

The X-ray powder diffraction pattern shows that the obtained sample is the crystal form A of the succinate salt of the compound of formula I. The crystal form A of the succinate salt of the compound of formula I has an X-ray powder diffraction pattern expressed by 2θ angles, and diffraction peaks and relative intensities of the X-ray powder diffraction pattern can further be shown in Table 29.

Table 29

| Diffraction peak number | 2θ angle (°) | Relative intensity (%) |
|---|---|---|
| 1 | 3.1700 | 86.47 |
| 2 | 4.7280 | 100.00 |

(continued)

| Diffraction peak number | 2θ angle (°) | Relative intensity (%) |
|---|---|---|
| 3 | 5.8389 | 42.93 |
| 4 | 7.8741 | 68.91 |
| 5 | 9.3480 | 33.01 |
| 6 | 11.7161 | 34.63 |
| 7 | 12.3943 | 13.72 |
| 8 | 16.6707 | 62.21 |
| 9 | 17.5845 | 27.41 |
| 10 | 19.9906 | 10.56 |
| 11 | 21.8996 | 26.33 |
| 12 | 23.5793 | 13.66 |
| 13 | 25.4028 | 15.64 |

In FIG. 73, the DSC pattern shows that the sample has three endothermic peaks at 70.0°C, 124.1°C, and 221.0°C, and one exothermic peak at 189.8°C.

In FIG. 74, the TGA pattern shows that the sample has a weight loss of 3.94% when heated to 150°C.

FIG. 128 is an XRPD pattern of the crystal form A of the succinate salt of the compound of formula I.

The $^1H$ NMR spectrum data of the crystal form A of the succinate salt is: $^1H$ NMR (400 MHz, DMSO-$d_6$) δ 9.12 (1H, s), 8.90 (1H, d), 7.96 (1H, t), 7.80 (1H, dd), 7.72 (1H, d), 7.60 (1H, t), 6.09 (1H, s), 5.60 (1H, m), 4.62 (2H, d), 2.42 (3H, s), 2.21 (3H, s), 1.61 (3H, d), 1.39-1.21 (4H, m). The $^1H$ NMR results show that the molar ratio of succinic acid to the compound of formula I is 1: 1 in the sample of the crystal form A of the succinate salt.

**Example 23: Preparation of crystal form B of succinate salt of compound of formula I**

The compound of formula I and an equimolar amount of succinic acid were suspended and stirred in acetone/n-heptane (1:3, v/v) at room temperature for 3 days, and then the solid was separated by centrifugation and vacuum dried at room temperature overnight.

The X-ray powder diffraction pattern shows that the obtained sample is the crystal form B of the succinate salt of the compound of formula I. The crystal form B of the succinate salt of the compound of formula I has an X-ray powder diffraction pattern expressed by 2θ angles, and diffraction peaks and relative intensities of the X-ray powder diffraction pattern can further be shown in Table 30.

Table 30

| Diffraction peak number | 2θ angle (°) | Relative intensity (%) |
|---|---|---|
| 1 | 5.7057 | 53.00 |
| 2 | 6.7550 | 8.45 |
| 3 | 9.2474 | 87.35 |
| 4 | 11.4224 | 75.05 |
| 5 | 12.3178 | 5.82 |
| 6 | 13.1359 | 3.84 |
| 7 | 13.5538 | 17.82 |
| 8 | 14.3636 | 2.23 |
| 9 | 15.1062 | 12.67 |
| 10 | 15.8019 | 6.12 |
| 11 | 16.3548 | 8.85 |
| 12 | 17.1711 | 100.00 |

(continued)

| Diffraction peak number | 2θ angle (°) | Relative intensity (%) |
|---|---|---|
| 13 | 18.6793 | 15.17 |
| 14 | 19.1310 | 12.89 |
| 15 | 19.5076 | 8.26 |
| 16 | 19.9919 | 12.86 |
| 17 | 20.5965 | 11.82 |
| 18 | 21.6001 | 5.61 |
| 19 | 21.9368 | 4.27 |
| 20 | 22.5861 | 5.81 |
| 21 | 22.9578 | 28.81 |
| 22 | 23.2550 | 20.09 |
| 23 | 23.6121 | 2.99 |
| 24 | 24.1087 | 12.65 |
| 25 | 25.1373 | 10.27 |
| 26 | 25.4891 | 12.78 |
| 27 | 25.7920 | 9.23 |
| 28 | 26.1262 | 12.30 |
| 29 | 26.6657 | 3.89 |
| 30 | 27.2599 | 2.16 |
| 31 | 28.9889 | 4.67 |
| 32 | 31.5060 | 3.86 |
| 33 | 34.2491 | 2.66 |

The X-ray powder diffraction pattern of the crystal form B of the succinate salt is shown in FIG. 75.

In FIG. 76, the DSC pattern shows that the sample has two endothermic peaks at 105.1°C and 223.3°C.

In FIG. 77, the TGA pattern shows that the sample has a weight loss of 4.44% when heated to 150°C.

The $^1H$ NMR spectrum data of the crystal form B of the succinate salt is: $^1H$ NMR (400 MHz, DMSO-$d_6$) δ 9.12 (1H, s), 8.90 (1H, d), 7.96 (1H, t), 7.80 (1H, dd), 7.72 (1H, d), 7.60 (1H, t), 6.09 (1H, s), 5.60 (1H, m), 4.61 (2H, d), 2.42 (5H, s), 2.22 (3H, s), 1.61 (3H, d), 1.35-1.24 (4H, m). The $^1H$ NMR results show that the molar ratio of succinic acid to the compound of formula I is 1:1 in the sample of the crystal form B of the succinate salt.

**Example 24: Preparation of crystal form A of ascorbate salt of compound of formula I**

The compound of formula I and an equimolar amount of L-ascorbic acid were suspended and stirred in IPA/$H_2O$ (19:1, v/v) at room temperature for 3 days, and then the solid was separated by centrifugation and vacuum dried at room temperature overnight.

The X-ray powder diffraction pattern shows that the obtained sample is the crystal form A of the ascorbate salt of the compound of formula I. The crystal form A of the ascorbate salt of the compound of formula I has an X-ray powder diffraction pattern expressed by 2θ angles, and diffraction peaks and relative intensities of the X-ray powder diffraction pattern can further be shown in Table 31.

Table 31

| Diffraction peak number | 2θ angle (°) | Relative intensity (%) |
|---|---|---|
| 1 | 5.7237 | 100.00 |
| 2 | 9.2032 | 18.95 |
| 3 | 11.3077 | 72.87 |

(continued)

| Diffraction peak number | 2θ angle (°) | Relative intensity (%) |
|---|---|---|
| 4 | 14.3899 | 12.14 |
| 5 | 15.5509 | 22.27 |
| 6 | 16.7168 | 15.25 |
| 7 | 17.5897 | 17.58 |
| 8 | 18.8322 | 18.71 |
| 9 | 20.3077 | 4.66 |
| 10 | 22.3311 | 5.36 |
| 11 | 25.9469 | 17.25 |

In FIG. 78, the DSC pattern shows that the sample has one endothermic peak at 185.7°C and one exothermic peak at 195.8°C.

In FIG. 79, the TGA pattern shows that the sample has a weight loss of 4.36% when heated to 150°C.

FIG. 129 is an XRPD pattern of the crystal form A of the ascorbate salt of the compound of formula I.

The $^1H$ NMR spectrum data of the crystal form A of the ascorbate salt is: $^1H$ NMR (400 MHz, $CD_3OD$) δ 9.18 (1H, s), 7.92 (1H, t), 7.74-7.65 (2H, m), 7.54 (1H, t), 6.35 (1H, s), 5.67 (1H, m), 4.72 (0.5H, d), 3.89 (0.5H, d), 3.67 (1H, d), 2.34 (3H, s), 1.68 (3H, d), 1.41-1.35 (4H, m). The $^1H$ NMR results show that the molar ratio of ascorbic acid to the compound of formula I is 0.5:1 in the sample of the crystal form A of the ascorbate salt.

**Example 25: Preparation of crystal form B of ascorbate salt of compound of formula I**

The compound of formula I and an equimolar amount of L-ascorbic acid were suspended and stirred in EtOAc at room temperature for 3 days, and then the solid was separated by centrifugation and vacuum dried at room temperature overnight.

The X-ray powder diffraction pattern shows that the obtained sample is the crystal form B of the ascorbate salt of the compound of formula I. The crystal form B of the ascorbate salt of the compound of formula I has an X-ray powder diffraction pattern expressed by 2θ angles, and diffraction peaks and relative intensities of the X-ray powder diffraction pattern can further be shown in Table 32.

Table 32

| Diffraction peak number | 2θ angle (°) | Relative intensity (%) |
|---|---|---|
| 1 | 3.1707 | 29.69 |
| 2 | 4.4733 | 23.68 |
| 3 | 5.5081 | 100.00 |
| 4 | 9.1133 | 6.80 |
| 5 | 11.0002 | 93.34 |
| 6 | 13.2393 | 4.60 |
| 7 | 15.2767 | 15.87 |
| 8 | 16.0915 | 20.51 |
| 9 | 16.6356 | 36.02 |
| 10 | 17.4728 | 35.23 |
| 11 | 19.8263 | 45.51 |
| 12 | 21.0910 | 6.18 |
| 13 | 21.5331 | 8.40 |
| 14 | 25.3038 | 10.56 |
| 15 | 25.9223 | 6.22 |

(continued)

| Diffraction peak number | 2θ angle (°) | Relative intensity (%) |
|---|---|---|
| 16 | 26.8139 | 3.34 |
| 17 | 28.0890 | 82.28 |
| 18 | 30.0538 | 40.01 |
| 19 | 35.5913 | 6.78 |
| 20 | 37.5593 | 3.42 |

The X-ray powder diffraction pattern of the crystal form B of the ascorbate salt is shown in FIG. 80.

In FIG. 81, the DSC pattern shows that the sample has one endothermic peak at 153.6°C and one exothermic peak at 190.3°C.

In FIG. 82, the TGA pattern shows that the sample has a weight loss of 1.33% when heated to 150°C.

The $^1$H NMR spectrum data of the crystal form B of the ascorbate salt is: $^1$H NMR (400 MHz, $CD_3OD$) δ 9.19 (1H, s), 7.92 (1H, t), 7.73-7.65 (2H, m), 7.53 (1H, t), 6.35 (1H, s), 5.67 (1H, m), 4.74 (1H, d), 3.89 (1H, m), 3.67 (2H, d), 2.34 (3H, s), 1.69 (3H, d), 1.42-1.35 (4H, m). The $^1$H NMR results show that the molar ratio of L-ascorbic acid to the compound of formula I is 1: 1 in the sample of the crystal form B of the ascorbate salt.

**Example 26: Preparation of crystal form A of adipate salt of compound of formula I**

The compound of formula I and an equimolar amount of adipic acid were suspended and stirred in MTBE at room temperature for 3 days, and then the solid was separated by centrifugation and vacuum dried at room temperature overnight.

The X-ray powder diffraction pattern shows that the obtained sample is the crystal form A of the adipate salt of the compound of formula I. The crystal form A of the adipate salt of the compound of formula I has an X-ray powder diffraction pattern expressed by 2θ angles, and diffraction peaks and relative intensities of the X-ray powder diffraction pattern can further be shown in Table 33.

Table 33

| Diffraction peak number | 2θ angle (°) | Relative intensity (%) |
|---|---|---|
| 1 | 8.4718 | 67.95 |
| 2 | 8.9976 | 61.80 |
| 3 | 11.4167 | 83.68 |
| 4 | 12.4070 | 27.06 |
| 5 | 13.6026 | 21.93 |
| 6 | 15.5779 | 23.86 |
| 7 | 16.2943 | 45.59 |
| 8 | 17.5244 | 47.58 |
| 9 | 18.2396 | 55.02 |
| 10 | 19.8412 | 100.00 |
| 11 | 21.2994 | 36.71 |
| 12 | 23.8124 | 10.35 |
| 13 | 24.6221 | 19.76 |
| 14 | 26.5540 | 9.38 |
| 15 | 27.7925 | 7.62 |
| 16 | 29.8266 | 6.29 |

In FIG. 83, the DSC pattern shows that the sample has one endothermic peak at 88.3°C.

In FIG. 84, the TGA pattern shows that the sample has a weight loss of 6.60% when heated to 150°C.

FIG. 130 is an XRPD pattern of the crystal form A of the adipate salt of the compound of formula I.

The $^{1}H$ NMR spectrum data of the crystal form A of the adipate salt is: $^{1}H$ NMR (400 MHz, DMSO-$d_6$) $\delta$ 9.12 (1H, s), 8.89 (1H, d), 7.96 (1H, t), 7.80 (1H, m), 7.72 (1H, d), 7.60 (1H, t), 6.09 (1H, s), 5.60 (1H, m), 4.61 (2H, d), 2.21 (7H, m), 1.61 (3H, d), 1.50 (4H, m), 1.35-1.25 (4H, m). The molar ratio of adipic acid to the compound of formula I is 1: 1 in the sample of the crystal form A of the adipate salt.

**Example 27: Preparation of crystal form A of *p*-toluenesulfonate salt of compound of formula I**

The compound of formula I and an equimolar amount of *p*-toluenesulfonic acid monohydrate were suspended and stirred in IPA/$H_2O$ (19:1, v/v) at room temperature for 3 days, and then the solid was separated by centrifugation and vacuum dried at room temperature overnight.

The X-ray powder diffraction pattern shows that the obtained sample is the crystal form A of the *p*-toluenesulfonate salt of the compound of formula I. The crystal form A of the p-toluenesulfonate salt of the compound of formula I has an X-ray powder diffraction pattern expressed by 2θ angles, and diffraction peaks and relative intensities of the X-ray powder diffraction pattern can further be shown in Table 34.

Table 34

| Diffraction peak number | 2θ angle (°) | Relative intensity (%) |
|---|---|---|
| 1 | 8.9180 | 22.08 |
| 2 | 9.4393 | 21.33 |
| 3 | 11.5147 | 5.35 |
| 4 | 12.3169 | 61.37 |
| 5 | 13.7084 | 1.36 |
| 6 | 15.9326 | 89.13 |
| 7 | 16.4387 | 100.00 |
| 8 | 17.2684 | 13.78 |
| 9 | 17.9133 | 24.06 |
| 10 | 18.3165 | 16.79 |
| 11 | 19.4915 | 5.98 |
| 12 | 20.6954 | 37.28 |
| 13 | 20.9526 | 30.72 |
| 14 | 21.1925 | 20.91 |
| 15 | 22.7769 | 12.78 |
| 16 | 23.1170 | 24.60 |
| 17 | 23.2986 | 22.76 |
| 18 | 24.3491 | 2.47 |
| 19 | 24.7620 | 14.93 |
| 20 | 25.5566 | 6.70 |
| 21 | 26.3685 | 44.14 |
| 22 | 26.8348 | 10.07 |
| 23 | 27.6967 | 10.29 |
| 24 | 29.2566 | 6.35 |
| 25 | 29.8415 | 2.65 |
| 26 | 30.3293 | 2.33 |
| 27 | 30.9189 | 1.35 |

(continued)

| Diffraction peak number | 2θ angle (°) | Relative intensity (%) |
|---|---|---|
| 28 | 31.7372 | 2.58 |
| 29 | 32.3362 | 2.30 |
| 30 | 33.5831 | 1.87 |
| 31 | 34.9009 | 1.18 |
| 32 | 36.2298 | 1.35 |
| 33 | 37.7435 | 0.63 |

The X-ray powder diffraction pattern of the crystal form A of the*p*-toluenesulfonate salt is shown in FIG. 85.
In FIG. 86, the DSC pattern shows that the sample has one endothermic peak at 150.9°C.
In FIG. 87, the TGA pattern shows that the sample has a weight loss of 0.76% when heated to 100°C, and a weight loss of 3.07% when heated from 100°C to 170°C.
The $^1H$ NMR spectrum data of the crystal form A of the *p*-toluenesulfonate salt is: $^1H$ NMR (400 MHz, DMSO-$d_6$) δ 9.38 (1H, s), 8.00 (1H, t), 7.87 (1H, dd), 7.77 (1H, d), 7.67 (1H, t), 7.47 (2H, dd), 7.11 (2H, d), 6.15 (1H, s), 5.81 (1H, t), 4.63 (2H, d), 2.44 (3H, s), 2.29 (3H, s), 1.68 (3H, d), 1.41-1.27 (4H, m). The molar ratio ofp-toluenesulfonic acid to the compound of formula I is 1:1 in the sample of the crystal form A of the p-toluenesulfonate salt.

**Example 28: Preparation of crystal form A of benzenesulfonate salt of compound of formula I**

The compound of formula I and an equimolar amount of benzenesulfonic acid were suspended and stirred in MTBE at room temperature for 3 days, and then the solid was separated by centrifugation and vacuum dried at room temperature overnight.
The X-ray powder diffraction pattern shows that the obtained sample is the crystal form A of the benzenesulfonate salt of the compound of formula I. The crystal form A of the benzenesulfonate salt of the compound of formula I has an X-ray powder diffraction pattern expressed by 2θ angles, and diffraction peaks and relative intensities of the X-ray powder diffraction pattern can further be shown in Table 35.

Table 35

| Diffraction peak number | 2θ angle (°) | Relative intensity (%) |
|---|---|---|
| 1 | 3.0977 | 63.58 |
| 2 | 6.5455 | 11.06 |
| 3 | 8.7674 | 17.07 |
| 4 | 10.8117 | 35.66 |
| 5 | 11.3821 | 49.24 |
| 6 | 11.9132 | 24.99 |
| 7 | 13.3227 | 10.58 |
| 8 | 14.0177 | 7.67 |
| 9 | 15.4042 | 9.34 |
| 10 | 16.2565 | 40.08 |
| 11 | 17.4115 | 29.89 |
| 12 | 18.3594 | 44.95 |
| 13 | 19.3888 | 32.82 |
| 14 | 19.9059 | 77.88 |
| 15 | 20.6784 | 53.41 |
| 16 | 21.3155 | 100.00 |
| 17 | 22.8963 | 18.69 |

(continued)

| Diffraction peak number | 2θ angle (°) | Relative intensity (%) |
|---|---|---|
| 18 | 25.8671 | 15.29 |
| 19 | 27.7592 | 9.57 |
| 20 | 32.2150 | 6.81 |

In FIG. 88, the DSC pattern shows that the sample has one endothermic peak at 111.7°C.

In FIG. 89, the TGA pattern shows that the sample has a weight loss of 2.55% when heated to 80°C, and a weight loss of 6.23% when heated from 80°C to 150°C.

FIG. 131 is an XRPD pattern of the crystal form A of the benzenesulfonate salt of the compound of formula I in Example 28.

The $^1$H NMR spectrum data of the crystal form A of the benzenesulfonate salt is: $^1$H NMR (400 MHz, DMSO-$d_6$) δ 9.40 (1H, s), 8.01 (1H, t), 7.87 (1H, dd), 7.77 (1H, d), 7.67 (1H, t), 7.62-7.56 (2H, m), 7.35-7.28 (3H, m), 6.16 (1H, s), 5.82 (1H, m), 4.64 (2H, d), 2.45 (3H, s), 1.68 (3H, d), 1.42-1.28 (4H, m). The molar ratio of benzenesulfonic acid to the compound of formula I is 1:1 in the sample of the crystal form A of the benzenesulfonate salt.

**Example 29: Preparation of crystal form A of oxalate salt of compound of formula I**

The compound of formula I and an equimolar amount of oxalic acid were suspended and stirred in Acetone/n-heptane (1:3, v/v) at room temperature for 3 days, and then the solid was separated by centrifugation and vacuum dried at room temperature overnight.

The X-ray powder diffraction pattern shows that the obtained sample is the crystal form A of the oxalate salt of the compound of formula I. The crystal form A of the oxalate salt of the compound of formula I has an X-ray powder diffraction pattern expressed by 2θ angles, and diffraction peaks and relative intensities of the X-ray powder diffraction pattern can further be shown in Table 36.

Table 36

| Diffraction peak number | 2θ angle (°) | Relative intensity (%) |
|---|---|---|
| 1 | 6.1579 | 65.04 |
| 2 | 7.0217 | 9.02 |
| 3 | 9.1873 | 7.84 |
| 4 | 10.4448 | 97.21 |
| 5 | 13.9480 | 19.90 |
| 6 | 15.1277 | 5.59 |
| 7 | 15.5499 | 5.85 |
| 8 | 16.7440 | 18.11 |
| 9 | 17.4987 | 100.00 |
| 10 | 18.1693 | 9.62 |
| 11 | 19.5112 | 11.28 |
| 12 | 20.0751 | 16.44 |
| 13 | 22.6414 | 5.26 |
| 14 | 23.5997 | 9.73 |
| 15 | 24.5061 | 29.87 |
| 16 | 25.2293 | 4.87 |
| 17 | 25.7921 | 12.09 |
| 18 | 26.5363 | 17.05 |
| 19 | 27.9052 | 3.88 |

(continued)

| Diffraction peak number | 2θ angle (°) | Relative intensity (%) |
|---|---|---|
| 20 | 28.4782 | 6.45 |
| 21 | 29.9326 | 1.60 |
| 22 | 32.6865 | 4.95 |

The X-ray powder diffraction pattern of the crystal form A of the oxalate salt is shown in FIG. 90.
In FIG. 91, the DSC pattern shows that the sample has two endothermic peaks at 103.4°C and 141.6°C.
In FIG. 92, the TGA pattern shows that the sample has a weight loss of 5.88% when heated to 100°C.
The $^1$H NMR spectrum data of the crystal form A of the oxalate salt is: $^1$H NMR (400 MHz, DMSO-$d_6$) δ 9.21 (1H, s), 7.98 (1H, t), 7.82 (1H, dd), 7.73 (1H, d), 7.62 (1H, t), 6.11 (1H, s), 5.67 (1H, m), 4.60 (2H, d), 2.28 (3H, s), 1.68 (3H, d), 1.37-1.22 (4H, m). The molar ratio of solvent EtOAc to the compound of formula I is 0.15:1 (approximately 2.3wt%) in the sample of the crystal form A of the oxalate salt. HPLC/IC results show that in the sample of the crystal form A of the oxalate salt, the molar ratio of oxalic acid to the compound of formula I is 1:1.

**Example 30: Preparation of crystal form A of 2-hydroxyethanesulfonate salt of compound of formula I**

The compound of formula I and an equimolar amount of 2-hydroxyethanesulfonic acid were suspended and stirred in EtOAc at room temperature for 3 days, and then the solid was separated by centrifugation and vacuum dried at room temperature overnight.
The X-ray powder diffraction pattern shows that the obtained sample is the crystal form A of the 2-hydroxyethanesulfonate salt of the compound of formula I. The crystal form A of the 2-hydroxyethanesulfonate salt of the compound of formula I has an X-ray powder diffraction pattern expressed by 2θ angles, and diffraction peaks and relative intensities of the X-ray powder diffraction pattern can further be shown in Table 37.

Table 37

| Diffraction peak number | 2θ angle (°) | Relative intensity (%) |
|---|---|---|
| 1 | 6.4673 | 28.94 |
| 2 | 9.8938 | 23.28 |
| 3 | 10.9559 | 21.78 |
| 4 | 13.1418 | 74.74 |
| 5 | 13.6076 | 30.46 |
| 6 | 14.5351 | 5.34 |
| 7 | 15.6934 | 20.61 |
| 8 | 18.3887 | 92.04 |
| 9 | 18.8170 | 100.00 |
| 10 | 19.3894 | 14.35 |
| 11 | 19.8344 | 6.67 |
| 12 | 20.4483 | 26.19 |
| 13 | 20.8232 | 15.84 |
| 14 | 21.1502 | 14.16 |
| 15 | 21.7048 | 53.93 |
| 16 | 22.0768 | 11.35 |
| 17 | 23.2544 | 17.44 |
| 18 | 24.0437 | 26.00 |
| 19 | 24.8212 | 12.74 |
| 20 | 26.0333 | 12.69 |

(continued)

| Diffraction peak number | 2θ angle (°) | Relative intensity (%) |
|---|---|---|
| 21 | 26.3559 | 12.33 |
| 22 | 26.7073 | 10.60 |
| 23 | 29.2874 | 3.69 |
| 24 | 30.6802 | 3.50 |
| 25 | 32.2293 | 4.79 |

The X-ray powder diffraction pattern of the crystal form A of the 2-hydroxyethanesulfonate salt is shown in FIG. 93.

In FIG. 94, the DSC pattern shows that the sample has an endothermic peak at 203.5°C.

In FIG. 95, the TGA pattern shows that the sample has a weight loss of 1.61% when heated to 150°C.

The $^1H$ NMR spectrum data of the crystal form A of the 2-hydroxyethanesulfonate salt is: $^1H$ NMR (400 MHz, DMSO-$d_6$) δ 9.39 (1H, s), 8.00 (1H, t), 7.86 (1H, dd), 7.77 (1H, d), 7.66 (1H, t), 6.15 (1H, s), 5.81 (1H, m), 4.65 (2H, d), 2.44 (3H, s), 2.36 (3H, dd), 1.68 (3H, d), 1.42-1.27 (4H, m), 1.05 (3H, s). The molar ratio of 2-hydroxyethanesulfonic acid to the compound of formula I is 1:1 in the sample of the crystal form A of the 2-hydroxyethanesulfonate salt.

**Example 31: Polymorph screening test**

1. Gas-solid permeation

The gas-solid permeation test was carried out with different solvents. About 20 mg of each sample of the compound of formula I was weighed into a 3 mL vial, and about 3 mL of solvent was added to a 20 mL vial. The 3 mL vial was then placed open in the 20 mL vial, and the 20 mL vial was sealed. The solid was collected after 10 days of standing at room temperature and subjected to XRPD testing. The test results are shown in Table 38. The crystal form A of the compound of formula I was obtained through the gas-solid permeation test.

Table 38 Summary of gas-solid permeation test

| Test number | Solvent | Result |
|---|---|---|
| 1 | ACN | Crystal form A of compound of formula I* |
| 2 | EtOAc | Crystal form A of compound of formula I* |
| 3 | MTBE | Crystal form A of compound of formula I |
| 4 | DMSO | Crystal form A of compound of formula I* |
| *: obtained by volatilization at room temperature after clarification. | | |

2. Gas-liquid permeation

The gas-liquid permeation test was carried out with different solvents. About 20 mg of each sample of the compound of formula I was weighed into a 3 mL vial, and 0.1 to 0.2 mL of a solvent was added for dissolution to obtain a clear solution. About 3 mL of anti-solvent was added to a 20 mL vial, and the 3 mL vial containing the clear solution was then placed open in the 20 mL vial, and the 20 mL vial was sealed and allowed to stand at room temperature. The resulting solid was collected and subjected to XRPD testing. The test results are shown in Table 39. The crystal form A of the compound of formula I was obtained through the gas-liquid permeation test.

Table 39 Summary of gas-liquid permeation test

| Test number | Solvent | Anti-solvent | Result |
|---|---|---|---|
| 1 | THF | *n*-Pentane | Crystal form A of compound of formula I |
| 2 | EtOAc | *n*-Hexane | Crystal form A of compound of formula I |
| 3 | IPA | | Crystal form A of compound of formula I |
| 4 | MEK | | Crystal form A of compound of formula I |

(continued)

| Test number | Solvent | Anti-solvent | Result |
|---|---|---|---|
| 5 | Acetone | MTBE | Crystal form A of compound of formula I |

3. Polymer induction

About 20 mg of each sample of the compound of formula I was weighed into a 3 mL vial, and 1.0 mL of ACN was added for dissolution. After dissolving, the mixture was filtered. 2 mg of mixed polymer A: polyvinylpyrrolidone, polyvinyl alcohol, polyvinyl chloride, polyvinyl acetate, hydroxypropylmethylcellulose, and methylcellulose (mixed by equal mass) was added thereto, and the vial was sealed with a sealing film with a few small holes punched in it, and left at room temperature to slowly volatilization to obtain the crystal form A of the compound of formula I.

4. Room temperature suspended stirring

The room temperature suspended stirring test was carried out with different solvents. About 20 mg of each sample of the compound of formula I was weighed into an HPLC glass vial, and 0.5 mL of the solvent listed in Table 40 was respectively added thereto. After the resulting turbid solution was magnetically stirred at room temperature for about 7 days, the solid sample was separated by centrifugation and subjected to XRPD testing. The test results are shown in Table 40. The crystal form A of the compound of formula I was obtained through the room temperature suspended stirring test.

Table 40 Summary of room temperature suspended stirring test

| Test number | Solvent (v/v) | Result |
|---|---|---|
| 1 | Anisole | Crystal form A of compound of formula I |
| 2 | MTBE | Crystal form A of compound of formula I |
| 3 | *n*-Heptane | Crystal form A of compound of formula I |
| 4 | $H_2O$ | Crystal form A of compound of formula I |
| 5 | $CHCl_3$/Anisole (1:4) | Crystal form A of compound of formula I |
| 6 | MeOH/$H_2O$ (1:4) | Crystal form A of compound of formula I |
| 7 | ACN/MTBE (1:9) | Crystal form A of compound of formula I |
| 8 | ACN/$H_2O$ (1:4) | Crystal form A of compound of formula I |
| 9 | EtOAc/*n*-Hexane (1:4) | Crystal form A of compound of formula I |
| 10 | Acetone/*n*-Heptane (1:4) | Crystal form A of compound of formula I |
| 11 | DCM/Cyclohexane (1:4) | Crystal form A of compound of formula I |
| 12 | 2-MeTHF/Cyclohexane (1:4) | Crystal form A of compound of formula I |
| 13 | IPAc/CPME (1:1) | Crystal form A of compound of formula I |
| 14 | ACN/Toluene (1:9) | Crystal form A of compound of formula I |
| 15 | DMF/$H_2O$ (1:9) | Crystal form A of compound of formula I |
| 16 | THF/*n*-Pentane (1:4) | Crystal form A of compound of formula I |
| 17 | 1,4-Dioxane/Cumene (1:4) | Crystal form A of compound of formula I |

5. 50°C suspended stirring

The 50°C suspended stirring test was carried out with different solvents. About 20 mg of each sample of the compound of formula I was weighed into an HPLC glass vial, and 0.5 mL of the solvent listed in Table 41 was respectively added thereto. After the resulting suspension was magnetically stirred at 50°C for about 3 days, the solid sample was separated by centrifugation and subjected to XRPD testing. The test results are shown in Table 41. The crystal form A of the compound of formula I was obtained through the 50°C suspended stirring test.

Table 41 Summary of 50°C suspended stirring test

| Test number | Solvent (v/v) | Result |
|---|---|---|
| 1 | CPME | Crystal form A of compound of formula I |
| 2 | *n*-Heptane | Crystal form A of compound of formula I |
| 3 | $H_2O$ | Crystal form A of compound of formula I |
| 4 | MIBK/Cyclohexane (1:4) | Crystal form A of compound of formula I |
| 5 | MTBE/Toluene (1:1) | Crystal form A of compound of formula I |
| 6 | 1,4-Dioxane/n-Heptane (1:4) | Crystal form A of compound of formula I |
| 7 | $CHCl_3$/*n*-Heptane (1:4) | Crystal form A of compound of formula I |
| 8 | IPAc/*n*-Hexane (1:4) | Crystal form A of compound of formula I |
| 9 | 2-MeTHF/CPME (1:9) | Crystal form A of compound of formula I |
| 10 | ACN/CPME (1:9) | Crystal form A of compound of formula I* |
| 11 | IPA/$H_2O$ (1:4) | Crystal form A of compound of formula I |
| *: After stirring at 50°C and room temperature, a clear solution was obtained and then transferred to 5°C for stirring. | | |

6. Temperature cycle

A total of 12 temperature cycle tests were carried out with different solvents. About 20 mg of each sample of the compound of formula I was weighed into an HPLC glass vial, and 0.5 mL of the solvent listed in Table 42 was respectively added thereto. After the resulting suspension was magnetically stirred under the temperature cycle (50°C to 5°C, 0.1°C/min, 2 cycles), the solid sample was separated by centrifugation and subjected to XRPD testing. The test results are shown in Table 42. The crystal form A of the compound of formula I was obtained through the temperature cycle test.

Table 42 Summary of temperature cycle test

| Test number | Solvent (v/v) | Result |
|---|---|---|
| 1 | MTBE | Crystal form A of compound of formula I |
| 2 | Chlorobenzene | Crystal form A of compound of formula I |
| 3 | Anisole | Crystal form A of compound of formula I |
| 4 | Toluene | Crystal form A of compound of formula I |
| 5 | EtOH/CPME (1:9) | Crystal form A of compound of formula I* |
| 6 | IPA/Cyclohexane (1:9) | Crystal form A of compound of formula I |
| 7 | MEK/*n*-Heptane (1:4) | Crystal form A of compound of formula I |
| 8 | THF/*n*-Hexane (1:4) | Crystal form A of compound of formula I |
| 9 | ACN/MTBE (1:9) | Crystal form A of compound of formula I* |
| 10 | IPAc/*n*-Heptane (1:4) | Crystal form A of compound of formula I |
| 11 | 1,4-Dioxane/CPME (1:9) | Crystal form A of compound of formula I |
| 12 | $H_2O$ | Crystal form A of compound of formula I |
| *: After the temperature cycle, the clear solution was obtained, and then volatilized at room temperature. | | |

7. Slow cooling

The slow cooling test was carried out with different solvent systems. About 20 mg of each sample of the compound of formula I was weighed into an HPLC vial, and 0.5 mL of the solvent listed in Table 43 was respectively added thereto. The mixture was stirred and equilibrated at 50°C for about 2 hours, and then filtered (using a 0.45 $\mu$m PTFE filter

head to filter) to obtain the supernatant. The resulting supernatant was placed in a bioincubator, and cooled from 50°C to 5°C at a rate of 0.05°C/min and maintained at a constant temperature of 5°C. The clear solution was transferred to a constant temperature of -20°C. The precipitated solid was collected and subjected to XRPD testing, and a sample of unprecipitated solid was transferred to room temperature for volatilization. The test results are shown in Table 43. The crystal form A of the compound of formula I was obtained through the slow cooling test.

Table 43 Summary of slow cooling test

| Test number | Solvent (v/v) | Result |
|---|---|---|
| 1 | CPME | Crystal form A of compound of formula I* |
| 2 | ACN/$H_2O$ (1:1) | Crystal form A of compound of formula I* |
| 3 | EtOAc/n-Hexane (1:1) | Crystal form A of compound of formula I |
| *: After slow cooling, the clear solution was obtained, and then volatilized at room temperature. | | |

8. Anti-solvent addition

The anti-solvent addition test was carried out with different solvents. About 20 mg of each sample of the compound of formula I was respectively weighed into a 20 mL vial, and the solid was completely dissolved with 0.1 to 0.5 mL of solvent (see Table 44). To this clear solution, the anti-solvent in Table 44 was added dropwise with stirring until solids precipitated out, or when the total volume of anti-solvent was added to 5 mL, the sample without solid precipitate was allowed to volatilize at room temperature. The precipitated solid was separated and subjected to XRPD testing. The test results are shown in Table 44. The crystal form A of the compound of formula I was obtained through the anti-solvent addition test.

Table 44 Summary of anti-solvent addition test

| Test number | Solvent | Anti-solvent | Result |
|---|---|---|---|
| 1 | Acetone | $H_2O$ | Crystal form A of compound of formula I |
| 2 | MeOH | | Crystal form A of compound of formula I |
| 3 | ACN | | Crystal form A of compound of formula I |
| 4 | DMSO | | Crystal form A of compound of formula I |
| 5 | THF | *n*-Heptane | Crystal form A of compound of formula I |
| 6 | 1,4-Dioxane | | Crystal form A of compound of formula I |
| 7 | EtOAc | | Crystal form A of compound of formula I |
| 8 | DCM | | Crystal form A of compound of formula I |
| 9 | Anisole | | Crystal form A of compound of formula I |
| 10 | 2-MeTHF | Toluene | Crystal form A of compound of formula I |
| 11 | IPAc | MTBE | Crystal form A of compound of formula I* |
| 12 | MEK | | Crystal form A of compound of formula I* |
| 13 | ACN | | Crystal form A of compound of formula I* |
| *: After adding the anti-solvent, the clear solution was obtained, and then volatilized at room temperature. | | | |

In the effect examples of the present disclosure, the preparation of control compounds 1 and 2 refers to patent WO2019122129A1. Their structures are as follows:

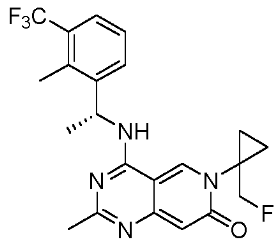

Control compound 1

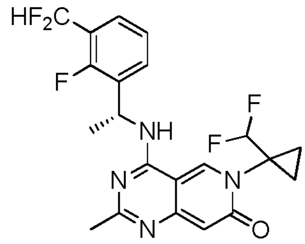

Control compound 2

**Effect example 1: Inhibition test of compound of formula I on KRAS G12C::SOS1 binding**

The compound to be tested was prepared into a 10 mM stock solution with DMSO, and the compound was serially diluted using 1X test buffer. 0.1 μL of the compound solution with different concentrations was transferred to a 384-well plate. 5 μL of GST-KRAS G12C was added to the 384-well plate, and centrifuged at 1000 rpm for 1 min. 5 μL of His-SOS1 was added to the 384-well plate, centrifuged at 1000 rpm for 1 min, and incubated at room temperature for 15 min.

After incubation, 10 μL of a mixed solution of anti-6his-Tb monoclonal antibody (Cisbio, Cat. No. 61HI2TLA) and anti-GST-XL665 monoclonal antibody (Cisbio, Cat. No. 61GSTXLA) was added to the test well, centrifuged at 1000 rpm for 1 min, and incubated at room temperature for 1 h.

After incubation, the fluorescence signal ratios at 665 nm and 615 nm were read on a multifunctional microplate reader (Perkin Elmer, Envision 2104), and the $IC_{50}$ values were calculated using Graphpad 5 software.

The inhibition results of the compound of formula I on KRAS G12C::SOS1 binding are shown in Table 45.

Table 45

| Test compound | $IC_{50}$ (nM) |
|---|---|
| Control compound 1 | 8.58 |
| Control compound 2 | 8.15 |
| Compound of formula I | 3.94 |

**Effect example 2: Inhibition test of compound of formula I on ERK phosphorylation level in DLD-1 cells**

Intracellular western blot quantitative analysis was used to detect the inhibitory level of compounds on ERK phosphorylation in DLD-1 cells.

DLD-1 cells (ATCC, CCL-221) were seeded in a T75 culture flask at $2.5 \times 10^6$ cells/flask, and cultured in a RPMI 1640 medium containing 10% FBS for 2 days. On day 3, the cells were seeded on a 384-well plate, and cultured overnight at 37°C, 5% $CO_2$. After overnight culture, the plate was added with serially diluted compounds (the final content of DMSO was 0.5%), the negative group was added with DMSO, and incubated in a 37°C, 5% $CO_2$ incubator.

The cells were fixed, washed once with PBS, treated to disrupt membranes, and blocked at room temperature for 1 h. The blocking solution was removed. The plate was added with primary antibody (CST, Cat. No. #4370S), and incubated overnight at 4°C. The plate was washed 3 times with PBST (PBS solution added with 0.05% Tween-20), and soaked for 2 min each time. The plate was added with secondary antibody (LI-COR, Cat. No. 926-32211), and incubated at room temperature in the dark. The plate was washed 3 times with PBST, and soaked for 2 min each time. The plate was centrifuged at 1000 rpm for 1 min, scanned on a two-color infrared laser imaging system (Odyssey® CLX) to read the signal.

$$\text{Relative signal} = \text{800-channel signal values/700-channel signal values.}$$

Relative expression level of ERK phosphorylation = (test compound - control compound I)/(DMSO group - control compound I)

$IC_{50}$ values were calculated using Graphpad 5 software.

The inhibition results of the compound of formula I on ERK phosphorylation level in DLD-1 cells are shown in Table 46.

Table 46

| Test compound | $IC_{50}$ (nM) |
|---|---|
| Control compound 1 | 72 |
| Compound of formula I | 56 |

**Effect example 3: Inhibition test of compound of formula I on 3D cell proliferation**

H358 cells were seeded in a T75 culture flask, and cultured in a RPMI 1640 medium containing 10% FBS for 2 days for subsequent experiments in which the cells would be cultured or seeded on a 384-well plate.

On day 1, the cells were seeded on the 384-well plate, with 40 μL of medium added per well and serially diluted compounds or DMSO added per well, and an additional well without seeded cells but with medium was set up as a blank control. After culture at 37°C, 5% $CO_2$ for 7 days, the plate was added with 3D CellTiter-Glo reagent (Promega, Cat. No. G9683) on day 8, shaken at 320 rpm for 20 min, and left at room temperature for 2 h. Luminescence signals were read on a multifunctional microplate reader. Cell viability inhibition rate was calculated as:

Cell viability inhibition rate = (DMSO group - test compound)/(DMSO group - blank control group) × 100%

$IC_{50}$ values were calculated using Graphpad 5 software.

The inhibition results of the compound of formula I on 3D cell proliferation in H358 cells are shown in Table 47.

Table 47

| Test compound | $IC_{50}$ (nM) |
|---|---|
| Control compound 1 | 13 |
| Compound of formula I | 11.5 |

**Effect example 4: Human liver microsome stability test of compound of formula I**

The human liver microsome stability test was performed by incubating the compound and human liver microsomes *in vitro.* First, the compound to be tested was prepared into a 10 mM stock solution in DMSO solvent, and then the compound was diluted to 0.5 mM with acetonitrile. Human liver microsomes (Corning) were diluted with PBS into a microsome/buffer solution, and the solution was used to dilute 0.5 mM compound into a working solution, in which the concentration of the compound was 1.5 μM, and the concentration of human liver microsomes was 0.75 mg/mL. A deep-well plate was taken, 30 μL of the working solution was added per well, and then 15 μL of pre-heated 6 mM NADPH solution was added thereto to initiate a reaction, and the reaction was incubated at 37°C. At 0, 5, 15, 30, and 45 min of the incubation, 135 μL of acetonitrile was added to the corresponding wells to terminate the reaction. After the reaction was terminated with acetonitrile at the last time point of 45 min, the deep-well plate was vortexed for 10 min (600 rpm/min), and then centrifuged for 15 min. After centrifugation, the supernatant was collected, and added with purified water in a ratio of 1:1 to perform LC-MS/MS detection. A ratio of a peak area of compound to a peak area of internal standard at each time point was obtained, and the peak area ratios of the compound at 5, 15, 30, and 45 min were compared with the peak area ratio at 0 min to calculate the remaining percentage of the compound at each time point. $T_{1/2}$ was calculated by using GraphPad 5 software.

The results of the human liver microsome stability test of the compound of formula I are shown in Table 48.

Table 48

| Compound | Remaining percentage (%) of compound after incubation for 30 min | $T_{1/2}$ (min) |
|---|---|---|
| Control compound 1 | 70.2 | 64.2 |
| Compound of formula I | 98.7 | 765 |

**Effect example 5: Inhibition test of compound of formula I on cytochrome P450**

The inhibitory potential of compounds on cytochrome P450 (CYP450) subtype CYP3A4 (two substrates of midazolam and testosterone) was detected. First, the compound to be tested was prepared into a 10 mM stock solution in

DMSO solvent, and the CYP3A4 inhibitor ketoconazole was prepared as 10 mM, 2.5 mM, and 2.5 mM stock solutions in DMSO solvent. The test compound and ketoconazole were diluted to 400-fold final concentration (compound: 10 μM, ketoconazole: 2.5 μM) with acetonitrile.

NADPH cofactor (10 mL of potassium phosphate buffer added with 66.7 mg NADPH) at 4-fold final concentration and substrates were prepared with potassium phosphate buffer (0.1 M, pH = 7.4), with CYP3A4 substrate midazolam at a final concentration of 320 μM and CYP3A4 substrate testosterone at a final concentration of 20 μM.

A human liver microsome solution at a concentration of 0.2 mg/mL was prepared with potassium phosphate buffer on ice. A solution of the compound to be tested and a control inhibitor (control compound) solution at 2-fold final concentration were prepared with the human liver microsome solution on ice. The test wells were added with 30 mL of the test compound solution and the control inhibitor solution respectively, and then added with 15 mL of the substrate for duplication. A 96-well assay plate and a NADPH solution were incubated at 37 °C for 5 min, and 15 μL of pre-heated 8 mM NADPH solution was added to the assay plate to initiate the reaction. A CYP3A4 assay plate was pre-incubated at 37°C for 5 min. The plate was added with 120 μL of acetonitrile to terminate the reaction, and after quenching, the plate was shaken on a shaker (IKA, MTS 2/4) for 10 min (600 rpm/min), and then centrifuged for 15 min. After centrifugation, the supernatant was collected, and added with purified water in a ratio of 1:1 to perform LC-MS/MS detection. A ratio of a peak area of compound to a peak area of internal standard was obtained, and the peak area ratio of the compound was compared with the peak area ratio of the control inhibitor to calculate the inhibition rate.

The results of the CYP450 enzyme inhibition test of the compound of formula I are shown in Table 49.

Table 49

| Compound (10 μM) | CYP inhibition rate (%) | |
|---|---|---|
| | CYP3A4 (midazolam) | CYP3A4 (testosterone) |
| Control compound 1 | 33.52 | 8.5 |
| Compound of formula I | 0 | 0 |

**Effect example 6: Plasma protein binding rate of compound of formula I**

Plasma protein binding rate of compounds was detected by equilibrium dialysis (HTDialysis, HTD 96b). The compound was prepared into a 0.5 nM stock solution with DMSO, and then 25-fold diluted with 0.05 M sodium phosphate buffer as a working solution. A blank 96-well plate was taken and preloaded with 380 μL of plasma per well. The plasma was then added with the working solution at 20 μL/well and mixed well, with the compound at a final concentration of 1 μM, containing 0.2% DMSO per well.

100 μL of 0.05 M sodium phosphate buffer was added to the receiver-side of each dialysis chamber (HTD 96b), and then 100 μL of plasma containing the compound was added to the supply-side. The dialysis chamber was covered with a plastic lid, shaken, and incubated at 37°C for 5 h.

After incubation, 25 μL of sample was taken from each of the supply-side and the receiver-side of the dialysis chamber, and placed in the blank 96-well plate. An equal volume of plasma was added to each of the supply-side samples while an equal volume of 0.05 M sodium phosphate buffer was added to each of the receiver-side samples, and mixed well. The 96-well plate was added with an acetonitrile solution containing internal standard at 200 μL per well, vortexed and shaken at 600 rpm for 10 min, and centrifuged at 5594 g for 15 min (Thermo Multifuge × 3R). 50 μL of the supernatant was then transferred to a new 96-well plate, and the samples were mixed with 50 μL of ultra-pure water for LC-MS/MS analysis.

Plasma protein binding rate and free fraction were calculated using the following formulas: % binding rate = 100 × ([supply-side concentration]$_{5h}$ - [receiver-side concentration]$_{5h}$)/[supply-side concentration]$_{5h}$. % free fraction = 100 - % binding rate

The free fractions of the compound of formula I in plasma are shown in Table 50.

Table 50

| Compound | Human (%) | Mouse (%) |
|---|---|---|
| Control compound 1 | 0.9 | 0.5 |
| Compound of formula I | 1.7 | 1.1 |

**Effect example 7: Mouse pharmacokinetic test of compound of formula I**

Male ICR mice (20 to 25 g) were used, and fasted overnight. Three mice were taken and orally administered by

gavage (10 mg/kg). Blood was collected before the administration, and at 15 min, 30 min, 1 h, 2 h, 4 h, 8 h, and 24 h after the administration. 6,800 g of the blood samples were centrifuged at 2 to 8°C for 6 min, and plasma was collected and stored at -80°C. The plasma at each time point was taken and added with an acetonitrile solution containing internal standard in 3 to 5 times the amount, vortexed and mixed for 1 min, and centrifuged at 13,000 rpm/min at 4°C for 10 min. The supernatant was collected, added with water in 3 times the amount, and mixed. An appropriate amount of the mixture was taken for LC-MS/MS analysis. The main pharmacokinetic parameters were analyzed using WinNonlin 7.0 software with non-compartmental model.

The results of the mouse pharmacokinetic test of the compound of formula I are shown in Table 51.

Table 51

| Compound | Pharmacokinetic parameters of mice (oral administration by gavage) | | | |
|---|---|---|---|---|
| | Cmax (ng/mL) | Tmax (hr) | $AUC_{0-t}$ (h * ng/mL) | $T_{1/2}$ (hr) |
| Control compound 1 | 176 | 0.25 | 365 | 1.35 |
| Control compound 2 | 669 | 0.50 | 1002 | 0.96 |
| Compound of formula I | 1442 | 1.00 | 3080 | 0.97 |

**Effect example 8: *In vivo* efficacy test of compound of formula I on Mia Paca-2 pancreatic cancer**

After one week of adaptive feeding of mice, Mia Paca-2 cells in a logarithmic phase were resuspended in serum-free DMEM, and then mixed with Matrigel in a ratio of 1: 1. 1 $\times$ $10^7$ Mia Paca-2 cells were inoculated subcutaneously at the right flank at 100 $\mu$L per mouse, and tumor growth was observed regularly. When the tumor grew to an average volume of 150 to 200 $mm^3$, the mice were randomly divided into a model group and an administration group (single drug, in combination with trametinib) based on the tumor size and body weight. The tumor size and body weight were measured and recorded before and during the administration. After the treatment, the tumor size in the model group was compared with that in the administration group to determine the efficacy.

The tumor inhibitory ability of the compound of formula I at the tumor weight level is shown in Table 52.

Table 52

| **Drug** | **Dose (mg/kg)** | **Average tumor weight at the end of the treatment (g)** | **Tumor weight inhibition rate (relative to the vehicle group)** |
|---|---|---|---|
| Vehicle | -- | 1.14 | -- |
| Control compound 2 | 50, BID | 0.46 | 59% |
| Compound of formula I | 25, BID | 0.35 | 70% |
| Compound of formula I | 50, BID | 0.27 | 76% |
| **Compound** of formula I + Trametinib | Compound of formula I: 25, BID Trametinib: 0.125, BID | 0.13 | 89% |
| Trametinib | 0.125, BID | 0.22 | 80% |
| "--" in the table means no testing. | | | |

The experimental results indicate that the compound of formula I of the present disclosure, alone or in combination with trametinib, has a significant inhibitory effect on Mia Paca-2 tumor growth, and the combined use is more effective than the single use.

**Effect example 9: *In vivo* efficacy test of NCI-H1975 non-small cell lung cancer**

NCI-H1975 tumor cells were cultured in an incubator at 37°C and 5% $CO_2$ using medium containing inactivated RPMI 1640 + 10% FBS. After reaching a confluency rate of 80 to 90%, the cells were passaged by subculturing. After one

week of adaptive feeding in mice, NCI-H1975 cells in logarithmic phase were inoculated subcutaneously into the right flank of mice at a concentration of 5 × $10^6$/100 μL. Tumor growth was regularly monitored until the tumors reached an average volume of 100 to 150 $mm^3$. The mice were then randomly divided into model group and administration group (combined with osimertinib) based on tumor size and weight of mice. Tumor size and animal weight were measured and recorded before and during the administration process. The differences in tumor size between the model group and administration group were compared to determine the efficacy. The Mann Whitney test method was used in Graph Pad 8.0 to compare the significance difference between the combined administration group and the single-drug group of osimertinib, as shown in Table 53.

Table 53 Synergistic anti-tumor effects of test compounds with osimertinib

| Drug | Dose (mg/kg) | Average tumor weight at the end of the treatment (g) | Tumor weight inhibition rate (relative to the vehicle group) |
|---|---|---|---|
| Vehicle | / | 4.1 | / |
| Compound of formula I + Osimertinib | Compound of formula I: 7.5, BID Osimertinib: 1, QD | 1.6 | 60% |
| Compound of formula I + Osimertinib | Compound of formula I: 25, BID Osimertinib: 1, QD | 0.1 | 98% |
| Osimertinib | 1, QD | 3.2 | 22% |

The results show that compared with the single-drug group of osimertinib, when the compound I-1 (7.5 mg/kg, BID or 25 mg/kg, BID) is administered in combination with osimertinib (1 mg/kg, QD) at the end of the treatment, the average tumor volume of the animal is significantly reduced, and the difference is statistically significant. The anti-tumor efficacy of the combination of the compound I-1 and osimertinib is significantly better than that of single-drug administration, and the combined administration of the compound I-1 (25 mg/kg, BID) and osimertinib (1 mg/kg, QD) has a synergistic effect.

**Effect example 10: *In vivo* efficacy test of compound of formula I on LOVO colorectal cancer**

After one week of adaptive feeding of mice, LOVO cells in a logarithmic phase were resuspended in serum-free F12K, 5 × $10^6$ LOVO cells were inoculated subcutaneously at the right flank at 100 μL per mouse, and tumor growth was observed regularly. When the tumor grew to an average volume of 150 to 200 $mm^3$, the mice were randomly divided into a model group and an administration group based on the tumor size and body weight. The tumor size and body weight were measured and recorded before and during the administration. After the treatment, the tumor size in the model group was compared with that in the administration group to determine the efficacy.

The tumor inhibitory ability of the compound of formula I at the tumor weight level is shown in Table 54.

Table 54

| Drug | Dose (mg/kg) | At the end of treatment Average tumor weight (g) | Tumor weight inhibition rate (relative to the vehicle group) |
|---|---|---|---|
| Vehicle | -- | 1.39 | -- |
| Control compound 2 | 50, BID | 1.08 | 23% |
| Compound of formula I | 50, BID | 0.75 | 46% |
| "--" in the table means no testing. | | | |

The experimental results indicate that the compound of the present disclosure has a significant inhibitory effect on LOVO tumor tissue growth, which is better than that of the control compound II.

**Effect example 11: Dynamic solubility**

Preparation of simulated gastric fluid (SGF)

100 mg of NaCl and 50 mg of Triton X-100 were weighed into a 50 mL volumetric flask, and purified water was added to complete dissolution. 816 μL of 1 M hydrochloric acid was added thereto, and the pH was adjusted to 1.8 with 1 M hydrochloric acid or 1 M NaOH solution. Purified water was added to make up to volume.

Preparation of fasted state simulated intestinal fluid (FaSSIF)

340 mg of anhydrous $NaH_2PO_4$, 42 mg of NaOH, and 620 mg of NaCl were weighed into a 100 mL volumetric flask. Purified water was added to complete dissolution. The pH was adjusted to 6.5 with 1 M hydrochloric acid or 1 M NaOH solution. Purified water was added to make up to volume. 110 mg of SIF powder was then weighed into a 50 mL volumetric flask, added with the above solution to complete dissolution and make up to volume.

Preparation of fed state simulated intestinal fluid (FeSSIF)

0.82 mL of glacial acetic acid, 400 mg of NaOH, and 1.18 g of NaCl were taken into a 100 mL volumetric flask. About 90 mL of purified water was added to complete dissolution, and the pH was adjusted to 5.0 with 1 M hydrochloric acid or 1 M NaOH solution. Purified water was added to make up to volume. 560 mg of SIF powder was then weighed into a 50 mL volumetric flask, added with the above solution to complete dissolution and make up to volume.

Rotating mixing was carried out at 37°C at a solid feed concentration of 10 mg/mL (calculated as the compound of formula I). The solubility of the crystal form A, the crystal form A of the fumarate salt, the crystal form B of the citrate salt, the crystal form A of the methanesulfonate salt, and the crystal form A of the ethanesulfonate salt of the compound of formula I in four systems water, SGF, FaSSIF, and FeSSIF was measured at different time points (1, 4, and 24 hours). After sampling at each time point, the samples were centrifuged (10000 rpm) and filtered (0.45 μm PTFE filter head), and the HPLC concentration and pH value of the filtrate were measured. The solid samples after centrifugation were tested for XRPD. The results of the solubility test are summarized in Table 55. The solubility curves are shown in FIG. @. The XRPD results for the samples after the solubility test are shown in FIG. 96 to FIG. 112. The results show that the crystal form A, the crystal form A of the fumarate salt, the crystal form B of the citrate salt, the crystal form A of the methanesulfonate salt, and the crystal form A of the ethanesulfonate salt of the compound of formula I all have high solubility in the four solvents. Among them, no change in crystal form is observed for the crystal form A, the crystal form A of the fumarate salt, and the crystal form B of the citrate salt of the compound of formula I after testing.

Table 55

| Starting material | Solvent | 1 hour | | 4 hours | | 24 hours | |
|---|---|---|---|---|---|---|---|
| | | S | pH | S | pH | S | pH |
| Crystal form A of compound of formula I | $H_2O$ | 0.14 | 5.4 | 0.11 | 6.0 | 0.11 | 5.8 |
| | SGF | 6.84 | 3.2 | 7.67 | 3.3 | 7.76 | 3.2 |
| | FaSSIF | 0.21 | 6.3 | 0.08 | 6.3 | 0.05 | 6.4 |
| | FeSSIF | 2.72 | 5.1 | 2.88 | 5.1 | 2.74 | 5.1 |
| Crystal form A of fumarate salt | $H_2O$ | 0.55 | 4.2 | 0.57 | 4.2 | 0.61 | 4.1 |
| | SGF | 1.96 | 1.8 | 1.94 | 2.0 | 2.09 | 1.7 |
| | FaSSIF | 0.36 | 4.8 | 0.32 | 4.6 | 0.32 | 4.3 |
| | FeSSIF | 3.51 | 4.6 | 2.39 | 4.8 | 2.18 | 4.7 |
| Crystal form B of citrate salt | $H_2O$ | 3.61 | 3.5 | 3.60 | 3.5 | 3.68 | 3.3 |
| | SGF | 9.13 | 2.5 | 9.40 | 2.6 | 9.29 | 2.4 |
| | FaSSIF | 0.79 | 4.5 | 1.18 | 3.9 | 1.61 | 3.6 |
| | FeSSIF | 4.09 | 4.8 | 3.52 | 4.6 | 2.96 | 4.5 |
| Crystal form A of methanesulfonate salt | $H_2O$ | 10.25 | 3.5 | 10.16 | 3.5 | 9.92 | 3.1 |
| | SGF | 9.76 | 1.7 | 9.88 | 1.8 | 10.13 | 1.6 |
| | FaSSIF | 1.44 | 4.3 | 1.61 | 4.4 | 1.83 | 4.0 |
| | FeSSIF | 4.04 | 4.8 | 4.16 | 4.8 | 4.24 | 4.8 |

(continued)

| Starting material | Solvent | 1 hour | | 4 hours | | 24 hours | |
|---|---|---|---|---|---|---|---|
| | | S | pH | S | pH | S | pH |
| Crystal form A of ethanesulfonate salt | $H_2O$ | 10.06 | 2.9 | 9.81 | 3.1 | 10.08 | 2.8 |
| | SGF | 10.42 | 1.6 | 10.13 | 1.5 | 10.11 | 1.5 |
| | FaSSIF | 0.62 | 4.5 | 1.61 | 4.2 | 2.52 | 3.7 |
| | FeSSIF | 2.79 | 4.8 | 3.62 | 4.8 | 3.70 | 4.7 |
| S: solubility (mg/mL). | | | | | | | |

**Effect example 12: Hygroscopicity**

The hygroscopicity of the crystal form A of the fumarate salt, the crystal form B of the citrate salt, the crystal form A of the methanesulfonate salt, the crystal form A of the ethanesulfonate salt, and the crystal form A of the compound of formula I was evaluated by a dynamic vapor sorption analyzer (DVS). Starting from 0%RH or room humidity, the test collected the mass change percentage of the sample as the humidity changed (0%RH to 95%RH) under constant temperature conditions of 25°C. The results of the DVS test and the XRPD results of the samples before and after the DVS test are shown in FIG. 113 to FIG. 122. When lower than 10% RH, the crystal form A of the compound of formula I and the crystal form A of the fumarate salt rapidly dehydrate, and when higher than 10% RH, the samples absorb more moisture and gain more weight with the increase of humidity. The crystal form B of the citrate salt, the crystal form A of the methanesulfonate salt, and the crystal form A of the ethanesulfonate salt are slightly hygroscopic. No change in crystal form is observed for all samples after the DVS test.

**Effect example 13: Solid state stability**

After leaving the crystal form A of the fumarate salt, the crystal form B of the citrate salt, the crystal form A of the methanesulfonate salt, the crystal form A of the ethanesulfonate salt, and the crystal form A of the compound of formula I respectively in an open condition for one week at 25°C/60% RH and 40°C/75% RH, the physical and chemical stability of the samples were measured by XRPD and HPLC. The XRPD results are listed in FIG. 123 to FIG. 127. The results in Table 56 show that no significant decrease is observed in all samples after the solid state stability test, and the crystal forms of the samples are consistent before and after the test. It shows that the crystal form A of the fumarate salt, the crystal form B of the citrate salt, the crystal form A of the methanesulfonate salt, the crystal form A of the ethanesulfonate salt, and the crystal form A of the compound of formula I have good stability.

Table 56 Summary of solid state stability evaluation

| **Crystal form (batch number)** | **Condition** | | **HPLC results** | |
|---|---|---|---|---|
| | | | Purity (area%) | Crystal form |
| Crystal form A of compound of formula I | Onset | | 97.06 | -- |
| | 25°C/60%RH/open | 1 week | 97.13 | Crystal form A of compound of formula I |
| | 40°C/75%RH/open | 1 week | 97.14 | |
| Crystal form A of fumarate salt | Onset | | 98.67 | -- |
| | 25°C/60%RH/open | 1 week | 98.67 | Crystal form A of fumarate salt |
| | 40°C/75%RH/open | 1 week | 98.70 | |
| Crystal form B of citrate salt | Onset | | 98.92 | -- |
| | 25°C/60%RH/open | 1 week | 99.00 | Crystal form A of citrate salt |
| | 40°C/75%RH/open | 1 week | 99.01 | |
| Crystal form A of methanesulfonate salt | Onset | | 99.18 | -- |
| | 25°C/60%RH/open | 1 week | 99.21 | Crystal form A of methanesulfonate salt |
| | 40°C/75%RH/open | 1 week | 99.26 | |

(continued)

| Crystal form (batch number) | Condition | | HPLC results | |
|---|---|---|---|---|
| | | | Purity (area%) | Crystal form |
| Crystal form A of ethanesulfonate salt | Onset | | 99.10 | -- |
| | 25°C/60%RH/open | 1 week | 99.15 | Crystal form A of ethanesulfonate salt |
| | 40°C/75%RH/open | 1 week | 99.29 | |

## Claims

**1.** A crystal form, wherein the crystal form is a crystal form A of a compound of formula I, a crystal form B of the compound of formula I, a crystal form A of a fumarate salt of the compound of formula I, a crystal form B of a fumarate salt of the compound of formula I, a crystal form C of a fumarate salt the compound of formula I, a crystal form A of a citrate salt of the compound of formula I, a crystal form B of a citrate salt of the compound of formula I, a crystal form A of a methanesulfonate salt of the compound of formula I, a crystal form A of an ethanesulfonate salt of the compound of formula I, a crystal form B of an ethanesulfonate salt of the compound of formula I, a crystal form A of a maleate salt of the compound of formula I, a crystal form B of a maleate salt of the compound of formula I, a crystal form A of an L-tartrate salt of the compound of formula I, a crystal form B of an L-tartrate salt of the compound of formula I, a crystal form C of an L-tartrate salt of the compound of formula I, a crystal form D of an L-tartrate salt of the compound of formula I, a crystal form A of a glycolate salt of the compound of formula I, a crystal form A of an L-malate salt of the compound of formula I, a crystal form B of an L-malate salt of the compound of formula I, a crystal form A of a hippurate salt of the compound of formula I, a crystal form A of a succinate salt of the compound of formula I, a crystal form B of a succinate salt of the compound of formula I, a crystal form A of an ascorbate salt of the compound of formula I, a crystal form B of an ascorbate salt of the compound of formula I, a crystal form A of an adipate salt of the compound of formula I, a crystal form A of a *p*-toluenesulfonate salt of the compound of formula I, a crystal form A of a benzenesulfonate salt of the compound of formula I, a crystal form A of an oxalate salt of the compound of formula I, or a crystal form A of a 2-hydroxyethanesulfonate salt of the compound of formula I;

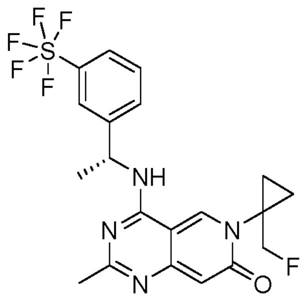

I

wherein the crystal form A of the compound of formula I has an X-ray powder diffraction pattern expressed by 2θ angles comprising diffraction peaks at: 12.06 ± 0.2°, 14.68 ± 0.2°, 18.13 ± 0.2°, 19.12 ± 0.2°, 20.25 ± 0.2°, 22.09 ± 0.2°, and 24.75 ± 0.2°;

the crystal form B of the compound of formula I has an X-ray powder diffraction pattern expressed by 2θ angles comprising diffraction peaks at: 12.30 ± 0.2°, 12.89 ± 0.2°, 14.49 ± 0.2°, 18.10 ± 0.2°, 18.70 ± 0.2°, 20.33 ± 0.2°, and 21.66 ± 0.2°;

the crystal form A of the fumarate salt of the compound of formula I has an X-ray powder diffraction pattern expressed by 2θ angles comprising diffraction peaks at: 6.86 ± 0.2°, 7.72 ± 0.2°, 16.47 ± 0.2°, 19.20 ± 0.2°, 19.63 ± 0.2°, 22.47 ± 0.2°, and 23.26 ± 0.2°;

the crystal form B of the fumarate salt of the compound of formula I has an X-ray powder diffraction pattern expressed by 2θ angles comprising diffraction peaks at: 4.78 ± 0.2°, 8.02 ± 0.2°, 9.68 ± 0.2°, 16.57 ± 0.2°, 17.93 ± 0.2°, 18.57 ± 0.2°, and 28.81 ± 0.2°;

the crystal form C of the fumarate salt of the compound of formula I has an X-ray powder diffraction pattern expressed by 2θ angles comprising diffraction peaks at: 6.85 ± 0.2°, 8.72 ± 0.2°, 13.13 ± 0.2°, 14.09 ± 0.2°, and 17.27 ± 0.2°;

the crystal form A of the citrate salt of the compound of formula I has an X-ray powder diffraction pattern expressed by 2θ angles comprising diffraction peaks at: 6.27 ± 0.2°, 10.79 ± 0.2°, 12.21 ± 0.2°, 12.58 ± 0.2°, 16.38 ± 0.2°, and 25.33 ± 0.2°;
the crystal form B of the citrate salt of the compound of formula I has an X-ray powder diffraction pattern expressed by 2θ angles comprising diffraction peaks at: 11.93 ± 0.2°, 15.45 ± 0.2°, 16.45 ± 0.2°, 17.60 ± 0.2°, 19.86 ± 0.2°, and 21.18 ± 0.2°, or comprising diffraction peaks at: 9.91 ± 0.2°, 12.79 ± 0.2°, 16.45 ± 0.2°, 17.60 ± 0.2°, and 20.88 ± 0.2°;
the crystal form A of the methanesulfonate salt of the compound of formula I has an X-ray powder diffraction pattern expressed by 2θ angles comprising diffraction peaks at: 7.28 ± 0.2°, 10.86 ± 0.2°, 12.71 ± 0.2°, 14.20 ± 0.2°, 14.58 ± 0.2°, 16.67 ± 0.2°, 18.70 ± 0.2°, and 19.96 ± 0.2°;
the crystal form A of the ethanesulfonate salt of the compound of formula I has an X-ray powder diffraction pattern expressed by 2θ angles comprising diffraction peaks at: 7.13 ± 0.2°, 10.80 ± 0.2°, 12.60 ± 0.2°, 14.26 ± 0.2°, 16.43 ± 0.2°, 18.28 ± 0.2°, 19.95 ± 0.2°, 21.45 ± 0.2°, and 23.26 ± 0.2°;
the crystal form B of the ethanesulfonate salt of the compound of formula I has an X-ray powder diffraction pattern expressed by 2θ angles comprising diffraction peaks at: 6.92 ± 0.2°, 9.86 ± 0.2°, 19.20 ± 0.2°, 20.10 ± 0.2°, 21.38 ± 0.2°, 25.27 ± 0.2°, and 29.85 ± 0.2°;
the crystal form A of the maleate salt of the compound of formula I has an X-ray powder diffraction pattern expressed by 2θ angles comprising diffraction peaks at: 4.46 ± 0.2°, 5.61 ± 0.2°, 11.23 ± 0.2°, 16.86 ± 0.2°, 18.30 ± 0.2°, 19.20 ± 0.2°, and 19.91 ± 0.2°;
the crystal form B of the maleate salt of the compound of formula I has an X-ray powder diffraction pattern expressed by 2θ angles comprising diffraction peaks at: 4.99 ± 0.2°, 10.46 ± 0.2°, 11.21 ± 0.2°, 16.82 ± 0.2°, 18.30 ± 0.2°, 18.92 ± 0.2°, and 19.50 ± 0.2°;
the crystal form A of the L-tartrate salt of the compound of formula I has an X-ray powder diffraction pattern expressed by 2θ angles comprising diffraction peaks at: 9.28 ± 0.2°, 11.38 ± 0.2°, 15.62 ± 0.2°, 19.54 ± 0.2°, 19.89 ± 0.2°, and 25.21 ± 0.2°;
the crystal form B of the L-tartrate salt of the compound of formula I has an X-ray powder diffraction pattern expressed by 2θ angles comprising diffraction peaks at: 4.22 ± 0.2°, 5.17 ± 0.2°, 5.77 ± 0.2°, 8.65 ± 0.2°, 11.51 ± 0.2°, 14.08 ± 0.2°, 16.59 ± 0.2°, 17.29 ± 0.2°, and 23.11 ± 0.2°;
the crystal form C of the L-tartrate salt of the compound of formula I has an X-ray powder diffraction pattern expressed by 2θ angles comprising diffraction peaks at: 5.77 ± 0.2°, 11.54 ± 0.2°, 16.50 ± 0.2°, 17.33 ± 0.2°, 18.46 ± 0.2°, 19.80 ± 0.2°, and 24.85 ± 0.2°;
the crystal form D of the L-tartrate salt of the compound of formula I has an X-ray powder diffraction pattern expressed by 2θ angles comprising diffraction peaks at: 5.80 ± 0.2°, 11.56 ± 0.2°, 15.74 ± 0.2°, 16.50 ± 0.2°, 17.38 ± 0.2°, 19.63 ± 0.2°, 23.23 ± 0.2°, 23.64 ± 0.2°, 24.86 ± 0.2°, and 25.28 ± 0.2°;
the crystal form A of the glycolate salt of the compound of formula I has an X-ray powder diffraction pattern expressed by 2θ angles comprising diffraction peaks at: 5.90 ± 0.2°, 7.21 ± 0.2°, 7.80 ± 0.2°, 11.42 ± 0.2°, 11.79 ± 0.2°, 17.72 ± 0.2°, 19.80 ± 0.2°, 23.10 ± 0.2°, and 23.71 ± 0.2°;
the crystal form A of the L-malate salt of the compound of formula I has an X-ray powder diffraction pattern expressed by 2θ angles comprising diffraction peaks at: 5.92 ± 0.2°, 11.82 ± 0.2°, 16.56 ± 0.2°, 17.68 ± 0.2°, 21.97 ± 0.2°, and 23.75 ± 0.2°;
the crystal form B of the L-malate salt of the compound of formula I has an X-ray powder diffraction pattern expressed by 2θ angles comprising diffraction peaks at: 6.69 ± 0.2°, 8.99 ± 0.2°, 9.30 ± 0.2°, 13.38 ± 0.2°, 17.00 ± 0.2°, 18.71 ± 0.2°, 21.18 ± 0.2°, and 26.89 ± 0.2°;
the crystal form A of the hippurate salt of the compound of formula I has an X-ray powder diffraction pattern expressed by 2θ angles comprising diffraction peaks at: 9.10 ± 0.2°, 10.41 ± 0.2°, 12.69 ± 0.2°, 15.00 ± 0.2°, 15.22 ± 0.2°, 16.93 ± 0.2°, 18.00 ± 0.2°, 18.71 ± 0.2°, 24.53 ± 0.2°, and 26.00 ± 0.2°;
the crystal form A of the succinate salt of the compound of formula I has an X-ray powder diffraction pattern expressed by 2θ angles comprising diffraction peaks at: 4.72 ± 0.2°, 5.83 ± 0.2°, 7.87 ± 0.2°, 9.34 ± 0.2°, 11.71 ± 0.2°, 12.39 ± 0.2°, 16.67 ± 0.2°, 17.58 ± 0.2°, 19.99 ± 0.2°, 21.89 ± 0.2°, 23.57 ± 0.2°, and 25.40 ± 0.2°;
the crystal form B of the succinate salt of the compound of formula I has an X-ray powder diffraction pattern expressed by 2θ angles comprising diffraction peaks at: 5.70 ± 0.2°, 9.24 ± 0.2°, 11.42 ± 0.2°, 13.55 ± 0.2°, 17.17 ± 0.2°, and 22.95 ± 0.2°;
the crystal form A of the ascorbate salt of the compound of formula I has an X-ray powder diffraction pattern expressed by 2θ angles comprising diffraction peaks at: 5.72 ± 0.2°, 9.20 ± 0.2°, 11.30 ± 0.2°, 15.55 ± 0.2°, 16.71 ± 0.2°, 17.58 ± 0.2°, 18.83 ± 0.2°, and 25.94 ± 0.2°;
the crystal form B of the ascorbate salt of the compound of formula I has an X-ray powder diffraction pattern expressed by 2θ angles comprising diffraction peaks at: 5.50 ± 0.2°, 11.00 ± 0.2°, 16.63 ± 0.2°, 17.47 ± 0.2°, 19.82 ± 0.2°, 28.08 ± 0.2°, and 30.05 ± 0.2°;

the crystal form A of the adipate salt of the compound of formula I has an X-ray powder diffraction pattern expressed by 2θ angles comprising diffraction peaks at: 8.47 ± 0.2°, 11.41 ± 0.2°, 15.57 ± 0.2°, 16.29 ± 0.2°, 17.52 ± 0.2°, 18.23 ± 0.2°, and 19.84 ± 0.2°;
the crystal form A of the p-toluenesulfonate salt of the compound of formula I has an X-ray powder diffraction pattern expressed by 2θ angles comprising diffraction peaks at: 8.91 ± 0.2°, 9.43 ± 0.2°, 12.31 ± 0.2°, 15.93 ± 0.2°, 16.43 ± 0.2°, 20.69 ± 0.2°, 20.95 ± 0.2°, 24.76 ± 0.2°, and 26.36 ± 0.2°;
the crystal form A of the benzenesulfonate salt of the compound of formula I has an X-ray powder diffraction pattern expressed by 2θ angles comprising diffraction peaks at: 10.81 ± 0.2°, 11.38 ± 0.2°, 16.25 ± 0.2°, 17.41 ± 0.2°, 18.35 ± 0.2°, 19.90 ± 0.2°, and 21.31 ± 0.2°;
the crystal form A of the oxalate salt of the compound of formula I has an X-ray powder diffraction pattern expressed by 2θ angles comprising diffraction peaks at: 6.15 ± 0.2°, 10.44 ± 0.2°, 13.94 ± 0.2°, 16.74 ± 0.2°, 17.49 ± 0.2°, and 24.50 ± 0.2°;
the crystal form A of the 2-hydroxyethanesulfonate salt of the compound of formula I has an X-ray powder diffraction pattern expressed by 2θ angles comprising diffraction peaks at: 6.46 ± 0.2°, 9.89 ± 0.2°, 10.95 ± 0.2°, 13.14 ± 0.2°, 18.38 ± 0.2°, 18.81 ± 0.2°, and 21.70 ± 0.2°.

**2.** The crystal form according to claim 1, wherein the crystal form A of the compound of formula I has an X-ray powder diffraction pattern expressed by 2θ angles comprising diffraction peaks at: 10.30 ± 0.2°, 12.06 ± 0.2°, 12.42 ± 0.2°, 14.68 ± 0.2°, 15.10 ± 0.2°, 17.78 ± 0.2°, 18.13 ± 0.2°, 19.12 ± 0.2°, 20.25 ± 0.2°, 21.76 ± 0.2°, 22.09 ± 0.2°, and 24.75 ± 0.2°;

and/or, the crystal form A of the compound of formula I is a hydrate, wherein the compound of formula I and water have a molar ratio of 1:(0.5 to 1.5), preferably 1:(1 to 1.5);
and/or, the crystal form A of the compound of formula I has a thermogravimetric analysis pattern with a weight loss when heated from an onset to 120 ± 5°C; preferably, the weight loss is 2% to 4%;
and/or, the crystal form A of the compound of formula I has a differential scanning calorimetry pattern with major endothermic peaks at 73.3 ± 3°C and/or 178.0 ± 3°C;
and/or, the crystal form B of the compound of formula I has an X-ray powder diffraction pattern expressed by 2θ angles comprising diffraction peaks at: 12.30 ± 0.2°, 12.89 ± 0.2°, 14.49 ± 0.2°, 15.32 ± 0.2°, 17.45 ± 0.2°, 18.10 ± 0.2°, 18.70 ± 0.2°, 20.33 ± 0.2°, 21.66 ± 0.2°, 22.01 ± 0.2°, 22.52 ± 0.2°, 23.23 ± 0.2°, 24.35 ± 0.2°, and 24.69 ± 0.2°;
and/or, the crystal form A of the fumarate salt has an X-ray powder diffraction pattern expressed by 2θ angles comprising diffraction peaks at: 6.86 ± 0.2°, 7.72 ± 0.2°, 9.57 ± 0.2°, 16.47 ± 0.2°, 19.20 ± 0.2°, 19.63 ± 0.2°, 22.47 ± 0.2°, 23.26 ± 0.2°, and 23.95 ± 0.2°; preferably, comprising diffraction peaks at: 6.86 ± 0.2°, 7.72 ± 0.2°, 9.57 ± 0.2°, 13.72 ± 0.2°, 15.45 ± 0.2°, 15.87 ± 0.2°, 16.47 ± 0.2°, 17.13 ± 0.2°, 17.47 ± 0.2°, 18.19 ± 0.2°, 18.72 ± 0.2°, 19.20 ± 0.2°, 19.63 ± 0.2°, 19.97 ± 0.2°, 22.47 ± 0.2°, 23.26 ± 0.2°, 23.95 ± 0.2°, 25.93 ± 0.2°, and 31.16 ± 0.2°;
and/or, in the crystal form A of the fumarate salt, the compound of formula I and fumaric acid have a molar ratio of 1: 1;
and/or, the crystal form A of the fumarate salt has a thermogravimetric analysis pattern with a weight loss when heated from an onset to 150 ± 5°C; preferably, the weight loss is 0% to 5%;
and/or, the crystal form A of the fumarate salt has a differential scanning calorimetry pattern with a major endothermic peak at 214.7 ± 3°C;
and/or, the crystal form A of the fumarate salt is a hydrate of the fumarate salt of the compound of formula I, wherein the compound of formula I and water have a molar ratio of 1:(0.5 to 1.5), preferably 1:(1 to 1.5);
and/or, the crystal form B of the fumarate salt has an X-ray powder diffraction pattern expressed by 2θ angles comprising diffraction peaks at: 4.78 ± 0.2°, 8.02 + 0.2°, 9.68 ± 0.2°, 11.93 ± 0.2°, 16.57 ± 0.2°, 17.93 ± 0.2°, 18.57 ± 0.2°, 19.41 ± 0.2°, 19.95 ± 0.2°, 21.10 ± 0.2°, and 28.81 ± 0.2°; preferably, comprising diffraction peaks at: 4.78 ± 0.2°, 5.98 ± 0.2°, 8.02 ± 0.2°, 9.68 ± 0.2°, 11.93 ± 0.2°, 12.44 ± 0.2°, 12.78 ± 0.2°, 14.01 ± 0.2°, 15.38 ± 0.2°, 15.80 ± 0.2°, 16.57 ± 0.2°, 17.36 ± 0.2°, 17.93 ± 0.2°, 18.57 ± 0.2°, 19.41 ± 0.2°, 19.95 ± 0.2°, 21.10 ± 0.2°, 23.97 ± 0.2°, 24.76 ± 0.2°, 25.74 ± 0.2°, 26.56 ± 0.2°, 28.81 ± 0.2°, and 29.48 ± 0.2°;
and/or, the crystal form B of the fumarate salt has a thermogravimetric analysis pattern with a weight loss when heated from an onset to 150 ± 5°C, preferably, the weight loss is 4% to 6%; with a weight loss when heated from 150 ± 5°C to 250 ± 5°C, preferably, the weight loss is 5% to 7%;
and/or, in the crystal form B of the fumarate salt, the compound of formula I and fumaric acid have a molar ratio of 1:1;
and/or, the crystal form B of the fumarate salt has a differential scanning calorimetry pattern with a major endothermic peak at 154.4 ± 3°C and an exothermic peak at 191.9 ± 3°C;

and/or, the crystal form C of the fumarate salt has an X-ray powder diffraction pattern expressed by 2θ angles comprising diffraction peaks at: 6.85 ± 0.2°, 8.72 ± 0.2°, 13.13 ± 0.2°, 14.09 ± 0.2°, 17.27 ± 0.2°, 17.90 ± 0.2°, 20.60 ± 0.2°, 21.42 ± 0.2°, 23.16 ± 0.2°, and 24.04 ± 0.2°; preferably, comprising diffraction peaks at: 6.85 ± 0.2°, 8.72 ± 0.2°, 9.90 ± 0.2°, 12.17 ± 0.2°, 13.13 ± 0.2°, 14.09 ± 0.2°, 14.29 ± 0.2°, 16.33 ± 0.2°, 17.27 ± 0.2°, 17.90 ± 0.2°, 20.60 ± 0.2°, 21.42 ± 0.2°, 23.16 ± 0.2°, and 24.04 ± 0.2°;

and/or, the crystal form C of the fumarate salt has a thermogravimetric analysis pattern with a weight loss when heated from an onset to 150 ± 5°C, preferably, the weight loss is 1% to 3%; with a weight loss when heated from 150 ± 5°C to 250 ± 5°C, preferably, the weight loss is 5% to 7%;

and/or, in the crystal form C of the fumarate salt, the compound of formula I and fumaric acid have a molar ratio of 1:1;

and/or, the crystal form C of the fumarate salt has a differential scanning calorimetry pattern with major endothermic peaks at 94.4 ± 3°C, 145.0 ± 3°C, and 161.2 ± 3°C and an exothermic peak at 190.4 ± 3°C;

and/or, the crystal form A of the citrate salt has an X-ray powder diffraction pattern expressed by 2θ angles comprising diffraction peaks at: 6.27 ± 0.2°, 10.79 ± 0.2°, 12.21 ± 0.2°, 12.58 ± 0.2°, 16.38 ± 0.2°, 18.33 ± 0.2°, and 25.33 ± 0.2°;

and/or, in the crystal form A of the citrate salt, the compound of formula I and citric acid have a molar ratio of 1:1;

and/or, the crystal form A of the citrate salt has a thermogravimetric analysis pattern with a weight loss when heated from an onset to 150 ± 5°C, preferably, the weight loss is 5% to 7%; with a weight loss when heated from 150 ± 5°C to 230 ± 5°C, preferably, the weight loss is 18% to 20%;

and/or, the crystal form A of the citrate salt has a differential scanning calorimetry pattern with major endothermic peaks at 99.1 ± 3°C and 137.9 ± 3°C;

and/or, the crystal form B of the citrate salt has an X-ray powder diffraction pattern expressed by 2θ angles comprising diffraction peaks at: 11.93 ± 0.2°, 12.79 ± 0.2°, 15.45 ± 0.2°, 16.45 ± 0.2°, 17.60 ± 0.2°, 19.86 ± 0.2°, 20.88 ± 0.2°, 21.18 ± 0.2°, 23.55 ± 0.2°, and 25.19 ± 0.2°, or comprising diffraction peaks at: 9.91 ± 0.2°, 12.79 ± 0.2°, 16.45 ± 0.2°, 17.60 ± 0.2°, 20.88 ± 0.2°, 24.34 ± 0.2°, and 24.70 ± 0.2°; preferably, comprising diffraction peaks at one or more of the following 2θ angles: 9.90 ± 0.2°, 11.93 ± 0.2°, 12.79 ± 0.2°, 15.45 ± 0.2°, 16.45 ± 0.2°, 17.60 ± 0.2°, 18.25 ± 0.2°, 19.34 ± 0.2°, 19.86 ± 0.2°, 20.88 ± 0.2°, 21.18 ± 0.2°, 23.55 ± 0.2°, 25.19 ± 0.2°, 27.72 ± 0.2°, 28.47 ± 0.2°, 29.97 ± 0.2°, 30.65 ± 0.2°, and 31.19 ± 0.2°;

and/or, in the crystal form B of the citrate salt, the compound of formula I and citric acid have a molar ratio of 1:1;

and/or, the crystal form B of the citrate salt has a thermogravimetric analysis pattern with a weight loss when heated from an onset to 150 ± 5°C, preferably, the weight loss is 0% to 2%;

and/or, the crystal form B of the citrate salt has a differential scanning calorimetry pattern with a major endothermic peak at 195.0 ± 3°C;

and/or, the crystal form A of the methanesulfonate salt has an X-ray powder diffraction pattern expressed by 2θ angles comprising diffraction peaks at: 7.28 ± 0.2°, 10.86 ± 0.2°, 12.71 ± 0.2°, 14.20 ± 0.2°, 14.58 ± 0.2°, 16.67 ± 0.2°, 18.70 ± 0.2°, 19.96 ± 0.2°, 21.57 ± 0.2°, and 21.93 ± 0.2°; preferably, comprising diffraction peaks at: 7.28 ± 0.2°, 10.86 ± 0.2°, 12.71 ± 0.2°, 14.20 ± 0.2°, 14.58 ± 0.2°, 16.67 ± 0.2°, 18.70 ± 0.2°, 19.96 ± 0.2°, 21.57 ± 0.2°, 21.93 ± 0.2°, 22.86 ± 0.2°, 23.39 ± 0.2°, 23.77 ± 0.2°, 24.50 ± 0.2°, 24.84 ± 0.2°, and 25.59 ± 0.2°;

and/or, in the crystal form A of the methanesulfonate salt, the compound of formula I and methanesulfonic acid have a molar ratio of 1:1;

and/or, the crystal form A of the methanesulfonate salt has a thermogravimetric analysis pattern with a weight loss when heated from an onset to 150 ± 5°C, preferably, the weight loss is 0% to 5%;

and/or, the crystal form A of the methanesulfonate salt has a differential scanning calorimetry pattern with a major endothermic peak at 206.0 ± 3°C;

and/or, the crystal form A of the ethanesulfonate salt has an X-ray powder diffraction pattern expressed by 2θ angles comprising diffraction peaks at: 7.13 ± 0.2°, 9.63 ± 0.2°, 10.80 ± 0.2°, 12.60 ± 0.2°, 14.03 ± 0.2°, 14.26 ± 0.2°, 16.43 ± 0.2°, 18.28 ± 0.2°, 18.73 ± 0.2°, 18.95 ± 0.2°, 19.96 ± 0.2°, 20.98 ± 0.2°, 21.45 ± 0.2°, 22.46 ± 0.2°, 22.95 ± 0.2°, 23.26 ± 0.2°, 24.40 ± 0.2°, 24.69 ± 0.2°, and 25.36 ± 0.2°;

and/or, in the crystal form A of the ethanesulfonate salt, the compound of formula I and ethanesulfonic acid have a molar ratio of 1:1;

and/or, the crystal form A of the ethanesulfonate salt has a thermogravimetric analysis pattern with a weight loss when heated from an onset to 150 ± 5°C, preferably, the weight loss is 0% to 5%;

and/or, the crystal form A of the ethanesulfonate salt has a differential scanning calorimetry pattern with a major endothermic peak at 218.7 ± 3°C;

and/or, the crystal form B of the ethanesulfonate salt has an X-ray powder diffraction pattern expressed by 2θ angles comprising diffraction peaks at: 6.92 ± 0.2°, 8.71 ± 0.2°, 9.86 ± 0.2°, 12.73 ± 0.2°, 15.01 ± 0.2°, 16.84 ± 0.2°, 19.20 ± 0.2°, 20.10 ± 0.2°, 21.38 ± 0.2°, 23.30 ± 0.2°, 25.27 ± 0.2°, and 29.85 ± 0.2°;

and/or, in the crystal form B of the ethanesulfonate salt, the compound of formula I and ethanesulfonic acid have a molar ratio of 1:1;
and/or, the crystal form B of the ethanesulfonate salt has a thermogravimetric analysis pattern with a weight loss when heated from an onset to 150 ± 5°C, preferably, the weight loss is 1% to 3%;
and/or, the crystal form B of the ethanesulfonate salt has a differential scanning calorimetry pattern with major endothermic peaks at 156.8 ± 3°C and 211.9 ± 3°C;
and/or, the crystal form A of the maleate salt has an X-ray powder diffraction pattern expressed by 2θ angles comprising diffraction peaks at: 4.46 ± 0.2°, 5.61 ± 0.2°, 11.23 ± 0.2°, 15.38 ± 0.2°, 16.86 ± 0.2°, 18.30 ± 0.2°, 19.20 ± 0.2°, 19.91 ± 0.2°, 20.42 ± 0.2°, and 23.00 ± 0.2°; preferably, comprising diffraction peaks at: 4.46 ± 0.2°, 5.61 ± 0.2°, 11.23 ± 0.2°, 11.86 ± 0.2°, 15.38 ± 0.2°, 16.86 ± 0.2°, 18.30 ± 0.2°, 19.20 ± 0.2°, 19.91 ± 0.2°, 20.42 ± 0.2°, 23.00 ± 0.2°, and 24.26 ± 0.2°;
and/or, in the crystal form A of the maleate salt, the compound of formula I and maleic acid have a molar ratio of 1:1;
and/or, the crystal form A of the maleate salt has a thermogravimetric analysis pattern with a weight loss when heated from an onset to 100 ± 5°C, preferably, the weight loss is 2% to 4%; with a weight loss when heated from 100 ± 5°C to 225 ± 5°C, preferably, the weight loss is 14% to 16%;
and/or, the crystal form A of the maleate salt has a differential scanning calorimetry pattern with major endothermic peaks at 160.4 ± 3°C and 219.1 ± 3°C;
and/or, the crystal form B of the maleate salt of the compound of formula I has an X-ray powder diffraction pattern expressed by 2θ angles comprising diffraction peaks at: 4.99 ± 0.2°, 6.99 ± 0.2°, 10.46 ± 0.2°, 11.21 ± 0.2°, 15.02 ± 0.2°, 15.60 ± 0.2°, 16.82 ± 0.2°, 18.30 ± 0.2°, 18.92 ± 0.2°, and 19.50 ± 0.2°;
and/or, in the crystal form B of the maleate salt, the compound of formula I and maleic acid have a molar ratio of 1:1;
and/or, the crystal form B of the maleate salt has a thermogravimetric analysis pattern with a weight loss when heated from an onset to 100 ± 5°C, preferably, the weight loss is 2% to 4%; with a weight loss when heated from 100 ± 5°C to 225 ± 5°C, preferably, the weight loss is 11% to 13%;
and/or, the crystal form B of the maleate salt has a differential scanning calorimetry pattern with major endothermic peaks at 152.9 ± 3°C and 220.7 ± 3°C;
and/or, the crystal form A of the L-tartrate salt has an X-ray powder diffraction pattern expressed by 2θ angles comprising diffraction peaks at: 9.28 ± 0.2°, 11.38 ± 0.2°, 12.97 ± 0.2°, 15.62 ± 0.2°, 16.95 ± 0.2°, 18.07 ± 0.2°, 19.54 ± 0.2°, 19.89 ± 0.2°, 22.94 ± 0.2°, 23.51 ± 0.2°, 25.21 ± 0.2°, 26.92 ± 0.2°, and 28.46 ± 0.2°;
and/or, in the crystal form A of the L-tartrate salt, the compound of formula I and L-tartaric acid have a molar ratio of 1:1;
and/or, the crystal form A of the L-tartrate salt has a thermogravimetric analysis pattern with a weight loss when heated from an onset to 150 ± 5°C, preferably, the weight loss is 2% to 4%;
and/or, the crystal form A of the L-tartrate salt has a differential scanning calorimetry pattern with major endothermic peaks at 82.2 ± 3°C and 213.2 ± 3°C;
and/or, the crystal form B of the L-tartrate salt has an X-ray powder diffraction pattern expressed by 2θ angles comprising diffraction peaks at: 4.22 ± 0.2°, 5.17 ± 0.2°, 5.77 ± 0.2°, 8.65 ± 0.2°, 10.09 ± 0.2°, 11.51 ± 0.2°, 14.08 ± 0.2°, 16.59 ± 0.2°, 17.29 ± 0.2°, 18.20 ± 0.2°, 18.64 ± 0.2°, 19.75 ± 0.2°, 20.22 ± 0.2°, 20.74 ± 0.2°, 21.95 ± 0.2°, and 23.11 ± 0.2°;
and/or, in the crystal form B of the L-tartrate salt, the compound of formula I and L-tartaric acid have a molar ratio of 1:1;
and/or, the crystal form B of the L-tartrate salt has a thermogravimetric analysis pattern with a weight loss when heated from an onset to 180 ± 5°C, preferably, the weight loss is 5% to 7%;
and/or, the crystal form B of the L-tartrate salt has a differential scanning calorimetry pattern with major endothermic peaks at 114.5 ± 3°C, 160.5 ± 3°C, 193.2 ± 3°C, and 219.5 ± 3°C;
and/or, the crystal form C of the L-tartrate salt has an X-ray powder diffraction pattern expressed by 2θ angles comprising diffraction peaks at: 4.60 ± 0.2°, 5.77 ± 0.2°, 11.54 ± 0.2°, 14.06 ± 0.2°, 16.50 ± 0.2°, 17.33 ± 0.2°, 18.46 ± 0.2°, 19.80 ± 0.2°, 21.91 ± 0.2°, 23.16 ± 0.2°, 24.85, and 25.27 ± 0.2°; preferably, comprising diffraction peaks at: 4.60 ± 0.2°, 5.77 ± 0.2°, 9.20 ± 0.2°, 11.54 ± 0.2°, 12.07 ± 0.2°, 12.56 ± 0.2°, 14.06 ± 0.2°, 14.68 ± 0.2°, 16.50 ± 0.2°, 17.33 ± 0.2°, 18.46 ± 0.2°, 19.10 ± 0.2°, 19.80 ± 0.2°, 20.56 ± 0.2°, 21.91 ± 0.2°, 23.16 ± 0.2°, 24.16 ± 0.2°, 24.85 ± 0.2°, and 25.27 ± 0.2°;
and/or, in the crystal form C of the L-tartrate salt, the compound of formula I and L-tartaric acid have a molar ratio of 1:0.5;
and/or, the crystal form C of the L-tartrate salt has a thermogravimetric analysis pattern with a weight loss when heated from an onset to 150 ± 5°C, preferably, the weight loss is 2% to 4%;
and/or, the crystal form C of the L-tartrate salt has a differential scanning calorimetry pattern with major endothermic peaks at 128.5 ± 3°C, 157.1 ± 3°C, and 220.5 ± 3°C;
and/or, the crystal form D of the L-tartrate salt has an X-ray powder diffraction pattern expressed by 2θ angles

comprising diffraction peaks at: 5.80 ± 0.2°, 11.56 ± 0.2°, 15.74 ± 0.2°, 16.50 ± 0.2°, 17.38 ± 0.2°, 18.24 ± 0.2°, 19.35 ± 0.2°, 19.63 ± 0.2°, 20.02 ± 0.2°, 23.23 ± 0.2°, 23.64 ± 0.2°, 24.86 ± 0.2°, and 25.28 ± 0.2°; preferably, comprising diffraction peaks at: 4.24 ± 0.2°, 5.80 ± 0.2°, 9.45 ± 0.2°, 11.56 ± 0.2°, 13.07 ± 0.2°, 15.74 ± 0.2°, 16.50 ± 0.2°, 17.38 ± 0.2°, 18.24 ± 0.2°, 19.35 ± 0.2°, 19.63 ± 0.2°, 20.02 ± 0.2°, 20.48 ± 0.2°, 20.70 ± 0.2°, 21.93 ± 0.2°, 23.23 ± 0.2°, 23.64 ± 0.2°, 24.86 ± 0.2°, and 25.28 ± 0.2°;

and/or, in the crystal form D of the L-tartrate salt, the compound of formula I and L-tartaric acid have a molar ratio of 1:1;

and/or, the crystal form D of the L-tartrate salt has a thermogravimetric analysis pattern with a weight loss when heated from an onset to 150 ± 5°C, preferably, the weight loss is 2% to 4%;

and/or, the crystal form D of the L-tartrate salt has a differential scanning calorimetry pattern with major endothermic peaks at 75.0± 3°C, 111.6 ± 3°C, 161.9 ± 3°C, 200.9 ± 3°C, and 218.8 ± 3°C;

and/or, the crystal form A of the glycolate salt has an X-ray powder diffraction pattern expressed by 2θ angles comprising diffraction peaks at: 5.90 ± 0.2°, 7.21 ± 0.2°, 7.80 ± 0.2°, 11.42 ± 0.2°, 11.79 ± 0.2°, 13.24 ± 0.2°, 14.74 ± 0.2°, 16.45 ± 0.2°, 17.72 ± 0.2°, 18.65 ± 0.2°, 19.80 ± 0.2°, 20.54 ± 0.2°, 21.96 ± 0.2°, 23.10 ± 0.2°, and 23.71 ± 0.2°; preferably, comprising diffraction peaks at: 5.90 ± 0.2°, 7.21 ± 0.2°, 7.80 ± 0.2°, 8.83 ± 0.2°, 9.34 ± 0.2°, 11.03 ± 0.2°, 11.42 ± 0.2°, 11.79 ± 0.2°, 12.18 ± 0.2°, 13.24 ± 0.2°, 13.49 ± 0.2°, 14.43 ± 0.2°, 14.74 ± 0.2°, 15.60 ± 0.2°, 15.86 ± 0.2°, 16.16 ± 0.2°, 16.45 ± 0.2°, 16.98 ± 0.2°, 17.72 ± 0.2°, 18.65 ± 0.2°, 19.80 ± 0.2°, 20.54 ± 0.2°, 21.96 ± 0.2°, 23.10 ± 0.2°, and 23.71 ± 0.2°;

and/or, in the crystal form A of the glycolate salt, the compound of formula I and glycolic acid have a molar ratio of 1:1;

and/or, the crystal form A of the glycolate salt has a thermogravimetric analysis pattern with a weight loss when heated from an onset to 150 ± 5°C, preferably, the weight loss is 2% to 4%;

and/or, the crystal form A of the glycolate salt has a differential scanning calorimetry pattern with a major endothermic peak at 110.1 ± 3°C;

and/or, the crystal form A of the L-malate salt has an X-ray powder diffraction pattern expressed by 2θ angles comprising diffraction peaks at: 5.92 ± 0.2°, 7.54 ± 0.2°, 8.32 ± 0.2°, 8.76 ± 0.2°, 11.82 ± 0.2°, 16.56 ± 0.2°, 17.68 ± 0.2°, 18.71 ± 0.2°, 19.69 ± 0.2°, 21.97 ± 0.2°, and 23.75 ± 0.2°; preferably, comprising diffraction peaks at: 5.92 ± 0.2°, 7.54 ± 0.2°, 8.32 ± 0.2°, 8.76 ± 0.2°, 10.35 ± 0.2°, 11.82 ± 0.2°, 12.40 ± 0.2°, 14.10 ± 0.2°, 16.56 ± 0.2°, 17.68 ± 0.2°, 18.71 ± 0.2°, 19.69 ± 0.2°, 20.81 ± 0.2°, 21.97 ± 0.2°, and 23.75 ± 0.2°;

and/or, in the crystal form A of the L-malate salt, the compound of formula I and L-malic acid have a molar ratio of 1:1;

and/or, the crystal form A of the L-malate salt has a thermogravimetric analysis pattern with a weight loss when heated from an onset to 50 ± 5°C, preferably, the weight loss is 2% to 4%; with a weight loss when heated from 50 ± 5°C to 100 ± 5°C, preferably, the weight loss is 1% to 3%; with a weight loss when heated from 100 ± 5°C to 150 ± 5°C, preferably, the weight loss is 0% to 2%;

and/or, the crystal form A of the L-malate salt has a differential scanning calorimetry pattern with major endothermic peaks at 72.9 ± 3°C, 122.2 ± 3°C, 142.1 ± 3°C, and 222.4 ± 3°C and an exothermic peak at 195.9 ± 3°C;

and/or, the crystal form B of the L-malate salt has an X-ray powder diffraction pattern expressed by 2θ angles comprising diffraction peaks at: 6.69 ± 0.2°, 8.99 ± 0.2°, 9.30 ± 0.2°, 10.69 ± 0.2°, 10.97 ± 0.2°, 11.29 ± 0.2°, 12.77 ± 0.2°, 13.02 ± 0.2°, 13.38 ± 0.2°, 14.64 ± 0.2°, 14.89 ± 0.2°, 15.89 ± 0.2°, 17.00 ± 0.2°, 17.55 ± 0.2°, 18.27 ± 0.2°, 18.71 ± 0.2°, 20.70 ± 0.2°, 21.18 ± 0.2°, 21.71 ± 0.2°, and 26.89 ± 0.2°;

and/or, in the crystal form B of the L-malate salt, the compound of formula I and L-malic acid have a molar ratio of 1:1;

and/or, the crystal form B of the L-malate salt has a thermogravimetric analysis pattern with a weight loss when heated from an onset to 150 ± 5°C, preferably, the weight loss is 2% to 4%;

and/or, the crystal form B of the L-malate salt has a differential scanning calorimetry pattern with a major endothermic peak at 134.8 ± 3°C;

and/or, the crystal form A of the hippurate salt has an X-ray powder diffraction pattern expressed by 2θ angles comprising diffraction peaks at: 8.78 ± 0.2°, 9.10 ± 0.2°, 10.41 ± 0.2°, 12.69 ± 0.2°, 12.91 ± 0.2°, 15.00 ± 0.2°, 15.22 ± 0.2°, 16.02 ± 0.2°, 16.57 ± 0.2°, 16.93 ± 0.2°, 17.69 ± 0.2°, 18.00 ± 0.2°, 18.71 ± 0.2°, 20.65 ± 0.2°, 21.27 ± 0.2°, 22.33 ± 0.2°, 24.53 ± 0.2°, and 26.00 ± 0.2°; preferably, comprising diffraction peaks at: 8.78 ± 0.2°, 9.10 ± 0.2°, 10.41 ± 0.2°, 12.69 ± 0.2°, 12.91 ± 0.2°, 15.00 ± 0.2°, 15.22 ± 0.2°, 16.02 ± 0.2°, 16.57 ± 0.2°, 16.93 ± 0.2°, 17.69 ± 0.2°, 18.00 ± 0.2°, 18.71 ± 0.2°, 20.40 ± 0.2°, 20.65 ± 0.2°, 20.89 ± 0.2°, 21.27 ± 0.2°, 22.33 ± 0.2°, 24.53 ± 0.2°, 24.87 ± 0.2°, 25.50 ± 0.2°, 26.00 ± 0.2°, 26.14 ± 0.2°, 27.64 ± 0.2°, 28.43 ± 0.2°, 29.31 ± 0.2°, 29.89 ± 0.2°, 31.11 ± 0.2°, and 32.36 ± 0.2°;

and/or, in the crystal form A of the hippurate salt, the compound of formula I and hippuric acid have a molar ratio of 1:1;

and/or, the crystal form A of the hippurate salt has a thermogravimetric analysis pattern with a weight loss when heated from an onset to 150 ± 5°C; preferably, the weight loss is 2% to 4%;
and/or, the crystal form A of the hippurate salt has a differential scanning calorimetry pattern with major endothermic peaks at 62.4 ± 3°C, 120.1 ± 3°C, and 207.0 ± 3°C;
and/or, in the crystal form A of the succinate salt, the compound of formula I and succinic acid have a molar ratio of 1:1;
and/or, the crystal form A of the succinate salt has a thermogravimetric analysis pattern with a weight loss when heated from an onset to 150 ± 5°C, preferably, the weight loss is 3% to 5%;
and/or, the crystal form A of the succinate salt has a differential scanning calorimetry pattern with major endothermic peaks at 70.0 ± 3°C, 124.1 ± 3°C, and 221.0 ± 3°C;
and/or, the crystal form B of the succinate salt has an X-ray powder diffraction pattern expressed by 2θ angles comprising diffraction peaks at: 5.70 ± 0.2°, 9.24 ± 0.2°, 11.42 ± 0.2°, 13.55 ± 0.2°, 15.10 ± 0.2°, 17.17 ± 0.2°, 18.67 ± 0.2°, 19.13 ± 0.2°, 19.99 ± 0.2°, 20.59 ± 0.2°, 22.95 ± 0.2°, 23.25 ± 0.2°, 24.10 ± 0.2°, 25.13 ± 0.2°, 25.48 ± 0.2°, and 26.12 ± 0.2°; preferably, comprising diffraction peaks at: 5.70 ± 0.2°, 6.75 ± 0.2°, 9.24 ± 0.2°, 11.42 ± 0.2°, 12.31 ± 0.2°, 13.13 ± 0.2°, 13.55 ± 0.2°, 15.10 ± 0.2°, 15.80 ± 0.2°, 16.35 ± 0.2°, 17.17 ± 0.2°, 18.67 ± 0.2°, 19.13 ± 0.2°, 19.50 ± 0.2°, 19.99 ± 0.2°, 20.59 ± 0.2°, 21.60 ± 0.2°, 21.93 ± 0.2°, 22.58 ± 0.2°, 22.95 ± 0.2°, 23.25 ± 0.2°, 24.10 ± 0.2°, 25.13 ± 0.2°, 25.48 ± 0.2°, 25.79 ± 0.2°, 26.12 ± 0.2°, 28.98 ± 0.2°, and 31.50 ± 0.2°;
and/or, in the crystal form B of the succinate salt, the compound of formula I and succinic acid have a molar ratio of 1:1;
and/or, the crystal form B of the succinate salt has a thermogravimetric analysis pattern with a weight loss when heated from an onset to 150 ± 5°C, preferably, the weight loss is 3% to 5%;
and/or, the crystal form B of the succinate salt has a differential scanning calorimetry pattern with major endothermic peaks at 105.1 ± 3°C and 222.3 ± 3°C;
and/or, the crystal form A of the ascorbate salt has an X-ray powder diffraction pattern expressed by 2θ angles comprising diffraction peaks at: 5.72 ± 0.2°, 9.20 ± 0.2°, 11.30 ± 0.2°, 14.38 ± 0.2°, 15.55 ± 0.2°, 16.71 ± 0.2°, 17.58 ± 0.2°, 18.83 ± 0.2°, 20.30 ± 0.2°, 22.33 ± 0.2°, and 25.94 ± 0.2°;
and/or, in the crystal form A of the ascorbate salt, the compound of formula I and ascorbic acid have a molar ratio of 1:0.5;
and/or, the crystal form A of the ascorbate salt has a thermogravimetric analysis pattern with a weight loss when heated from an onset to 150 ± 5°C, preferably, the weight loss is 3% to 5%;
and/or, the crystal form A of the ascorbate salt has a differential scanning calorimetry pattern with a major endothermic peak at 185.7 ± 3°C and one exothermic peak at 195.8 ± 3°C;
and/or, the crystal form B of the ascorbate salt has an X-ray powder diffraction pattern expressed by 2θ angles comprising diffraction peaks at: 4.47 ± 0.2°, 5.50 ± 0.2°, 9.11 ± 0.2°, 11.00 ± 0.2°, 15.27 ± 0.2°, 16.09 ± 0.2°, 16.63 ± 0.2°, 17.47 ± 0.2°, 19.82 ± 0.2°, 25.30 ± 0.2°, 28.08 ± 0.2°, and 30.05 ± 0.2°; preferably, comprising diffraction peaks at: 4.47 ± 0.2°, 5.50 ± 0.2°, 9.11 ± 0.2°, 11.00 ± 0.2°, 13.23 ± 0.2°, 15.27 ± 0.2°, 16.09 ± 0.2°, 16.63 ± 0.2°, 17.47 ± 0.2°, 19.82 ± 0.2°, 21.09 ± 0.2°, 21.53 ± 0.2°, 25.30 ± 0.2°, 25.92 ± 0.2°, 26.81 ± 0.2°, 28.08 ± 0.2°, and 30.05 ± 0.2°;
and/or, in the crystal form B of the ascorbate salt, the compound of formula I and ascorbic acid have a molar ratio of 1:1;
and/or, the crystal form B of the ascorbate salt has a thermogravimetric analysis pattern with a weight loss when heated from an onset to 150 ± 5°C, preferably, the weight loss is 0% to 2%;
and/or, the crystal form B of the ascorbate salt has a differential scanning calorimetry pattern with a major endothermic peak at 153.6 ± 3°C;
and/or, the crystal form A of the adipate salt has an X-ray powder diffraction pattern expressed by 2θ angles comprising diffraction peaks at: 8.47 ± 0.2°, 8.99 ± 0.2°, 11.41 ± 0.2°, 12.40 ± 0.2°, 13.60 ± 0.2°, 15.57 ± 0.2°, 16.29 ± 0.2°, 17.52 ± 0.2°, 18.23 ± 0.2°, 19.84 ± 0.2°, 21.29 ± 0.2°, 23.81 ± 0.2°, 24.62 ± 0.2°, 26.55 ± 0.2°, 27.79 ± 0.2°, and 29.82 ± 0.2°;
and/or, in the crystal form A of the adipate salt, the compound of formula I and adipic acid have a molar ratio of 1:1;
and/or, the crystal form A of the adipate salt has a thermogravimetric analysis pattern with a weight loss when heated from an onset to 150 ± 5°C, preferably, the weight loss is 6% to 8%;
and/or, the crystal form A of the adipate salt has a differential scanning calorimetry pattern with a major endothermic peak at 88.3 ± 3°C;
and/or, the crystal form A of the p-toluenesulfonate salt has an X-ray powder diffraction pattern expressed by 2θ angles comprising diffraction peaks at: 8.91 ± 0.2°, 9.43 ± 0.2°, 12.31 ± 0.2°, 15.93 ± 0.2°, 16.43 ± 0.2°, 17.26 ± 0.2°, 17.91 ± 0.2°, 18.31 ± 0.2°, 20.69 ± 0.2°, 20.95 ± 0.2°, 23.11 ± 0.2°, 23.29 ± 0.2°, 24.76 ± 0.2°, and 26.36 ± 0.2°; preferably, comprising diffraction peaks at: 8.91 ± 0.2°, 9.43 ± 0.2°, 11.51 ± 0.2°, 12.31 ± 0.2°, 13.70 ±

0.2°, 15.93 ± 0.2°, 16.43 ± 0.2°, 17.26 ± 0.2°, 17.91 ± 0.2°, 18.31 ± 0.2°, 19.49 ± 0.2°, 20.69 ± 0.2°, 20.95 ± 0.2°, 21.19 ± 0.2°, 22.77 ± 0.2°, 23.11 ± 0.2°, 23.29 ± 0.2°, 24.34 ± 0.2°, 24.76 ± 0.2°, 25.55 ± 0.2°, 26.36 ± 0.2°, 26.83 ± 0.2°, 27.69 ± 0.2°, and 29.25 ± 0.2°;

and/or, in the crystal form A of the *p*-toluenesulfonate salt, the compound of formula I and *p*-toluenesulfonic acid have a molar ratio of 1:1;

and/or, the crystal form A of the *p*-toluenesulfonate salt has a thermogravimetric analysis pattern with a weight loss when heated from an onset to 100 ± 5°C, preferably, the weight loss is 0% to 2%, with a weight loss of 2% to 4% when heated from 100°C to 170°C;

and/or, the crystal form A of the p-toluenesulfonate salt has a differential scanning calorimetry pattern with a major endothermic peak at 150.9 ± 3°C;

and/or, the crystal form A of the benzenesulfonate salt has an X-ray powder diffraction pattern expressed by 2θ angles comprising diffraction peaks at: 6.54 ± 0.2°, 8.76 ± 0.2°, 10.81 ± 0.2°, 11.38 ± 0.2°, 11.91 ± 0.2°, 13.32 ± 0.2°, 14.01 ± 0.2°, 15.40 ± 0.2°, 16.25 ± 0.2°, 17.41 ± 0.2°, 18.35 ± 0.2°, 19.38 ± 0.2°, 19.90 ± 0.2°, 20.67 ± 0.2°, 21.31 ± 0.2°, 22.89 ± 0.2°, 25.86 ± 0.2°, 27.75 ± 0.2°, and 32.21 ± 0.2°;

and/or, in the crystal form A of the benzenesulfonate salt, the compound of formula I and benzenesulfonic acid have a molar ratio of 1:1;

and/or, the crystal form A of the benzenesulfonate salt has a thermogravimetric analysis pattern with a weight loss when heated from an onset to 80 ± 5°C, preferably, the weight loss is 2% to 4%; with a weight loss when heated from 80°C to 150°C, preferably, the weight loss is 5% to 7%;

and/or, the crystal form A of the benzenesulfonate salt has a differential scanning calorimetry pattern with major endothermic peaks at 111.7 ± 3°C and 122.6 ± 3°C;

and/or, the crystal form A of the oxalate salt has an X-ray powder diffraction pattern expressed by 2θ angles comprising diffraction peaks at: 6.15 ± 0.2°, 7.02 ± 0.2°, 10.44 ± 0.2°, 13.94 ± 0.2°, 16.74 ± 0.2°, 17.49 ± 0.2°, 20.07 ± 0.2°, 24.50 ± 0.2°, 25.79 ± 0.2°, and 26.53 ± 0.2°; preferably, comprising diffraction peaks at: 6.15 ± 0.2°, 7.02 ± 0.2°, 9.18 ± 0.2°, 10.44 ± 0.2°, 13.94 ± 0.2°, 15.12 ± 0.2°, 15.54 ± 0.2°, 16.74 ± 0.2°, 17.49 ± 0.2°, 18.16 ± 0.2°, 19.51 ± 0.2°, 20.07 ± 0.2°, 22.64 ± 0.2°, 23.59 ± 0.2°, 24.50 ± 0.2°, 25.22 ± 0.2°, 25.79 ± 0.2°, 26.53 ± 0.2°, 27.90 ± 0.2°, and 28.47 ± 0.2°;

and/or, in the crystal form A of the oxalate salt, the compound of formula I and oxalic acid have a molar ratio of 1:1;

and/or, the crystal form A of the oxalate salt has a thermogravimetric analysis pattern with a weight loss when heated from an onset to 100 ± 5°C; preferably, the weight loss is 2% to 7%;

and/or, the crystal form A of the oxalate salt has a differential scanning calorimetry pattern with major endothermic peaks at 103.4 ± 3°C and 141.6 ± 3°C;

and/or, the crystal form A of the 2-hydroxyethanesulfonate salt has an X-ray powder diffraction pattern expressed by 2θ angles comprising diffraction peaks at: 6.46 ± 0.2°, 9.89 ± 0.2°, 10.95 ± 0.2°, 13.14 ± 0.2°, 13.60 ± 0.2°, 15.69 ± 0.2°, 18.38 ± 0.2°, 18.81 ± 0.2°, 20.44 ± 0.2°, and 21.70 ± 0.2°;

and/or, in the crystal form A of the 2-hydroxyethanesulfonate salt, the compound of formula I and 2-hydroxyethanesulfonic acid have a molar ratio of 1:1;

and/or, the crystal form A of the 2-hydroxyethanesulfonate salt has a thermogravimetric analysis pattern with a weight loss when heated from an onset to 150 ± 5°C, preferably, the weight loss is 0% to 3%;

and/or, the crystal form A of the 2-hydroxyethanesulfonate salt has a differential scanning calorimetry pattern with a major endothermic peak at 203.5 ± 3°C.

**3.** The crystal form according to claim 1, wherein the crystal form A of the compound of formula I has an X-ray powder diffraction pattern expressed by 2θ angles comprising diffraction peaks at: 6.02 ± 0.2°, 7.78 ± 0.2°, 10.30 ± 0.2°, 11.42 ± 0.2°, 12.06 ± 0.2°, 12.42 ± 0.2°, 14.68 ± 0.2°, 15.10 ± 0.2°, 16.61 ± 0.2°, 17.78 ± 0.2°, 18.13 ± 0.2°, 18.48 ± 0.2°, 19.12 ± 0.2°, 19.98 ± 0.2°, 20.25 ± 0.2°, 21.76 ± 0.2°, 22.09 ± 0.2°, and 24.75 ± 0.2°;

and/or, the crystal form A of the compound of formula I has a thermogravimetric analysis pattern with a weight loss of 3.24% when heated from an onset to 120 ± 5°C;

and/or, the crystal form A of the compound of formula I is a hydrate of the compound of formula I, wherein the compound of formula I and water have a molar ratio of 1:1, 1:1.1, 1:1.2, 1:1.3, 1:1.4, or 1:1.5; most preferably 1:1 or 1:1.5;

and/or, the crystal form B of the compound of formula I has an X-ray powder diffraction pattern expressed by 2θ angles comprising diffraction peaks at: 10.08 ± 0.2°, 10.30 ± 0.2°, 11.58 ± 0.2°, 12.30 ± 0.2°, 12.89 ± 0.2°, 14.49 ± 0.2°, 15.32 ± 0.2°, 16.39 ± 0.2°, 16.89 ± 0.2°, 17.45 ± 0.2°, 18.10 ± 0.2°, 18.21 ± 0.2°, 18.70 ± 0.2°, 19.84 ± 0.2°, 20.33 ± 0.2°, 21.05 ± 0.2°, 21.66 ± 0.2°, 22.01 ± 0.2°, 22.52 ± 0.2°, 23.23 ± 0.2°, 24.35 ± 0.2°, and 24.69 ± 0.2°;

and/or, the crystal form A of the fumarate salt has an X-ray powder diffraction pattern expressed by 2θ angles

comprising diffraction peaks at: 6.86 ± 0.2°, 7.72 ± 0.2°, 9.57 ± 0.2°, 12.90 ± 0.2°, 13.17 ± 0.2°, 13.72 ± 0.2°, 15.45 ± 0.2°, 15.87 ± 0.2°, 16.47 ± 0.2°, 17.13 ± 0.2°, 17.47 ± 0.2°, 18.19 ± 0.2°, 18.72 ± 0.2°, 19.20 ± 0.2°, 19.63 ± 0.2°, 19.97 ± 0.2°, 20.89 ± 0.2°, 22.47 ± 0.2°, 23.26 ± 0.2°, 23.95 ± 0.2°, 25.93 ± 0.2°, 26.28 ± 0.2°, 27.23 ± 0.2°, 29.54 ± 0.2°, 29.82 ± 0.2°, and 31.16 ± 0.2°;

and/or, the crystal form A of the fumarate salt has a thermogravimetric analysis pattern with a weight loss of 3.35% when heated from an onset to 150 ± 5°C;

and/or, the crystal form A of the fumarate salt is a hydrate of the fumarate salt of the compound of formula I, wherein the compound of formula I and water have a molar ratio of 1:1, 1:1.1, 1:1.2, 1:1.3, 1:1.4, or 1:1.5; most preferably 1:1 or 1:1.5;

and/or, the crystal form B of the fumarate salt has an X-ray powder diffraction pattern expressed by 2θ angles comprising diffraction peaks at: 4.78 ± 0.2°, 5.98 ± 0.2°, 8.02 ± 0.2°, 9.68 ± 0.2°, 11.55 ± 0.2°, 11.93 ± 0.2°, 12.44 ± 0.2°, 12.78 ± 0.2°, 14.01 ± 0.2°, 15.24 ± 0.2°, 15.38 ± 0.2°, 15.80 ± 0.2°, 16.57 ± 0.2°, 17.36 ± 0.2°, 17.93 ± 0.2°, 18.57 ± 0.2°, 19.41 ± 0.2°, 19.95 ± 0.2°, 21.10 ± 0.2°, 22.15 ± 0.2°, 22.53 ± 0.2°, 22.89 ± 0.2°, 23.97 ± 0.2°, 24.76 ± 0.2°, 25.18 ± 0.2°, 25.74 ± 0.2°, 26.56 ± 0.2°, 28.81 ± 0.2°, and 29.48 ± 0.2°;

and/or, the crystal form B of the fumarate salt has a thermogravimetric analysis pattern with a weight loss of 5.64% when heated from an onset to 150 ± 5°C and a weight loss of 6.76% when heated from 150 ± 5°C to 250 ± 5°C;

and/or, the crystal form C of the fumarate salt has a thermogravimetric analysis pattern with a weight loss of 1.69% when heated from an onset to 150 ± 5°C and a weight loss of 6.21% when heated from 150 ± 5°C to 250 ± 5°C;

and/or, the crystal form A of the citrate salt has a thermogravimetric analysis pattern with a weight loss of 5.72% when heated from an onset to 150 ± 5°C and a weight loss of 19.25% when heated from 150 ± 5°C to 230 ± 5°C;

and/or, the crystal form B of the citrate salt has an X-ray powder diffraction pattern expressed by 2θ angles comprising diffraction peaks at one or more of the following 2θ angles: 9.90 ± 0.2°, 11.93 ± 0.2°, 12.79 ± 0.2°, 15.45 ± 0.2°, 16.45 ± 0.2°, 16.72 ± 0.2°, 17.60 ± 0.2°, 18.25 ± 0.2°, 19.34 ± 0.2°, 19.86 ± 0.2°, 20.88 ± 0.2°, 21.18 ± 0.2°, 23.22 ± 0.2°, 23.55 ± 0.2°, 24.35 ± 0.2°, 24.68 ± 0.2°, 25.19 ± 0.2°, 27.72 ± 0.2°, 28.47 ± 0.2°, 29.97 ± 0.2°, 30.65 ± 0.2°, and 31.19; preferably, comprising diffraction peaks at one or more of the following 2θ angles: 9.90 ± 0.2°, 10.38 ± 0.2°, 11.78 ± 0.2°, 11.93 ± 0.2°, 12.79 ± 0.2°, 15.19 ± 0.2°, 15.45 ± 0.2°, 16.45 ± 0.2°, 16.72 ± 0.2°, 17.60 ± 0.2°, 18.25 ± 0.2°, 19.34 ± 0.2°, 19.86 ± 0.2°, 20.88 ± 0.2°, 21.18 ± 0.2°, 23.22 ± 0.2°, 23.55 ± 0.2°, 24.35 ± 0.2°, 24.68 ± 0.2°, 25.19 ± 0.2°, 25.75 ± 0.2°, 27.72 ± 0.2°, 28.47 ± 0.2°, 29.97 ± 0.2°, 30.65 ± 0.2°, and 31.19;

and/or, the crystal form B of the citrate salt has a thermogravimetric analysis pattern with a weight loss of 1.26% when heated from an onset to 150 ± 5°C;

and/or, the crystal form A of the methanesulfonate salt has an X-ray powder diffraction pattern expressed by 2θ angles comprising diffraction peaks at: 7.28 ± 0.2°, 9.84 ± 0.2°, 10.86 ± 0.2°, 12.71 ± 0.2°, 14.20 ± 0.2°, 14.58 ± 0.2°, 16.67 ± 0.2°, 18.70 ± 0.2°, 18.92 ± 0.2°, 19.96 ± 0.2°, 20.38 ± 0.2°, 21.31 ± 0.2°, 21.57 ± 0.2°, 21.93 ± 0.2°, 22.86 ± 0.2°, 23.39 ± 0.2°, 23.77 ± 0.2°, 24.50 ± 0.2°, 24.84 ± 0.2°, and 25.59 ± 0.2°;

and/or, the crystal form A of the methanesulfonate salt has a thermogravimetric analysis pattern with a weight loss of 2.14% when heated from an onset to 150 ± 5°C;

and/or, the crystal form A of the ethanesulfonate salt has a thermogravimetric analysis pattern with a weight loss of 0.87% when heated from an onset to 150 ± 5°C;

and/or, the crystal form B of the ethanesulfonate salt has a thermogravimetric analysis pattern with a weight loss of 2.17% when heated from an onset to 150 ± 5°C;

and/or, the crystal form A of the maleate salt has a thermogravimetric analysis pattern with a weight loss of 3.14% when heated from an onset to 100 ± 5°C and a weight loss of 14.49% when heated from 100 ± 5°C to 225 ± 5°C;

and/or, the crystal form B of the maleate salt has a thermogravimetric analysis pattern with a weight loss of 2.84% when heated from an onset to 100 ± 5°C and a weight loss of 12.43% when heated from 100 ± 5°C to 225 ± 5°C;

and/or, the crystal form A of the L-tartrate salt has a thermogravimetric analysis pattern with a weight loss of 3.18% when heated from an onset to 150 ± 5°C;

and/or, the crystal form B of the L-tartrate salt has an X-ray powder diffraction pattern expressed by 2θ angles comprising diffraction peaks at: 4.22 ± 0.2°, 5.17 ± 0.2°, 5.77 ± 0.2°, 8.65 ± 0.2°, 10.09 ± 0.2°, 11.51 ± 0.2°, 12.58 ± 0.2°, 14.08 ± 0.2°, 14.91 ± 0.2°, 15.59 ± 0.2°, 16.41 ± 0.2°, 16.59 ± 0.2°, 17.29 ± 0.2°, 18.20 ± 0.2°, 18.64 ± 0.2°, 19.75 ± 0.2°, 20.22 ± 0.2°, 20.74 ± 0.2°, 21.95 ± 0.2°, 23.11 ± 0.2°, 24.84 ± 0.2°, 25.27 ± 0.2°, and 25.56 ± 0.2°;

and/or, the crystal form B of the L-tartrate salt has a thermogravimetric analysis pattern with a weight loss of 5.57% when heated from an onset to 180 ± 5°C;

and/or, the crystal form C of the L-tartrate salt has a thermogravimetric analysis pattern with a weight loss of 3.16% when heated from an onset to 150 ± 5°C;

and/or, the crystal form D of the L-tartrate salt has a thermogravimetric analysis pattern with a weight loss of 2.72% when heated from an onset to 150 ± 5°C;

and/or, the crystal form A of the glycolate salt has a thermogravimetric analysis pattern with a weight loss of 3.40% when heated from an onset to 150 ± 5°C;
and/or, the crystal form A of the L-malate salt has a thermogravimetric analysis pattern with a weight loss of 2.88% when heated from an onset to 50 ± 5°C, a weight loss of 2.46% when heated from 50 ± 5°C to 100 ± 5°C, and a weight loss of 1.31% when heated from 100 ± 5°C to 150 ± 5°C;
and/or, the crystal form B of the L-malate salt has a thermogravimetric analysis pattern with a weight loss of 3.40% when heated from an onset to 150 ± 5°C;
and/or, the crystal form A of the hippurate salt has a thermogravimetric analysis pattern with a weight loss of 3.04% when heated from an onset to 150 ± 5°C;
and/or, the crystal form A of the succinate salt has a thermogravimetric analysis pattern with a weight loss of 3.94% when heated from an onset to 150 ± 5°C;
and/or, the crystal form B of the succinate salt has a thermogravimetric analysis pattern with a weight loss of 4.44% when heated from an onset to 150 ± 5°C;
and/or, the crystal form A of the ascorbate salt has a thermogravimetric analysis pattern with a weight loss of 4.36% when heated from an onset to 150 ± 5°C;
and/or, the crystal form B of the ascorbate salt has a thermogravimetric analysis pattern with a weight loss of 1.33% when heated from an onset to 150 ± 5°C;
and/or, the crystal form A of the adipate salt has a thermogravimetric analysis pattern with a weight loss of 6.60% when heated from an onset to 150 ± 5°C;
and/or, the crystal form A of the *p*-toluenesulfonate salt has a thermogravimetric analysis pattern with a weight loss of 0.76% when heated from an onset to 100 ± 5°C and a weight loss of 3.07% when heated from 100°C to 170°C;
and/or, the crystal form A of the benzenesulfonate salt has a thermogravimetric analysis pattern with a weight loss of 2.55% when heated from an onset to 80 ± 5°C and a weight loss of 6.23% when heated from 80°C to 150°C;
and/or, the crystal form A of the oxalate salt has a thermogravimetric analysis pattern with a weight loss of 5.88% when heated from an onset to 100 ± 5°C;
and/or, the crystal form A of the 2-hydroxyethanesulfonate salt has an X-ray powder diffraction pattern expressed by 2θ angles comprising diffraction peaks at: 6.46 ± 0.2°, 9.89 ± 0.2°, 10.95 ± 0.2°, 13.14 ± 0.2°, 13.60 ± 0.2°, 15.69 ± 0.2°, 18.38 ± 0.2°, 18.81 ± 0.2°, 20.44 ± 0.2°, 21.70 ± 0.2°, 23.25 ± 0.2°, 24.04 ± 0.2°, 24.82 ± 0.2°, 26.03 ± 0.2°, 26.35 ± 0.2°, and 26.70 ± 0.2°;
and/or, the crystal form A of the 2-hydroxyethanesulfonate salt has a thermogravimetric analysis pattern with a weight loss of 1.61% when heated from an onset to 150 ± 5°C.

**4.** The crystal form according to claim 1, wherein the crystal form A of the compound of formula I has an X-ray powder diffraction pattern expressed by 2θ angles with diffraction peaks and relative intensities as shown in Table 8;

Table 8

| Diffraction peak number | 2θ angle (°) | Relative intensity (%) |
|---|---|---|
| 1 | 6.0228 | 3.59 |
| 2 | 7.7830 | 3.30 |
| 3 | 10.3092 | 11.15 |
| 4 | 11.4209 | 6.30 |
| 5 | 12.0638 | 100.00 |
| 6 | 12.4274 | 23.84 |
| 7 | 14.6867 | 28.86 |
| 8 | 15.1087 | 11.55 |
| 9 | 16.6189 | 9.65 |
| 10 | 17.7871 | 17.72 |
| 11 | 18.1363 | 28.30 |
| 12 | 18.4865 | 27.27 |
| 13 | 19.1245 | 26.11 |
| 14 | 19.9890 | 17.55 |

(continued)

| Diffraction peak number | 2θ angle (°) | Relative intensity (%) |
|---|---|---|
| 15 | 20.2538 | 26.72 |
| 16 | 21.7638 | 17.22 |
| 17 | 22.0984 | 25.18 |
| 18 | 23.4584 | 2.72 |
| 19 | 24.7554 | 13.48 |
| 20 | 26.1969 | 4.94 |
| 21 | 29.0571 | 1.31 |
| 22 | 30.4824 | 1.85 |
| 23 | 31.0426 | 1.54 |
| 24 | 32.4179 | 1.62 |
| 25 | 35.9264 | 1.20 |

and/or, the crystal form B of the compound of formula I has an X-ray powder diffraction pattern expressed by 2θ angles with diffraction peaks and relative intensities as shown in Table 10;

Table 10

| Diffraction peak number | 2θ angle (°) | Relative intensity (%) |
|---|---|---|
| 1 | 8.2865 | 2.76 |
| 2 | 9.2885 | 2.12 |
| 3 | 10.0872 | 6.27 |
| 4 | 10.3092 | 10.73 |
| 5 | 11.5894 | 7.31 |
| 6 | 12.3098 | 30.33 |
| 7 | 12.8969 | 100.00 |
| 8 | 14.4978 | 66.58 |
| 9 | 15.3275 | 14.46 |
| 10 | 16.3902 | 9.76 |
| 11 | 16.8991 | 19.27 |
| 12 | 17.4565 | 24.35 |
| 13 | 18.1094 | 94.08 |
| 14 | 18.2100 | 91.83 |
| 15 | 18.7070 | 58.39 |
| 16 | 19.8490 | 12.99 |
| 17 | 20.3335 | 65.71 |
| 18 | 21.0502 | 10.48 |
| 19 | 21.6664 | 30.40 |
| 20 | 22.0197 | 13.74 |
| 21 | 22.5234 | 14.20 |
| 22 | 23.2319 | 17.04 |
| 23 | 24.3595 | 23.56 |

(continued)

| Diffraction peak number | 2θ angle (°) | Relative intensity (%) |
|---|---|---|
| 24 | 24.6980 | 15.70 |
| 25 | 25.4100 | 7.92 |
| 26 | 25.7299 | 5.40 |
| 27 | 25.9974 | 4.74 |
| 28 | 26.6367 | 6.53 |
| 29 | 27.3167 | 6.58 |
| 30 | 28.6459 | 6.47 |
| 31 | 29.1507 | 2.52 |
| 32 | 30.7774 | 6.28 |
| 33 | 31.6249 | 2.31 |
| 34 | 32.8463 | 1.84 |
| 35 | 34.6151 | 1.98 |
| 36 | 35.8761 | 2.23 |
| 37 | 37.5599 | 1.89 |

and/or, the crystal form A of the fumarate salt has an X-ray powder diffraction pattern expressed by 2θ angles with diffraction peaks and relative intensities as shown in Table 11;

Table 11

| Diffraction peak number | 2θ angle (°) | Relative intensity (%) |
|---|---|---|
| 1 | 6.8635 | 16.34 |
| 2 | 7.7252 | 48.44 |
| 3 | 9.5770 | 15.24 |
| 4 | 11.9972 | 1.28 |
| 5 | 12.9002 | 5.22 |
| 6 | 13.1749 | 3.47 |
| 7 | 13.7294 | 12.06 |
| 8 | 14.7803 | 2.02 |
| 9 | 15.4533 | 5.22 |
| 10 | 15.8774 | 6.84 |
| 11 | 16.4762 | 36.15 |
| 12 | 16.7209 | 2.77 |
| 13 | 17.1307 | 11.61 |
| 14 | 17.4731 | 6.35 |
| 15 | 18.1952 | 10.02 |
| 16 | 18.7222 | 9.03 |
| 17 | 19.2097 | 18.81 |
| 18 | 19.6315 | 20.13 |
| 19 | 19.9729 | 5.41 |
| 20 | 20.6384 | 1.38 |

(continued)

| Diffraction peak number | 2θ angle (°) | Relative intensity (%) |
|---|---|---|
| 21 | 20.8956 | 3.60 |
| 22 | 22.4788 | 87.04 |
| 23 | 22.7668 | 13.84 |
| 24 | 23.2638 | 100.00 |
| 25 | 23.5487 | 6.31 |
| 26 | 23.9591 | 18.73 |
| 27 | 25.5844 | 2.38 |
| 28 | 25.9303 | 14.48 |
| 29 | 26.2872 | 5.24 |
| 30 | 26.5499 | 1.63 |
| 31 | 27.2374 | 3.53 |
| 32 | 27.9091 | 1.38 |
| 33 | 28.9544 | 1.68 |
| 34 | 29.5480 | 4.81 |
| 35 | 29.8239 | 6.24 |
| 36 | 30.5191 | 1.77 |
| 37 | 31.1617 | 15.39 |
| 38 | 32.6027 | 1.68 |
| 39 | 33.6475 | 1.74 |
| 40 | 34.9521 | 1.90 |
| 41 | 35.7055 | 3.05 |
| 42 | 37.0550 | 2.07 |
| 43 | 39.2410 | 1.65 |

and/or, the crystal form B of the fumarate salt has an X-ray powder diffraction pattern expressed by 2θ angles with diffraction peaks and relative intensities as shown in Table 12;

Table 12

| Diffraction peak number | 2θ angle (°) | Relative intensity (%) |
|---|---|---|
| 1 | 3.1306 | 30.47 |
| 2 | 4.7842 | 88.15 |
| 3 | 5.9872 | 23.25 |
| 4 | 8.0240 | 35.07 |
| 5 | 9.6881 | 59.76 |
| 6 | 11.5567 | 18.92 |
| 7 | 11.9324 | 40.62 |
| 8 | 12.4487 | 19.30 |
| 9 | 12.7894 | 19.80 |
| 10 | 14.0148 | 14.98 |
| 11 | 15.2481 | 23.64 |

(continued)

| Diffraction peak number | 2θ angle (°) | Relative intensity (%) |
|---|---|---|
| 12 | 15.3870 | 25.91 |
| 13 | 15.8013 | 17.28 |
| 14 | 16.5798 | 49.63 |
| 15 | 17.3697 | 19.13 |
| 16 | 17.9353 | 35.64 |
| 17 | 18.5729 | 39.25 |
| 18 | 19.4112 | 21.04 |
| 19 | 19.9508 | 16.56 |
| 20 | 21.1098 | 24.00 |
| 21 | 22.1541 | 16.63 |
| 22 | 22.5310 | 20.66 |
| 23 | 22.8977 | 18.36 |
| 24 | 23.9784 | 40.14 |
| 25 | 24.7603 | 18.73 |
| 26 | 25.1801 | 12.42 |
| 27 | 25.7408 | 8.11 |
| 28 | 26.5670 | 9.32 |
| 29 | 27.0251 | 4.34 |
| 30 | 28.8193 | 100.00 |
| 31 | 29.4817 | 22.24 |
| 32 | 33.3756 | 2.57 |

and/or, the crystal form C of the fumarate salt has an X-ray powder diffraction pattern expressed by 2θ angles with diffraction peaks and relative intensities as shown in Table 13;

Table 13

| Diffraction peak number | 2θ angle (°) | Relative intensity (%) |
|---|---|---|
| 1 | 6.8589 | 65.48 |
| 2 | 8.7271 | 75.22 |
| 3 | 9.9037 | 12.52 |
| 4 | 12.1726 | 13.47 |
| 5 | 13.1356 | 42.02 |
| 6 | 14.0951 | 100.00 |
| 7 | 14.2993 | 91.55 |
| 8 | 16.3367 | 18.89 |
| 9 | 17.2742 | 74.17 |
| 10 | 17.9016 | 47.93 |
| 11 | 20.6039 | 39.40 |
| 12 | 21.4276 | 39.71 |
| 13 | 23.1627 | 45.22 |

(continued)

| Diffraction peak number | 2θ angle (°) | Relative intensity (%) |
|---|---|---|
| 14 | 24.0403 | 36.91 |
| 15 | 25.4177 | 11.45 |
| 16 | 29.8822 | 8.71 |

and/or, the crystal form A of the citrate salt has an X-ray powder diffraction pattern expressed by 2θ angles with diffraction peaks and relative intensities as shown in Table 14;

Table 14

| Diffraction peak number | 2θ angle (°) | Relative intensity (%) |
|---|---|---|
| 1 | 6.2756 | 100.00 |
| 2 | 10.7946 | 4.33 |
| 3 | 12.2105 | 7.49 |
| 4 | 12.5828 | 22.11 |
| 5 | 16.3816 | 10.38 |
| 6 | 18.3385 | 2.67 |
| 7 | 25.3323 | 20.65 |
| 8 | 29.0006 | 0.90 |

and/or, the crystal form B of the citrate salt has an X-ray powder diffraction pattern expressed by 2θ angles with diffraction peaks and relative intensities as shown in Table 15;

Table 15

| Diffraction peak number | 2θ angle (°) | Relative intensity (%) |
|---|---|---|
| 1 | 8.1218 | 1.02 |
| 2 | 9.9016 | 14.74 |
| 3 | 10.0614 | 8.51 |
| 4 | 10.3896 | 3.73 |
| 5 | 11.7789 | 14.10 |
| 6 | 11.9292 | 17.11 |
| 7 | 12.7906 | 11.73 |
| 8 | 15.1889 | 6.82 |
| 9 | 15.4521 | 27.86 |
| 10 | 16.4518 | 33.98 |
| 11 | 16.7237 | 12.65 |
| 12 | 17.6030 | 32.39 |
| 13 | 18.2515 | 8.85 |
| 14 | 19.3428 | 7.74 |
| 15 | 19.8659 | 100.00 |
| 16 | 20.0922 | 9.69 |
| 17 | 20.8796 | 28.12 |
| 18 | 21.1802 | 51.78 |

(continued)

| Diffraction peak number | 2θ angle (°) | Relative intensity (%) |
|---|---|---|
| 19 | 21.6692 | 2.76 |
| 20 | 22.3401 | 0.84 |
| 21 | 23.2208 | 6.47 |
| 22 | 23.5512 | 17.52 |
| 23 | 23.6983 | 13.72 |
| 24 | 23.9640 | 3.65 |
| 25 | 24.3547 | 9.13 |
| 26 | 24.6846 | 7.83 |
| 27 | 25.1969 | 30.57 |
| 28 | 25.7570 | 5.66 |
| 29 | 26.0958 | 2.76 |
| 30 | 26.5982 | 2.22 |
| 31 | 27.0313 | 1.92 |
| 32 | 27.7238 | 8.24 |
| 33 | 28.4748 | 9.85 |
| 34 | 29.9717 | 5.53 |
| 35 | 30.6555 | 10.80 |
| 36 | 31.1973 | 5.24 |
| 37 | 33.1554 | 2.03 |
| 38 | 33.8397 | 1.46 |
| 39 | 34.8016 | 2.27 |
| 40 | 35.7488 | 1.00 |
| 41 | 36.3921 | 1.20 |
| 42 | 37.5999 | 0.63 |
| 43 | 38.3018 | 1.16 |

and/or, the crystal form A of the methanesulfonate salt has an X-ray powder diffraction pattern expressed by 2θ angles with diffraction peaks and relative intensities as shown in Table 16;

Table 16

| Diffraction peak number | 2θ angle (°) | Relative intensity (%) |
|---|---|---|
| 1 | 7.2862 | 89.34 |
| 2 | 9.8423 | 7.40 |
| 3 | 10.8616 | 56.83 |
| 4 | 12.7185 | 67.06 |
| 5 | 14.2081 | 47.55 |
| 6 | 14.5823 | 45.24 |
| 7 | 16.6720 | 57.90 |
| 8 | 18.7082 | 58.29 |
| 9 | 18.9279 | 40.54 |

(continued)

| Diffraction peak number | 2θ angle (°) | Relative intensity (%) |
|---|---|---|
| 10 | 19.9608 | 100.00 |
| 11 | 20.3837 | 14.37 |
| 12 | 21.3164 | 25.10 |
| 13 | 21.5738 | 51.13 |
| 14 | 21.9370 | 81.96 |
| 15 | 22.8600 | 10.38 |
| 16 | 23.3925 | 18.64 |
| 17 | 23.7735 | 15.02 |
| 18 | 24.5069 | 28.44 |
| 19 | 24.8441 | 13.03 |
| 20 | 25.5949 | 15.81 |
| 21 | 26.2468 | 5.61 |
| 22 | 27.3285 | 4.14 |
| 23 | 28.2624 | 5.37 |
| 24 | 29.7468 | 6.93 |
| 25 | 30.8227 | 9.05 |
| 26 | 31.9822 | 3.70 |
| 27 | 34.1619 | 5.00 |
| 28 | 35.6490 | 2.56 |
| 29 | 38.2044 | 4.63 |

and/or, the crystal form A of the ethanesulfonate salt has an X-ray powder diffraction pattern expressed by 2θ angles with diffraction peaks and relative intensities as shown in Table 17;

Table 17

| Diffraction peak number | 2θ angle (°) | Relative intensity (%) |
|---|---|---|
| 1 | 7.1331 | 100.00 |
| 2 | 7.7512 | 2.43 |
| 3 | 9.6314 | 6.73 |
| 4 | 10.8074 | 16.62 |
| 5 | 12.6099 | 15.69 |
| 6 | 13.4343 | 3.51 |
| 7 | 14.0370 | 28.74 |
| 8 | 14.2676 | 55.92 |
| 9 | 16.4327 | 39.29 |
| 10 | 16.8493 | 3.36 |
| 11 | 18.2789 | 23.50 |
| 12 | 18.7338 | 9.33 |
| 13 | 18.9545 | 5.06 |
| 14 | 19.7019 | 7.62 |

(continued)

| Diffraction peak number | 2θ angle (°) | Relative intensity (%) |
|---|---|---|
| 15 | 19.9629 | 30.11 |
| 16 | 20.9824 | 7.83 |
| 17 | 21.4515 | 83.20 |
| 18 | 22.4679 | 7.89 |
| 19 | 22.9554 | 10.07 |
| 20 | 23.2675 | 31.01 |
| 21 | 24.4085 | 11.46 |
| 22 | 24.6912 | 9.87 |
| 23 | 25.3570 | 7.70 |
| 24 | 26.3787 | 3.47 |
| 25 | 26.6481 | 5.75 |
| 26 | 27.9500 | 1.93 |
| 27 | 29.2757 | 2.78 |
| 28 | 30.0459 | 2.37 |
| 29 | 31.3811 | 3.35 |
| 30 | 32.5227 | 1.46 |
| 31 | 33.4655 | 4.69 |
| 32 | 34.1098 | 1.06 |
| 33 | 34.6685 | 2.96 |
| 34 | 35.0228 | 3.01 |
| 35 | 37.2615 | 6.38 |
| 36 | 38.2123 | 1.67 |
| 37 | 39.2030 | 0.70 |

and/or, the crystal form B of the ethanesulfonate salt has an X-ray powder diffraction pattern expressed by 2θ angles with diffraction peaks and relative intensities as shown in Table 18;

Table 18

| Diffraction peak number | 2θ angle (°) | Relative intensity (%) |
|---|---|---|
| 1 | 3.1872 | 80.75 |
| 2 | 5.9961 | 37.13 |
| 3 | 6.9234 | 80.06 |
| 4 | 8.7147 | 23.19 |
| 5 | 9.8666 | 100.00 |
| 6 | 12.7362 | 13.27 |
| 7 | 15.0169 | 10.98 |
| 8 | 16.8410 | 10.47 |
| 9 | 19.2082 | 24.35 |
| 10 | 20.1052 | 60.59 |
| 11 | 21.3893 | 13.13 |

(continued)

| Diffraction peak number | 2θ angle (°) | Relative intensity (%) |
|---|---|---|
| 12 | 23.3054 | 3.54 |
| 13 | 25.2706 | 16.94 |
| 14 | 29.8583 | 6.01 |

and/or, the crystal form A of the maleate salt has an X-ray powder diffraction pattern expressed by 2θ angles with diffraction peaks and relative intensities as shown in Table 19;

Table 19

| Diffraction peak number | 2θ angle (°) | Relative intensity (%) |
|---|---|---|
| 1 | 4.4667 | 54.95 |
| 2 | 5.6155 | 59.30 |
| 3 | 9.0632 | 3.30 |
| 4 | 11.2334 | 100.00 |
| 5 | 11.8651 | 7.21 |
| 6 | 15.3855 | 12.47 |
| 7 | 16.8641 | 28.25 |
| 8 | 18.3097 | 19.29 |
| 9 | 19.2073 | 24.01 |
| 10 | 19.9183 | 5.92 |
| 11 | 20.4217 | 5.60 |
| 12 | 23.0040 | 9.90 |
| 13 | 24.2613 | 7.02 |
| 14 | 25.9499 | 2.87 |
| 15 | 31.3936 | 2.21 |
| 16 | 35.0184 | 0.72 |

and/or, the crystal form B of the maleate salt has an X-ray powder diffraction pattern expressed by 2θ angles with diffraction peaks and relative intensities as shown in Table 20;

Table 20

| Diffraction peak number | 2θ angle (°) | Relative intensity (%) |
|---|---|---|
| 1 | 3.1784 | 74.33 |
| 2 | 4.9956 | 100.00 |
| 3 | 6.9966 | 18.03 |
| 4 | 10.4659 | 27.05 |
| 5 | 11.2179 | 47.74 |
| 6 | 15.0246 | 6.39 |
| 7 | 15.6048 | 4.21 |
| 8 | 16.8234 | 14.78 |
| 9 | 18.3075 | 14.91 |
| 10 | 18.9268 | 14.55 |

(continued)

| Diffraction peak number | 2θ angle (°) | Relative intensity (%) |
|---|---|---|
| 11 | 19.5029 | 12.94 |

and/or, the crystal form A of the L-tartrate salt has an X-ray powder diffraction pattern expressed by 2θ angles with diffraction peaks and relative intensities as shown in Table 21;

Table 21

| Diffraction peak number | 2θ angle (°) | Relative intensity (%) |
|---|---|---|
| 1 | 9.2807 | 51.00 |
| 2 | 11.3838 | 27.35 |
| 3 | 12.9746 | 5.62 |
| 4 | 15.6270 | 53.82 |
| 5 | 16.9513 | 6.71 |
| 6 | 18.0771 | 13.13 |
| 7 | 19.5410 | 100.00 |
| 8 | 19.8984 | 93.04 |
| 9 | 22.9497 | 7.07 |
| 10 | 23.5183 | 14.41 |
| 11 | 25.2139 | 32.14 |
| 12 | 26.9208 | 12.77 |
| 13 | 28.4640 | 14.98 |
| 14 | 32.3397 | 5.56 |
| 15 | 34.2393 | 0.99 |

and/or, the crystal form B of the L-tartrate salt has an X-ray powder diffraction pattern expressed by 2θ angles with diffraction peaks and relative intensities as shown in Table 22;

Table 22

| Diffraction peak number | 2θ angle (°) | Relative intensity (%) |
|---|---|---|
| 1 | 4.2215 | 38.68 |
| 2 | 5.1706 | 40.36 |
| 3 | 5.7735 | 100.00 |
| 4 | 7.4112 | 14.46 |
| 5 | 8.6523 | 32.84 |
| 6 | 10.0921 | 19.37 |
| 7 | 11.5196 | 76.58 |
| 8 | 12.5832 | 11.88 |
| 9 | 14.0808 | 22.24 |
| 10 | 14.9152 | 11.32 |
| 11 | 15.2866 | 7.81 |
| 12 | 15.5985 | 12.72 |
| 13 | 16.4184 | 32.78 |

(continued)

| Diffraction peak number | 2θ angle (°) | Relative intensity (%) |
|---|---|---|
| 14 | 16.5916 | 35.33 |
| 15 | 17.2969 | 67.11 |
| 16 | 18.2001 | 18.09 |
| 17 | 18.6485 | 27.40 |
| 18 | 19.7513 | 19.59 |
| 19 | 20.2201 | 22.35 |
| 20 | 20.7442 | 16.32 |
| 21 | 21.9572 | 12.21 |
| 22 | 23.1125 | 42.52 |
| 23 | 23.5448 | 7.98 |
| 24 | 24.0135 | 5.03 |
| 25 | 24.8468 | 16.48 |
| 26 | 25.2782 | 12.13 |
| 27 | 25.5658 | 9.78 |
| 28 | 27.1150 | 2.89 |
| 29 | 28.4479 | 2.91 |
| 30 | 29.7783 | 2.26 |

and/or, the crystal form C of the L-tartrate salt has an X-ray powder diffraction pattern expressed by 2θ angles with diffraction peaks and relative intensities as shown in Table 23;

Table 23

| Diffraction peak number | 2θ angle (°) | Relative intensity (%) |
|---|---|---|
| 1 | 4.6038 | 31.81 |
| 2 | 5.7732 | 38.83 |
| 3 | 9.2002 | 10.19 |
| 4 | 11.5430 | 30.16 |
| 5 | 12.0755 | 10.27 |
| 6 | 12.5641 | 10.04 |
| 7 | 14.0631 | 15.56 |
| 8 | 14.6835 | 8.49 |
| 9 | 16.5088 | 100.00 |
| 10 | 17.3391 | 28.18 |
| 11 | 18.4699 | 31.10 |
| 12 | 19.1098 | 8.56 |
| 13 | 19.8012 | 20.09 |
| 14 | 20.5691 | 7.70 |
| 15 | 21.9156 | 17.52 |
| 16 | 23.1670 | 16.09 |
| 17 | 24.1677 | 9.73 |

(continued)

| Diffraction peak number | 2θ angle (°) | Relative intensity (%) |
|---|---|---|
| 18 | 24.8512 | 31.65 |
| 19 | 25.2758 | 21.42 |
| 20 | 27.1192 | 2.21 |
| 21 | 28.4266 | 5.91 |
| 22 | 28.9705 | 3.24 |
| 23 | 29.5693 | 2.56 |
| 24 | 30.9591 | 2.05 |
| 25 | 31.9485 | 1.58 |

and/or, the crystal form D of the L-tartrate salt has an X-ray powder diffraction pattern expressed by 2θ angles with diffraction peaks and relative intensities as shown in Table 24;

Table 24

| Diffraction peak number | 2θ angle (°) | Relative intensity (%) |
|---|---|---|
| 1 | 4.2422 | 42.07 |
| 2 | 5.8020 | 53.33 |
| 3 | 8.7267 | 17.54 |
| 4 | 9.4584 | 26.30 |
| 5 | 11.1738 | 21.81 |
| 6 | 11.5661 | 59.25 |
| 7 | 12.5842 | 13.25 |
| 8 | 13.0799 | 19.41 |
| 9 | 14.1192 | 16.86 |
| 10 | 15.7446 | 30.43 |
| 11 | 16.5077 | 44.95 |
| 12 | 17.3896 | 34.63 |
| 13 | 17.6339 | 16.98 |
| 14 | 18.2421 | 26.29 |
| 15 | 19.3518 | 52.51 |
| 16 | 19.6337 | 100.00 |
| 17 | 20.0205 | 46.45 |
| 18 | 20.4894 | 23.24 |
| 19 | 20.7079 | 21.69 |
| 20 | 21.9343 | 15.39 |
| 21 | 23.2390 | 25.50 |
| 22 | 23.6445 | 25.23 |
| 23 | 24.1736 | 8.79 |
| 24 | 24.8661 | 28.61 |
| 25 | 25.2811 | 34.19 |
| 26 | 26.3682 | 9.14 |

(continued)

| Diffraction peak number | 2θ angle (°) | Relative intensity (%) |
|---|---|---|
| 27 | 27.0293 | 13.90 |
| 28 | 27.4344 | 6.60 |
| 29 | 27.8155 | 6.32 |
| 30 | 28.4773 | 11.93 |
| 31 | 29.3186 | 4.94 |
| 32 | 29.6527 | 4.60 |
| 33 | 30.4539 | 3.66 |
| 34 | 31.7699 | 2.15 |
| 35 | 32.5069 | 5.18 |
| 36 | 34.3724 | 2.69 |
| 37 | 35.2995 | 4.90 |
| 38 | 35.8595 | 3.58 |
| 39 | 36.8085 | 2.50 |

and/or, the crystal form A of the glycolate salt has an X-ray powder diffraction pattern expressed by 2θ angles with diffraction peaks and relative intensities as shown in Table 25;

Table 25

| Diffraction peak number | 2θ angle (°) | Relative intensity (%) |
|---|---|---|
| 1 | 3.1271 | 72.60 |
| 2 | 5.9057 | 100.00 |
| 3 | 7.2124 | 72.19 |
| 4 | 7.8025 | 86.03 |
| 5 | 8.8362 | 43.25 |
| 6 | 9.3497 | 27.02 |
| 7 | 11.0324 | 28.05 |
| 8 | 11.4234 | 47.43 |
| 9 | 11.7978 | 72.11 |
| 10 | 12.1867 | 20.13 |
| 11 | 13.2497 | 29.84 |
| 12 | 13.4903 | 21.04 |
| 13 | 14.4342 | 23.68 |
| 14 | 14.7436 | 40.29 |
| 15 | 15.6065 | 23.20 |
| 16 | 15.8646 | 24.09 |
| 17 | 16.1643 | 28.68 |
| 18 | 16.4561 | 34.85 |
| 19 | 16.9834 | 19.90 |
| 20 | 17.7296 | 59.46 |
| 21 | 18.6510 | 23.46 |

(continued)

| Diffraction peak number | 2θ angle (°) | Relative intensity (%) |
|---|---|---|
| 22 | 19.8034 | 42.99 |
| 23 | 20.5404 | 25.02 |
| 24 | 21.9693 | 26.27 |
| 25 | 23.1042 | 36.55 |
| 26 | 23.7184 | 49.07 |
| 27 | 26.1802 | 8.05 |
| 28 | 26.7988 | 6.89 |
| 29 | 27.4725 | 7.21 |

and/or, the crystal form A of the L-malate salt has an X-ray powder diffraction pattern expressed by 2θ angles with diffraction peaks and relative intensities as shown in Table 26;

Table 26

| Diffraction peak number | 2θ angle (°) | Relative intensity (%) |
|---|---|---|
| 1 | 3.1073 | 84.43 |
| 2 | 4.1818 | 70.54 |
| 3 | 5.3014 | 64.57 |
| 4 | 5.9205 | 80.23 |
| 5 | 7.5428 | 49.60 |
| 6 | 8.3245 | 44.27 |
| 7 | 8.7635 | 40.59 |
| 8 | 10.3509 | 26.87 |
| 9 | 11.8207 | 100.00 |
| 10 | 12.4052 | 26.35 |
| 11 | 14.1024 | 33.87 |
| 12 | 16.5624 | 93.55 |
| 13 | 17.6811 | 46.90 |
| 14 | 18.7116 | 34.53 |
| 15 | 19.6954 | 24.29 |
| 16 | 20.8139 | 17.33 |
| 17 | 21.9726 | 63.79 |
| 18 | 23.7515 | 46.99 |
| 19 | 24.6273 | 16.55 |
| 20 | 25.5067 | 11.46 |
| 21 | 26.6295 | 9.10 |
| 22 | 27.1413 | 6.62 |
| 23 | 29.4502 | 7.39 |
| 24 | 35.5830 | 2.34 |

and/or, the crystal form B of the L-malate salt has an X-ray powder diffraction pattern expressed by 2θ angles with diffraction peaks and relative intensities as shown in Table 27;

Table 27

| Diffraction peak number | 2θ angle (°) | Relative intensity (%) |
|---|---|---|
| 1 | 6.6946 | 100.00 |
| 2 | 8.9934 | 7.32 |
| 3 | 9.3090 | 5.00 |
| 4 | 10.6973 | 5.76 |
| 5 | 10.9766 | 9.36 |
| 6 | 11.2990 | 4.77 |
| 7 | 12.7765 | 3.43 |
| 8 | 13.0261 | 7.42 |
| 9 | 13.3853 | 27.84 |
| 10 | 14.6423 | 8.81 |
| 11 | 14.8965 | 7.02 |
| 12 | 15.8948 | 3.96 |
| 13 | 17.0023 | 19.26 |
| 14 | 17.5519 | 2.89 |
| 15 | 18.2765 | 4.87 |
| 16 | 18.7177 | 14.52 |
| 17 | 19.6178 | 1.64 |
| 18 | 20.7001 | 4.16 |
| 19 | 21.1891 | 11.85 |
| 20 | 21.7134 | 4.87 |
| 21 | 24.0216 | 2.04 |
| 22 | 24.8829 | 1.93 |
| 23 | 25.6150 | 1.84 |
| 24 | 26.1470 | 1.78 |
| 25 | 26.8997 | 7.51 |
| 26 | 28.6034 | 0.31 |
| 27 | 29.6357 | 1.07 |
| 28 | 33.0994 | 0.92 |
| 29 | 35.0393 | 0.44 |

and/or, the crystal form A of the hippurate salt has an X-ray powder diffraction pattern expressed by 2θ angles with diffraction peaks and relative intensities as shown in Table 28;

Table 28

| Diffraction peak number | 2θ angle (°) | Relative intensity (%) |
|---|---|---|
| 1 | 3.0729 | 26.97 |
| 2 | 8.7876 | 51.26 |
| 3 | 9.1056 | 74.10 |
| 4 | 10.4103 | 73.32 |
| 5 | 12.6933 | 100.00 |

(continued)

| Diffraction peak number | 2θ angle (°) | Relative intensity (%) |
|---|---|---|
| 6 | 12.9176 | 43.28 |
| 7 | 15.0080 | 57.45 |
| 8 | 15.2256 | 47.92 |
| 9 | 16.0243 | 33.19 |
| 10 | 16.5774 | 24.31 |
| 11 | 16.9340 | 60.96 |
| 12 | 17.6939 | 57.54 |
| 13 | 18.0061 | 83.99 |
| 14 | 18.7195 | 47.71 |
| 15 | 20.4015 | 21.63 |
| 16 | 20.6500 | 44.41 |
| 17 | 20.8900 | 39.95 |
| 18 | 21.2771 | 19.32 |
| 19 | 22.3325 | 16.90 |
| 20 | 23.4499 | 2.64 |
| 21 | 24.5380 | 38.84 |
| 22 | 24.8788 | 14.80 |
| 23 | 25.5077 | 11.39 |
| 24 | 26.0053 | 42.91 |
| 25 | 26.1476 | 40.31 |
| 26 | 27.6400 | 14.93 |
| 27 | 28.4368 | 9.35 |
| 28 | 29.3126 | 7.26 |
| 29 | 29.8946 | 13.69 |
| 30 | 31.1147 | 7.88 |
| 31 | 32.3693 | 4.21 |
| 32 | 33.5114 | 2.00 |
| 33 | 34.8745 | 1.39 |
| 34 | 36.6788 | 4.85 |
| 35 | 38.9536 | 3.25 |

and/or, the crystal form A of the succinate salt has an X-ray powder diffraction pattern expressed by 2θ angles with diffraction peaks and relative intensities as shown in Table 29;

Table 29

| Diffraction peak number | 2θ angle (°) | Relative intensity (%) |
|---|---|---|
| 1 | 3.1700 | 86.47 |
| 2 | 4.7280 | 100.00 |
| 3 | 5.8389 | 42.93 |
| 4 | 7.8741 | 68.91 |

(continued)

| Diffraction peak number | 2θ angle (°) | Relative intensity (%) |
|---|---|---|
| 5 | 9.3480 | 33.01 |
| 6 | 11.7161 | 34.63 |
| 7 | 12.3943 | 13.72 |
| 8 | 16.6707 | 62.21 |
| 9 | 17.5845 | 27.41 |
| 10 | 19.9906 | 10.56 |
| 11 | 21.8996 | 26.33 |
| 12 | 23.5793 | 13.66 |
| 13 | 25.4028 | 15.64 |

and/or, the crystal form B of the succinate salt has an X-ray powder diffraction pattern expressed by 2θ angles with diffraction peaks and relative intensities as shown in Table 30;

Table 30

| Diffraction peak number | 2θ angle (°) | Relative intensity (%) |
|---|---|---|
| 1 | 5.7057 | 53.00 |
| 2 | 6.7550 | 8.45 |
| 3 | 9.2474 | 87.35 |
| 4 | 11.4224 | 75.05 |
| 5 | 12.3178 | 5.82 |
| 6 | 13.1359 | 3.84 |
| 7 | 13.5538 | 17.82 |
| 8 | 14.3636 | 2.23 |
| 9 | 15.1062 | 12.67 |
| 10 | 15.8019 | 6.12 |
| 11 | 16.3548 | 8.85 |
| 12 | 17.1711 | 100.00 |
| 13 | 18.6793 | 15.17 |
| 14 | 19.1310 | 12.89 |
| 15 | 19.5076 | 8.26 |
| 16 | 19.9919 | 12.86 |
| 17 | 20.5965 | 11.82 |
| 18 | 21.6001 | 5.61 |
| 19 | 21.9368 | 4.27 |
| 20 | 22.5861 | 5.81 |
| 21 | 22.9578 | 28.81 |
| 22 | 23.2550 | 20.09 |
| 23 | 23.6121 | 2.99 |
| 24 | 24.1087 | 12.65 |
| 25 | 25.1373 | 10.27 |

(continued)

| Diffraction peak number | 2θ angle (°) | Relative intensity (%) |
|---|---|---|
| 26 | 25.4891 | 12.78 |
| 27 | 25.7920 | 9.23 |
| 28 | 26.1262 | 12.30 |
| 29 | 26.6657 | 3.89 |
| 30 | 27.2599 | 2.16 |
| 31 | 28.9889 | 4.67 |
| 32 | 31.5060 | 3.86 |
| 33 | 34.2491 | 2.66 |

and/or, the crystal form A of the ascorbate salt has an X-ray powder diffraction pattern expressed by 2θ angles with diffraction peaks and relative intensities as shown in Table 31;

Table 31

| Diffraction peak number | 2θ angle (°) | Relative intensity (%) |
|---|---|---|
| 1 | 5.7237 | 100.00 |
| 2 | 9.2032 | 18.95 |
| 3 | 11.3077 | 72.87 |
| 4 | 14.3899 | 12.14 |
| 5 | 15.5509 | 22.27 |
| 6 | 16.7168 | 15.25 |
| 7 | 17.5897 | 17.58 |
| 8 | 18.8322 | 18.71 |
| 9 | 20.3077 | 4.66 |
| 10 | 22.3311 | 5.36 |
| 11 | 25.9469 | 17.25 |

and/or, the crystal form B of the ascorbate salt has an X-ray powder diffraction pattern expressed by 2θ angles with diffraction peaks and relative intensities as shown in Table 32;

Table 32

| Diffraction peak number | 2θ angle (°) | Relative intensity (%) |
|---|---|---|
| 1 | 3.1707 | 29.69 |
| 2 | 4.4733 | 23.68 |
| 3 | 5.5081 | 100.00 |
| 4 | 9.1133 | 6.80 |
| 5 | 11.0002 | 93.34 |
| 6 | 13.2393 | 4.60 |
| 7 | 15.2767 | 15.87 |
| 8 | 16.0915 | 20.51 |
| 9 | 16.6356 | 36.02 |
| 10 | 17.4728 | 35.23 |

(continued)

| Diffraction peak number | 2θ angle (°) | Relative intensity (%) |
|---|---|---|
| 11 | 19.8263 | 45.51 |
| 12 | 21.0910 | 6.18 |
| 13 | 21.5331 | 8.40 |
| 14 | 25.3038 | 10.56 |
| 15 | 25.9223 | 6.22 |
| 16 | 26.8139 | 3.34 |
| 17 | 28.0890 | 82.28 |
| 18 | 30.0538 | 40.01 |
| 19 | 35.5913 | 6.78 |
| 20 | 37.5593 | 3.42 |

and/or, the crystal form A of the adipate salt has an X-ray powder diffraction pattern expressed by 2θ angles with diffraction peaks and relative intensities as shown in Table 33;

Table 33

| Diffraction peak number | 2θ angle (°) | Relative intensity (%) |
|---|---|---|
| 1 | 8.4718 | 67.95 |
| 2 | 8.9976 | 61.80 |
| 3 | 11.4167 | 83.68 |
| 4 | 12.4070 | 27.06 |
| 5 | 13.6026 | 21.93 |
| 6 | 15.5779 | 23.86 |
| 7 | 16.2943 | 45.59 |
| 8 | 17.5244 | 47.58 |
| 9 | 18.2396 | 55.02 |
| 10 | 19.8412 | 100.00 |
| 11 | 21.2994 | 36.71 |
| 12 | 23.8124 | 10.35 |
| 13 | 24.6221 | 19.76 |
| 14 | 26.5540 | 9.38 |
| 15 | 27.7925 | 7.62 |
| 16 | 29.8266 | 6.29 |

and/or, the crystal form A of the *p*-toluenesulfonate salt has an X-ray powder diffraction pattern expressed by 2θ angles with diffraction peaks and relative intensities as shown in Table 34;

Table 34

| Diffraction peak number | 2θ angle (°) | Relative intensity (%) |
|---|---|---|
| 1 | 8.9180 | 22.08 |
| 2 | 9.4393 | 21.33 |
| 3 | 11.5147 | 5.35 |

(continued)

| Diffraction peak number | 2θ angle (°) | Relative intensity (%) |
|---|---|---|
| 4 | 12.3169 | 61.37 |
| 5 | 13.7084 | 1.36 |
| 6 | 15.9326 | 89.13 |
| 7 | 16.4387 | 100.00 |
| 8 | 17.2684 | 13.78 |
| 9 | 17.9133 | 24.06 |
| 10 | 18.3165 | 16.79 |
| 11 | 19.4915 | 5.98 |
| 12 | 20.6954 | 37.28 |
| 13 | 20.9526 | 30.72 |
| 14 | 21.1925 | 20.91 |
| 15 | 22.7769 | 12.78 |
| 16 | 23.1170 | 24.60 |
| 17 | 23.2986 | 22.76 |
| 18 | 24.3491 | 2.47 |
| 19 | 24.7620 | 14.93 |
| 20 | 25.5566 | 6.70 |
| 21 | 26.3685 | 44.14 |
| 22 | 26.8348 | 10.07 |
| 23 | 27.6967 | 10.29 |
| 24 | 29.2566 | 6.35 |
| 25 | 29.8415 | 2.65 |
| 26 | 30.3293 | 2.33 |
| 27 | 30.9189 | 1.35 |
| 28 | 31.7372 | 2.58 |
| 29 | 32.3362 | 2.30 |
| 30 | 33.5831 | 1.87 |
| 31 | 34.9009 | 1.18 |
| 32 | 36.2298 | 1.35 |
| 33 | 37.7435 | 0.63 |

and/or, the crystal form A of the benzenesulfonate salt has an X-ray powder diffraction pattern expressed by 2θ angles with diffraction peaks and relative intensities as shown in Table 35;

Table 35

| Diffraction peak number | 2θ angle (°) | Relative intensity (%) |
|---|---|---|
| 1 | 3.0977 | 63.58 |
| 2 | 6.5455 | 11.06 |
| 3 | 8.7674 | 17.07 |
| 4 | 10.8117 | 35.66 |

(continued)

| Diffraction peak number | 2θ angle (°) | Relative intensity (%) |
|---|---|---|
| 5 | 11.3821 | 49.24 |
| 6 | 11.9132 | 24.99 |
| 7 | 13.3227 | 10.58 |
| 8 | 14.0177 | 7.67 |
| 9 | 15.4042 | 9.34 |
| 10 | 16.2565 | 40.08 |
| 11 | 17.4115 | 29.89 |
| 12 | 18.3594 | 44.95 |
| 13 | 19.3888 | 32.82 |
| 14 | 19.9059 | 77.88 |
| 15 | 20.6784 | 53.41 |
| 16 | 21.3155 | 100.00 |
| 17 | 22.8963 | 18.69 |
| 18 | 25.8671 | 15.29 |
| 19 | 27.7592 | 9.57 |
| 20 | 32.2150 | 6.81 |

and/or, the crystal form A of the oxalate salt has an X-ray powder diffraction pattern expressed by 2θ angles with diffraction peaks and relative intensities as shown in Table 36;

Table 36

| Diffraction peak number | 2θ angle (°) | Relative intensity (%) |
|---|---|---|
| 1 | 6.1579 | 65.04 |
| 2 | 7.0217 | 9.02 |
| 3 | 9.1873 | 7.84 |
| 4 | 10.4448 | 97.21 |
| 5 | 13.9480 | 19.90 |
| 6 | 15.1277 | 5.59 |
| 7 | 15.5499 | 5.85 |
| 8 | 16.7440 | 18.11 |
| 9 | 17.4987 | 100.00 |
| 10 | 18.1693 | 9.62 |
| 11 | 19.5112 | 11.28 |
| 12 | 20.0751 | 16.44 |
| 13 | 22.6414 | 5.26 |
| 14 | 23.5997 | 9.73 |
| 15 | 24.5061 | 29.87 |
| 16 | 25.2293 | 4.87 |
| 17 | 25.7921 | 12.09 |
| 18 | 26.5363 | 17.05 |

(continued)

| Diffraction peak number | 2θ angle (°) | Relative intensity (%) |
|---|---|---|
| 19 | 27.9052 | 3.88 |
| 20 | 28.4782 | 6.45 |
| 21 | 29.9326 | 1.60 |
| 22 | 32.6865 | 4.95 |

and/or, the crystal form A of the 2-hydroxyethanesulfonate salt has an X-ray powder diffraction pattern expressed by 2θ angles with diffraction peaks and relative intensities as shown in Table 37;

Table 37

| Diffraction peak number | 2θ angle (°) | Relative intensity (%) |
|---|---|---|
| 1 | 6.4673 | 28.94 |
| 2 | 9.8938 | 23.28 |
| 3 | 10.9559 | 21.78 |
| 4 | 13.1418 | 74.74 |
| 5 | 13.6076 | 30.46 |
| 6 | 14.5351 | 5.34 |
| 7 | 15.6934 | 20.61 |
| 8 | 18.3887 | 92.04 |
| 9 | 18.8170 | 100.00 |
| 10 | 19.3894 | 14.35 |
| 11 | 19.8344 | 6.67 |
| 12 | 20.4483 | 26.19 |
| 13 | 20.8232 | 15.84 |
| 14 | 21.1502 | 14.16 |
| 15 | 21.7048 | 53.93 |
| 16 | 22.0768 | 11.35 |
| 17 | 23.2544 | 17.44 |
| 18 | 24.0437 | 26.00 |
| 19 | 24.8212 | 12.74 |
| 20 | 26.0333 | 12.69 |
| 21 | 26.3559 | 12.33 |
| 22 | 26.7073 | 10.60 |
| 23 | 29.2874 | 3.69 |
| 24 | 30.6802 | 3.50 |
| 25 | 32.2293 | 4.79 |

**5.** The crystal form according to any one of claims 1 to 4, wherein the X-ray powder diffraction pattern of the crystal form A of the compound of formula I expressed by 2θ angles is basically shown in FIG. 4;

and/or, the differential scanning calorimetry pattern of the crystal form A of the compound of formula I is basically shown in FIG. 5;

and/or, the thermogravimetric analysis pattern of the crystal form A of the compound of formula I is basically

shown in FIG. 6;
and/or, the X-ray powder diffraction pattern of the crystal form B of the compound of formula I expressed by 2θ angles is basically shown in FIG. 10;
and/or, the X-ray powder diffraction pattern of the crystal form A of the fumarate salt of the compound of formula I expressed by 2θ angles is basically shown in FIG. 11;
and/or, the differential scanning calorimetry pattern of the crystal form A of the fumarate salt is basically shown in FIG. 12;
and/or, the thermogravimetric analysis pattern of the crystal form A of the fumarate salt is basically shown in FIG. 13;
and/or, the X-ray powder diffraction pattern of the crystal form B of the fumarate salt of the compound of formula I expressed by 2θ angles is basically shown in FIG. 16;
and/or, the differential scanning calorimetry pattern of the crystal form B of the fumarate salt is basically shown in FIG. 17;
and/or, the thermogravimetric analysis pattern of the crystal form B of the fumarate salt is basically shown in FIG. 18;
and/or, the X-ray powder diffraction pattern of the crystal form C of the fumarate salt of the compound of formula I expressed by 2θ angles is basically shown in FIG. 19;
and/or, the differential scanning calorimetry pattern of the crystal form C of the fumarate salt is basically shown in FIG. 20;
and/or, the thermogravimetric analysis pattern of the crystal form C of the fumarate salt is basically shown in FIG. 21;
and/or, the X-ray powder diffraction pattern of the crystal form A of the citrate salt of the compound of formula I expressed by 2θ angles is basically shown in FIG. 22;
and/or, the differential scanning calorimetry pattern of the crystal form A of the citrate salt is basically shown in FIG. 23;
and/or, the thermogravimetric analysis pattern of the crystal form A of the citrate salt is basically shown in FIG. 24;
and/or, the X-ray powder diffraction pattern of the crystal form B of the citrate salt expressed by 2θ angles is basically shown in FIG. 25;
and/or, the differential scanning calorimetry pattern of the crystal form B of the citrate salt is basically shown in FIG. 27;
and/or, the thermogravimetric analysis pattern of the crystal form B of the citrate salt is basically shown in FIG. 28;
and/or, the X-ray powder diffraction pattern of the crystal form A of the methanesulfonate salt expressed by 2θ angles is basically shown in FIG. 30;
and/or, the differential scanning calorimetry pattern of the crystal form A of the methanesulfonate salt is basically shown in FIG. 31;
and/or, the thermogravimetric analysis pattern of the crystal form A of the methanesulfonate salt is basically shown in FIG. 32;
and/or, the X-ray powder diffraction pattern of the crystal form A of the ethanesulfonate salt of the compound of formula I expressed by 2θ angles is basically shown in FIG. 35;
and/or, the differential scanning calorimetry pattern of the crystal form A of the ethanesulfonate salt of the compound of formula I is basically shown in FIG. 37;
and/or, the thermogravimetric analysis pattern of the crystal form A of the ethanesulfonate salt of the compound of formula I is basically shown in FIG. 38;
and/or, the X-ray powder diffraction pattern of the crystal form B of the ethanesulfonate salt expressed by 2θ angles is basically shown in FIG. 40;
and/or, the differential scanning calorimetry pattern of the crystal form B of the ethanesulfonate salt is basically shown in FIG. 41;
and/or, the thermogravimetric analysis pattern of the crystal form B of the ethanesulfonate salt is basically shown in FIG. 42;
and/or, the X-ray powder diffraction pattern of the crystal form A of the maleate salt expressed by 2θ angles is basically shown in FIG. 43;
and/or, the differential scanning calorimetry pattern of the crystal form A of the maleate salt is basically shown in FIG. 44;
and/or, the thermogravimetric analysis pattern of the crystal form A of the maleate salt is basically shown in FIG. 45;
and/or, the X-ray powder diffraction pattern of the crystal form B of the maleate salt expressed by 2θ angles is basically shown in FIG. 46;
and/or, the differential scanning calorimetry pattern of the crystal form B of the maleate salt is basically shown in

FIG. 47;
and/or, the thermogravimetric analysis pattern of the crystal form B of the maleate salt is basically shown in FIG. 48;
and/or, the X-ray powder diffraction pattern of the crystal form A of the L-tartrate salt expressed by 2θ angles is basically shown in FIG. 49;
and/or, the differential scanning calorimetry pattern of the crystal form A of the L-tartrate salt is basically shown in FIG. 50;
and/or, the thermogravimetric analysis pattern of the crystal form A of the L-tartrate salt is basically shown in FIG. 51;
and/or, the X-ray powder diffraction pattern of the crystal form B of the L-tartrate salt expressed by 2θ angles is basically shown in FIG. 52;
and/or, the differential scanning calorimetry pattern of the crystal form B of the L-tartrate salt is basically shown in FIG. 53;
and/or, the thermogravimetric analysis pattern of the crystal form B of the L-tartrate salt is basically shown in FIG. 54;
and/or, the X-ray powder diffraction pattern of the crystal form C of the L-tartrate salt expressed by 2θ angles is basically shown in FIG. 55;
and/or, the differential scanning calorimetry pattern of the crystal form C of the L-tartrate salt is basically shown in FIG. 56;
and/or, the thermogravimetric analysis pattern of the crystal form C of the L-tartrate salt is basically shown in FIG. 57;
and/or, the X-ray powder diffraction pattern of the crystal form D of the L-tartrate salt expressed by 2θ angles is basically shown in FIG. 58;
and/or, the differential scanning calorimetry pattern of the crystal form D of the L-tartrate salt is basically shown in FIG. 59;
and/or, the thermogravimetric analysis pattern of the crystal form D of the L-tartrate salt is basically shown in FIG. 60;
and/or, the X-ray powder diffraction pattern of the crystal form A of the glycolate salt expressed by 2θ angles is basically shown in FIG. 61;
and/or, the differential scanning calorimetry pattern of the crystal form A of the glycolate salt is basically shown in FIG. 62;
and/or, the thermogravimetric analysis pattern of the crystal form A of the glycolate salt is basically shown in FIG. 63;
and/or, the X-ray powder diffraction pattern of the crystal form A of the L-malate salt expressed by 2θ angles is basically shown in FIG. 64;
and/or, the differential scanning calorimetry pattern of the crystal form A of the L-malate salt is basically shown in FIG. 65;
and/or, the thermogravimetric analysis pattern of the crystal form A of the L-malate salt is basically shown in FIG. 66;
and/or, the X-ray powder diffraction pattern of the crystal form B of the L-malate salt expressed by 2θ angles is basically shown in FIG. 67;
and/or, the differential scanning calorimetry pattern of the crystal form B of the L-malate salt is basically shown in FIG. 68;
and/or, the thermogravimetric analysis pattern of the crystal form B of the L-malate salt is basically shown in FIG. 69;
and/or, the X-ray powder diffraction pattern of the crystal form A of the hippurate salt expressed by 2θ angles is basically shown in FIG. 70;
and/or, the differential scanning calorimetry pattern of the crystal form A of the hippurate salt is basically shown in FIG. 71;
and/or, the thermogravimetric analysis pattern of the crystal form A of the hippurate salt is basically shown in FIG. 72;
and/or, the X-ray powder diffraction pattern of the crystal form A of the succinate salt expressed by 2θ angles is basically shown in FIG. 128;
and/or, the differential scanning calorimetry pattern of the crystal form A of the succinate salt is basically shown in FIG. 73;
and/or, the thermogravimetric analysis pattern of the crystal form A of the succinate salt is basically shown in FIG. 74;
and/or, the X-ray powder diffraction pattern of the crystal form B of the succinate salt expressed by 2θ angles is

basically shown in FIG. 75;
and/or, the differential scanning calorimetry pattern of the crystal form B of the succinate salt is basically shown in FIG. 76;
and/or, the thermogravimetric analysis pattern of the crystal form B of the succinate salt is basically shown in FIG. 77;
and/or, the X-ray powder diffraction pattern of the crystal form A of the ascorbate salt expressed by 2θ angles is basically shown in FIG. 129;
and/or, the differential scanning calorimetry pattern of the crystal form A of the ascorbate salt is basically shown in FIG. 78;
and/or, the thermogravimetric analysis pattern of the crystal form A of the ascorbate salt is basically shown in FIG. 79;
and/or, the X-ray powder diffraction pattern of the crystal form B of the ascorbate salt expressed by 2θ angles is basically shown in FIG. 80;
and/or, the differential scanning calorimetry pattern of the crystal form B of the ascorbate salt is basically shown in FIG. 81;
and/or, the thermogravimetric analysis pattern of the crystal form B of the ascorbate salt is basically shown in FIG. 82;
and/or, the X-ray powder diffraction pattern of the crystal form A of the adipate salt expressed by 2θ angles is basically shown in FIG. 130;
and/or, the differential scanning calorimetry pattern of the crystal form A of the adipate salt is basically shown in FIG. 83;
and/or, the thermogravimetric analysis pattern of the crystal form A of the adipate salt is basically shown in FIG. 84;
and/or, the X-ray powder diffraction pattern of the crystal form A of the*p*-toluenesulfonate salt expressed by 2θ angles is basically shown in FIG. 85;
and/or, the differential scanning calorimetry pattern of the crystal form A of the *p*-toluenesulfonate salt is basically shown in FIG. 86;
and/or, the thermogravimetric analysis pattern of the crystal form A of the *p*-toluenesulfonate salt is basically shown in FIG. 87;
and/or, the X-ray powder diffraction pattern of the crystal form A of the benzenesulfonate salt expressed by 2θ angles is basically shown in FIG. 131;
and/or, the differential scanning calorimetry pattern of the crystal form A of the benzenesulfonate salt is basically shown in FIG. 88;
and/or, the thermogravimetric analysis pattern of the crystal form A of the benzenesulfonate salt is basically shown in FIG. 89;
and/or, the X-ray powder diffraction pattern of the crystal form A of the oxalate salt expressed by 2θ angles is basically shown in FIG. 90;
and/or, the differential scanning calorimetry pattern of the crystal form A of the oxalate salt is basically shown in FIG. 91;
and/or, the thermogravimetric analysis pattern of the crystal form A of the oxalate salt is basically shown in FIG. 92;
and/or, the X-ray powder diffraction pattern of the crystal form A of the 2-hydroxyethanesulfonate salt expressed by 2θ angles is basically shown in FIG. 93;
and/or, the differential scanning calorimetry pattern of the crystal form A of the 2-hydroxyethanesulfonate salt is basically shown in FIG. 94;
and/or, the thermogravimetric analysis pattern of the crystal form A of the 2-hydroxyethanesulfonate salt is basically shown in FIG. 95.

**6.** A pharmaceutically acceptable salt of a compound of formula I, wherein the pharmaceutically acceptable salt is a salt formed by the compound of formula I and an acid; the acid is hydrochloric acid, sulfuric acid, maleic acid, aspartic acid, phosphoric acid, fumaric acid, tartaric acid, citric acid, glucuronic acid, glycolic acid, malic acid, hippuric acid, gluconic acid, lactic acid, succinic acid, ascorbic acid, adipic acid, *p*-toluenesulfonic acid, methanesulfonic acid, benzenesulfonic acid, oxalic acid, 2-hydroxyethanesulfonic acid, ethanesulfonic acid, gentisic acid, or benzoic acid;

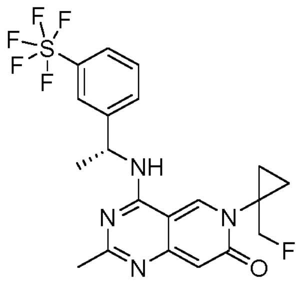

I

7. The pharmaceutically acceptable salt of the compound of formula I according to claim 6, wherein the acid is fumaric acid, citric acid, methanesulfonic acid, ethanesulfonic acid, maleic acid, L-tartaric acid, glycolic acid, L-malic acid, hippuric acid, succinic acid, ascorbic acid, adipic acid, *p*-toluenesulfonic acid, benzenesulfonic acid, oxalic acid, or 2-hydroxyethanesulfonic acid;
and/or, the compound of formula I and the acid have a molar ratio of 1:(0.5 to 1.2), such as 1:0.5, 1:0.6, 1:0.7, 1:0.8, 1:0.9, 1:1, 1:1.1, or 1:1.2.

8. The pharmaceutically acceptable salt of the compound of formula I according to claim 7, wherein the pharmaceutically acceptable salt of the compound of formula I is any one of the following pharmaceutically acceptable salts:

(1) a fumarate salt of the compound of formula I; wherein the compound of formula I and fumaric acid have a molar ratio of 1:1;
(2) a citrate salt of the compound of formula I; wherein the compound of formula I and citric acid have a molar ratio of 1:(1 to 1.2), such as 1:1, 1:1.1, or 1:1.2;
(3) a methanesulfonate salt of the compound of formula I; wherein the compound of formula I and methanesulfonic acid have a molar ratio of 1:(0.8 to 1), such as 1:1, 1:0.9, or 1:0.8;
(4) an ethanesulfonate salt of the compound of formula I; wherein the compound of formula I and ethanesulfonic acid have a molar ratio of 1:1;
(5) a maleate salt of the compound of formula I; wherein the compound of formula I and maleic acid have a molar ratio of 1:1;
(6) an L-tartrate salt of the compound of formula I; wherein the compound of formula I and L-tartaric acid have a molar ratio of 1:(0.5 to 1.1), such as 1:0.5, 1:0.6, 1:0.7, 1:0.8, 1:0.9, 1:1, or 1:1.1;
(7) a glycolate salt of the compound of formula I; wherein the compound of formula I and glycolic acid have a molar ratio of (1 to 1.1), such as 1:1 or 1:1.1;
(8) an L-malate salt of the compound of formula I; wherein the compound of formula I and L-malic acid have a molar ratio of 1:(1 to 1.1), such as 1:1 or 1:1.1;
(9) a hippurate salt of the compound of formula I; wherein the compound of formula I and hippuric acid have a molar ratio of 1:1;
(10) a succinate salt of the compound of formula I; wherein the compound of formula I and succinic acid have a molar ratio of 1:(0.8 to 1.2), such as 1:0.8, 1:0.9, 1:1, or 1:1.2;
(11) an ascorbate salt of the compound of formula I; wherein the compound of formula I and ascorbic acid have a molar ratio of 1:(0.5 to 1.2), such as 1:0.5, 1:0.6, 1:0.7, 1:0.8, 1:0.9, 1:1, or 1:1.2;
(12) an adipate salt of the compound of formula I; wherein the compound of formula I and adipic acid have a molar ratio of 1:1;
(13) a *p*-toluenesulfonate salt of the compound of formula I; wherein the compound of formula I and *p*-toluenesulfonic acid have a molar ratio of 1:1;
(14) a benzenesulfonate salt of the compound of formula I; wherein the compound of formula I and benzenesulfonic acid have a molar ratio of 1:1;
(15) an oxalate salt of the compound of formula I; wherein the compound of formula I and oxalic acid have a molar ratio of 1:1;
(16) a 2-hydroxyethanesulfonate salt of the compound of formula I; wherein the compound of formula I and 2-hydroxyethanesulfonic acid have a molar ratio of 1:(1 to 1.1), such as 1:1 or 1:1.1.

9. A preparation method for the pharmaceutically acceptable salt of the compound of formula I according to any one of claims 6 to 8, wherein the preparation method comprises: carrying out a salt-forming reaction of the compound of formula I with an acid in a solvent to obtain the pharmaceutically acceptable salt of the compound of formula I; wherein

the acid is hydrochloric acid, sulfuric acid, maleic acid, aspartic acid, phosphoric acid, fumaric acid, tartaric acid, citric acid, glucuronic acid, glycolic acid, malic acid, hippuric acid, gluconic acid, lactic acid, succinic acid, ascorbic acid, adipic acid, *p*-toluenesulfonic acid, methanesulfonic acid, benzenesulfonic acid, oxalic acid, 2-hydroxyethanesulfonic acid, ethanesulfonic acid, gentisic acid, or benzoic acid;

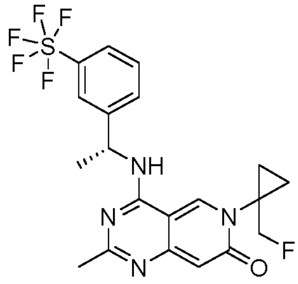

I ;

preferably, the preparation method satisfies one or more of the following conditions:

(1) the solvent is one or more of water, ethyl acetate, methyl *tert*-butyl ether, acetone, *n*-heptane, and isopropanol; preferably one or more of water, ethyl acetate, methyl *tert*-butyl ether, acetone, and *n*-heptane;
(2) the compound of formula I and the acid have a molar ratio of 1:(1 ± 0.5); preferably, 1:(1 ± 0.2) or 1:(1 ± 0.1), for example, 1:1;
(3) the acid is fumaric acid, citric acid, methanesulfonic acid, ethanesulfonic acid, maleic acid, L-tartaric acid, glycolic acid, L-malic acid, hippuric acid, succinic acid, ascorbic acid, adipic acid, *p*-toluenesulfonic acid, benzenesulfonic acid, oxalic acid, or 2-hydroxyethanesulfonic acid;

preferably, when the crystal form is a crystal form A of the compound of formula I, the preparation method comprises the following steps: crystallizing the compound of formula I in a solvent M, and separating solids; the solvent M is an alkane solvent; the alkane solvent is preferably *n*-hexane; the crystallization is carried out at room temperature, preferably 40 to 60°C, such as 50°C;
when the crystal form is the crystal form A of the fumarate salt of the compound of formula I, the crystal form B of the fumarate salt of the compound of formula I, the crystal form C of the fumarate salt the compound of formula I, the crystal form A of the citrate salt of the compound of formula I, the crystal form B of the citrate salt of the compound of formula I, the crystal form A of the methanesulfonate salt of the compound of formula I, the crystal form A of the ethanesulfonate salt of the compound of formula I, the crystal form B of the ethanesulfonate salt of the compound of formula I, the crystal form A of the maleate salt of the compound of formula I, the crystal form B of the maleate salt of the compound of formula I, the crystal form A of the L-tartrate salt of the compound of formula I, the crystal form B of the L-tartrate salt of the compound of formula I, the crystal form C of the L-tartrate salt of the compound of formula I, the crystal form D of the L-tartrate salt of the compound of formula I, the crystal form A of the glycolate salt of the compound of formula I, the crystal form A of the L-malate salt of the compound of formula I, the crystal form B of the L-malate salt of the compound of formula I, the crystal form A of the hippurate salt of the compound of formula I, the crystal form A of the succinate salt of the compound of formula I, the crystal form B of the succinate salt of the compound of formula I, the crystal form A of the ascorbate salt of the compound of formula I, the crystal form B of the ascorbate salt of the compound of formula I, the crystal form A of the adipate salt of the compound of formula I, the crystal form A of the*p*-toluene sulfonate salt of the compound of formula I, the crystal form A of the benzenesulfonate salt of the compound of formula I, the crystal form A of the oxalate salt of the compound of formula I, or the crystal form A of the 2-hydroxyethanesulfonate salt of the compound of formula I, the preparation method comprises the following steps: reacting the compound of formula I with an acid in a solvent N, and separating solids;
the solvent N is one or more of an alcohol solvent, an alkane solvent, an ester solvent, and water; the solvent N is preferably a mixture of isopropanol and water, *n*-hexane, or ethyl acetate; more preferably, the isopropanol and water have a volume ratio of 19:1 ± 5; the reaction is carried out at room temperature, preferably 10 to 30°C; the acid is fumaric acid, citric acid, methanesulfonic acid, ethanesulfonic acid, maleic acid, L-tartaric acid, glycolic acid, L-malic acid, hippuric acid, succinic acid, ascorbic acid, adipic acid, *p*-toluenesulfonic acid, benzenesulfonic acid, 2-hydroxyethanesulfonic acid, oxalic acid, or 2-hydroxyethanesulfonic acid.

**10.** A pharmaceutical composition I, wherein the pharmaceutical composition I comprises a substance A and a

pharmaceutically acceptable carrier, and the substance A is one or more of the crystal form A of the compound of formula I, the crystal form B of the compound of formula I, the crystal form A of the fumarate salt of the compound of formula I, the crystal form B of the fumarate salt of the compound of formula I, the crystal form C of the fumarate salt the compound of formula I, the crystal form A of the citrate salt of the compound of formula I, the crystal form B of the citrate salt of the compound of formula I, the crystal form A of the methanesulfonate salt of the compound of formula I, the crystal form A of the ethanesulfonate salt of the compound of formula I, the crystal form B of the ethanesulfonate salt of the compound of formula I, the crystal form A of the maleate salt of the compound of formula I, the crystal form B of the maleate salt of the compound of formula I, the crystal form A of the L-tartrate salt of the compound of formula I, the crystal form B of the L-tartrate salt of the compound of formula I, the crystal form C of the L-tartrate salt of the compound of formula I, the crystal form D of the L-tartrate salt of the compound of formula I, the crystal form A of the glycolate salt of the compound of formula I, the crystal form A of the L-malate salt of the compound of formula I, the crystal form B of the L-malate salt of the compound of formula I, the crystal form A of the hippurate salt of the compound of formula I, the crystal form A of the succinate salt of the compound of formula I, the crystal form B of the succinate salt of the compound of formula I, the crystal form A of the ascorbate salt of the compound of formula I, the crystal form B of the ascorbate salt of the compound of formula I, the crystal form A of the adipate salt of the compound of formula I, the crystal form A of the p-toluenesulfonate salt of the compound of formula I, the crystal form A of the benzenesulfonate salt of the compound of formula I, the crystal form A of the oxalate salt of the compound of formula I, or the crystal form A of the 2-hydroxyethanesulfonate salt of the compound of formula I according to any one of claims 1 to 5, and the pharmaceutically acceptable salt of the compound of formula I according to any one of claims 6 to 8.

**11.** A pharmaceutical composition II, wherein the pharmaceutical composition II comprises a substance **A** and at least one other pharmacologically active inhibitor;

the substance **A** is as defined in claim 10;
preferably, the other pharmacologically activity inhibitor is an inhibitor of MEK, an inhibitor of EGFR, or an inhibitor of KRAS;
more preferably, the inhibitor of MEK is trametinib;
more preferably, the inhibitor of EGFR is osimertinib or gefitinib, preferably osimertinib;
more preferably, the inhibitor of KRAS is MRTX-849, AMG-510, or JDQ443.

**12.** A use of a substance **A,** the pharmaceutical composition I according to claim 10, or the pharmaceutical composition II according to claim 11, wherein the use is selected from:

(1) preparing a drug for inhibiting an interaction between SOS1 and RAS family proteins;
(2) preparing a drug for preventing and/or treating a disease related to or mediated by SOS1 and RAS family proteins, and the substance **A** is as defined in claim 10.

**13.** The use according to claim 12, wherein it satisfies one or more of the following conditions:

(1) the disease related to or mediated by SOS1 and RAS family proteins comprises but is not limited to: cancer and RASopathies; the RASopathies are preferably Noonan syndrome, cardiofaciocutaneous syndrome, hereditary gingival fibromatosis type 1, neurofibromatosis type 1 (NF1), capillary malformation-arteriovenous malformation syndrome, Costello syndrome, and Legius syndrome, more preferably neurofibromatosis type 1;
the cancer is preferably selected from melanoma, skin cancer, liver cancer, kidney cancer, lung cancer, nasopharyngeal cancer, gastric cancer, esophageal cancer, colorectal cancer, gallbladder carcinoma, cholangiocarcinoma, choriocarcinoma, pancreatic cancer, polycythemia vera, pediatric tumor, cervical cancer, ovarian cancer, breast cancer, bladder cancer, urothelial carcinoma, ureteral tumor, prostate cancer, seminoma, testicular cancer, leukemia, head and neck tumor, endometrial cancer, thyroid cancer, lymphoma, sarcoma, osteoma, neuroblastoma, neuroblastoma, brain tumor, myeloma, astrocytoma, glioblastoma, and glioma;
(2) the drug comprises the substance **A** and trametinib or osimertinib;
(3) the RAS family protein can be KRAS, such as KRAS G12C, KRAS G12D, and KRAS G12V.

**14.** The use according to claim 13, wherein the cancer is selected from colorectal cancer, lung cancer, and pancreatic cancer; the lung cancer is preferably non-small cell lung cancer.

**15.** The use according to claim 12, wherein it satisfies one or more of the following conditions:

(1) the use comprises a use of the substance **A** in combination with an inhibitor of MEK;

(2) the use comprises a use of the substance **A** in combination with an inhibitor of EGFR;
(3) the use comprises a use of the substance **A** in combination with an inhibitor of KRAS;

preferably, the use comprises a use of the substance **A** in combination with trametinib or osimertinib.

**16.** A use of a substance **A,** the pharmaceutical composition I according to claim 10, or the pharmaceutical composition II according to claim 11, wherein the use is selected from:

(1) inhibiting an interaction between SOS1 and RAS family proteins;
(2) preventing and/or treating a disease related to SOS1 and RAS family proteins;

the substance **A** is as defined in claim 10.

**17.** A method for inhibiting SOS1 and RAS family proteins, or preventing and/or treating a disease related to or mediated by SOS1 and RAS family proteins, comprising the steps of: administering to a subject in need thereof a therapeutically effective amount of a substance **A;**

the substance **A** is as defined in claim 10;
preferably, the method comprises using the substance **A** in combination with an inhibitor of MEK, an inhibitor of EGFR, or an inhibitor of KRAS;
more preferably, the method comprises using the substance **A** in combination with trametinib or osimertinib.

**18.** A quality detection method for a substance **B,** wherein the method comprises the following steps: eluting a test substance in a silica gel chromatographic column using high-performance liquid chromatography;

the test substance comprises the substance **B**; wherein the substance **B** is one or more of the compound of formula I, the crystal form A of the compound of formula I, the crystal form B of the compound of formula I, the crystal form A of the fumarate salt of the compound of formula I, the crystal form B of the fumarate salt of the compound of formula I, the crystal form C of the fumarate salt the compound of formula I, the crystal form A of the citrate salt of the compound of formula I, the crystal form B of the citrate salt of the compound of formula I, the crystal form A of the methanesulfonate salt of the compound of formula I, the crystal form A of the ethanesulfonate salt of the compound of formula I, the crystal form B of the ethanesulfonate salt of the compound of formula I, the crystal form A of the maleate salt of the compound of formula I, the crystal form B of the maleate salt of the compound of formula I, the crystal form A of the L-tartrate salt of the compound of formula I, the crystal form B of the L-tartrate salt of the compound of formula I, the crystal form C of the L-tartrate salt of the compound of formula I, the crystal form D of the L-tartrate salt of the compound of formula I, the crystal form A of the glycolate salt of the compound of formula I, the crystal form A of the L-malate salt of the compound of formula I, the crystal form B of the L-malate salt of the compound of formula I, the crystal form A of the hippurate salt of the compound of formula I, the crystal form A of the succinate salt of the compound of formula I, the crystal form B of the succinate salt of the compound of formula I, the crystal form A of the ascorbate salt of the compound of formula I, the crystal form B of the ascorbate salt of the compound of formula I, the crystal form A of the adipate salt of the compound of formula I, the crystal form A of the*p*-toluenesulfonate salt of the compound of formula I, the crystal form A of the benzenesulfonate salt of the compound of formula I, the crystal form A of the oxalate salt of the compound of formula I, the crystal form A of the 2-hydroxyethanesulfonate salt of the compound of formula I according to any one of claims 1 to 5, and the pharmaceutically acceptable salt of the compound of formula I according to claim 6 or 7;
the mobile phase used for eluting is a mobile phase A and a mobile phase B;
the mobile phase A is an aqueous solution of 0.1 $\pm$ 0.05% ammonia;
the mobile phase B is acetonitrile;
in the elution procedure, the mobile phase A and the mobile phase B have a volume ratio of 1: 1.5 to 9;
preferably, the elution procedure used for eluting is:

| Time/min | Mobile phase B/% |
|---|---|
| 0.0 | 15 |
| 8.0 | 90 |
| 10.0 | 90 |

(continued)

| Time/min | Mobile phase B/% |
|---|---|
| 10.1 | 15 |
| 13.0 | 15 |

preferably, the quality detection refers to a purity test, a dynamic solubility test, and a stability test; preferably, the detection method satisfies one or more of the following conditions:

(1) the injection volume for eluting is 5 ± 1 μL;
(2) the flow rate for eluting is 1.0 ± 0.2 mL/min;
(3) the wavelength of the UV detector for eluting is 228 ± 5 nm;
(4) the model of the chromatographic column is Waters XBridge C18, 4.6 mm/150 mm/3 μm;
(5) the column temperature of the chromatographic column is room temperature, preferably 30°C;
(6) the diluent of the test substance is ACN/$H_2O$ (1:1 ± 0.5).

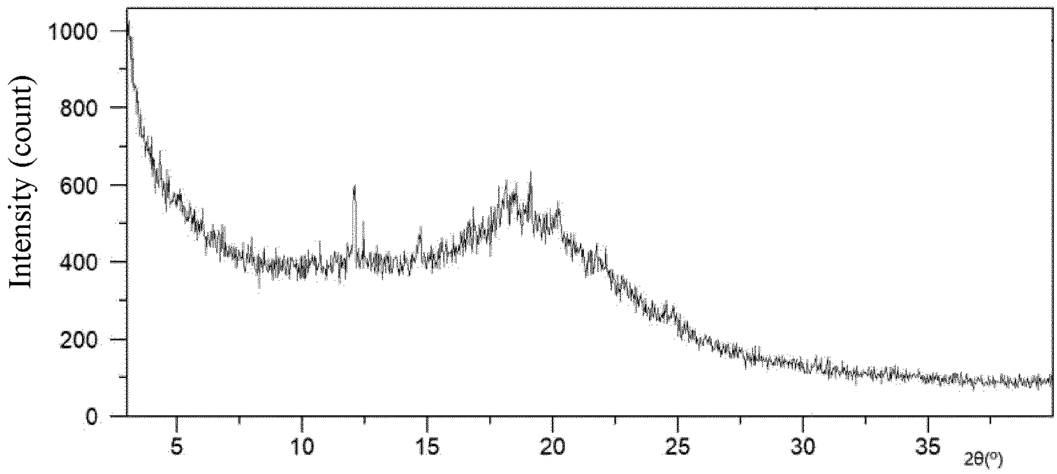


FIG. 1

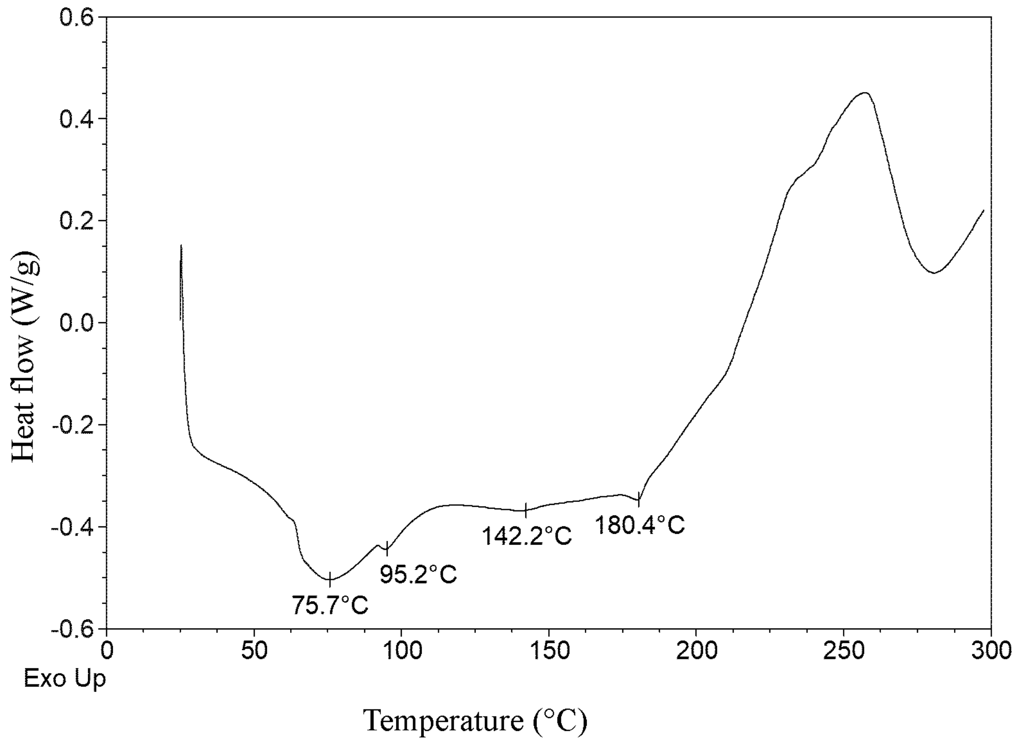
0.6
0.4
0.2
0.0
-0.2
-0.4
-0.6
Heat flow (W/g)
0
50
100
150
200
250
300
Exo Up
Temperature (°C)
75.7°C
95.2°C
142.2°C
180.4°C

FIG. 2

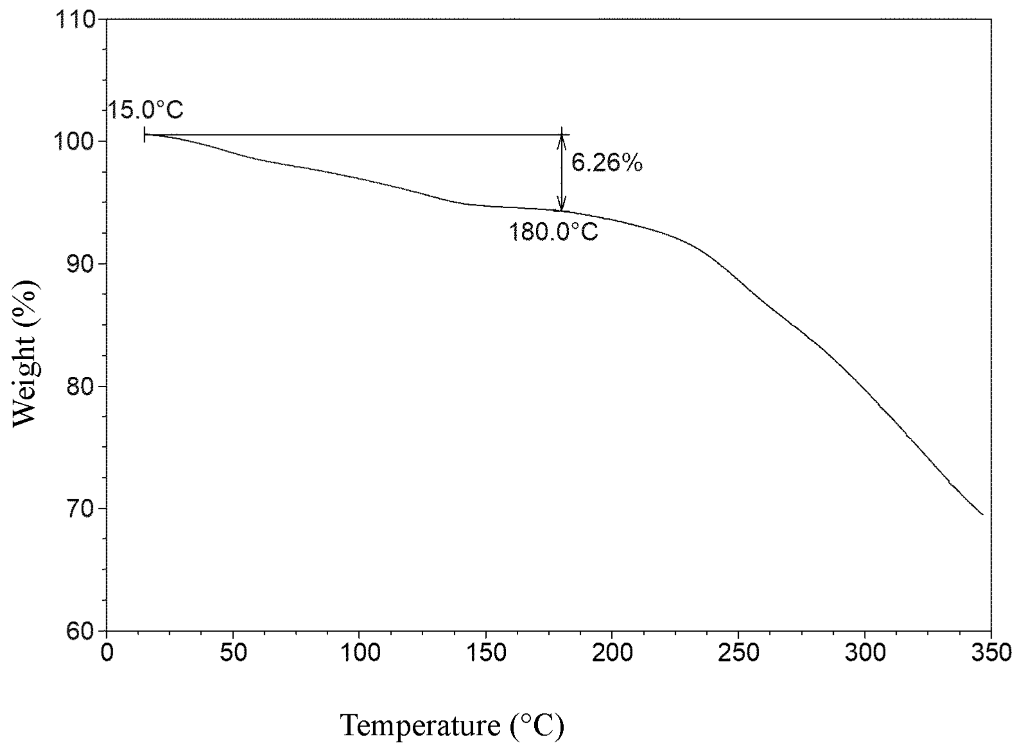
110
100
90
80
70
60
Weight (%)
0
50
100
150
200
250
300
350
Temperature (°C)
15.0°C
6.26%
180.0°C

FIG. 3

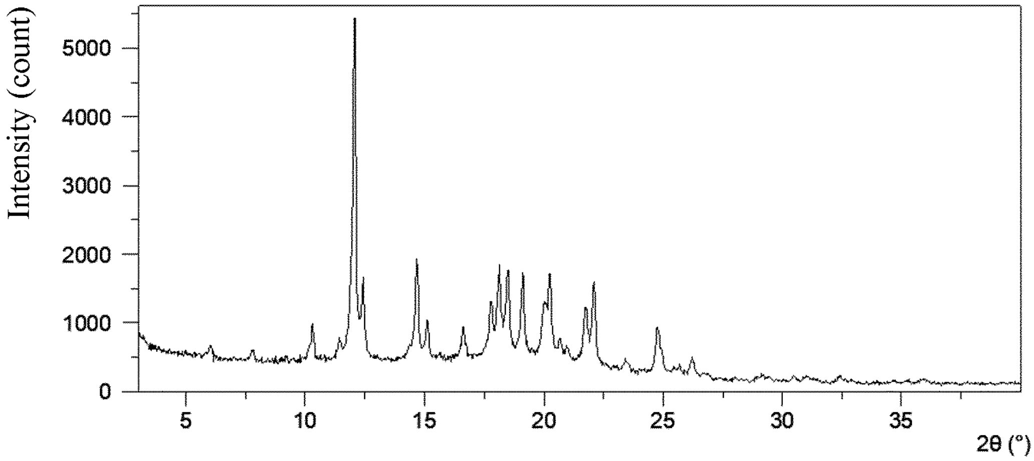
Intensity (count)
5000
4000
3000
2000
1000
0
5
10
15
20
25
30
35
2θ (°)

FIG. 4

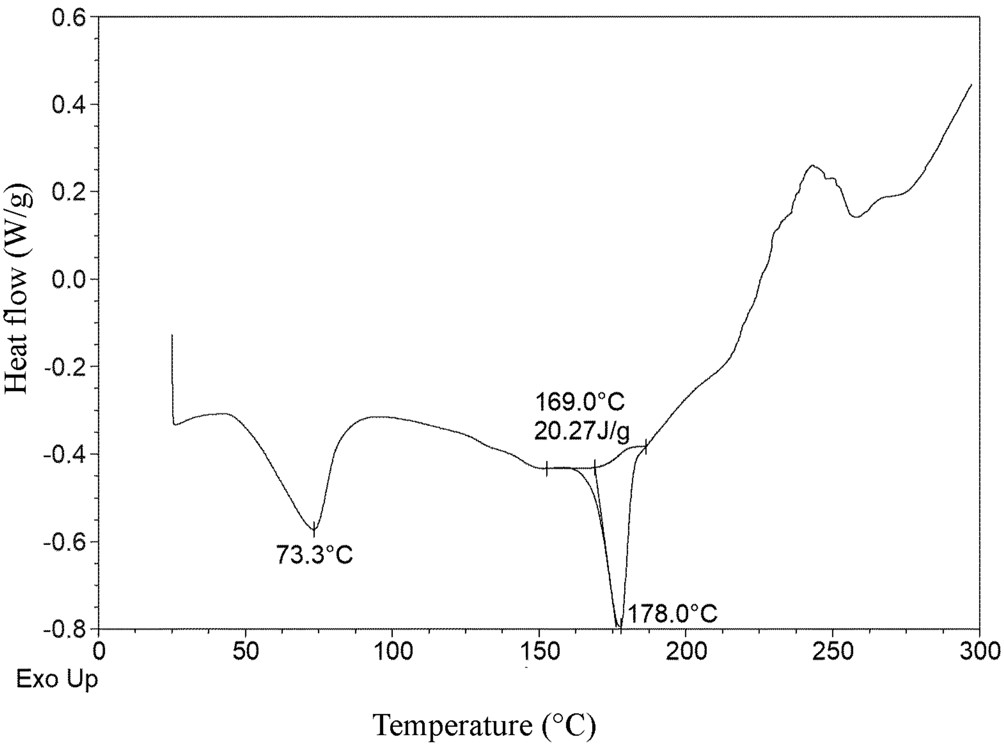
0.6
0.4
0.2
0.0
-0.2
-0.4
-0.6
-0.8
Heat flow (W/g)
169.0°C
20.27J/g
73.3°C
178.0°C
0
50
100
150
200
250
300
Exo Up
Temperature (°C)

FIG. 5

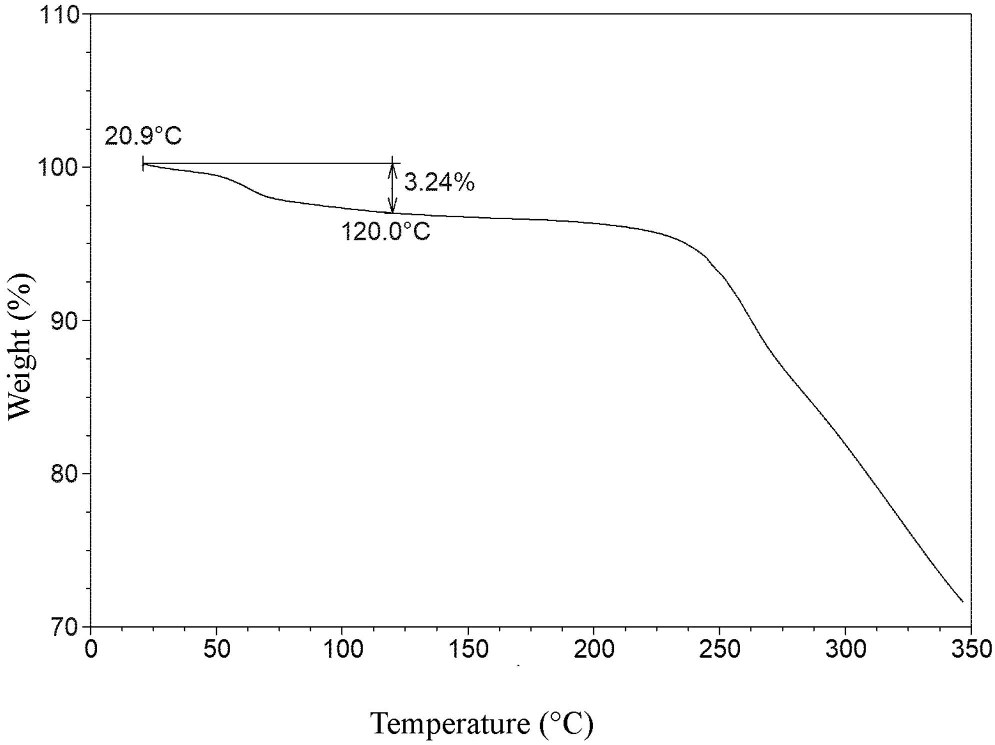
110
100
90
80
70
Weight (%)
20.9°C
3.24%
120.0°C
0
50
100
150
200
250
300
350
Temperature (°C)

FIG. 6

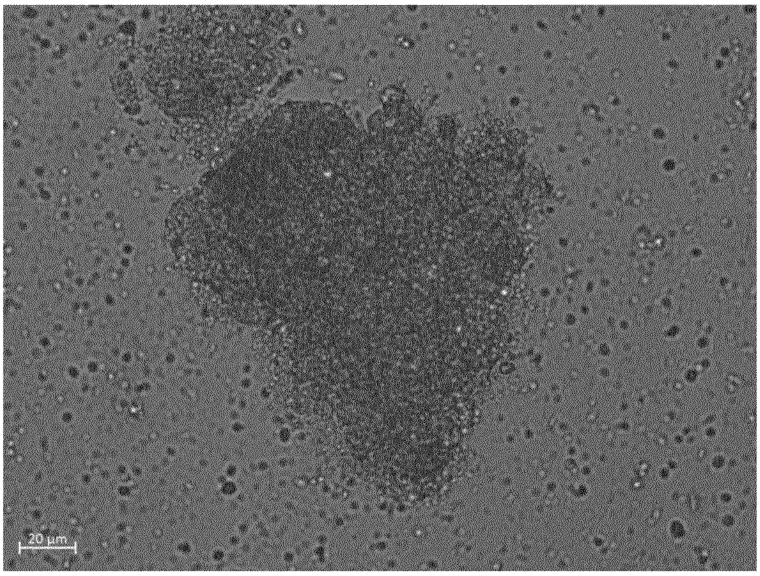
20 µm

FIG. 7

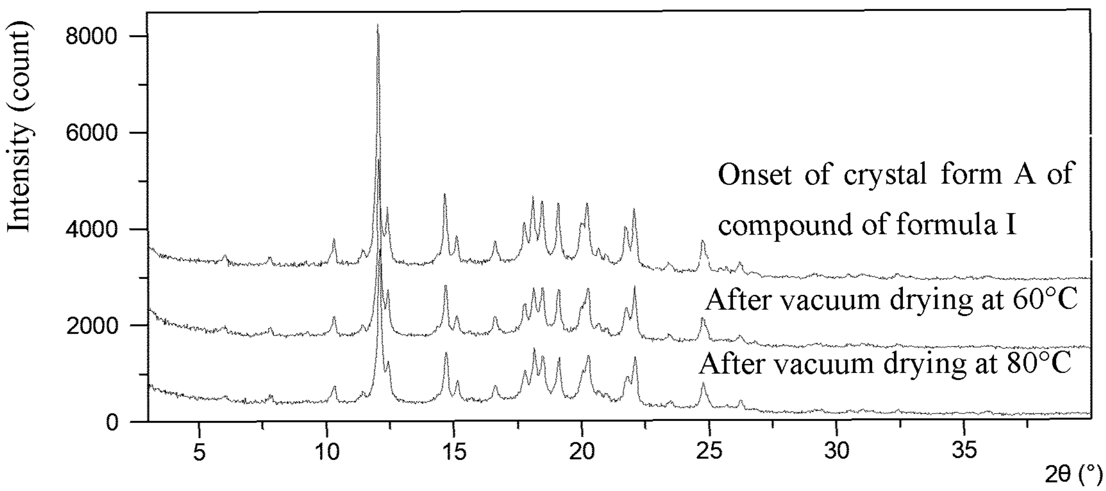


FIG. 8

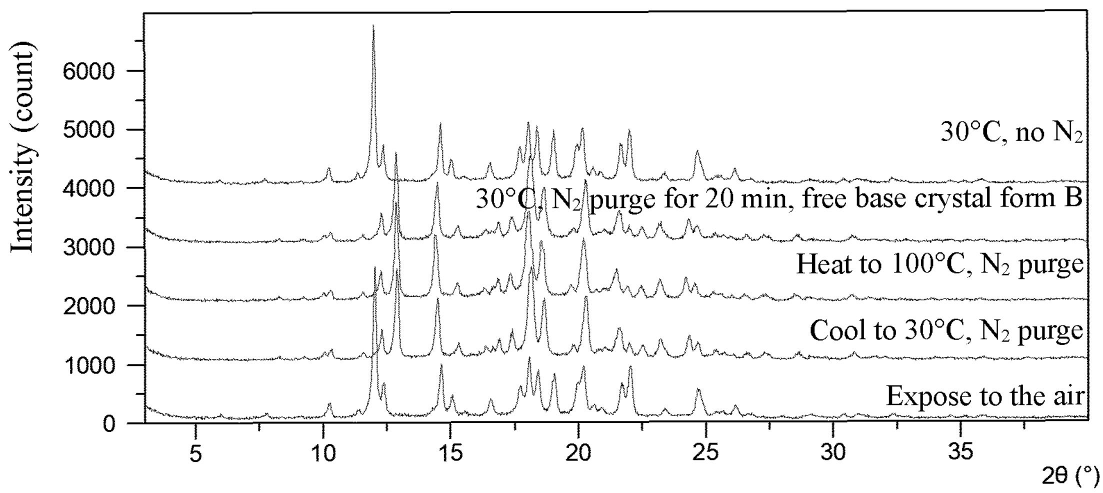


FIG. 9

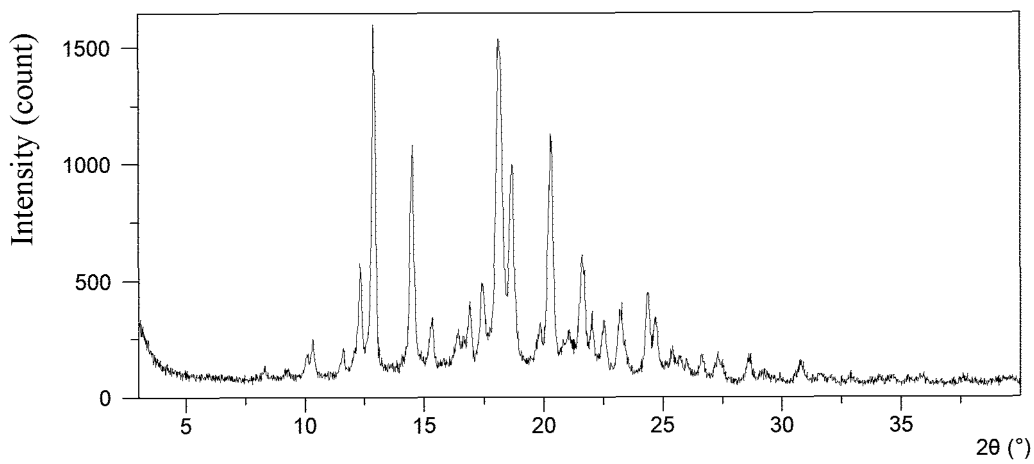


FIG. 10

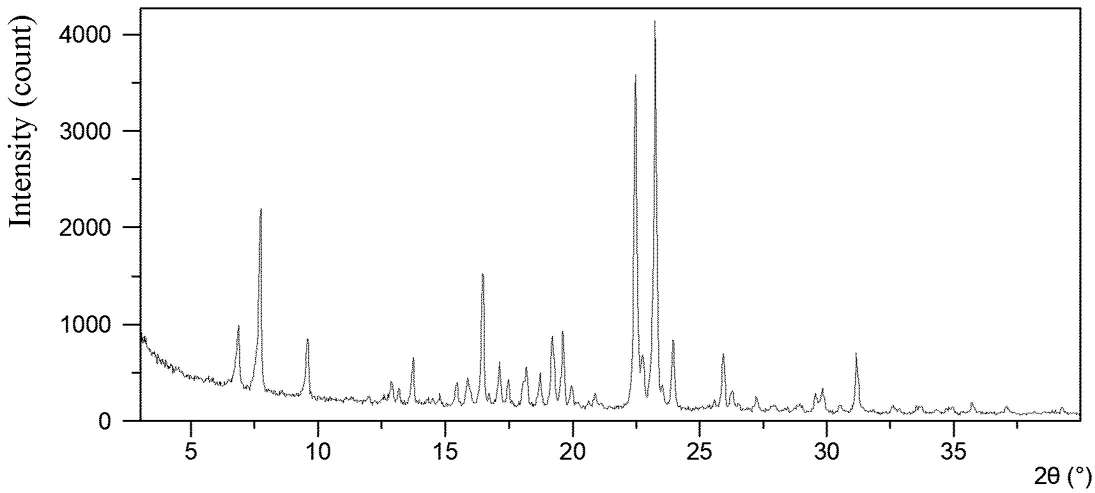
Intensity (count)
4000
3000
2000
1000
0
5
10
15
20
25
30
35
2θ (°)

FIG. 11

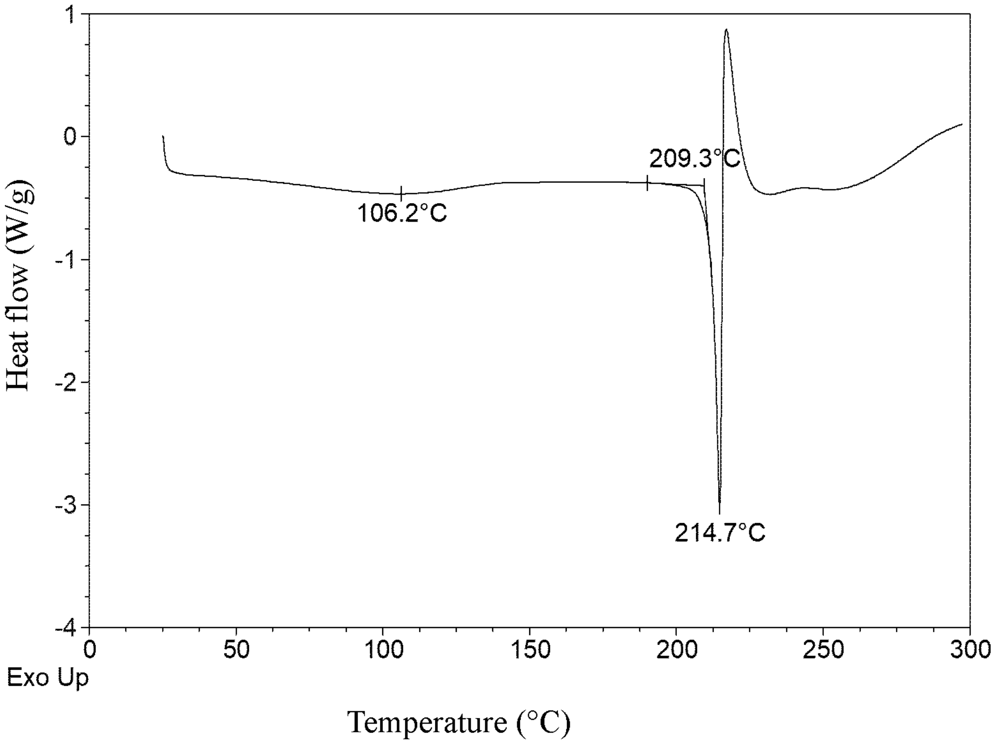
Heat flow (W/g)
1
0
-1
-2
-3
-4
209.3°C
106.2°C
214.7°C
0
50
100
150
200
250
300
Exo Up
Temperature (°C)

FIG. 12

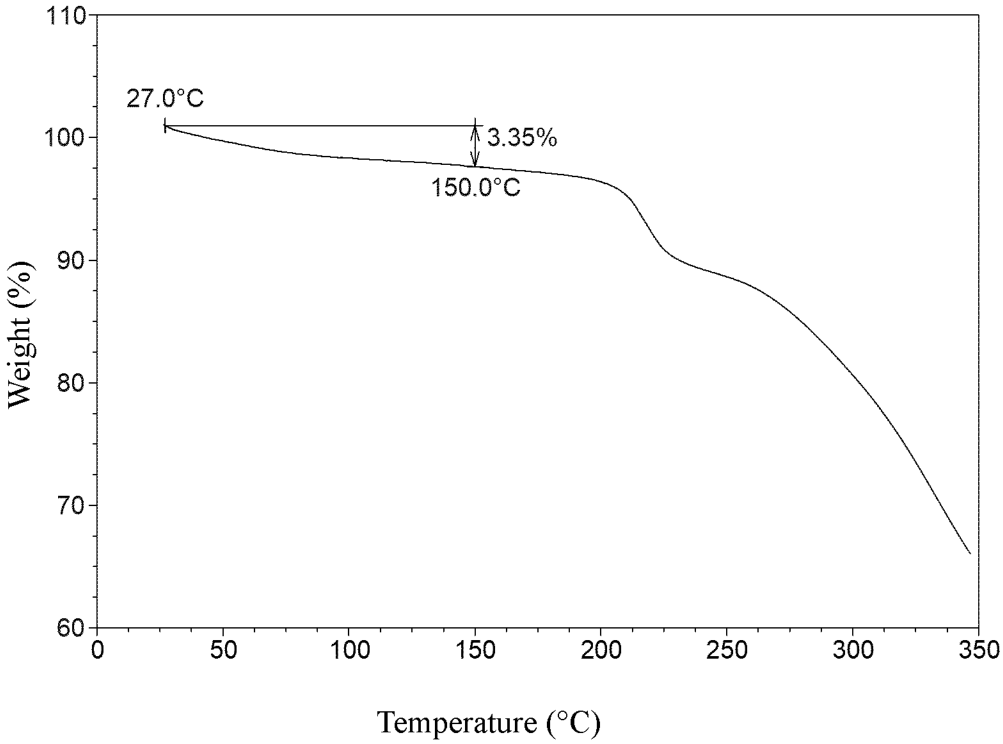
110
100
90
80
70
60
Weight (%)
0
50
100
150
200
250
300
350
Temperature (°C)
27.0°C
3.35%
150.0°C

FIG. 13

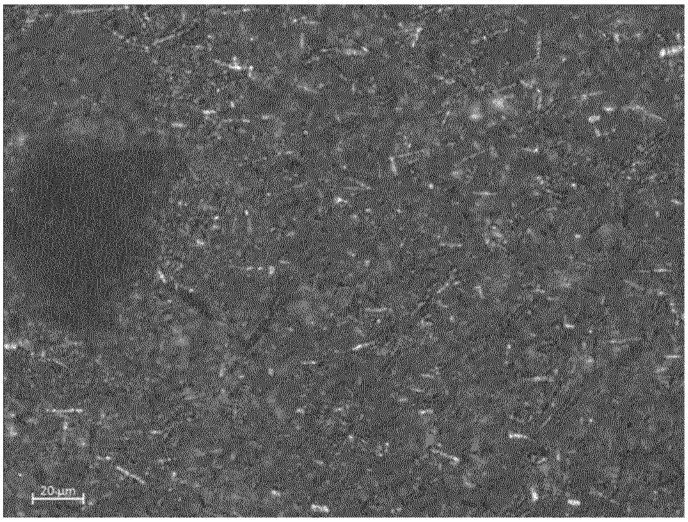
20 µm

FIG. 14

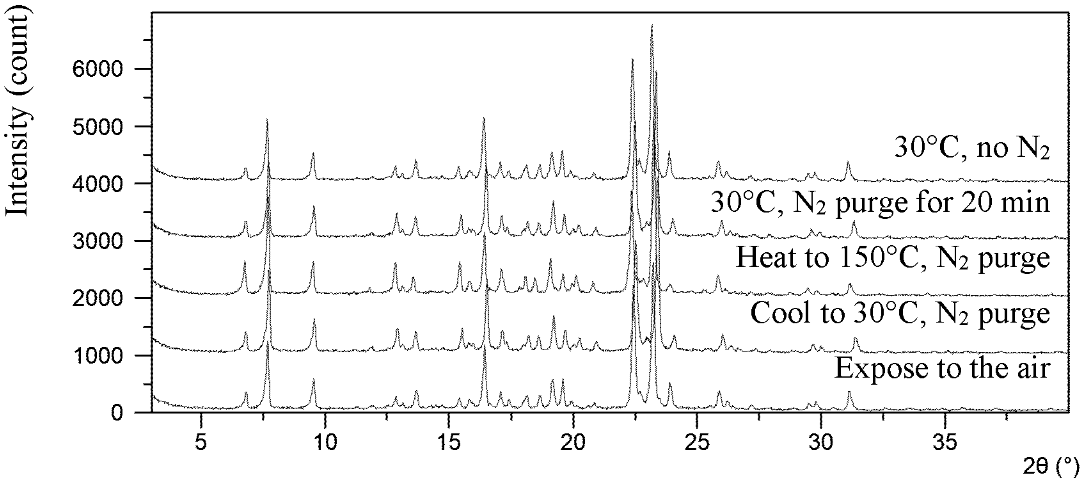


FIG. 15

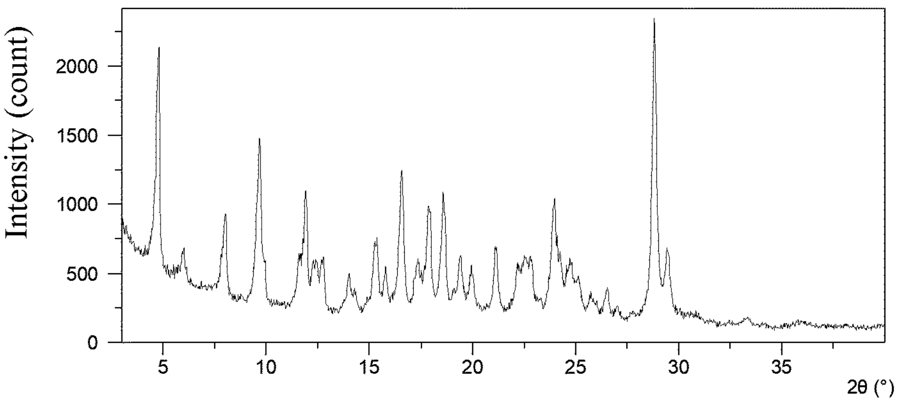


FIG. 16

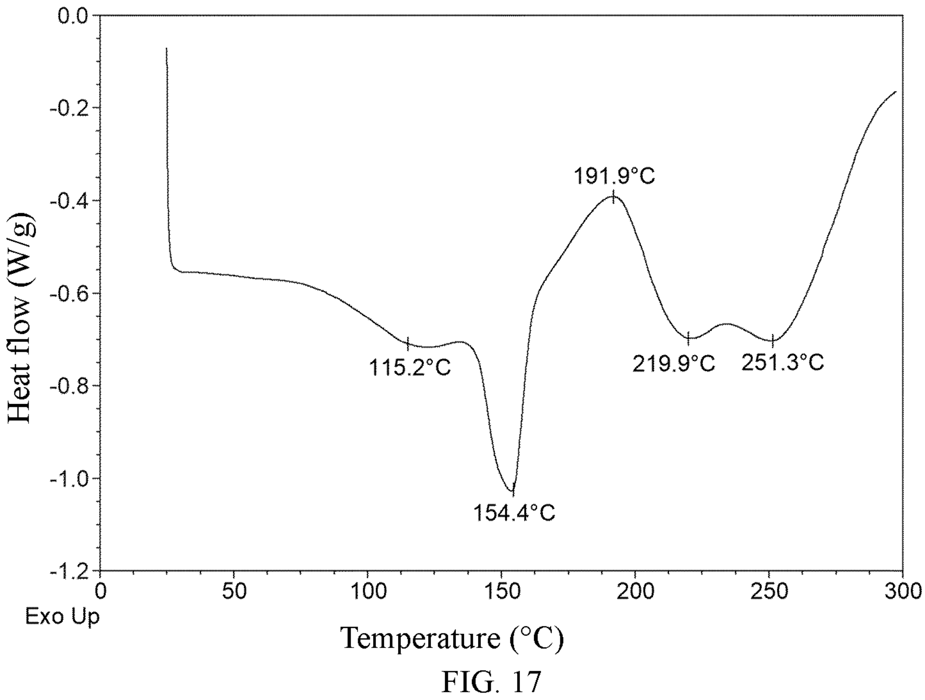


FIG. 17

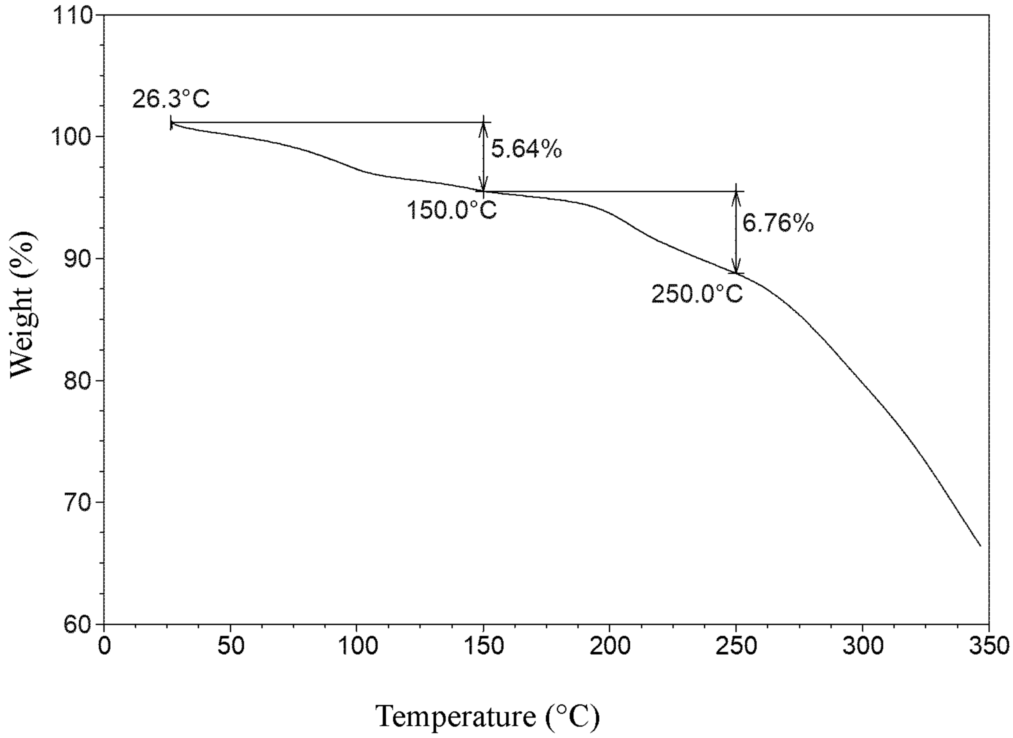
110
100
90
80
70
60
Weight (%)
26.3°C
5.64%
150.0°C
6.76%
250.0°C
0
50
100
150
200
250
300
350
Temperature (°C)

FIG. 18

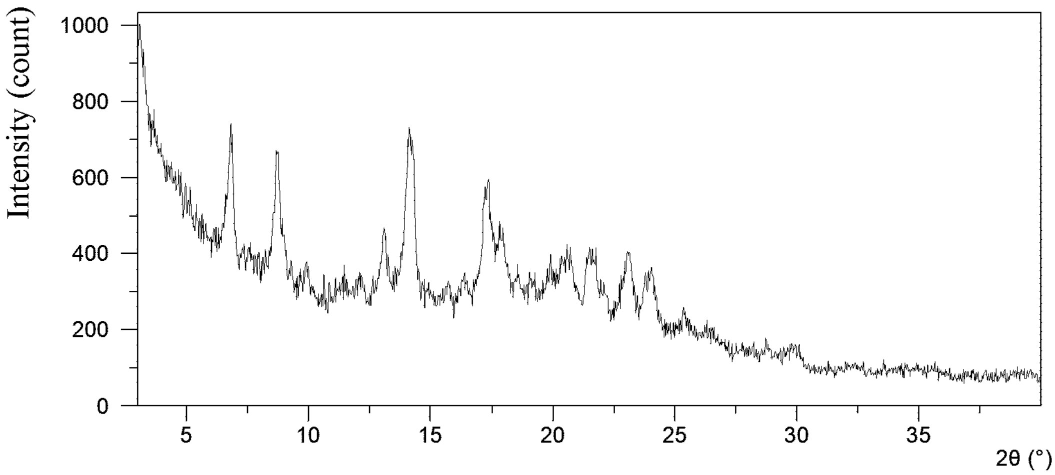
Intensity (count)
1000
800
600
400
200
0
5
10
15
20
25
30
35
2θ (°)

FIG. 19

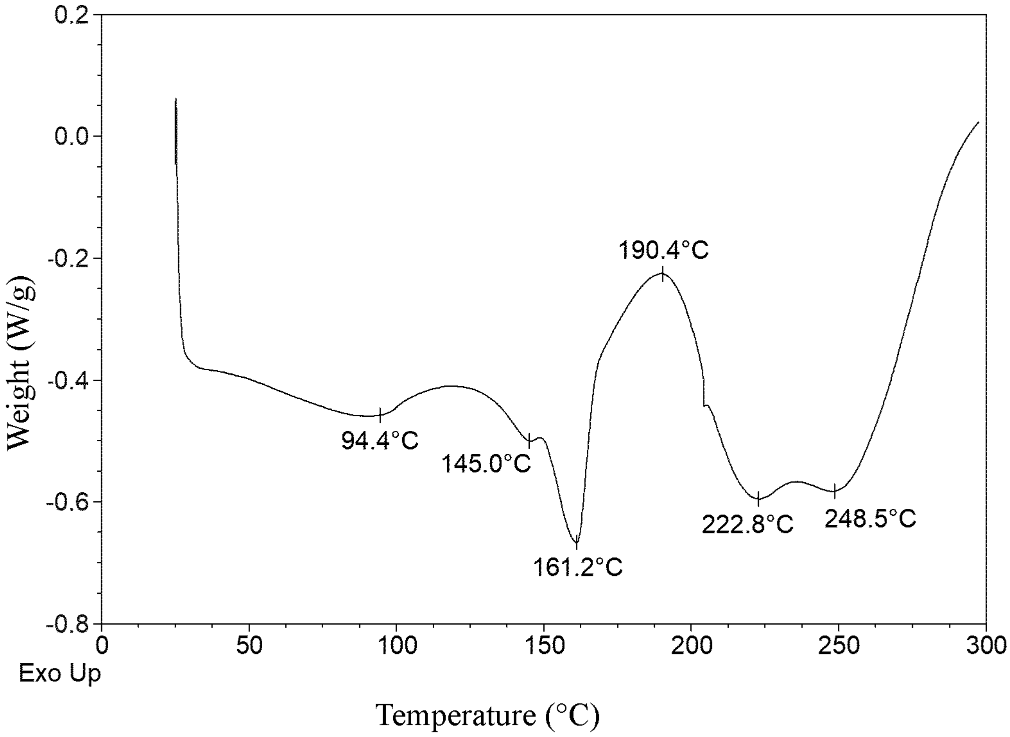
0.2
0.0
-0.2
-0.4
-0.6
-0.8
Weight (W/g)
190.4°C
94.4°C
145.0°C
161.2°C
222.8°C
248.5°C
0
50
100
150
200
250
300
Exo Up
Temperature (°C)

FIG. 20

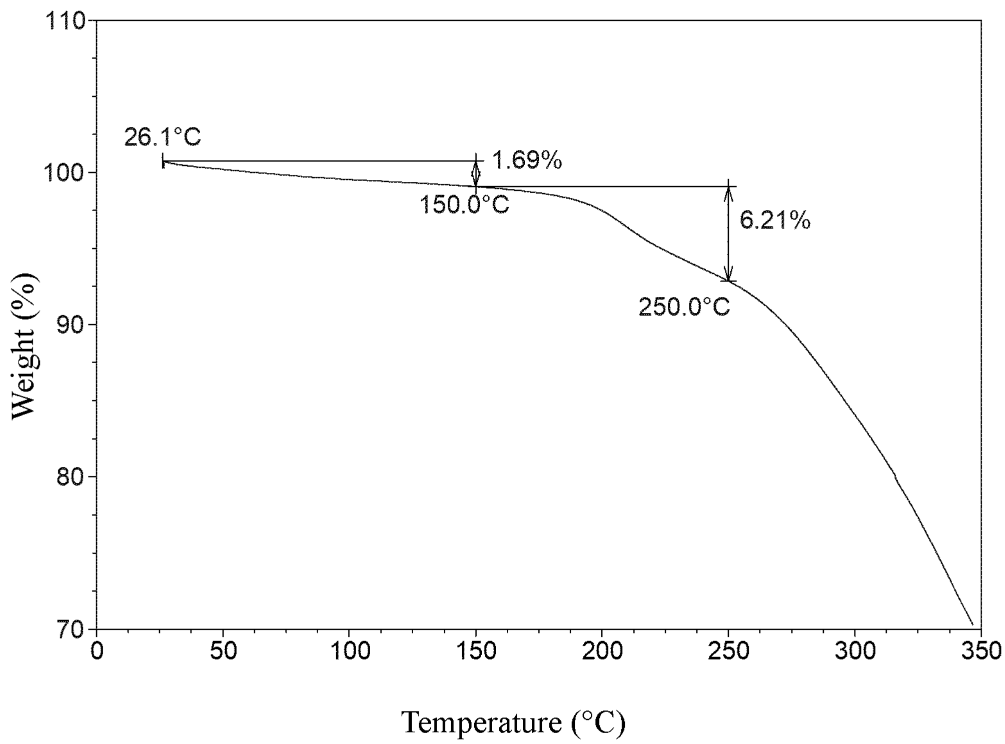
110
100
90
80
70
Weight (%)
26.1°C
1.69%
150.0°C
6.21%
250.0°C
0
50
100
150
200
250
300
350
Temperature (°C)

FIG. 21

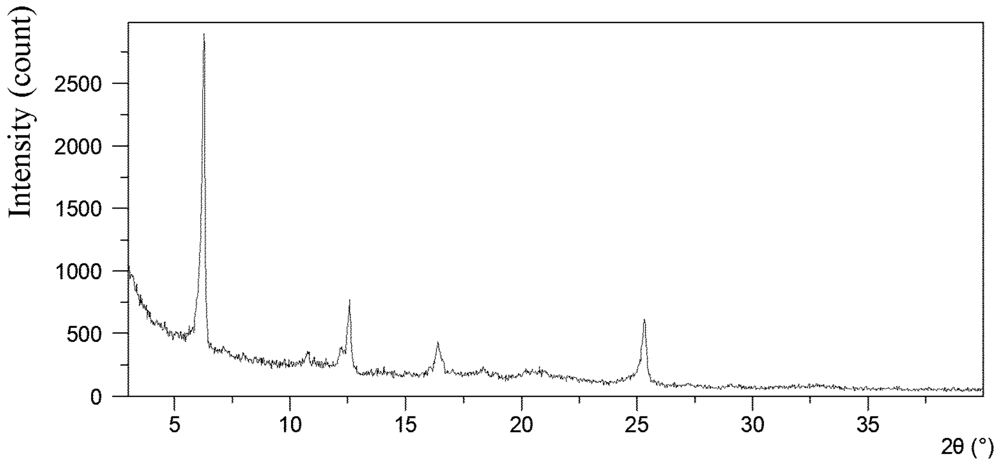

Intensity (count)
2500
2000
1500
1000
500
0
5
10
15
20
25
30
35
2θ (°)


FIG. 22

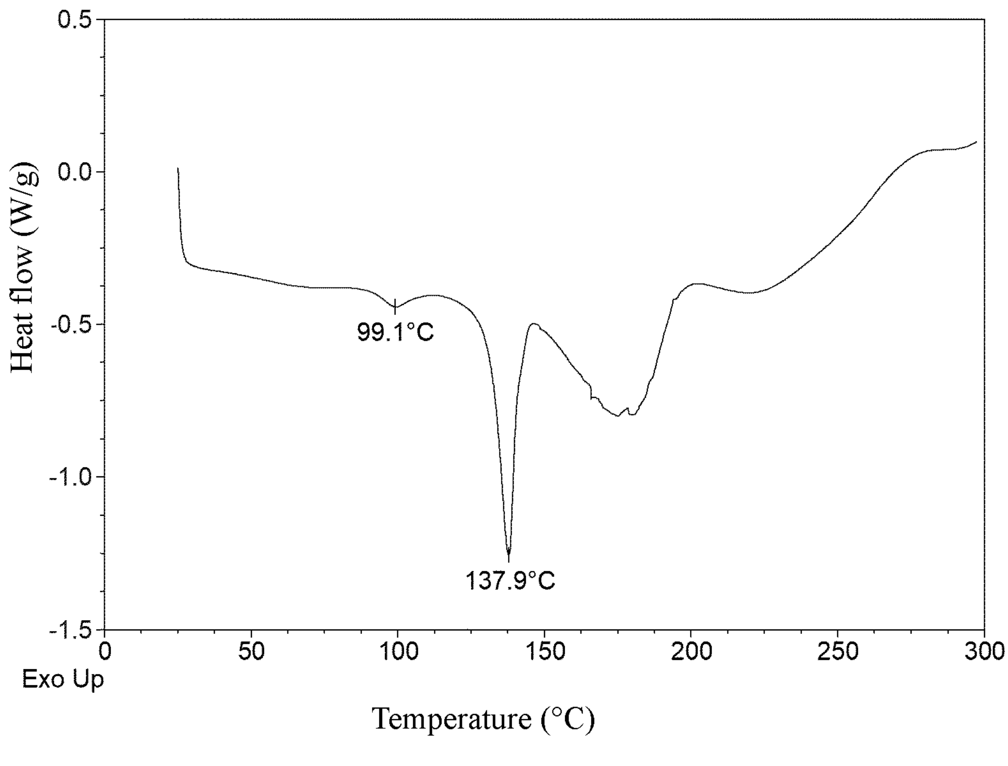

0.5
0.0
-0.5
-1.0
-1.5
Heat flow (W/g)
99.1°C
137.9°C
0
50
100
150
200
250
300
Exo Up
Temperature (°C)


FIG. 23

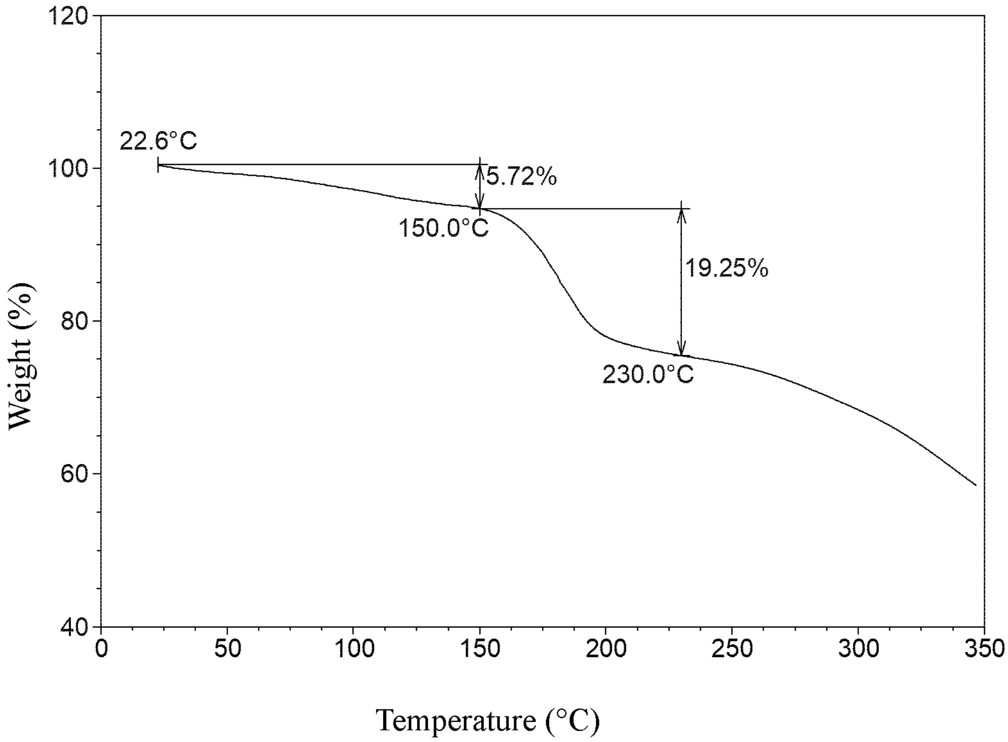

Weight (%)
120
100
80
60
40
22.6°C
5.72%
150.0°C
19.25%
230.0°C
0
50
100
150
200
250
300
350
Temperature (°C)


FIG. 24

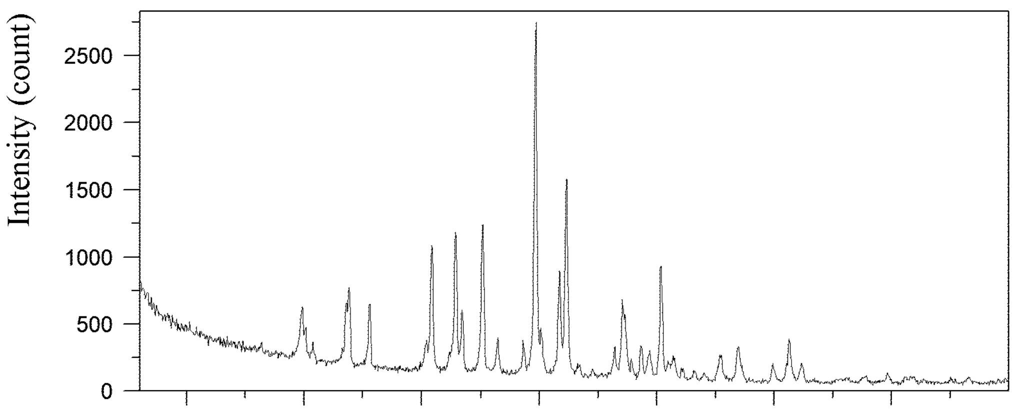

Intensity (count)
2500
2000
1500
1000
500
0
5
10
15
20
25
30
35
2θ (°)


FIG. 25

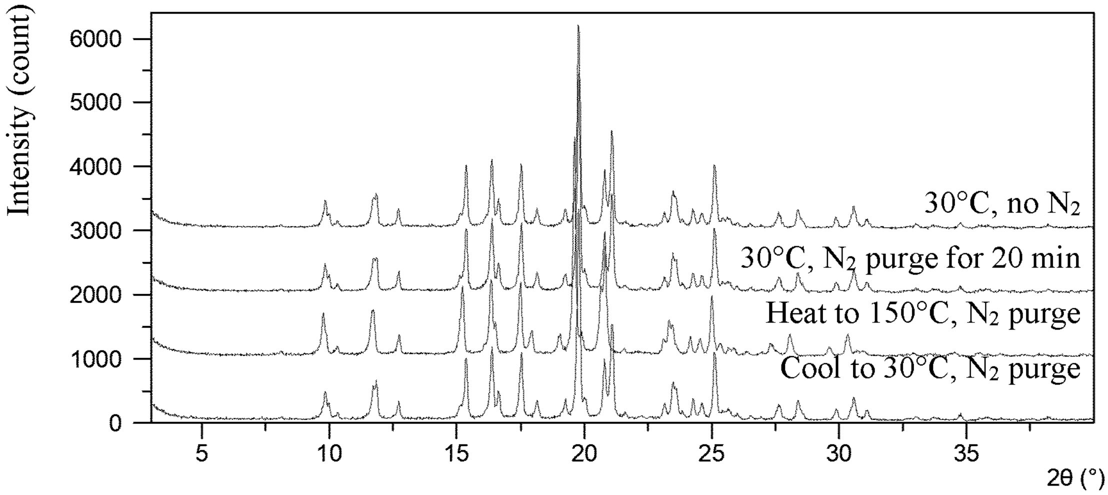
Intensity (count)
6000
5000
4000
3000
2000
1000
0
5
10
15
20
25
30
35
2θ (°)
30°C, no N2
30°C, N2 purge for 20 min
Heat to 150°C, N2 purge
Cool to 30°C, N2 purge

FIG. 26

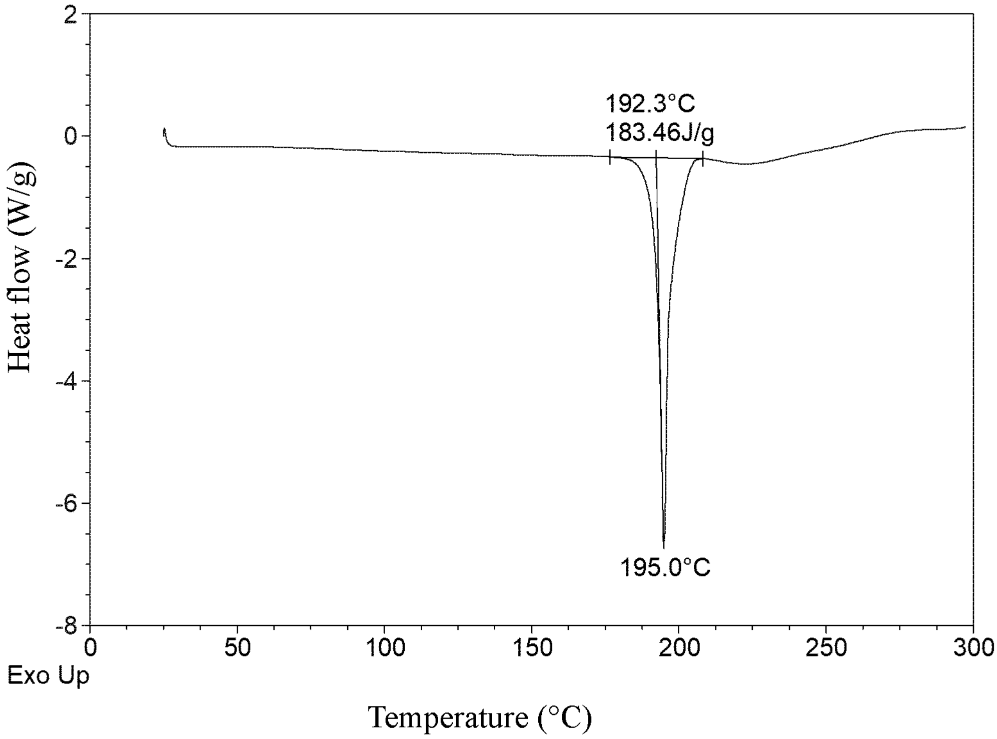
2
0
-2
-4
-6
-8
Heat flow (W/g)
192.3°C
183.46J/g
195.0°C
0
50
100
150
200
250
300
Exo Up
Temperature (°C)

FIG. 27

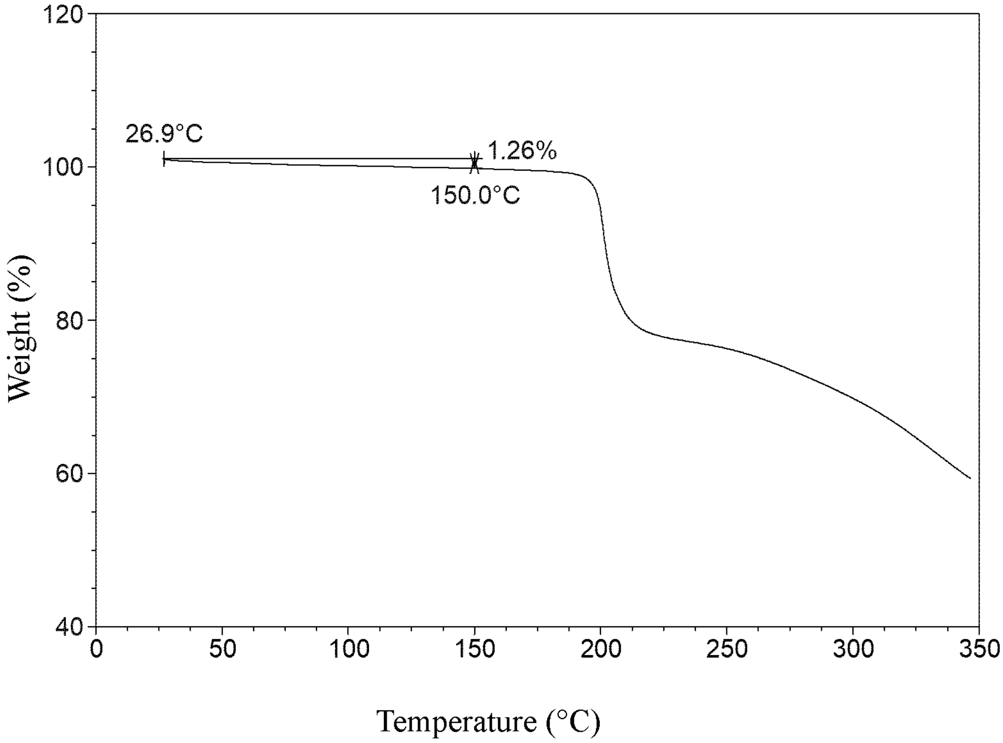
120
100
80
60
40
Weight (%)
26.9°C
1.26%
150.0°C
0
50
100
150
200
250
300
350
Temperature (°C)

FIG. 28

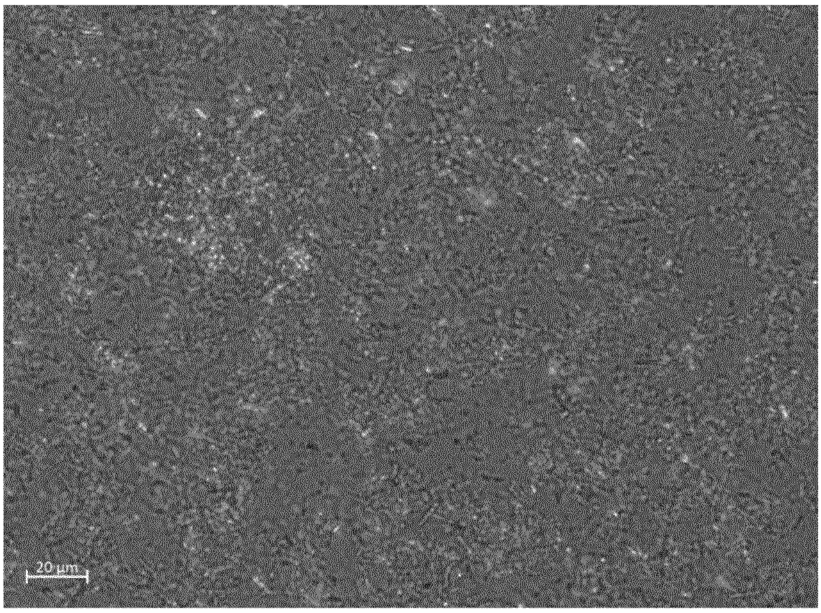
20 µm

FIG. 29

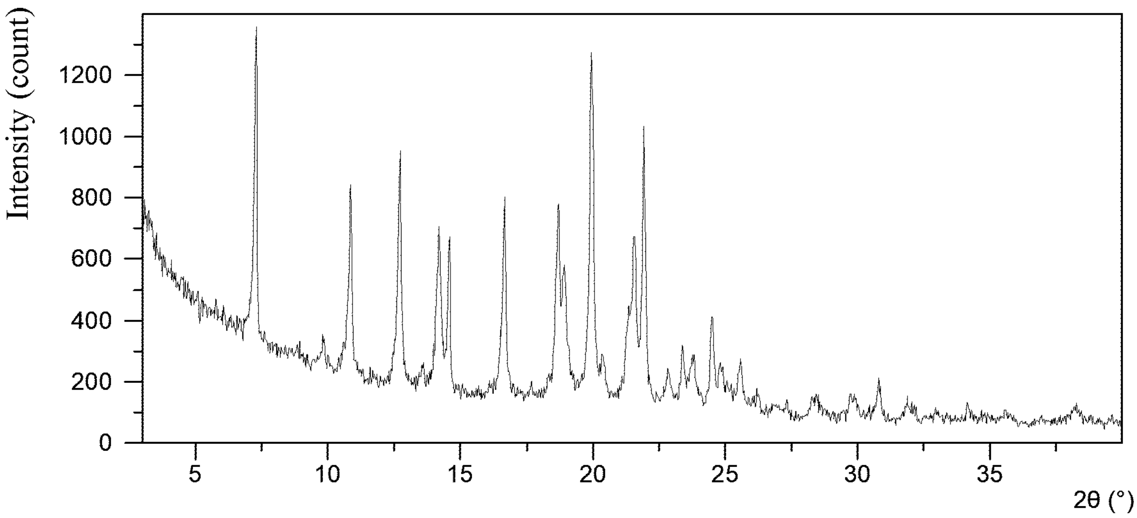
Intensity (count)
1200
1000
800
600
400
200
0
5
10
15
20
25
30
35
2θ (°)

FIG. 30

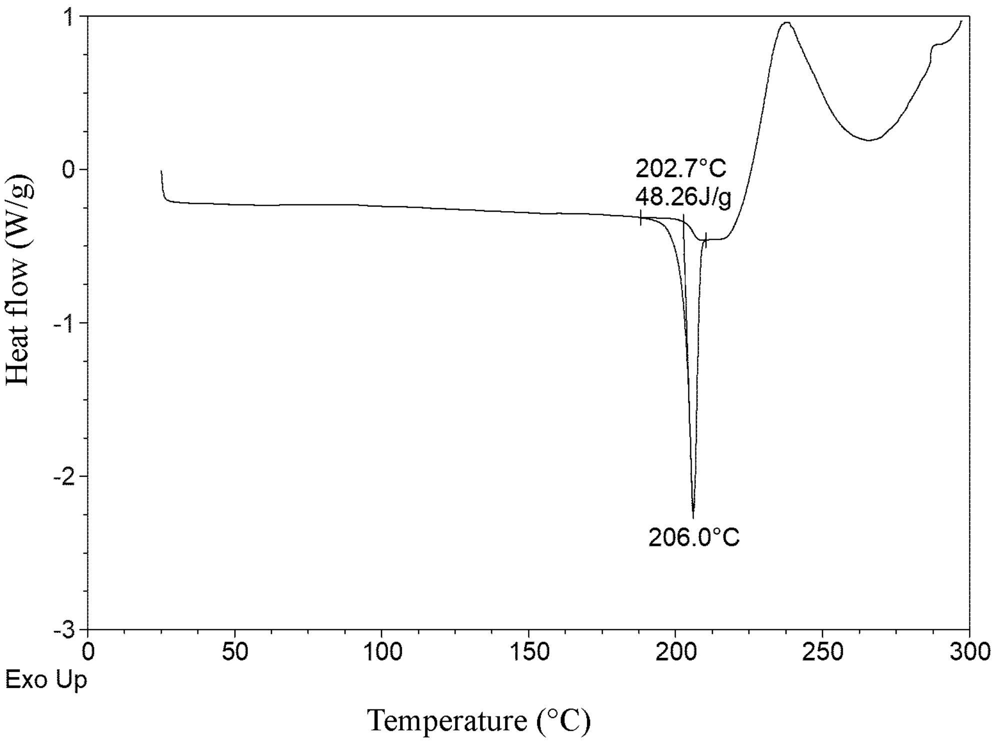
1
0
-1
-2
-3
Heat flow (W/g)
202.7°C
48.26J/g
206.0°C
0
50
100
150
200
250
300
Exo Up
Temperature (°C)

FIG. 31

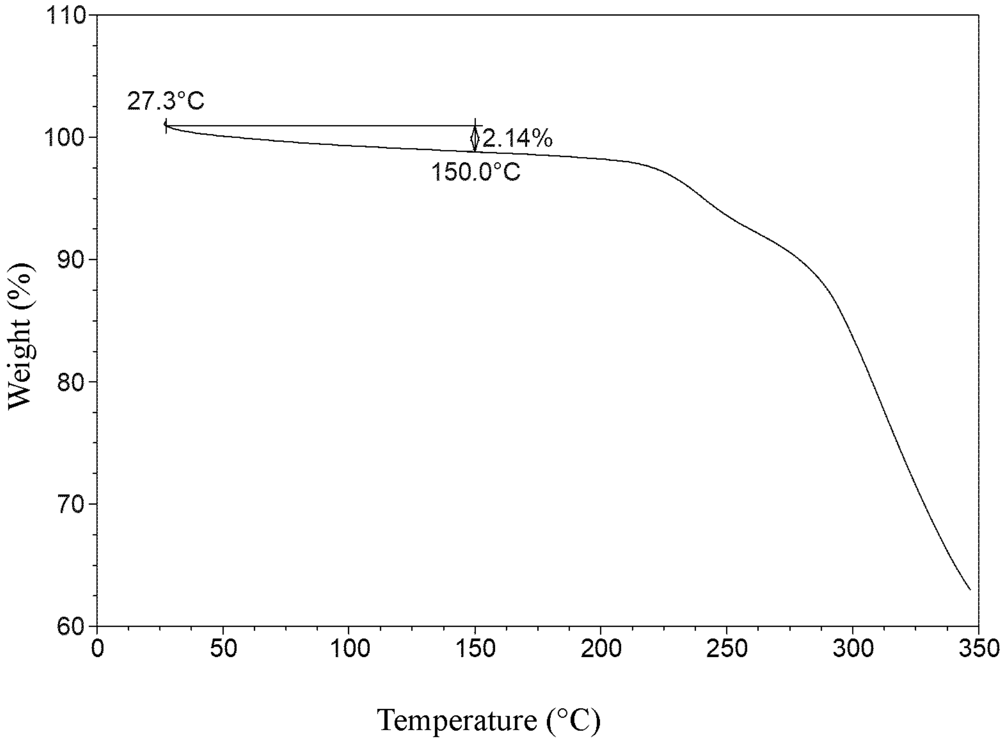
110
100
90
80
70
60
Weight (%)
27.3°C
2.14%
150.0°C
0
50
100
150
200
250
300
350
Temperature (°C)

FIG. 32

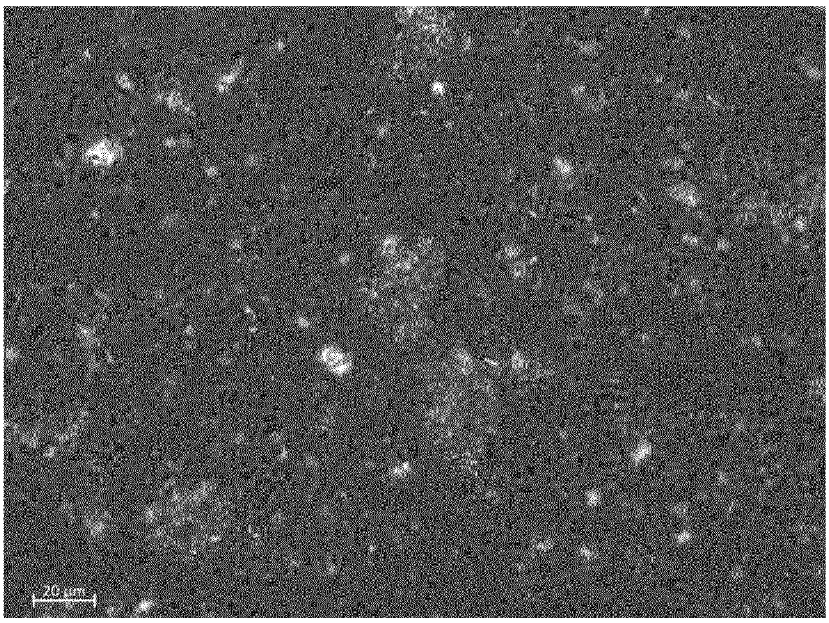
20 µm

FIG. 33

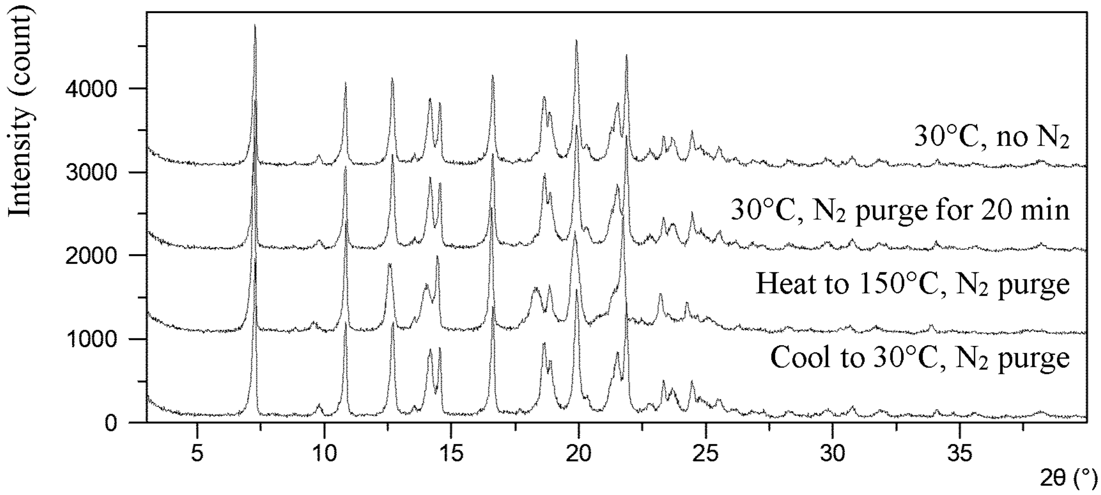


FIG. 34

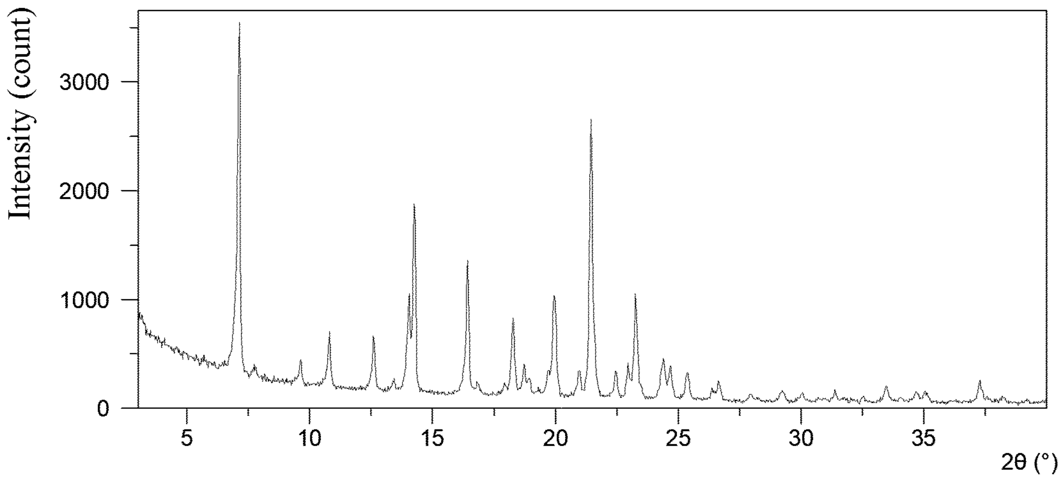


FIG. 35

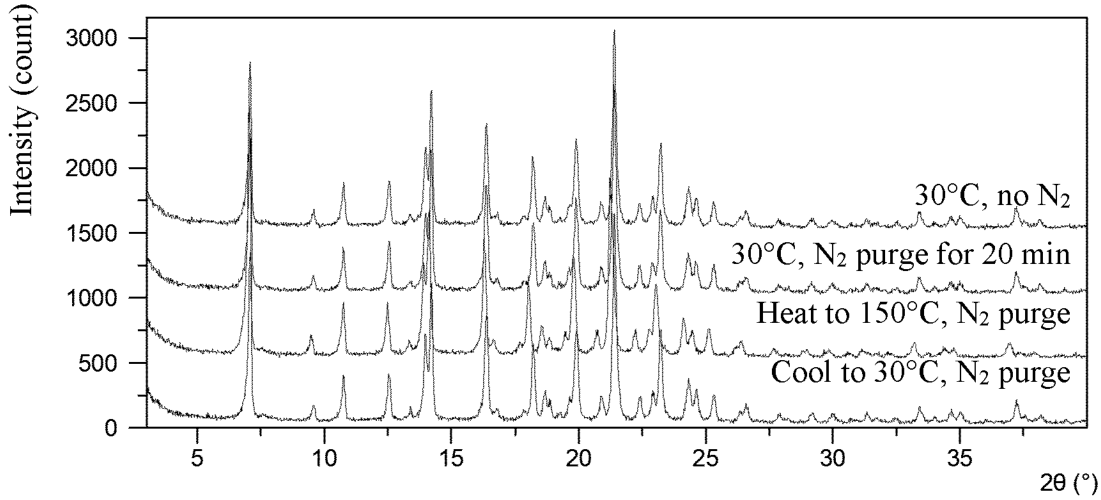


FIG. 36

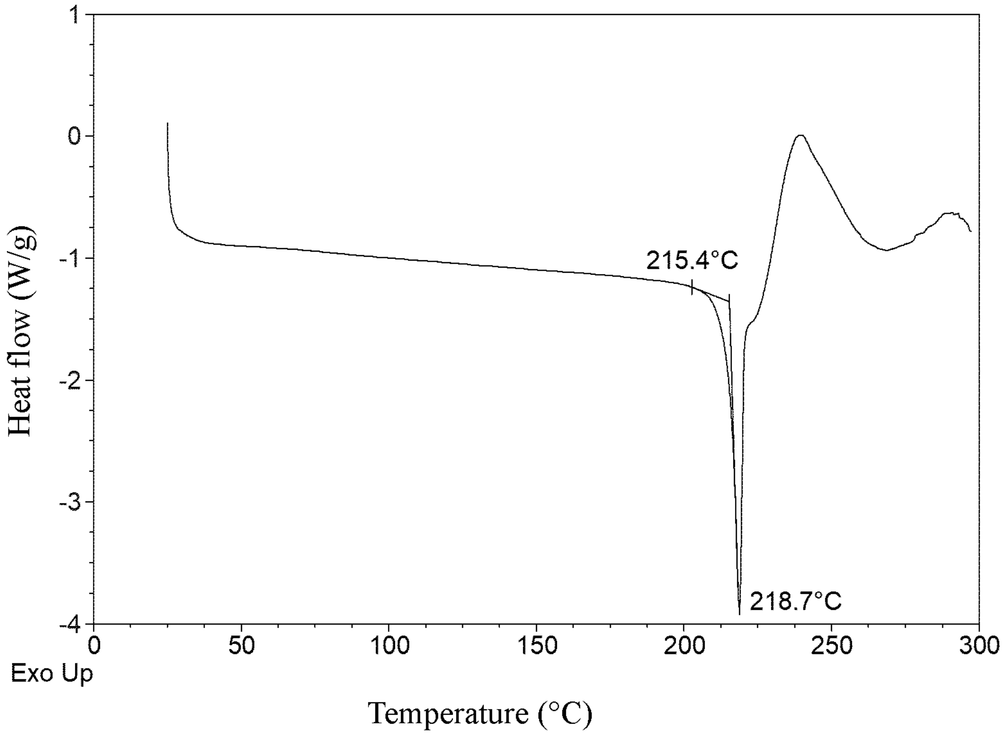

Heat flow (W/g)
Temperature (°C)
Exo Up
215.4°C
218.7°C
1
0
-1
-2
-3
-4
0
50
100
150
200
250
300


FIG. 37

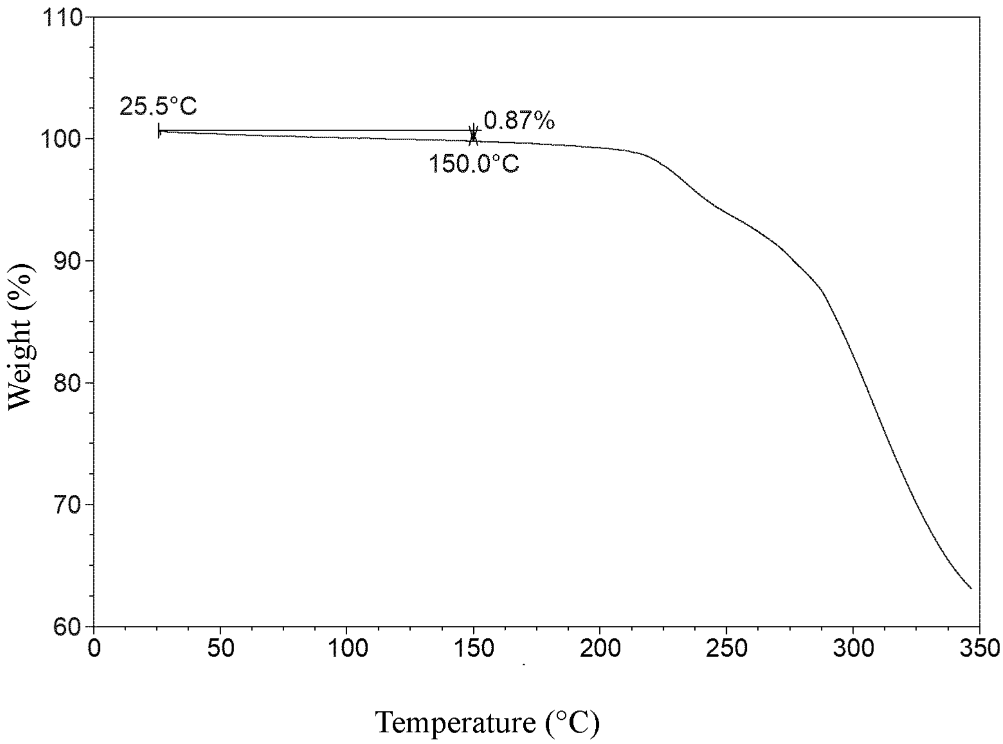

Weight (%)
Temperature (°C)
25.5°C
0.87%
150.0°C
110
100
90
80
70
60
0
50
100
150
200
250
300
350


FIG. 38

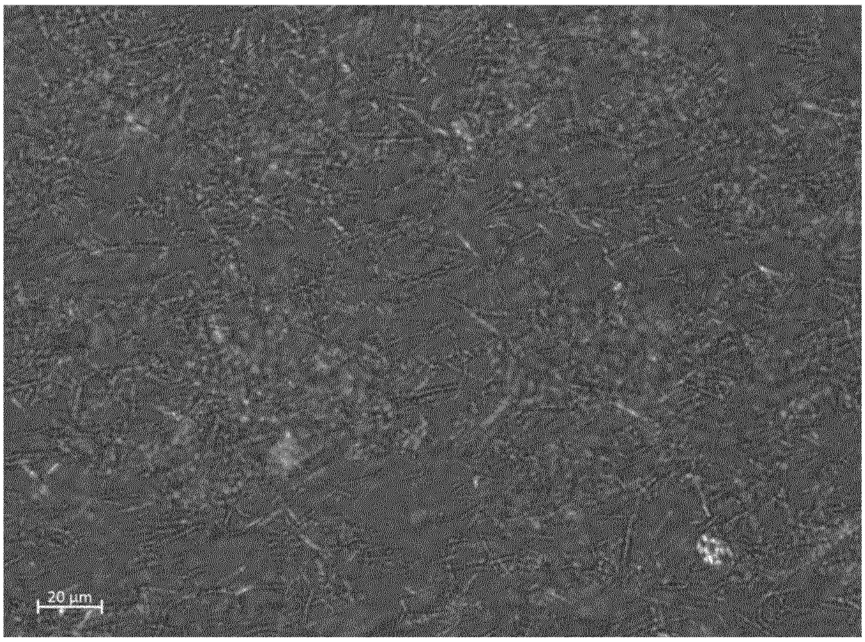
20 µm

FIG. 39

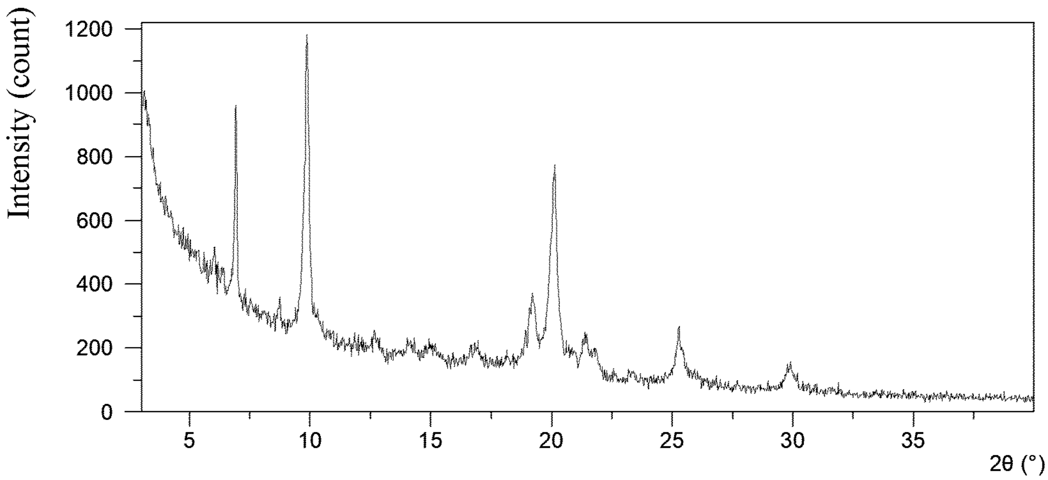
Intensity (count)
1200
1000
800
600
400
200
0
5
10
15
20
25
30
35
2θ (°)

FIG. 40

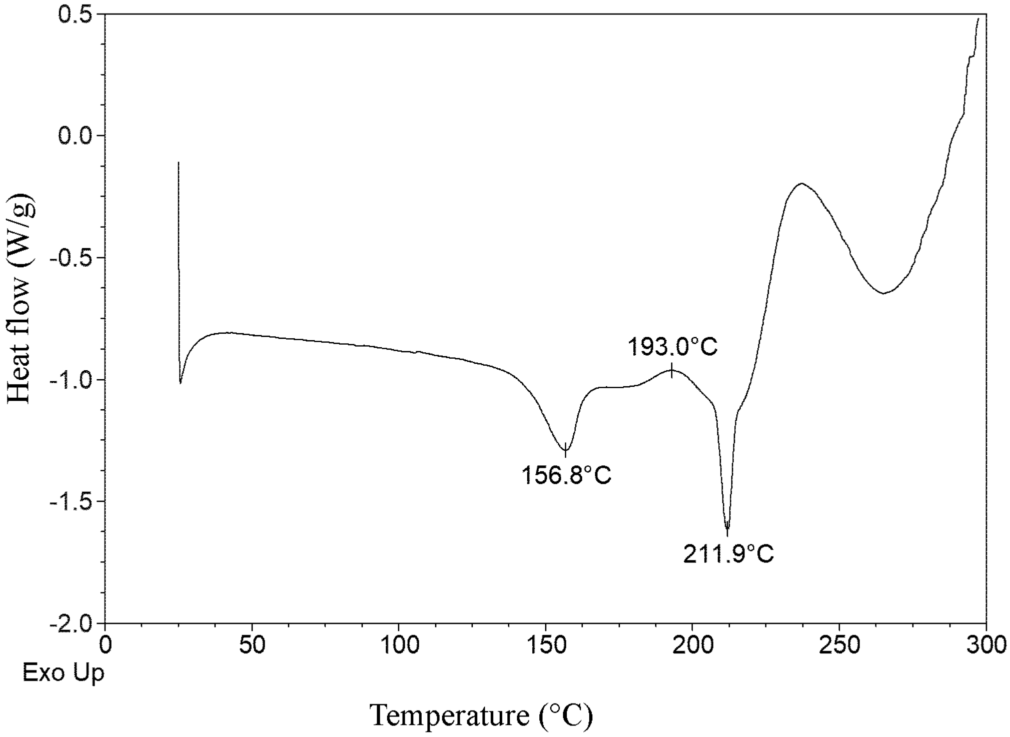
0.5
0.0
-0.5
-1.0
-1.5
-2.0
Heat flow (W/g)
193.0°C
156.8°C
211.9°C
0
50
100
150
200
250
300
Exo Up
Temperature (°C)

FIG. 41

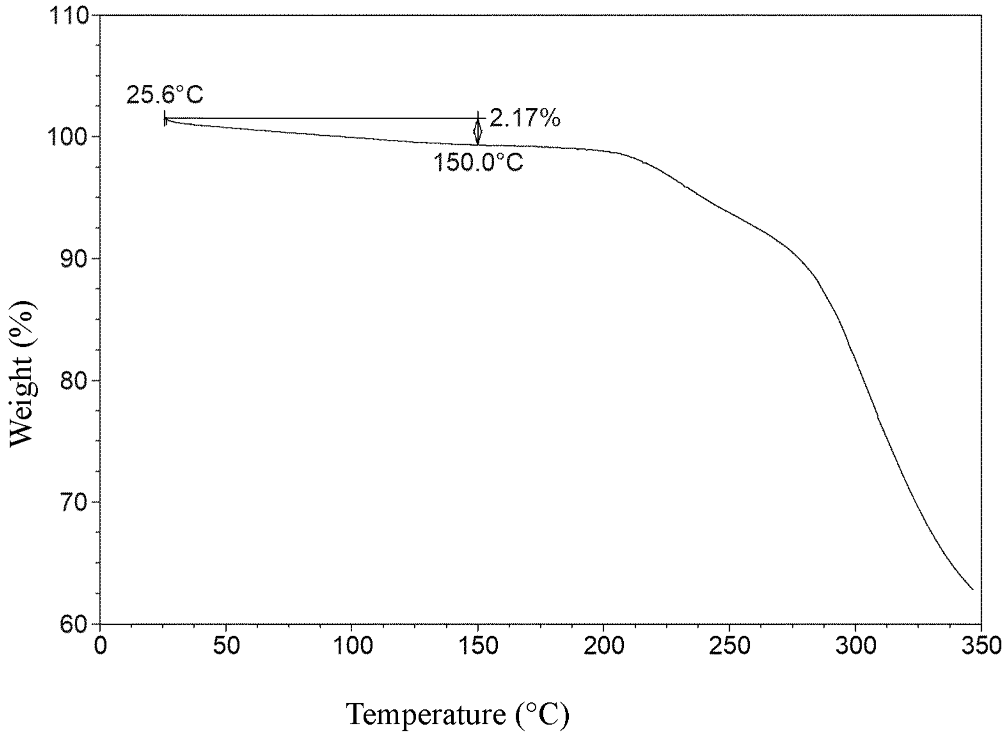
110
100
90
80
70
60
Weight (%)
25.6°C
2.17%
150.0°C
0
50
100
150
200
250
300
350
Temperature (°C)

FIG. 42

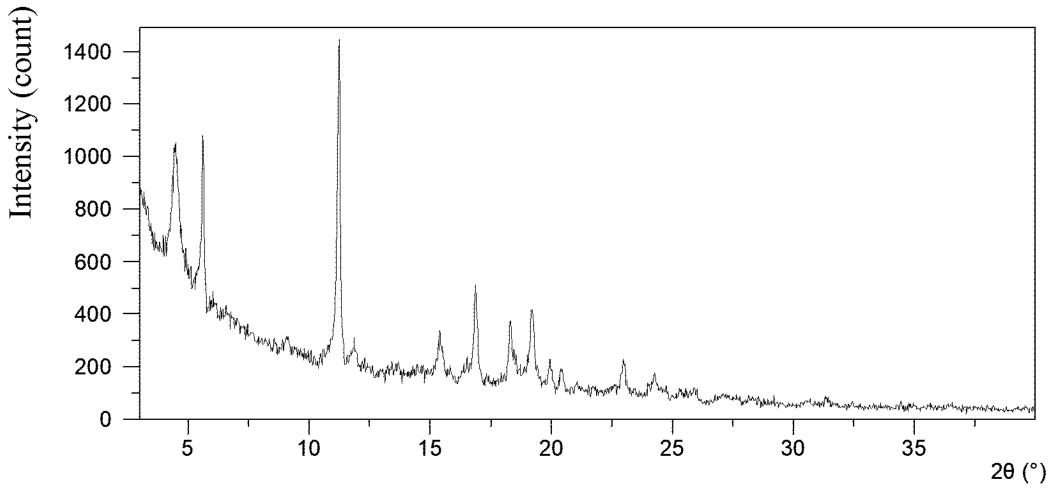
Intensity (count)
1400
1200
1000
800
600
400
200
0
5
10
15
20
25
30
35
2θ (°)

FIG. 43

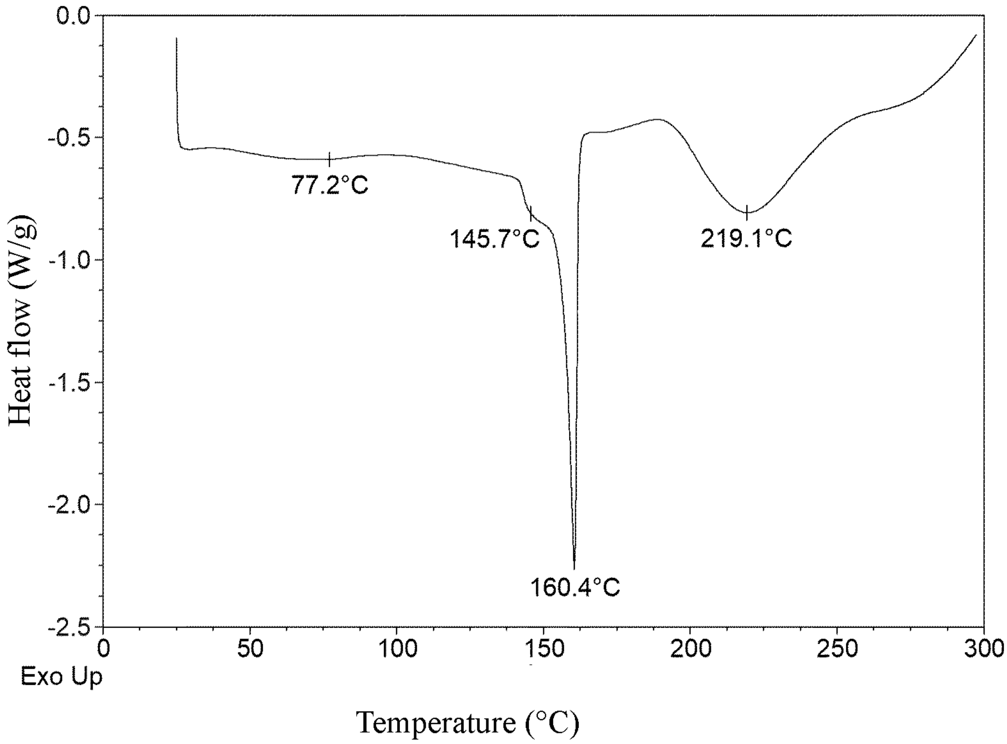
0.0
-0.5
-1.0
-1.5
-2.0
-2.5
Heat flow (W/g)
77.2°C
145.7°C
219.1°C
160.4°C
0
50
100
150
200
250
300
Exo Up
Temperature (°C)

FIG. 44

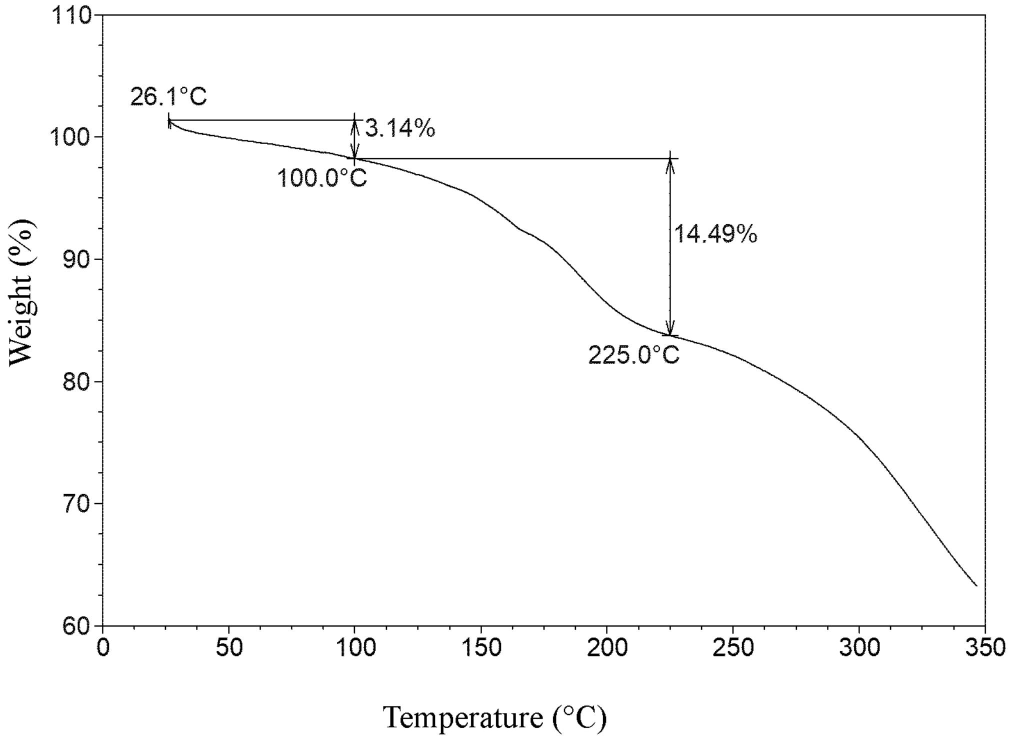
110
100
90
80
70
60
Weight (%)
26.1°C
3.14%
100.0°C
14.49%
225.0°C
0
50
100
150
200
250
300
350
Temperature (°C)

FIG. 45

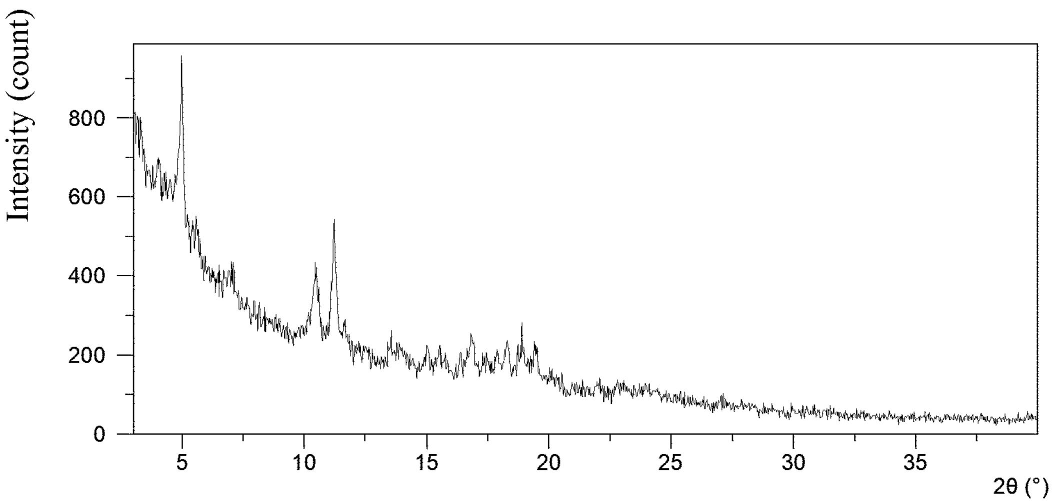
Intensity (count)
800
600
400
200
0
5
10
15
20
25
30
35
2θ (°)

FIG. 46

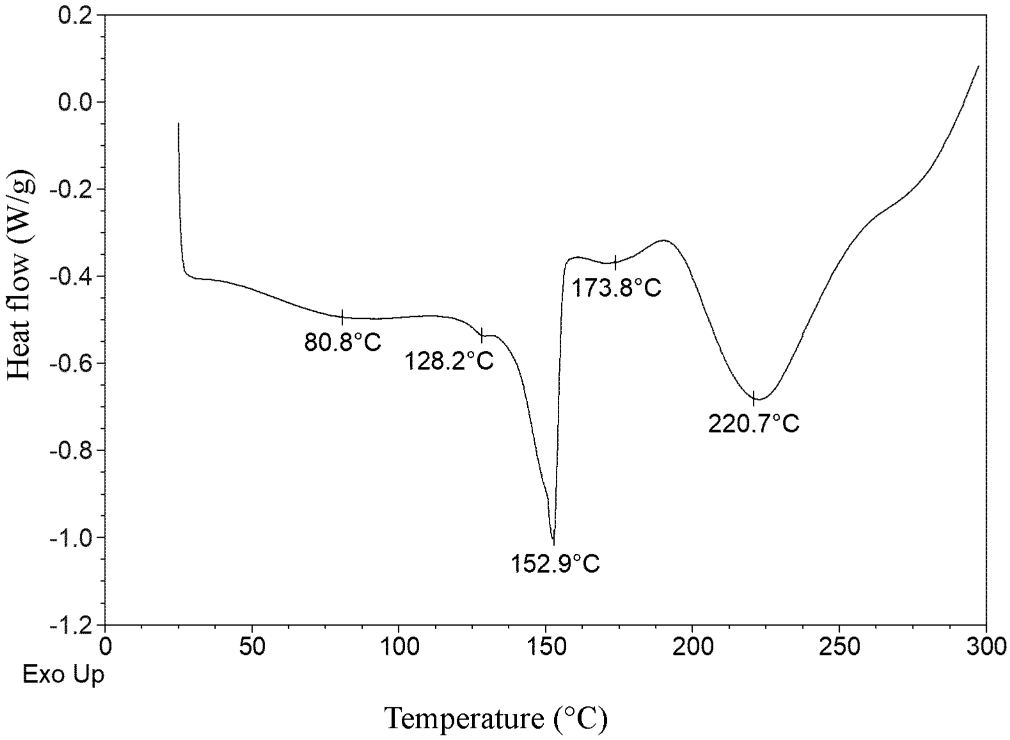
0.2
0.0
-0.2
-0.4
-0.6
-0.8
-1.0
-1.2
Heat flow (W/g)
80.8°C
128.2°C
152.9°C
173.8°C
220.7°C
0
50
100
150
200
250
300
Exo Up
Temperature (°C)

FIG. 47

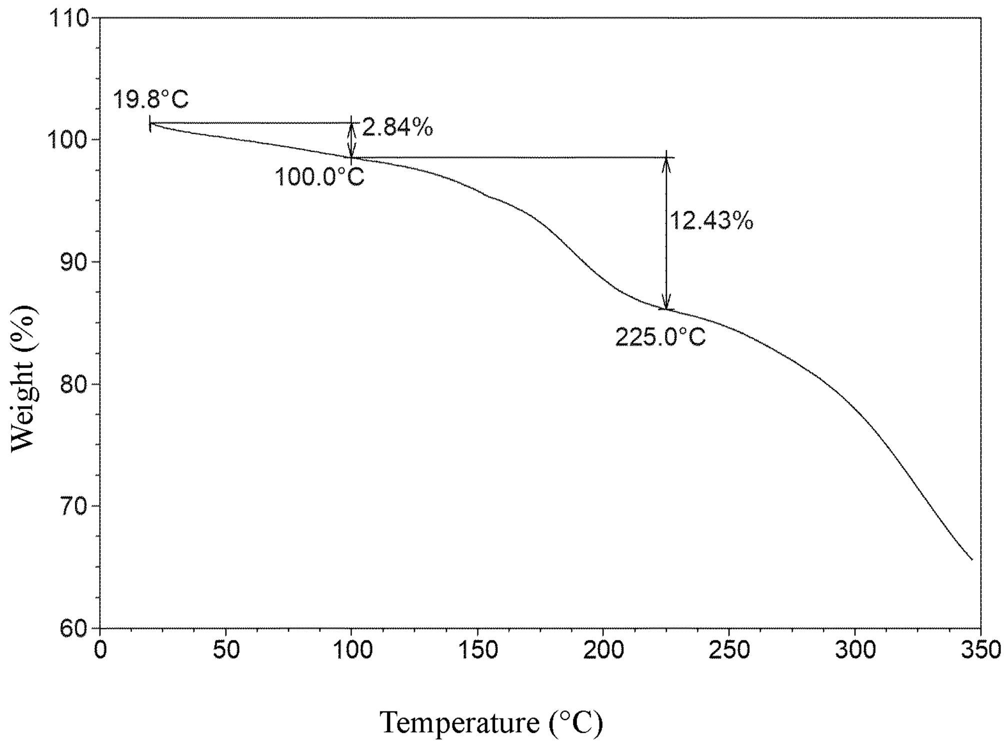
110
100
90
80
70
60
Weight (%)
19.8°C
2.84%
100.0°C
12.43%
225.0°C
0
50
100
150
200
250
300
350
Temperature (°C)

FIG. 48

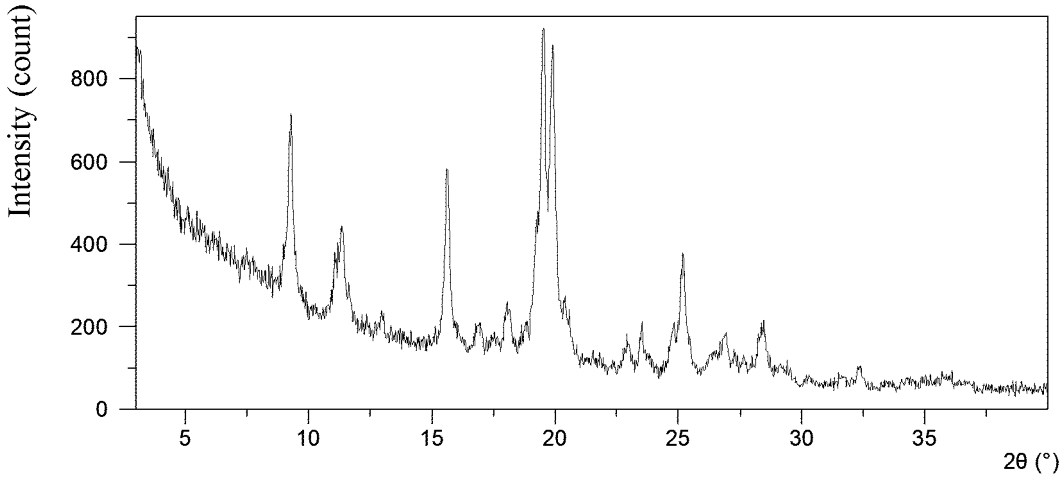
Intensity (count)
800
600
400
200
0
5
10
15
20
25
30
35
2θ (°)

FIG. 49

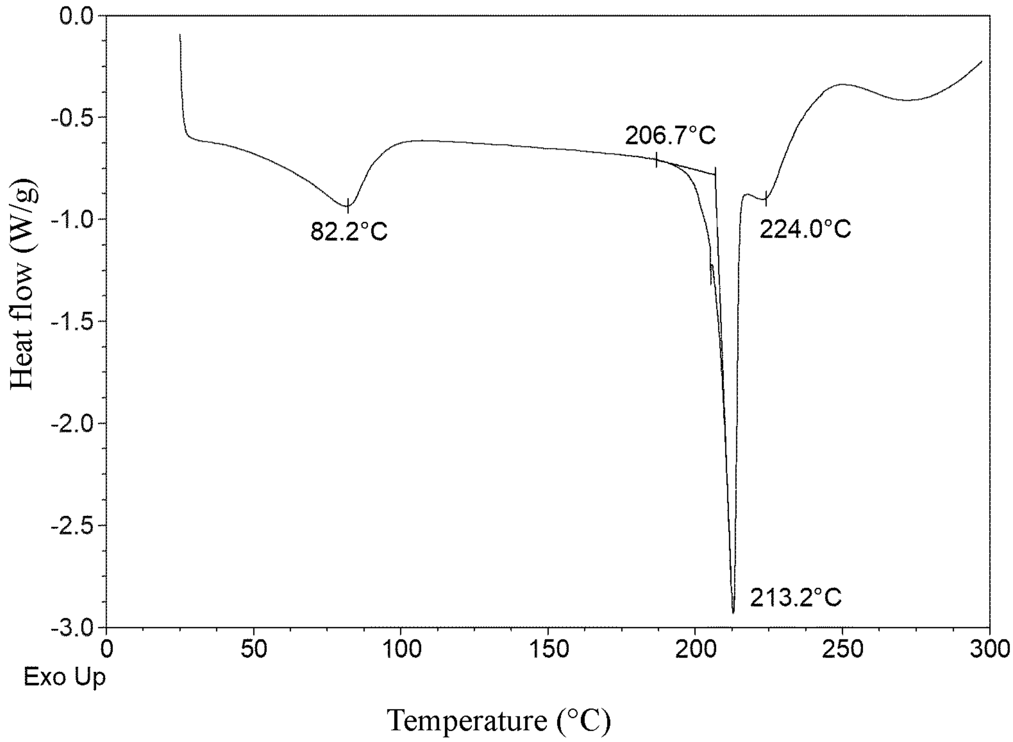
0.0
-0.5
-1.0
-1.5
-2.0
-2.5
-3.0
Heat flow (W/g)
206.7°C
82.2°C
224.0°C
213.2°C
0
50
100
150
200
250
300
Exo Up
Temperature (°C)

FIG. 50

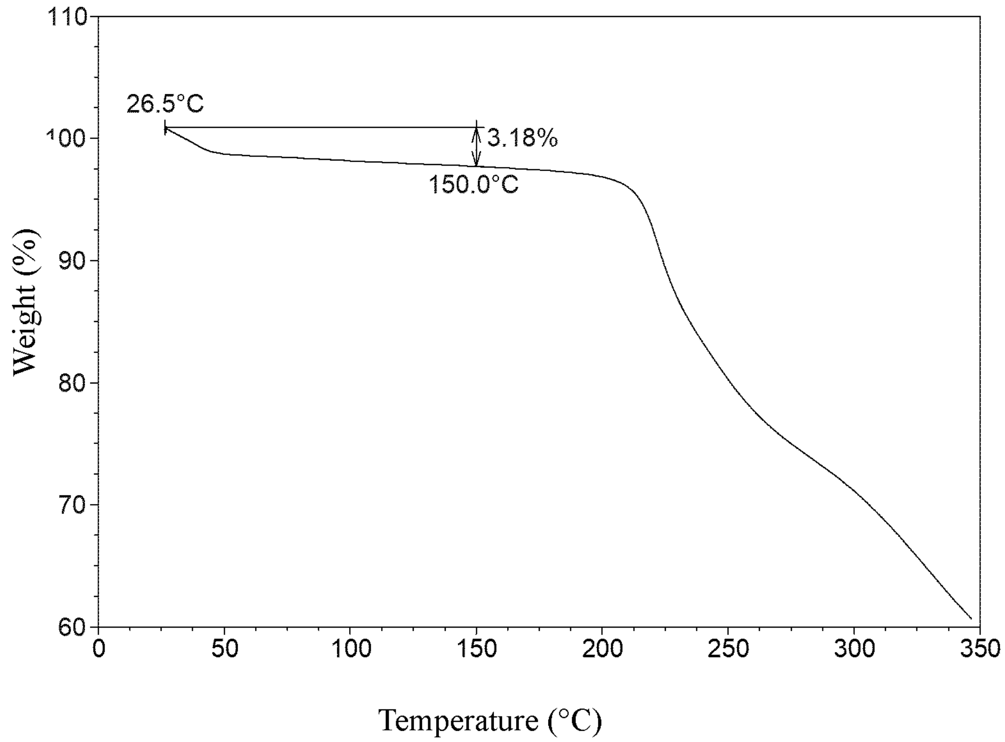
110
100
90
80
70
60
0
50
100
150
200
250
300
350
Weight (%)
Temperature (°C)
26.5°C
3.18%
150.0°C

FIG. 51

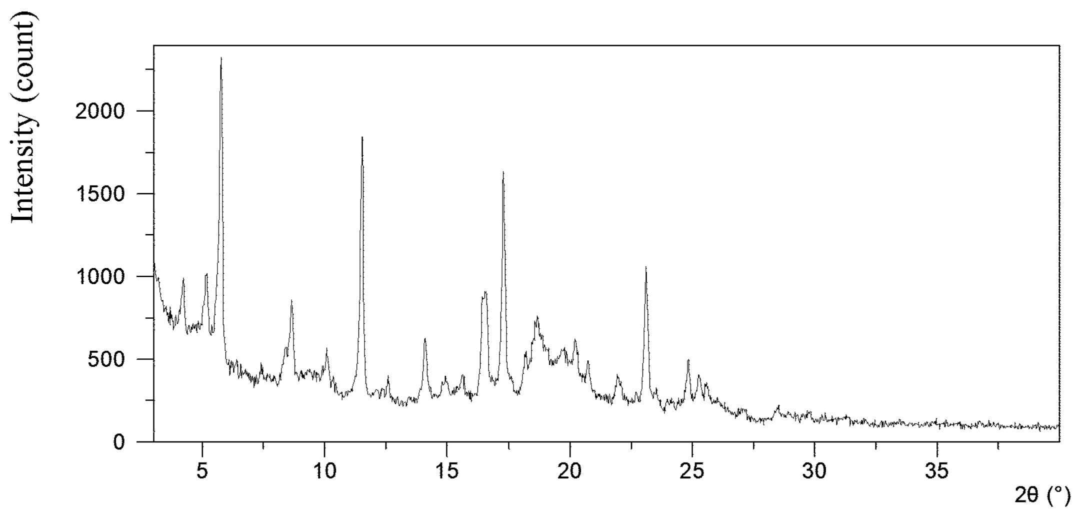
Intensity (count)
2000
1500
1000
500
0
5
10
15
20
25
30
35
2θ (°)

FIG. 52

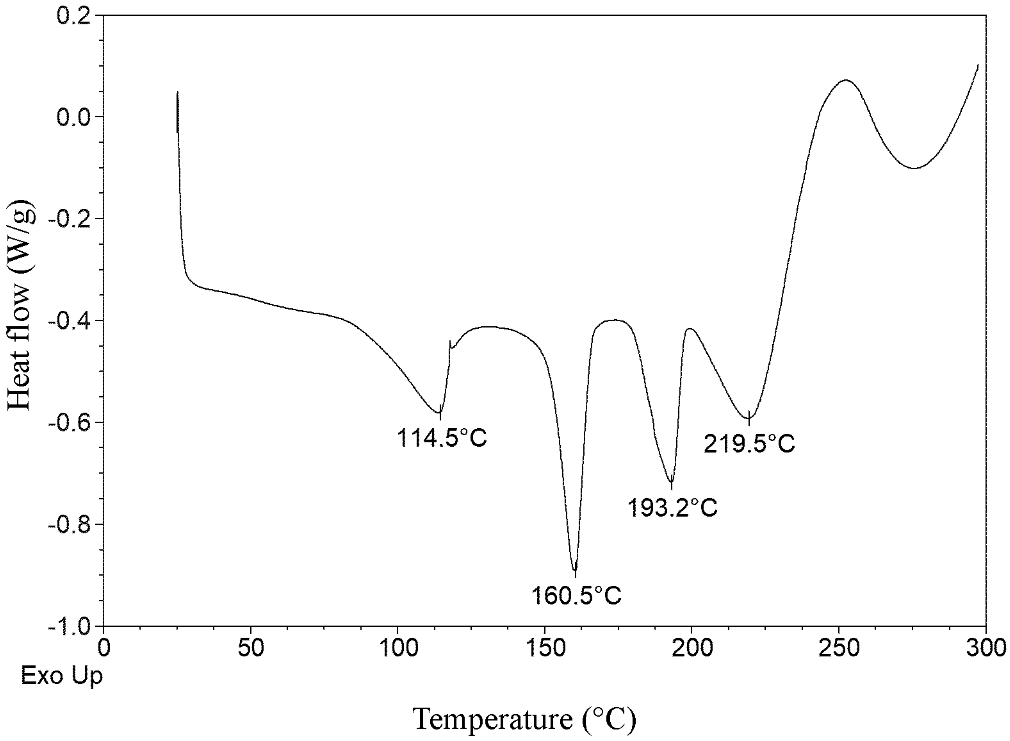
0.2
0.0
-0.2
-0.4
-0.6
-0.8
-1.0
Heat flow (W/g)
114.5°C
160.5°C
193.2°C
219.5°C
0
50
100
150
200
250
300
Exo Up
Temperature (°C)

FIG. 53

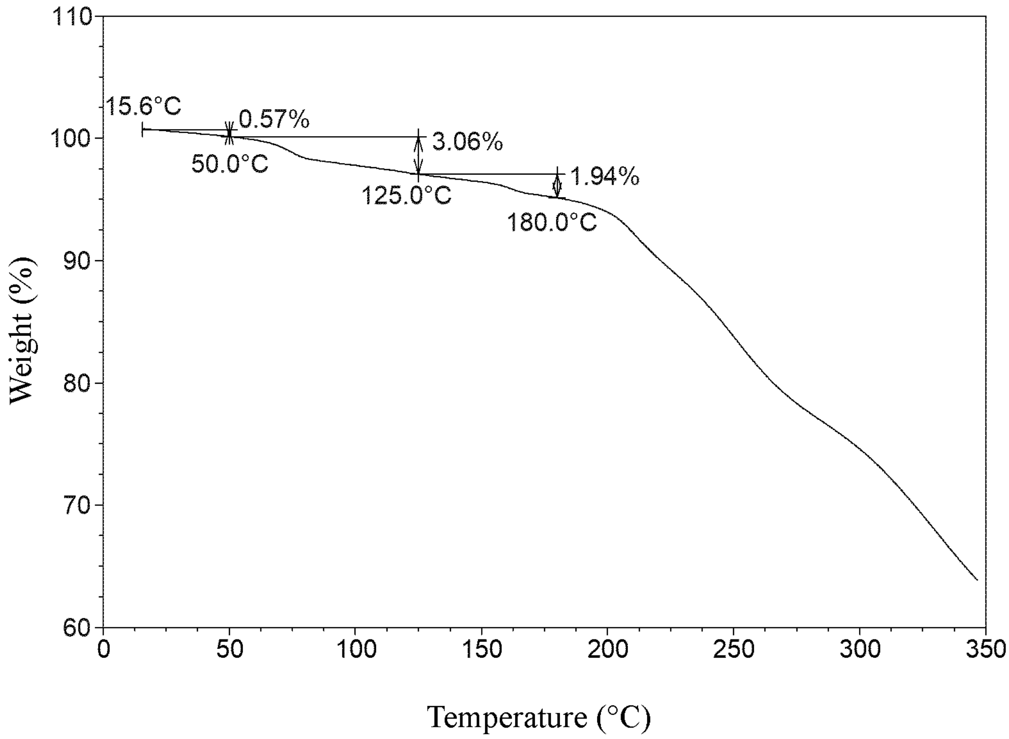
110
100
90
80
70
60
Weight (%)
15.6°C
0.57%
50.0°C
3.06%
125.0°C
1.94%
180.0°C
0
50
100
150
200
250
300
350
Temperature (°C)

FIG. 54

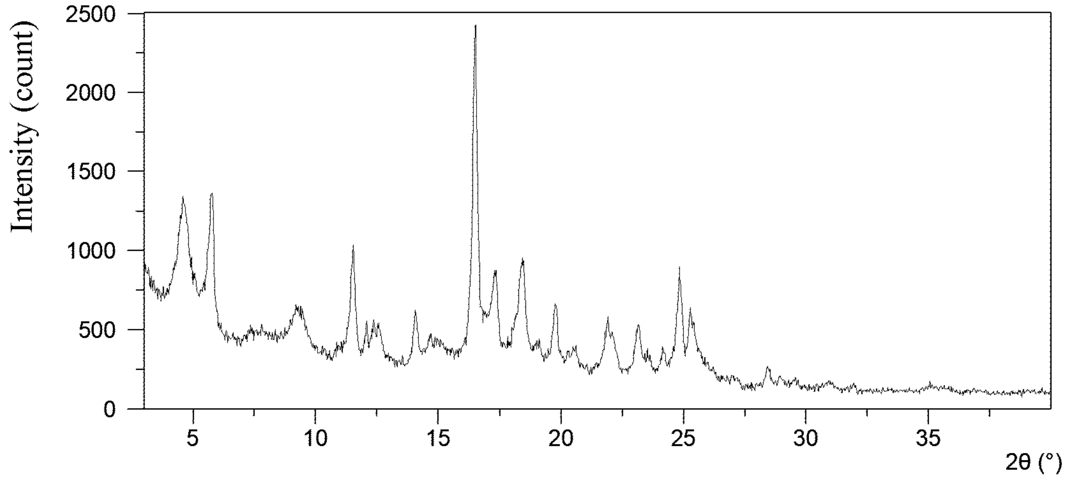
Intensity (count)
2500
2000
1500
1000
500
0
5
10
15
20
25
30
35
2θ (°)

FIG. 55

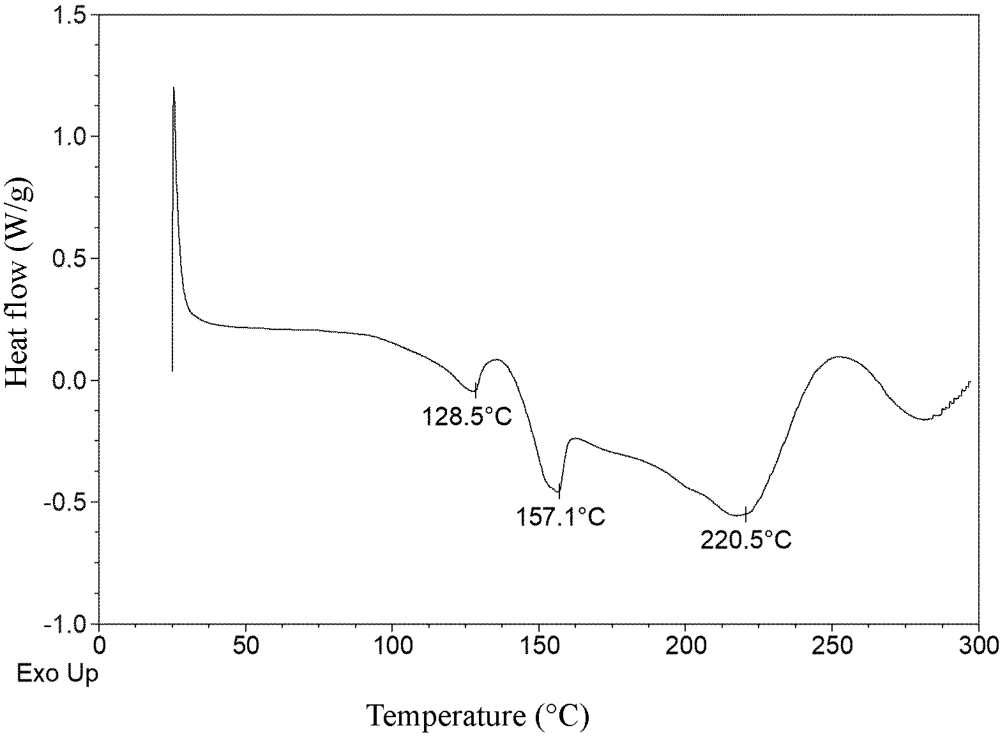
Heat flow (W/g)
1.5
1.0
0.5
0.0
-0.5
-1.0
0
50
100
150
200
250
300
Exo Up
128.5°C
157.1°C
220.5°C
Temperature (°C)

FIG. 56

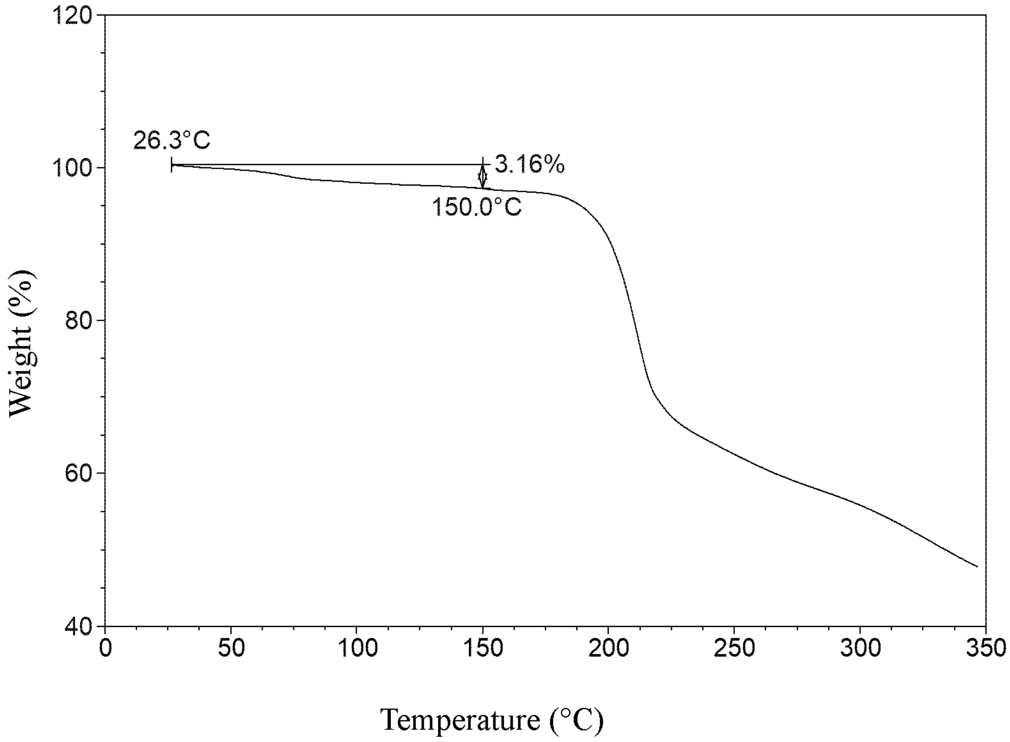
120
100
80
60
40
Weight (%)
26.3°C
3.16%
150.0°C
0
50
100
150
200
250
300
350
Temperature (°C)

FIG. 57

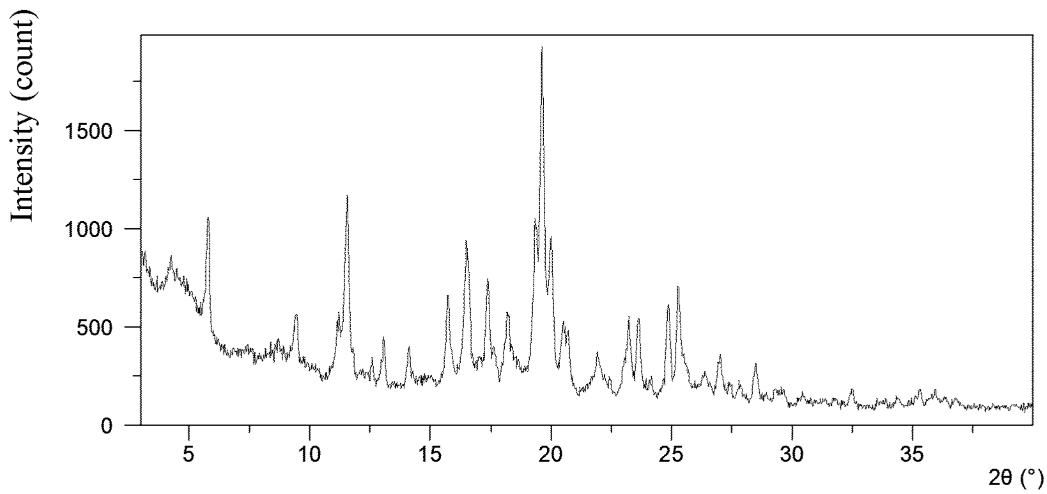
Intensity (count)
1500
1000
500
0
5
10
15
20
25
30
35
2θ (°)

FIG. 58

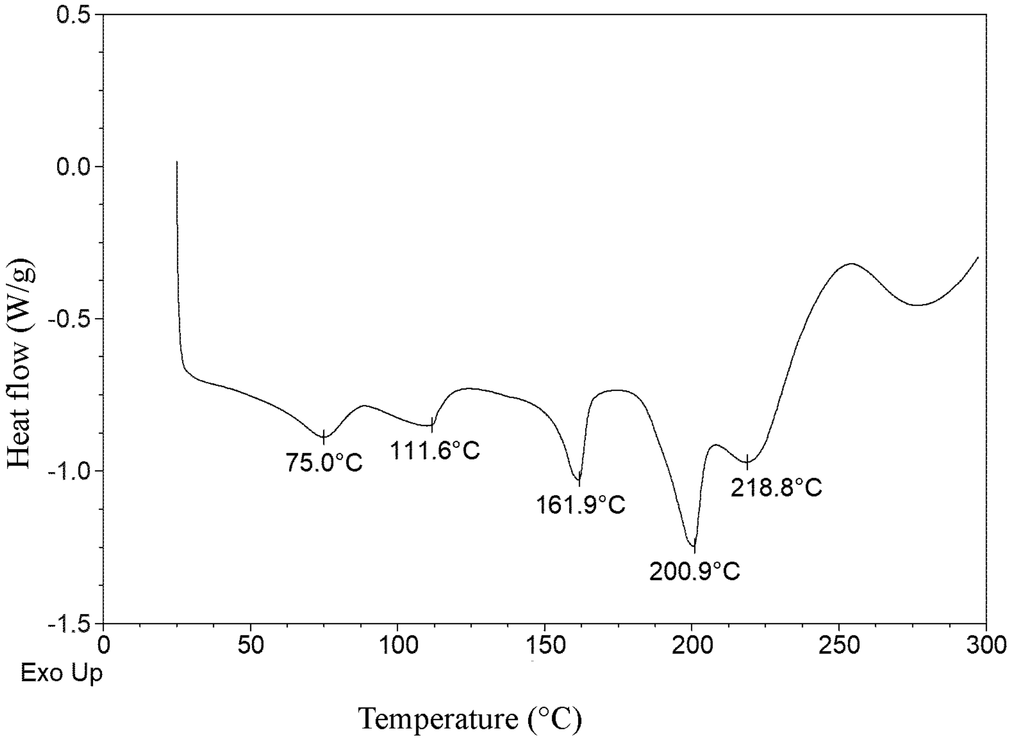
0.5
0.0
-0.5
-1.0
-1.5
Heat flow (W/g)
75.0°C
111.6°C
161.9°C
200.9°C
218.8°C
0
50
100
150
200
250
300
Exo Up
Temperature (°C)

FIG. 59

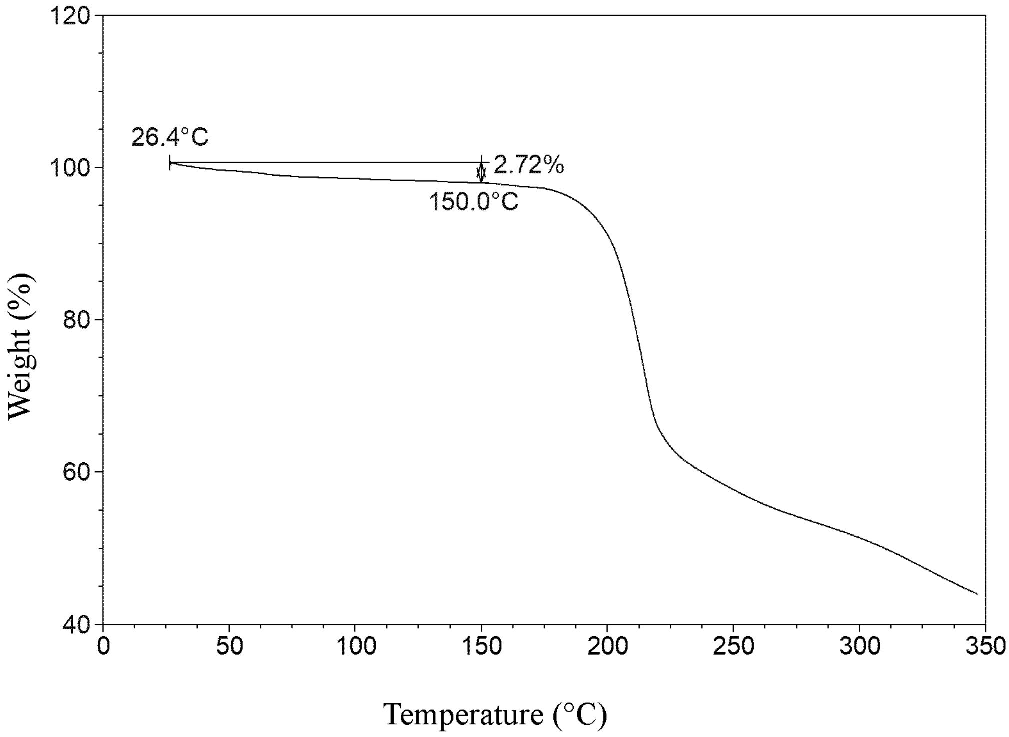
120
100
80
60
40
Weight (%)
26.4°C
2.72%
150.0°C
0
50
100
150
200
250
300
350
Temperature (°C)

FIG. 60

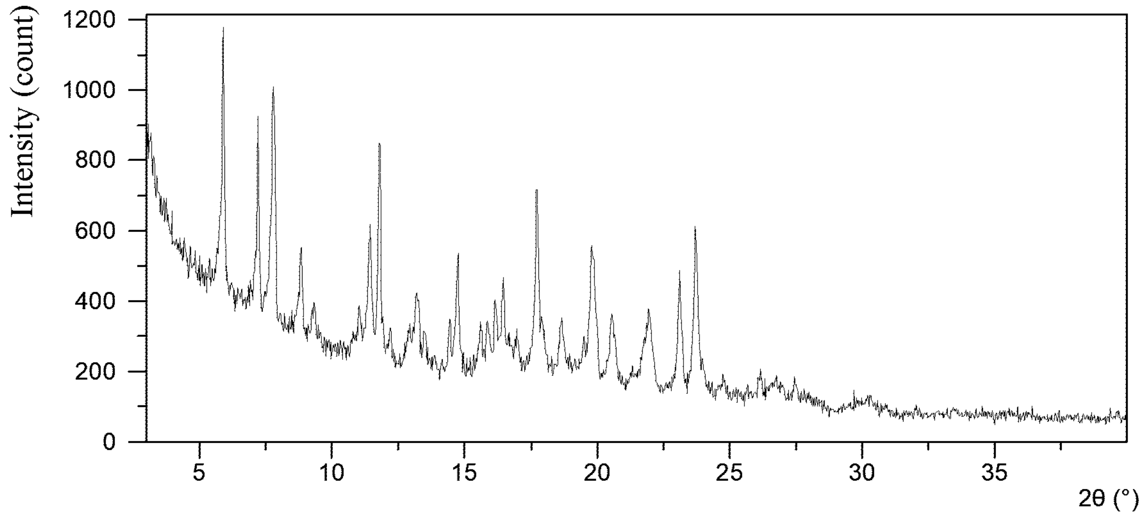
Intensity (count)
1200
1000
800
600
400
200
0
5
10
15
20
25
30
35
2θ (°)

FIG. 61

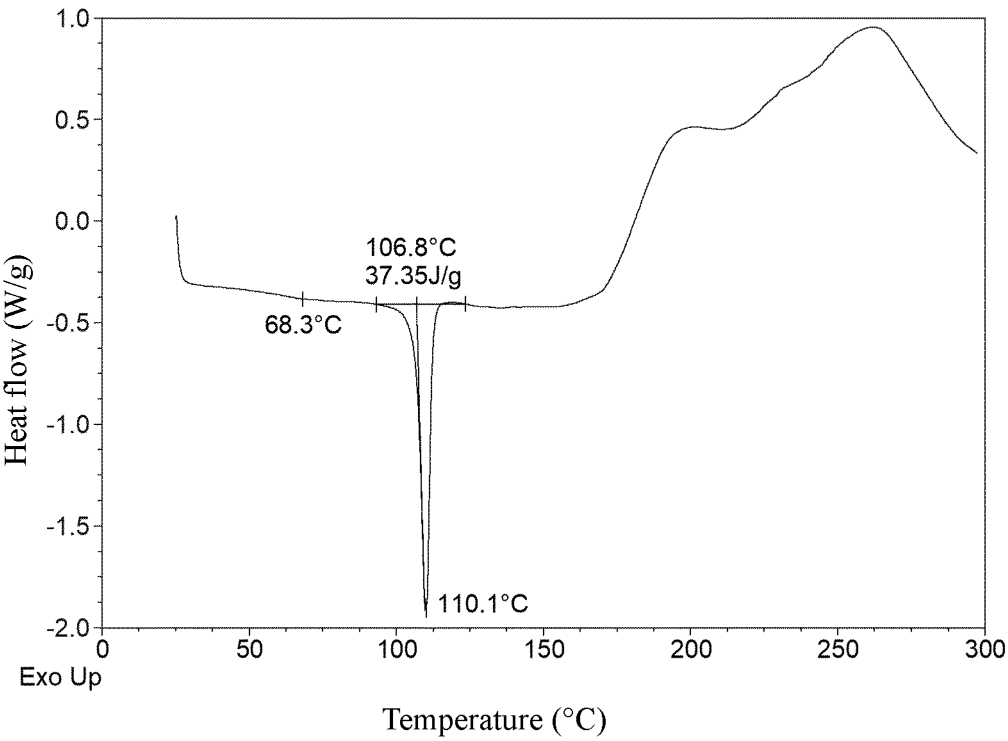
1.0
0.5
0.0
-0.5
-1.0
-1.5
-2.0
Heat flow (W/g)
106.8°C
37.35J/g
68.3°C
110.1°C
0
50
100
150
200
250
300
Exo Up
Temperature (°C)

FIG. 62

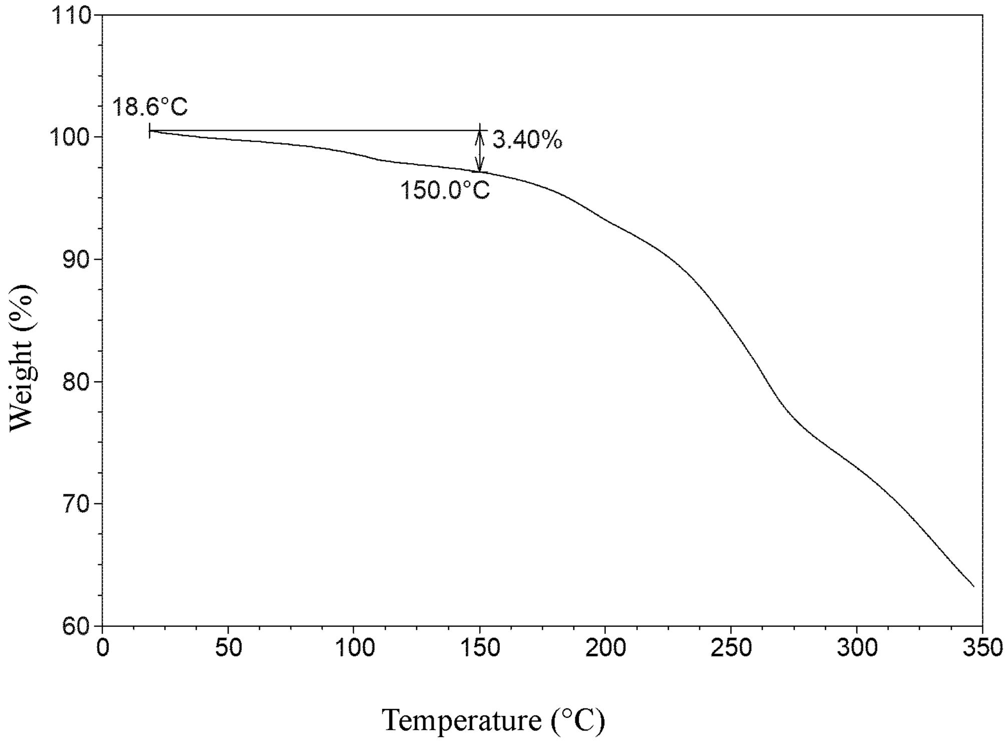
110
100
90
80
70
60
Weight (%)
18.6°C
3.40%
150.0°C
0
50
100
150
200
250
300
350
Temperature (°C)

FIG. 63

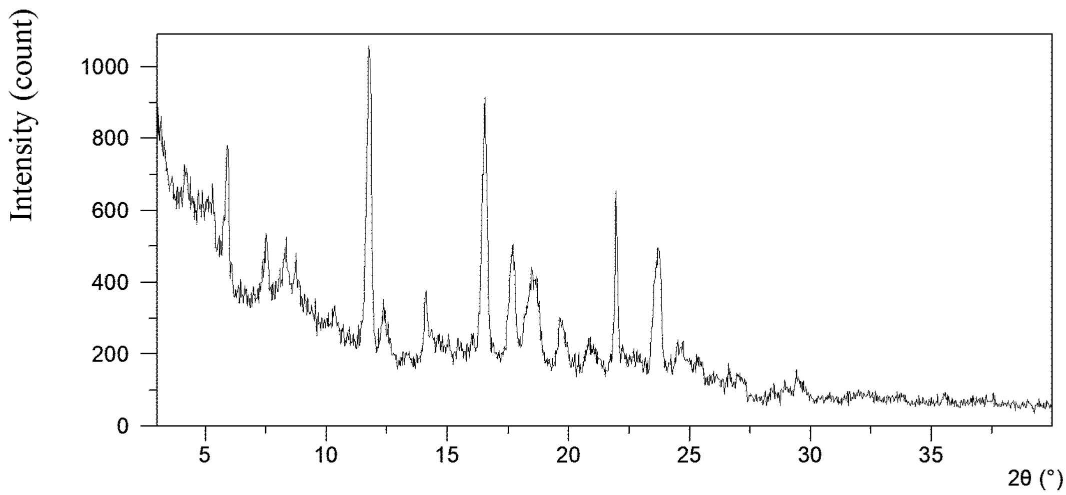
Intensity (count)
1000
800
600
400
200
0
5
10
15
20
25
30
35
2θ (°)

FIG. 64

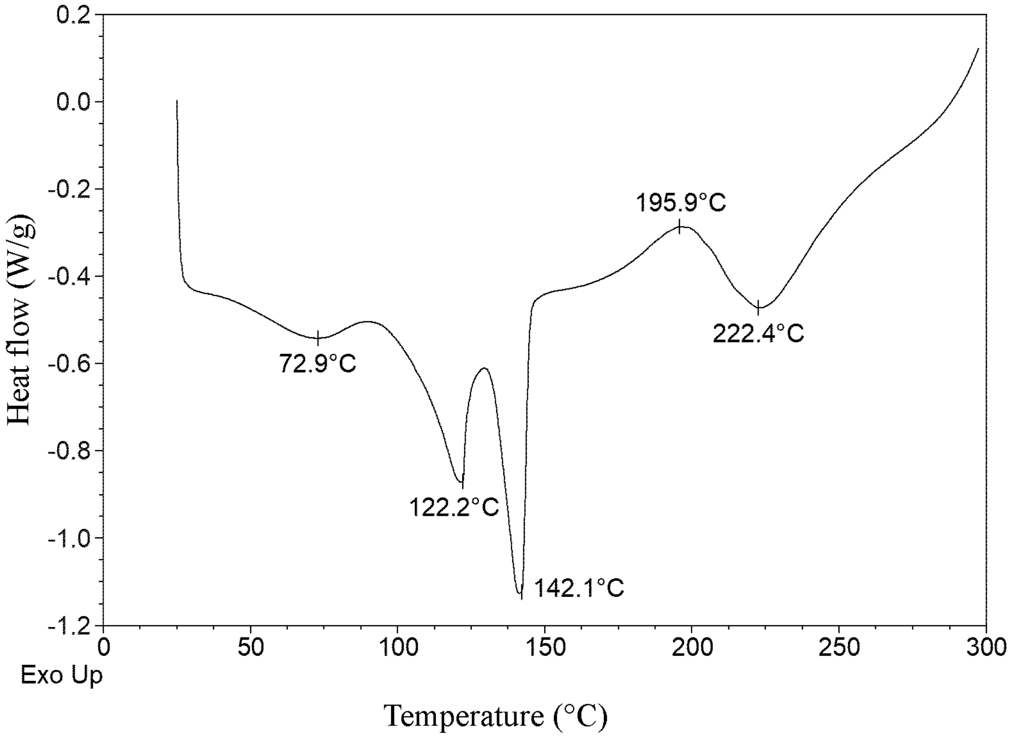
0.2
0.0
-0.2
-0.4
-0.6
-0.8
-1.0
-1.2
Heat flow (W/g)
195.9°C
222.4°C
72.9°C
122.2°C
142.1°C
0
50
100
150
200
250
300
Exo Up
Temperature (°C)

FIG. 65

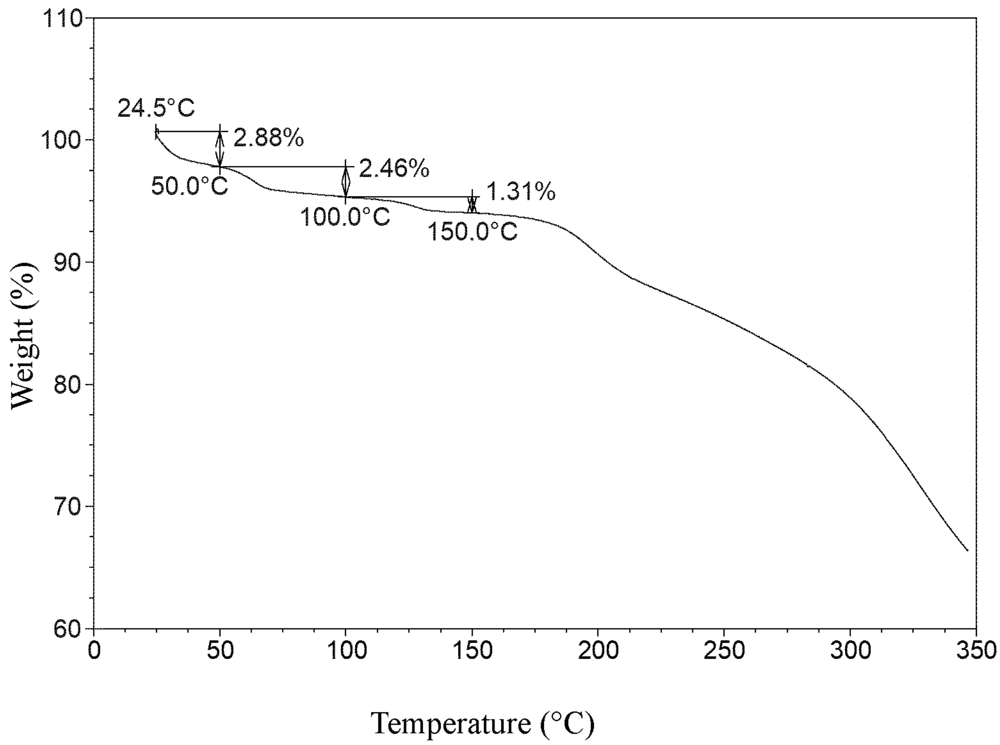
110
100
90
80
70
60
Weight (%)
24.5°C
2.88%
50.0°C
2.46%
100.0°C
1.31%
150.0°C
0
50
100
150
200
250
300
350
Temperature (°C)

FIG. 66

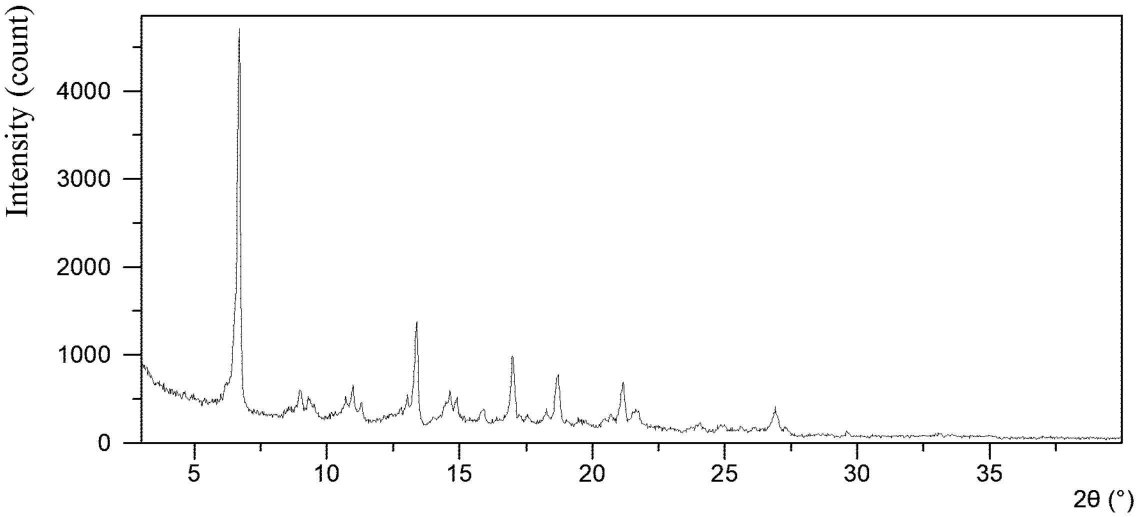
Intensity (count)
4000
3000
2000
1000
0
5
10
15
20
25
30
35
2θ (°)

FIG. 67

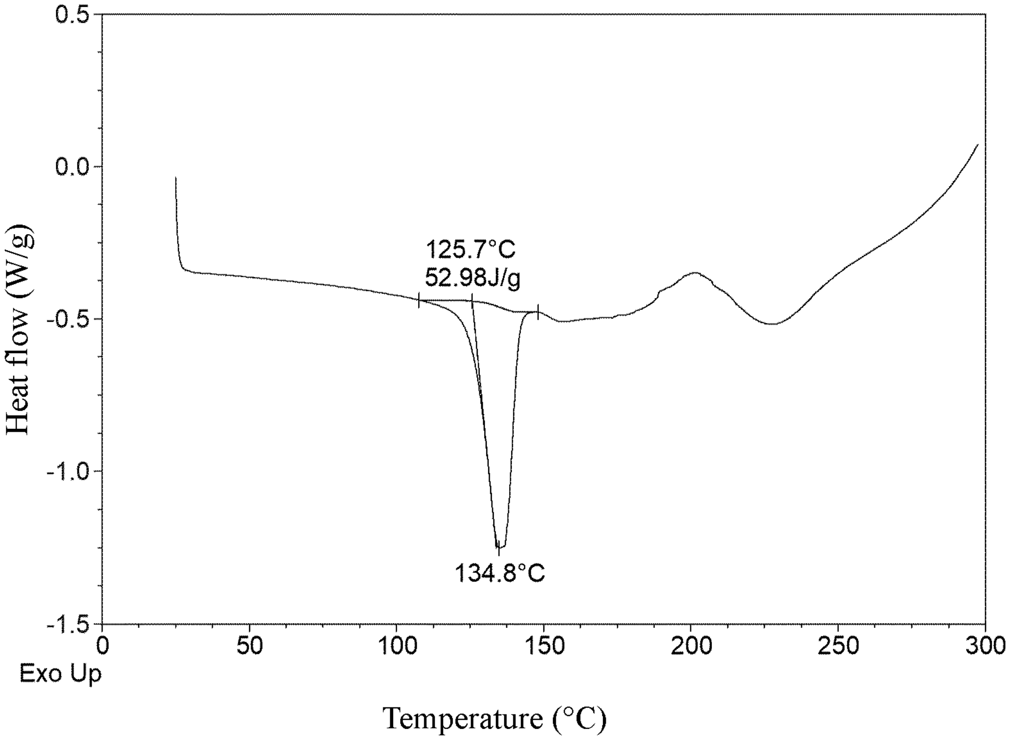
0.5
0.0
-0.5
-1.0
-1.5
Heat flow (W/g)
125.7°C
52.98J/g
134.8°C
0
50
100
150
200
250
300
Exo Up
Temperature (°C)

FIG. 68

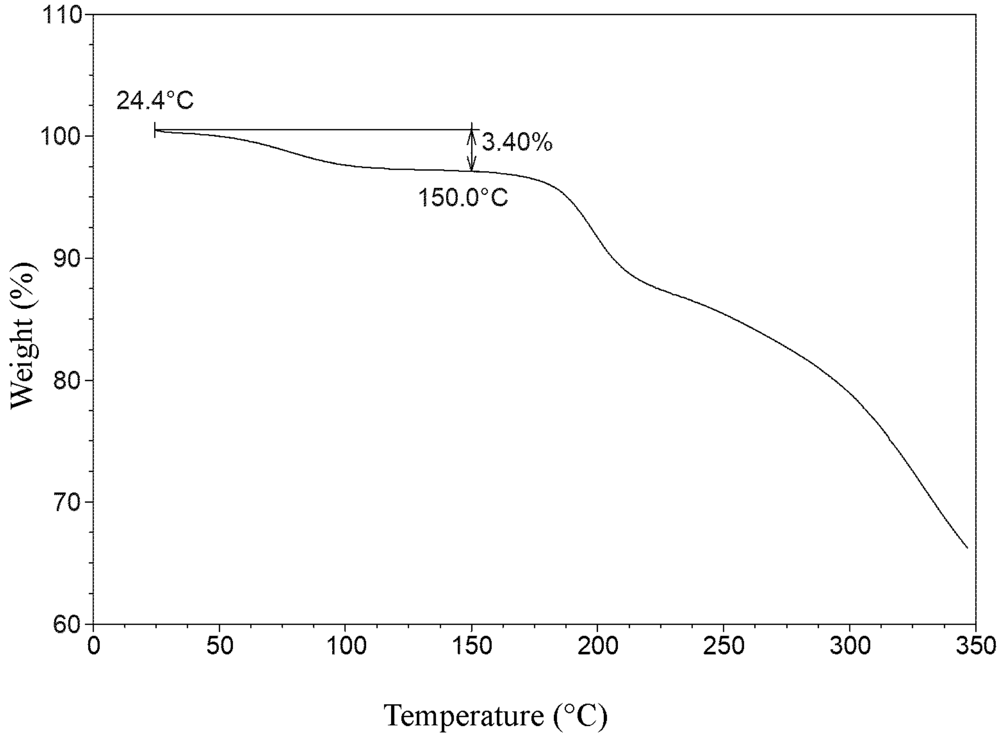
110
100
90
80
70
60
Weight (%)
24.4°C
3.40%
150.0°C
0
50
100
150
200
250
300
350
Temperature (°C)

FIG. 69

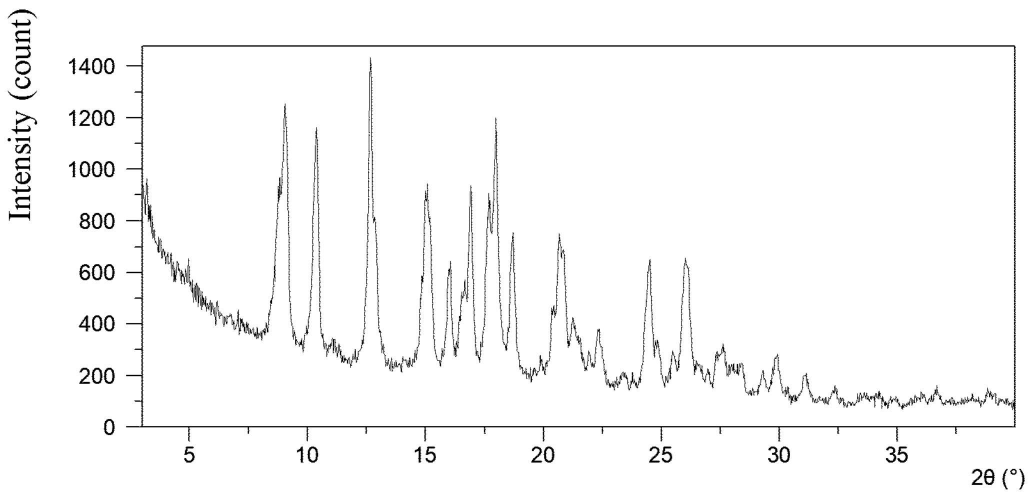
Intensity (count)
1400
1200
1000
800
600
400
200
0
5
10
15
20
25
30
35
2θ (°)

FIG. 70

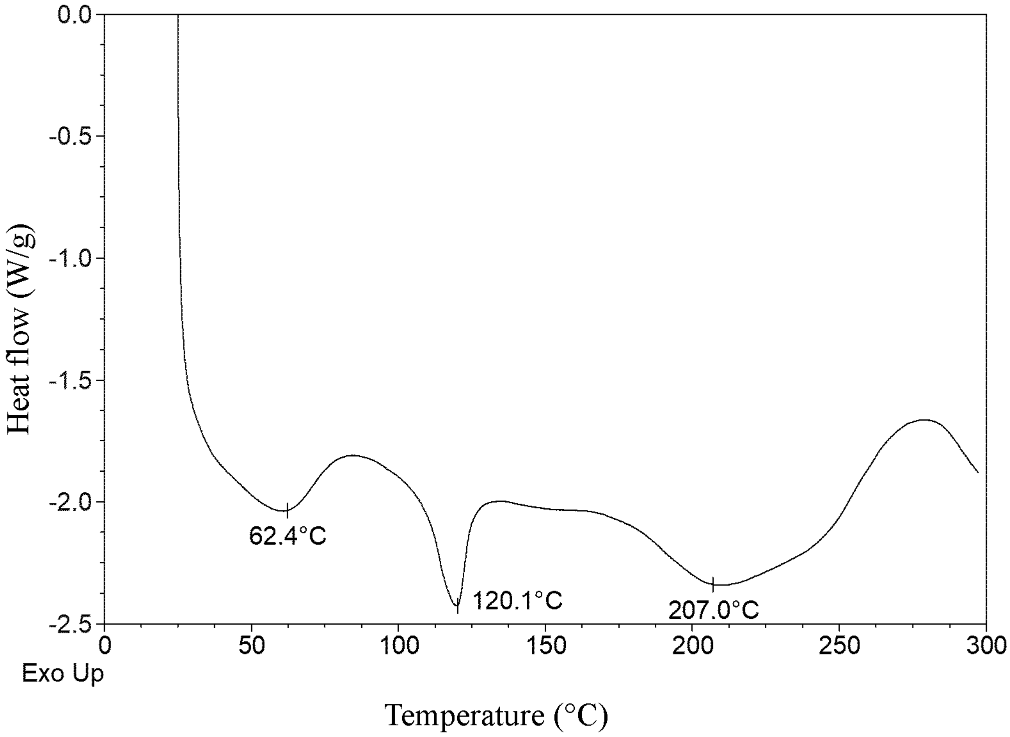
0.0
-0.5
-1.0
-1.5
-2.0
-2.5
Heat flow (W/g)
62.4°C
120.1°C
207.0°C
0
50
100
150
200
250
300
Exo Up
Temperature (°C)

FIG. 71

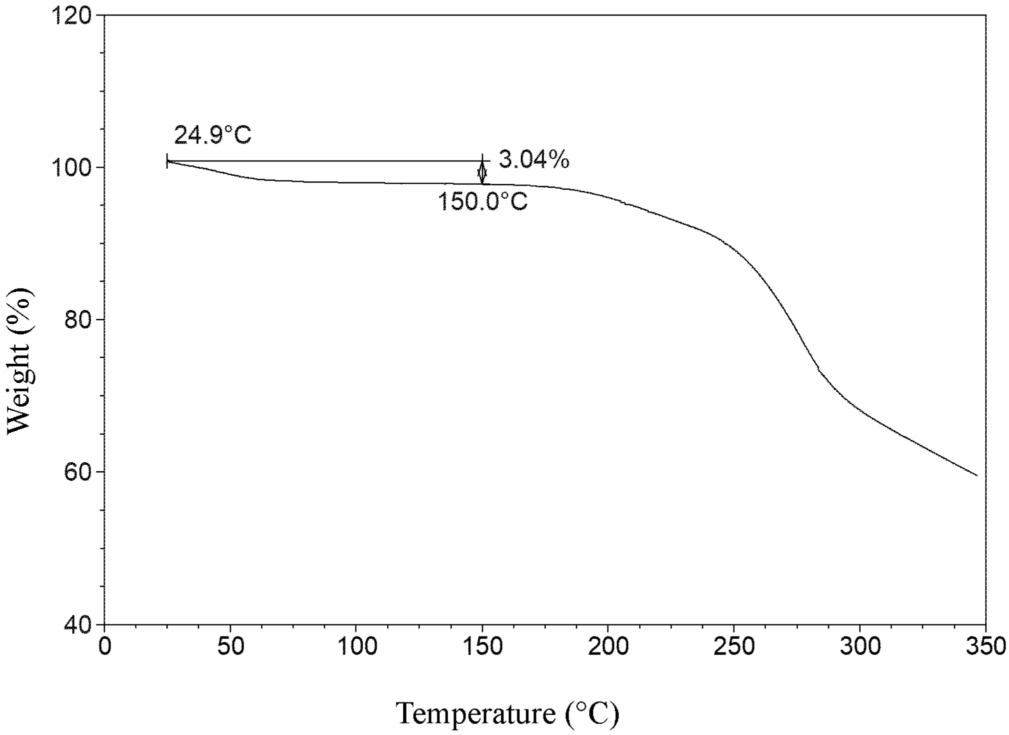
120
100
80
60
40
Weight (%)
24.9°C
3.04%
150.0°C
0
50
100
150
200
250
300
350
Temperature (°C)

FIG. 72

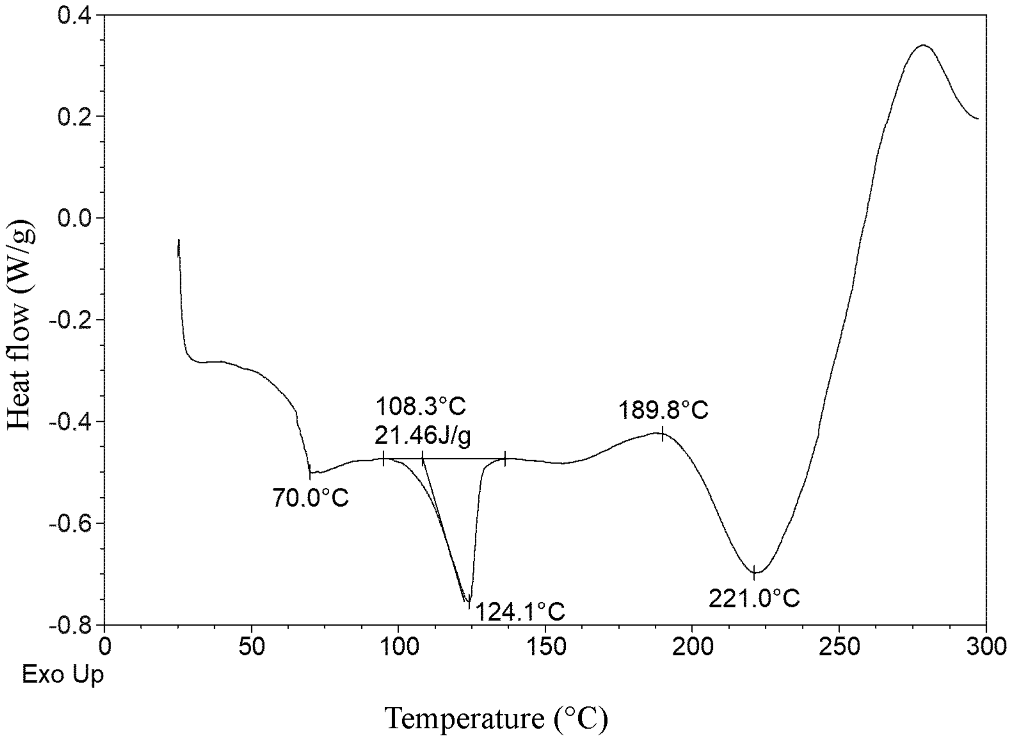
0.4
0.2
0.0
-0.2
-0.4
-0.6
-0.8
Heat flow (W/g)
108.3°C
21.46J/g
189.8°C
70.0°C
124.1°C
221.0°C
0
50
100
150
200
250
300
Exo Up
Temperature (°C)

FIG. 73

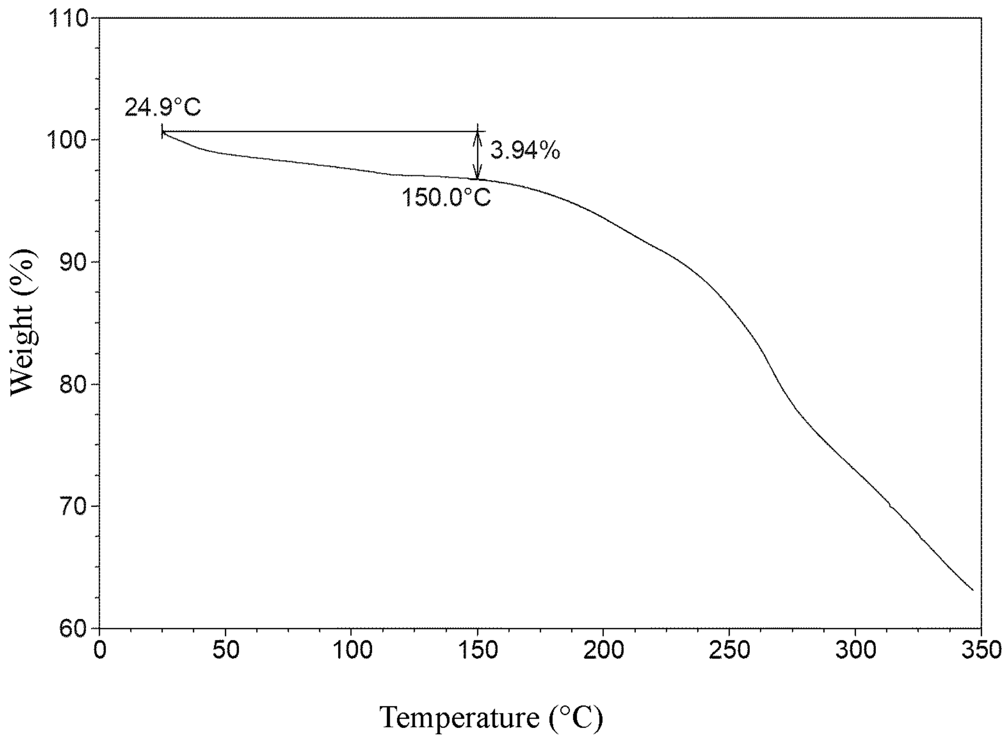
110
100
90
80
70
60
Weight (%)
24.9°C
3.94%
150.0°C
0
50
100
150
200
250
300
350
Temperature (°C)

FIG. 74

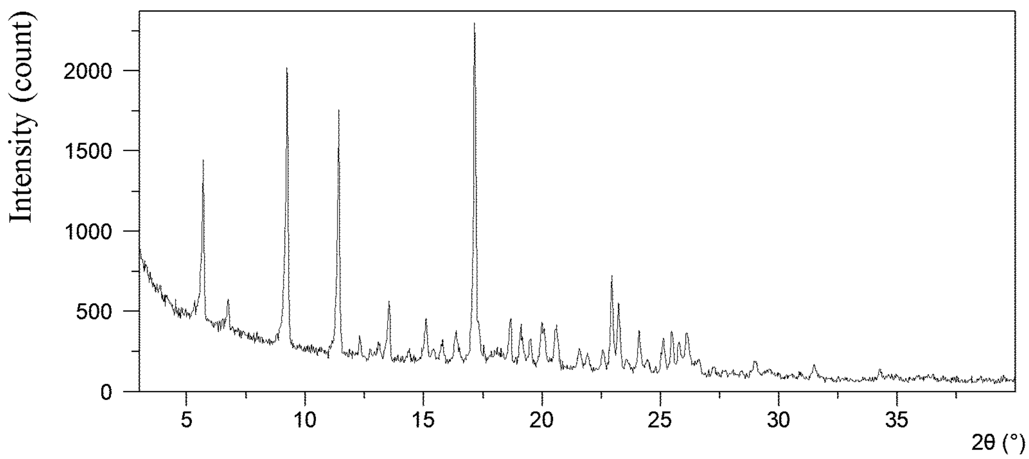
Intensity (count)
2000
1500
1000
500
0
5
10
15
20
25
30
35
2θ (°)

FIG. 75

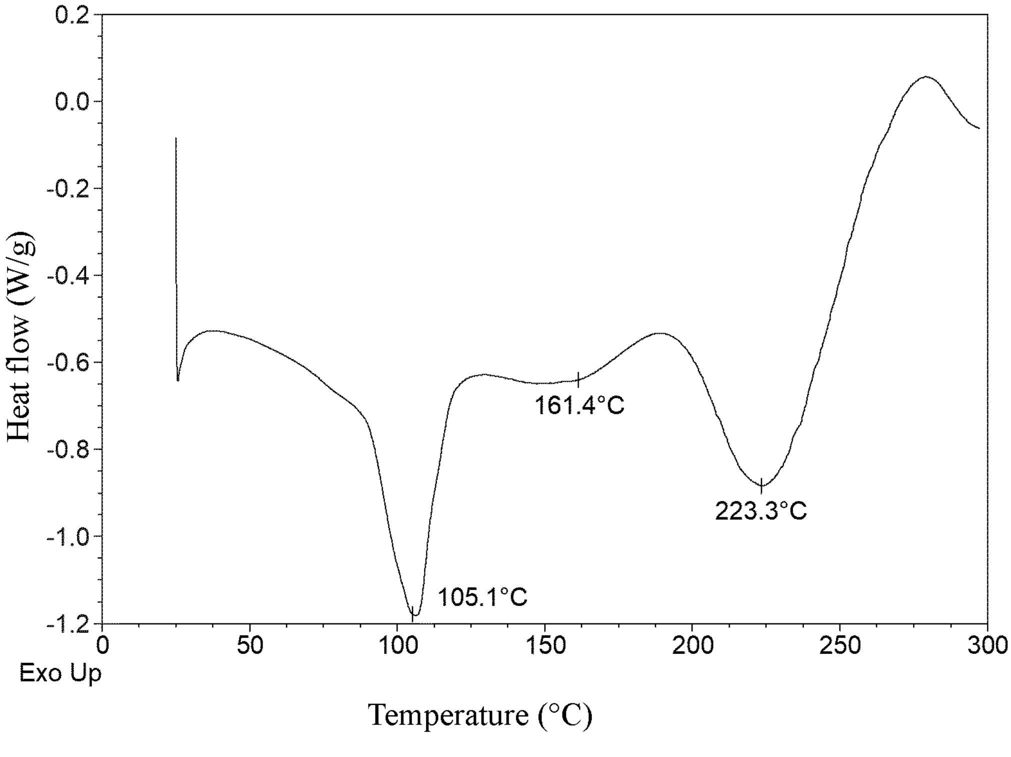
0.2
0.0
-0.2
-0.4
-0.6
-0.8
-1.0
-1.2
Heat flow (W/g)
161.4°C
223.3°C
105.1°C
0
50
100
150
200
250
300
Exo Up
Temperature (°C)

FIG. 76

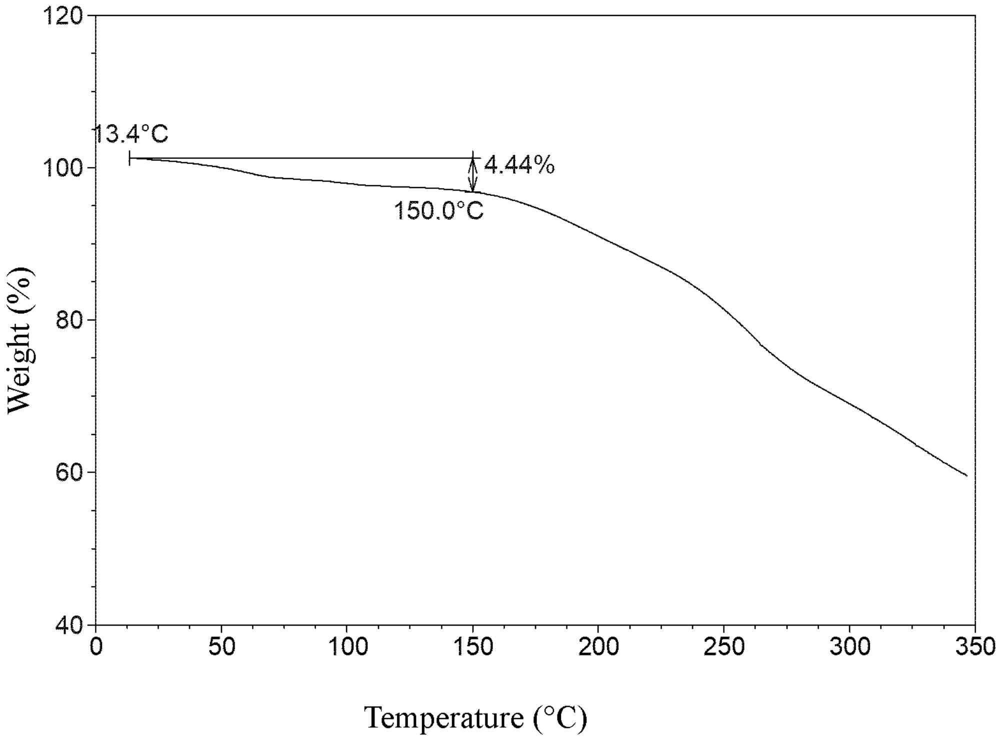

120
100
80
60
40
Weight (%)
13.4°C
4.44%
150.0°C
0
50
100
150
200
250
300
350
Temperature (°C)


FIG. 77

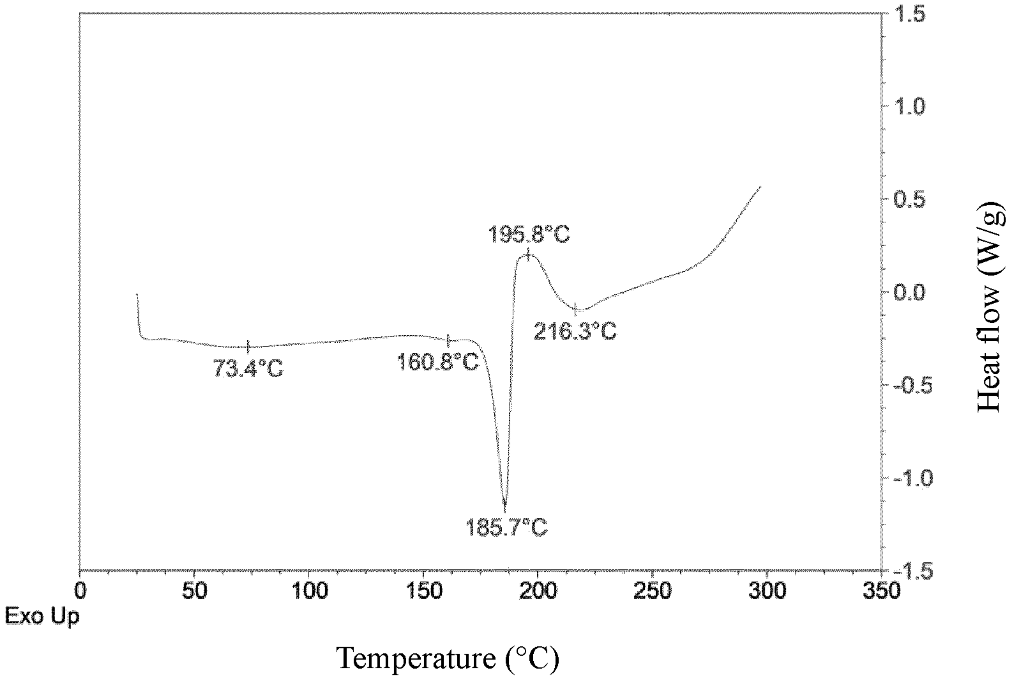

1.5
1.0
0.5
0.0
-0.5
-1.0
-1.5
Heat flow (W/g)
195.8°C
216.3°C
73.4°C
160.8°C
185.7°C
0
50
100
150
200
250
300
350
Exo Up
Temperature (°C)


FIG. 78

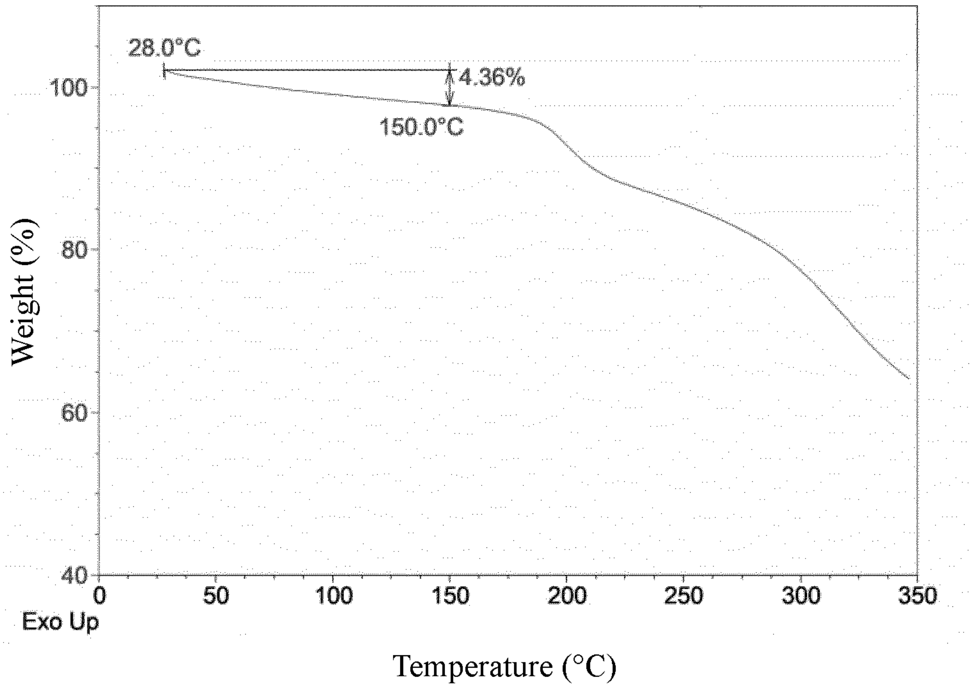
28.0°C
4.36%
150.0°C
Weight (%)
100
80
60
40
0
50
100
150
200
250
300
350
Exo Up
Temperature (°C)

FIG. 79

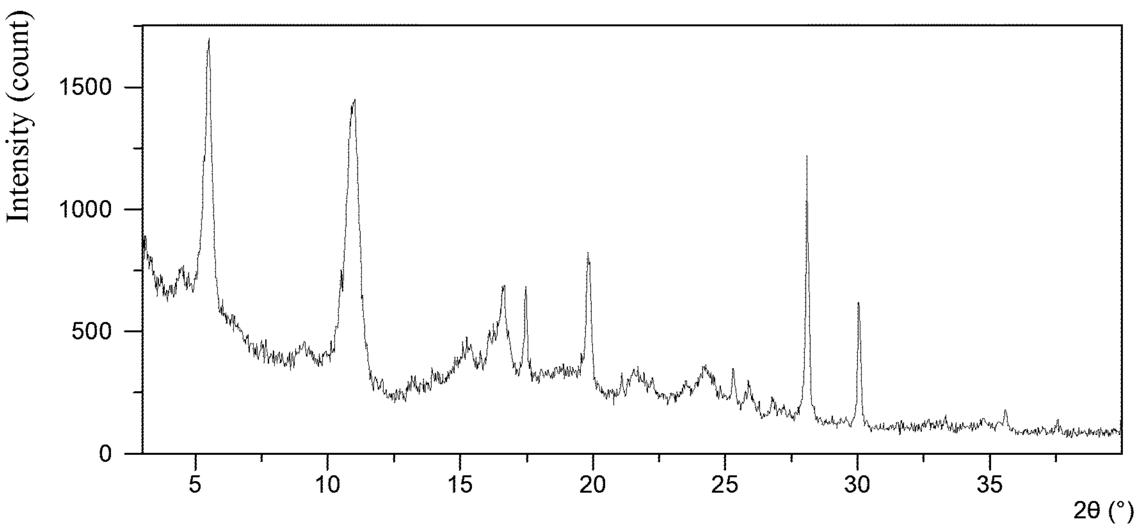
Intensity (count)
1500
1000
500
0
5
10
15
20
25
30
35
2θ (°)

FIG. 80

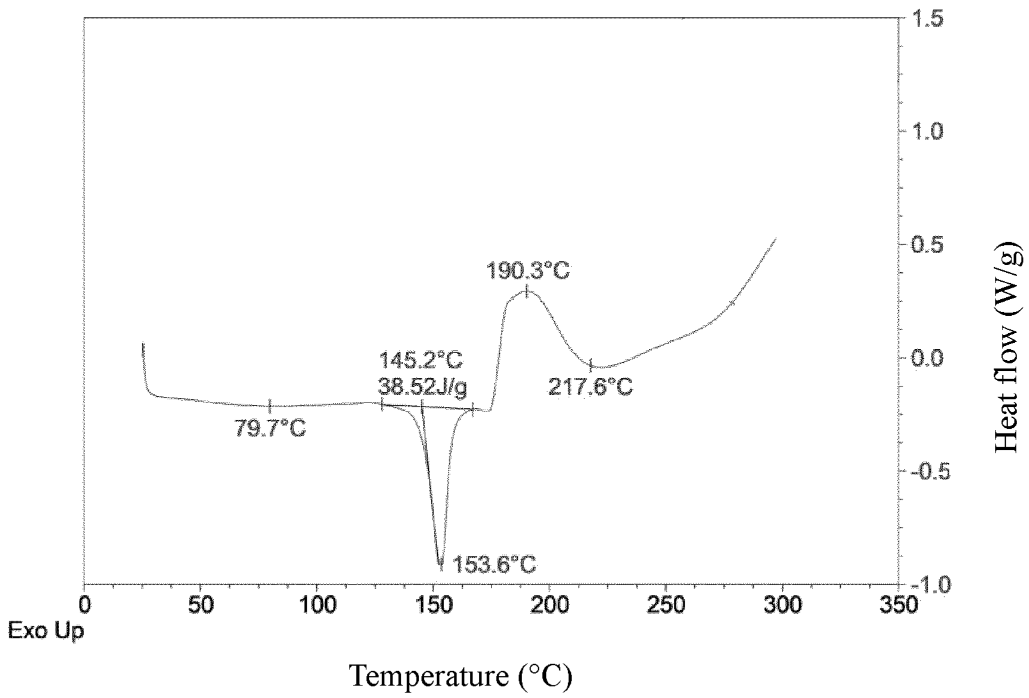
1.5
1.0
0.5
0.0
-0.5
-1.0
Heat flow (W/g)
190.3°C
145.2°C
38.52J/g
217.6°C
79.7°C
153.6°C
0
50
100
150
200
250
300
350
Exo Up
Temperature (°C)

FIG. 81

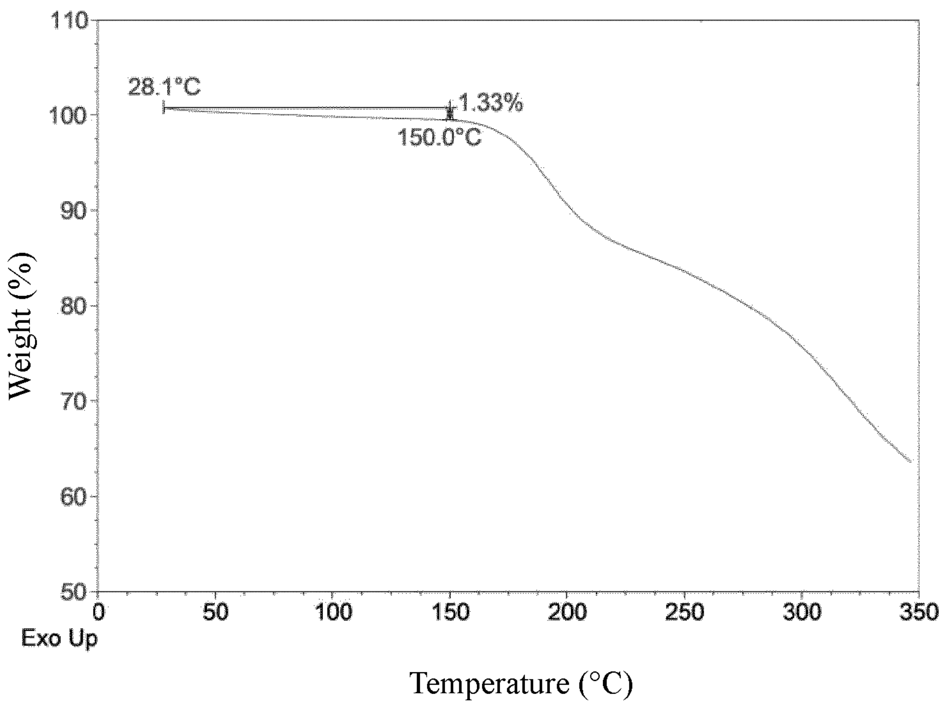
110
100
90
80
70
60
50
Weight (%)
28.1°C
1.33%
150.0°C
0
50
100
150
200
250
300
350
Exo Up
Temperature (°C)

FIG. 82

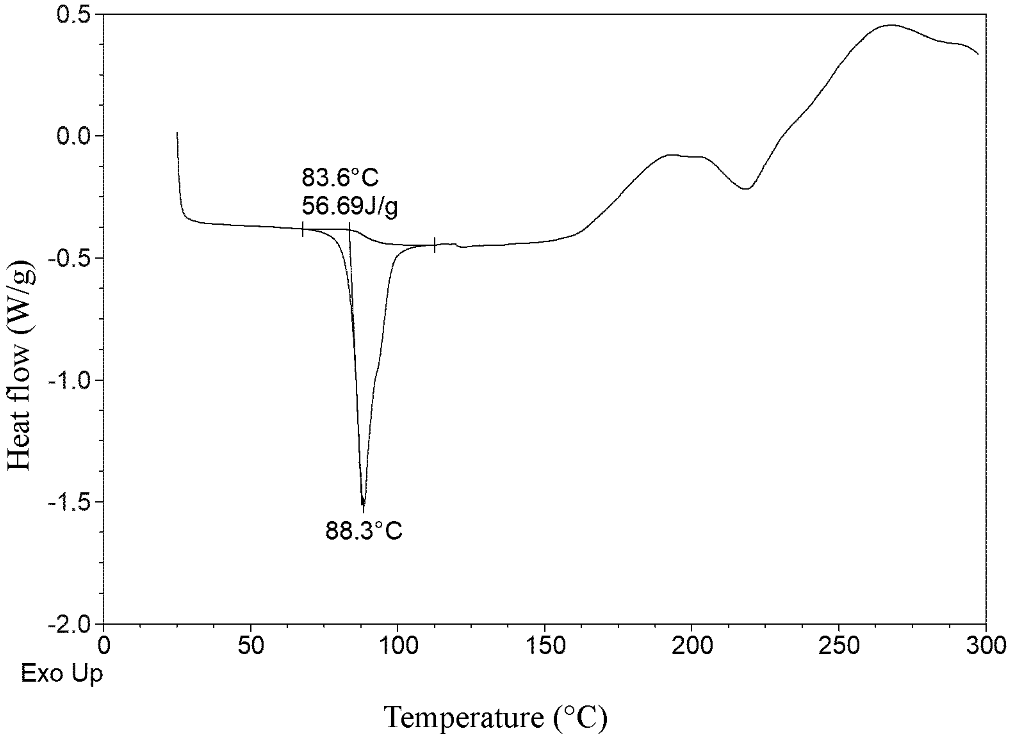

Heat flow (W/g)
Temperature (°C)
Exo Up
83.6°C
56.69J/g
88.3°C
0.5
0.0
-0.5
-1.0
-1.5
-2.0
0
50
100
150
200
250
300


FIG. 83

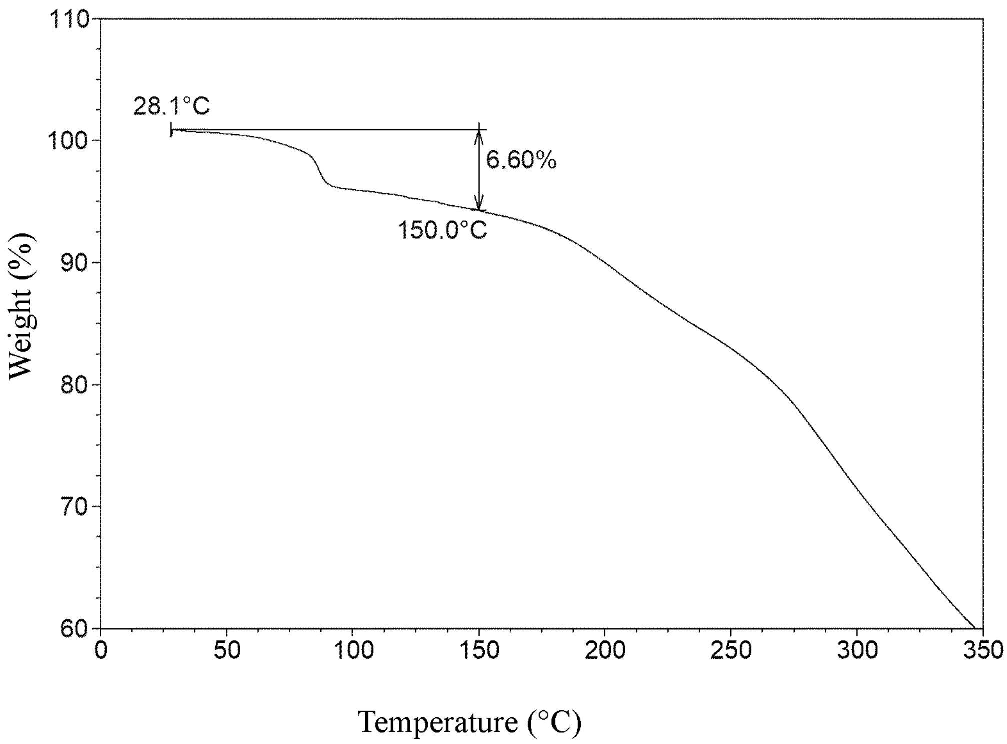

Weight (%)
Temperature (°C)
28.1°C
6.60%
150.0°C
110
100
90
80
70
60
0
50
100
150
200
250
300
350


FIG. 84

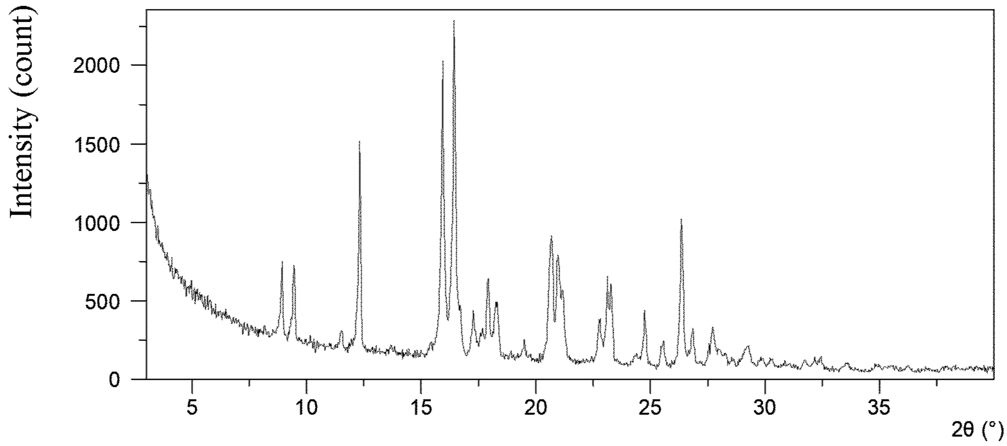
Intensity (count)
2000
1500
1000
500
0
5
10
15
20
25
30
35
2θ (°)

FIG. 85

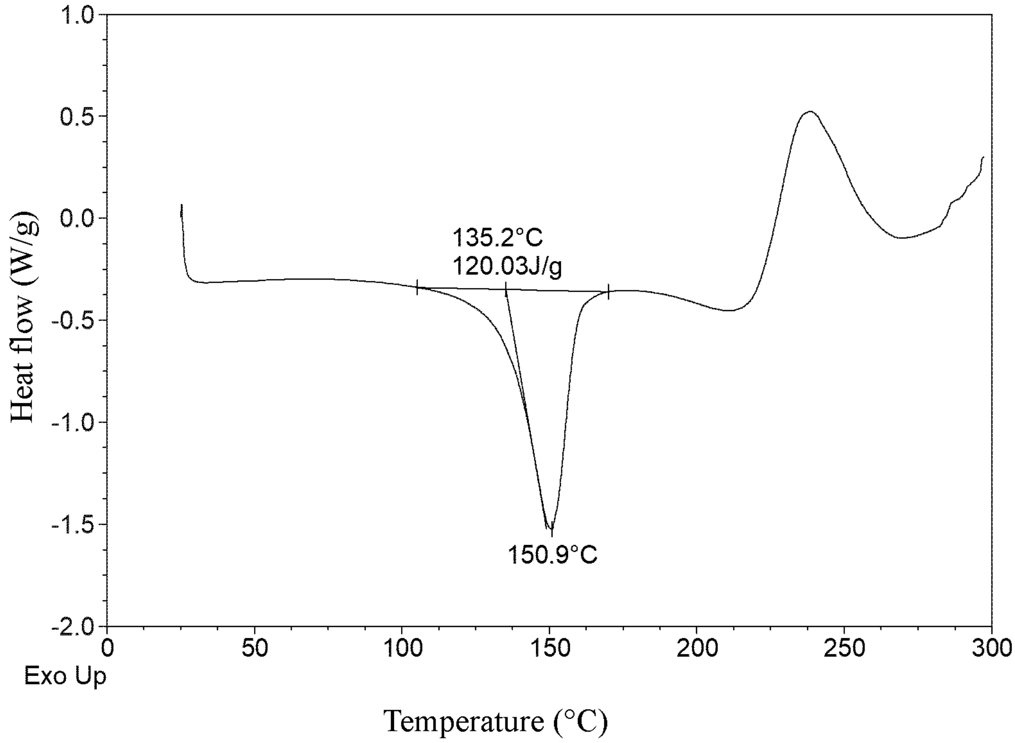
1.0
0.5
0.0
-0.5
-1.0
-1.5
-2.0
Heat flow (W/g)
135.2°C
120.03J/g
150.9°C
0
50
100
150
200
250
300
Exo Up
Temperature (°C)

FIG. 86

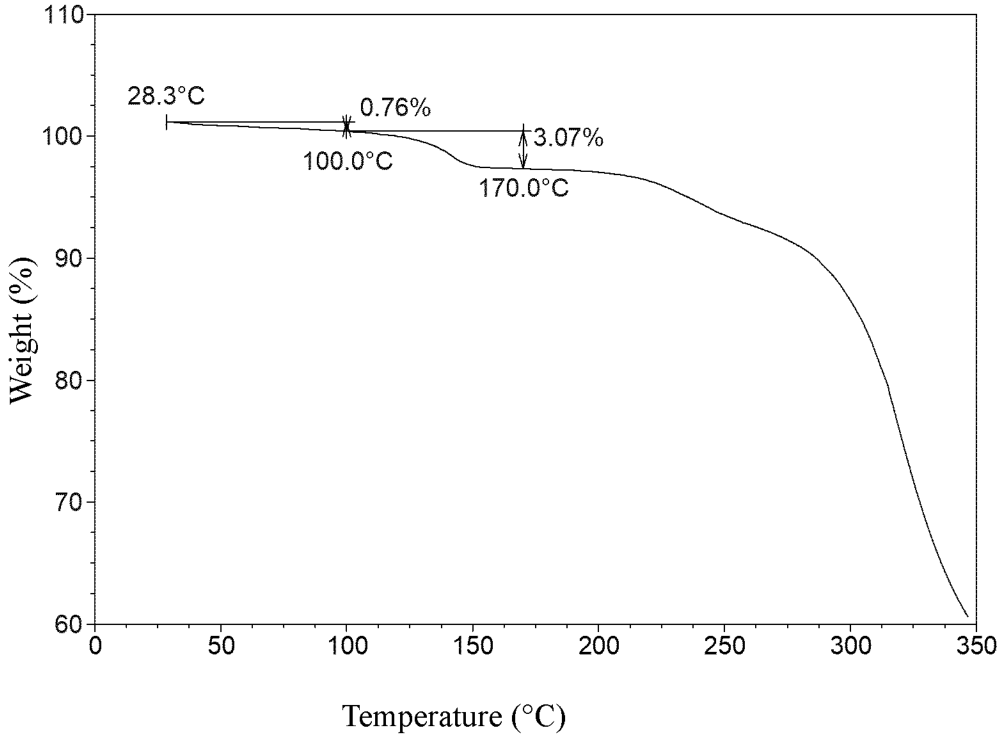
110
100
90
80
70
60
Weight (%)
28.3°C
0.76%
100.0°C
3.07%
170.0°C
0
50
100
150
200
250
300
350
Temperature (°C)

FIG. 87

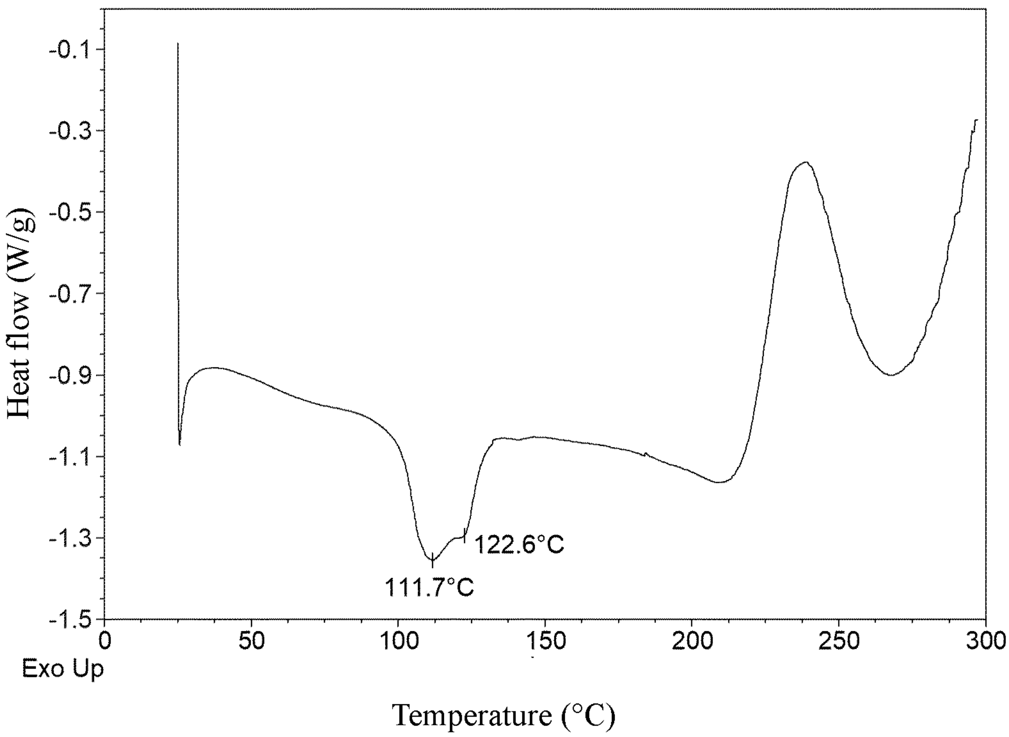
-0.1
-0.3
-0.5
-0.7
-0.9
-1.1
-1.3
-1.5
Heat flow (W/g)
122.6°C
111.7°C
0
50
100
150
200
250
300
Exo Up
Temperature (°C)

FIG. 88

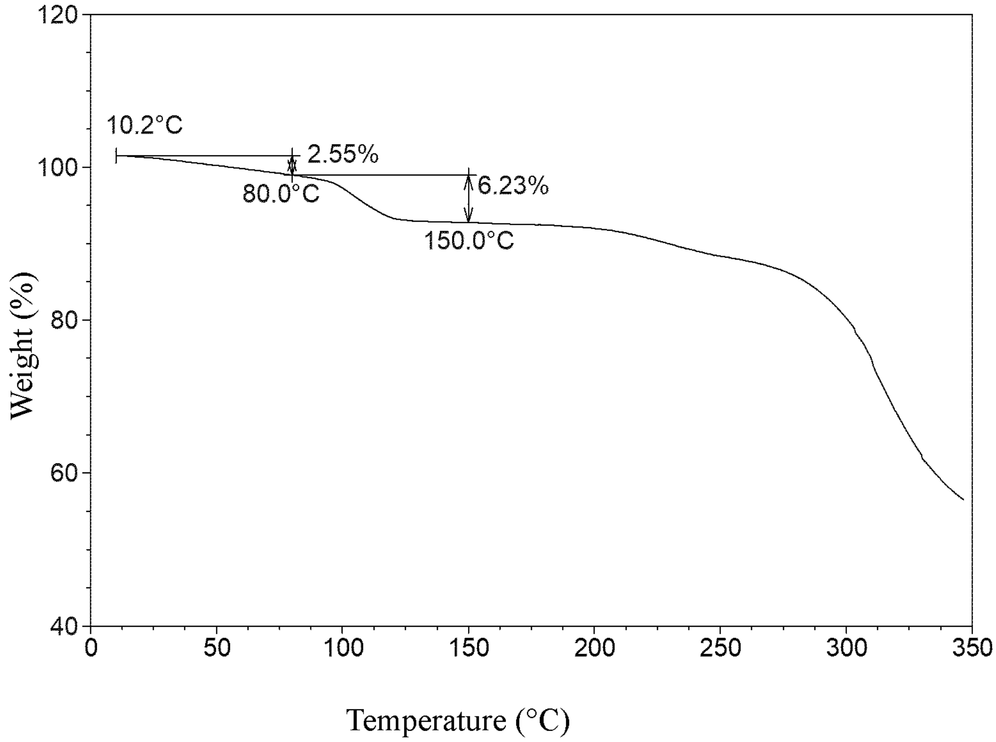
120
100
80
60
40
0
50
100
150
200
250
300
350
10.2°C
2.55%
80.0°C
6.23%
150.0°C
Weight (%)
Temperature (°C)

FIG. 89

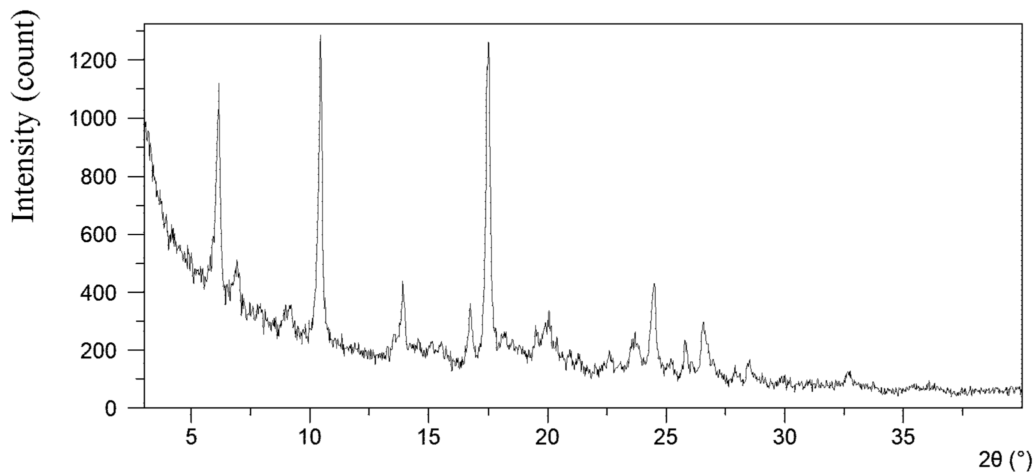
1200
1000
800
600
400
200
0
5
10
15
20
25
30
35
Intensity (count)
2θ (°)

FIG. 90

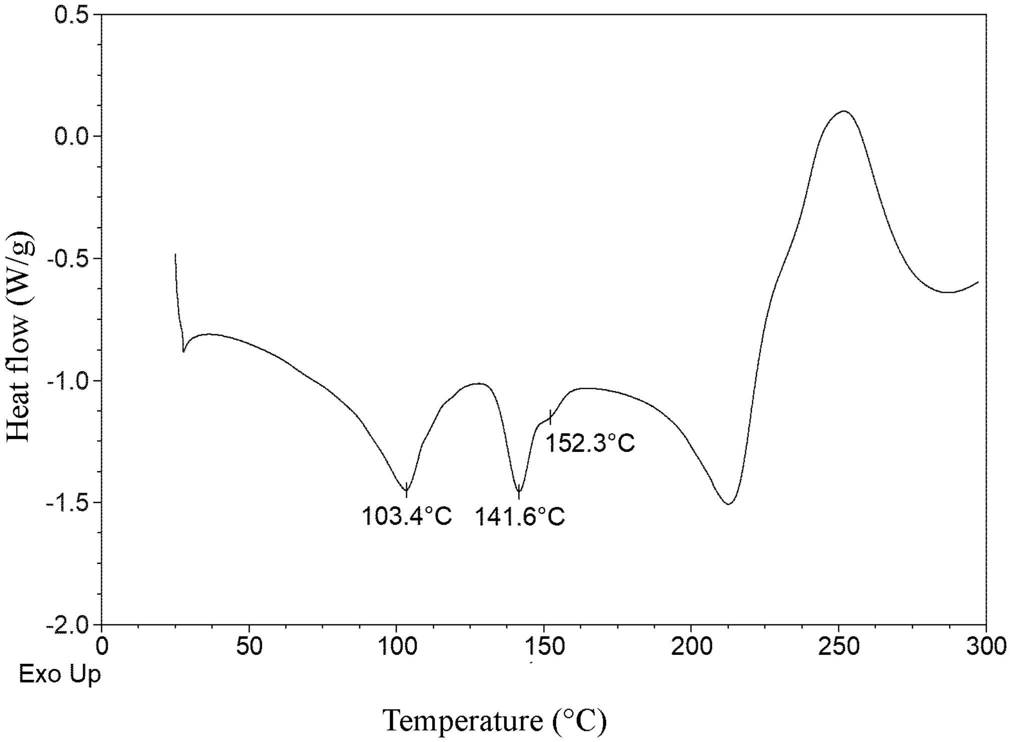
0.5
0.0
-0.5
-1.0
-1.5
-2.0
Heat flow (W/g)
103.4°C
141.6°C
152.3°C
0
50
100
150
200
250
300
Exo Up
Temperature (°C)

FIG. 91

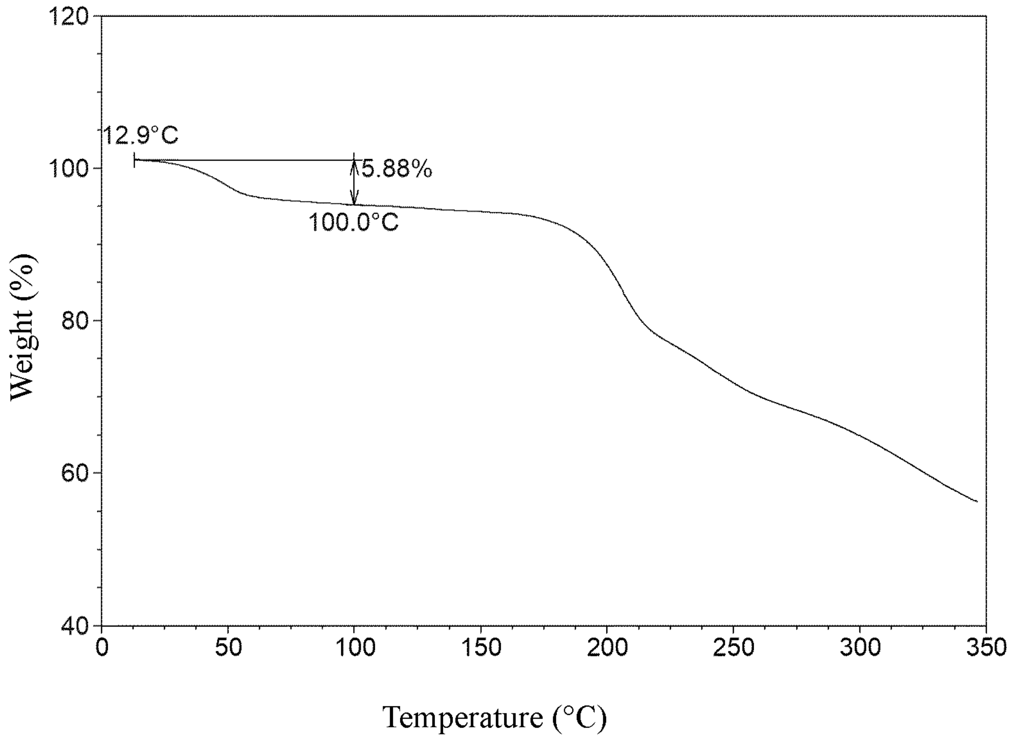
120
100
80
60
40
Weight (%)
12.9°C
5.88%
100.0°C
0
50
100
150
200
250
300
350
Temperature (°C)

FIG. 92

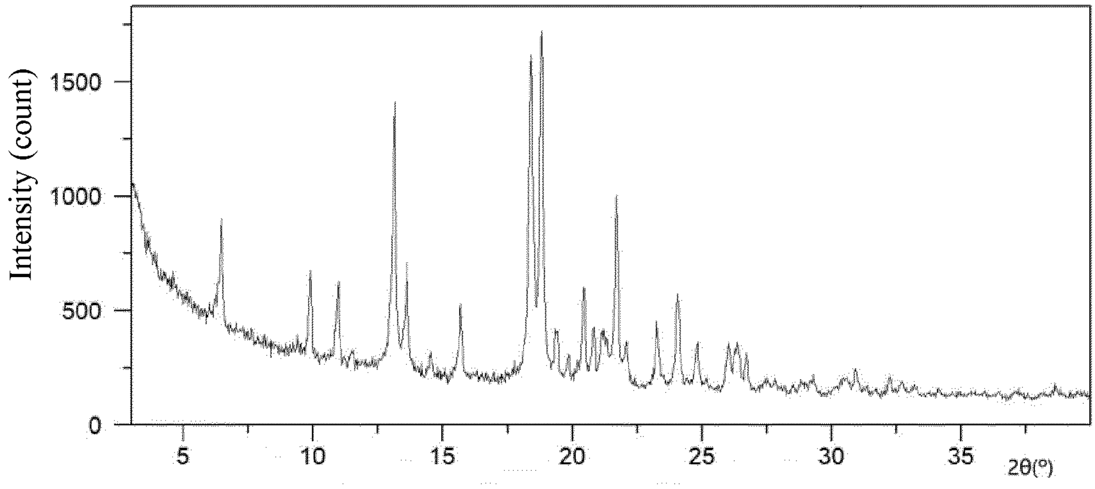
Intensity (count)
1500
1000
500
0
5
10
15
20
25
30
35
2θ(°)

FIG. 93

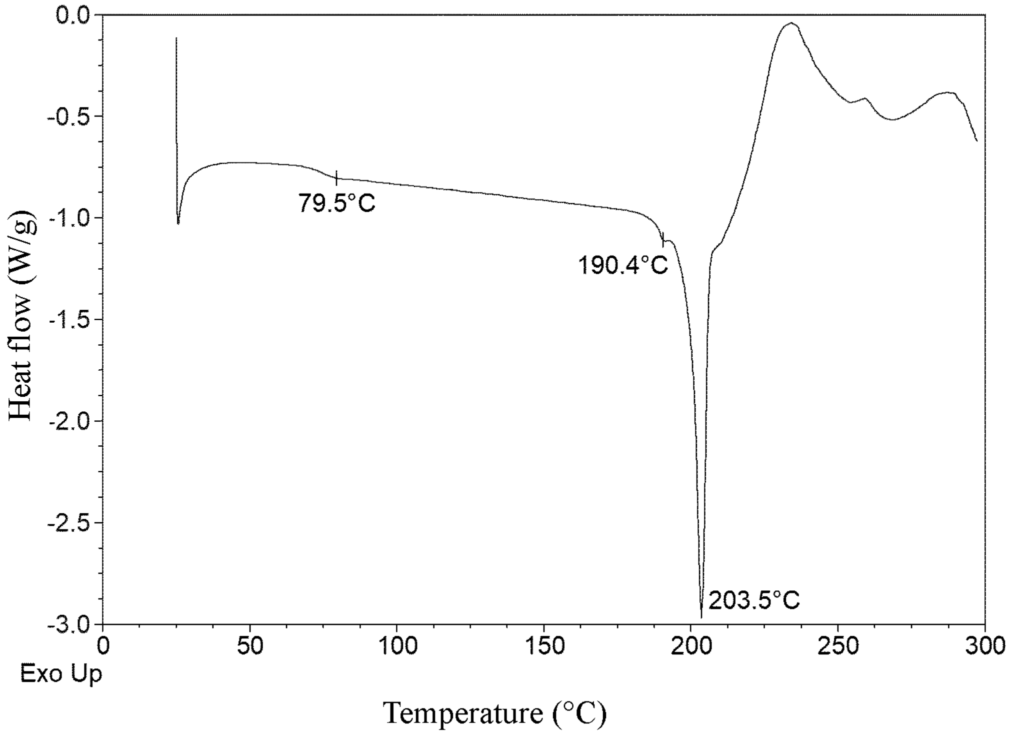
0.0
-0.5
-1.0
-1.5
-2.0
-2.5
-3.0
Heat flow (W/g)
79.5°C
190.4°C
203.5°C
0
50
100
150
200
250
300
Exo Up
Temperature (°C)

FIG. 94

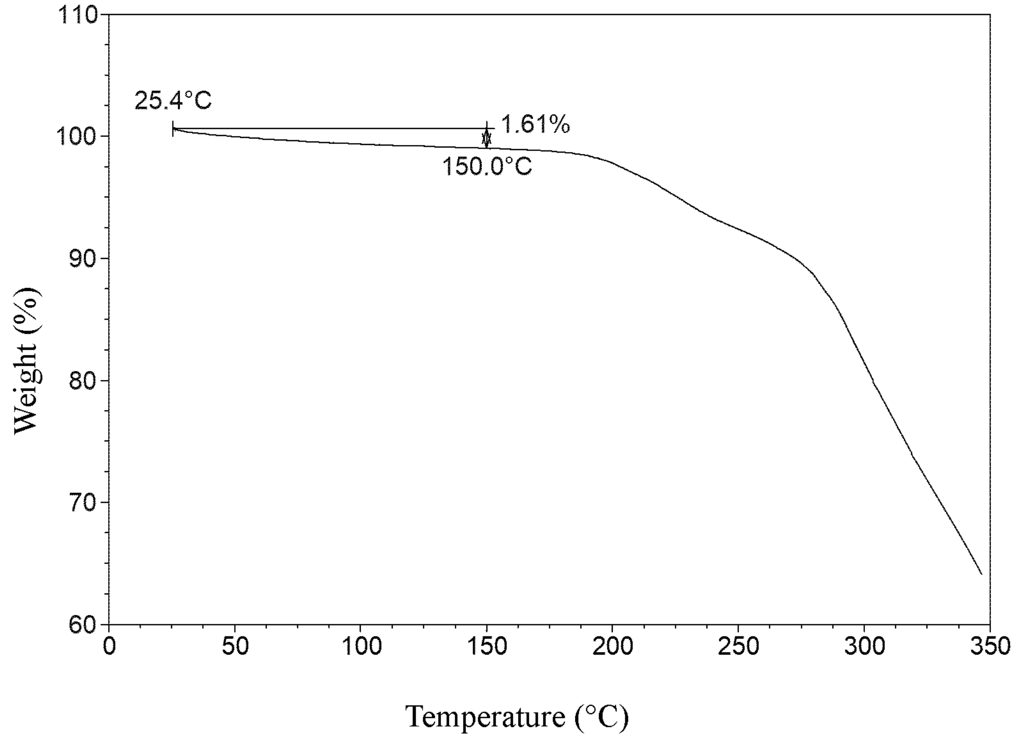
110
100
90
80
70
60
Weight (%)
25.4°C
1.61%
150.0°C
0
50
100
150
200
250
300
350
Temperature (°C)

FIG. 95

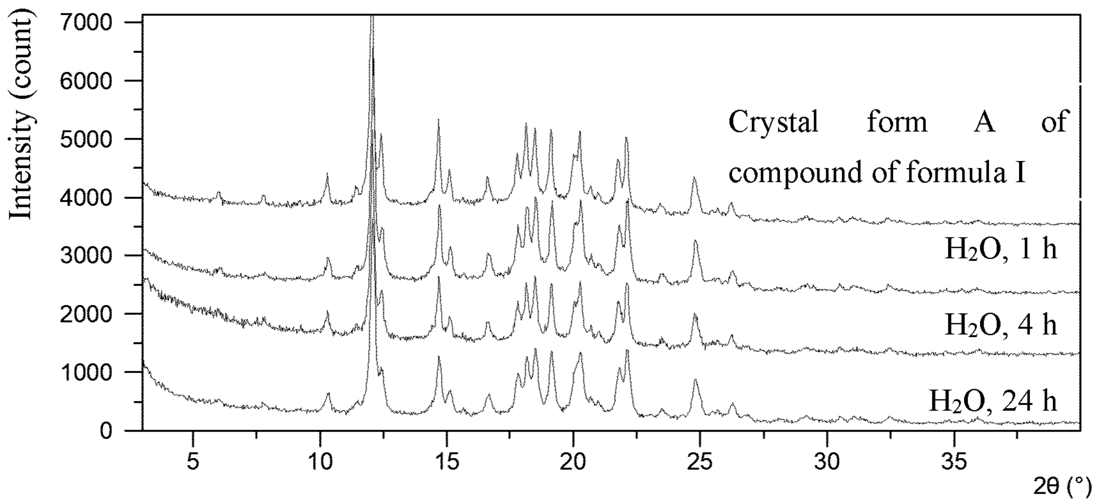
Intensity (count)
7000
6000
5000
4000
3000
2000
1000
0
Crystal form A of compound of formula I
H2O, 1 h
H2O, 4 h
H2O, 24 h
5
10
15
20
25
30
35
2θ (°)

FIG. 96

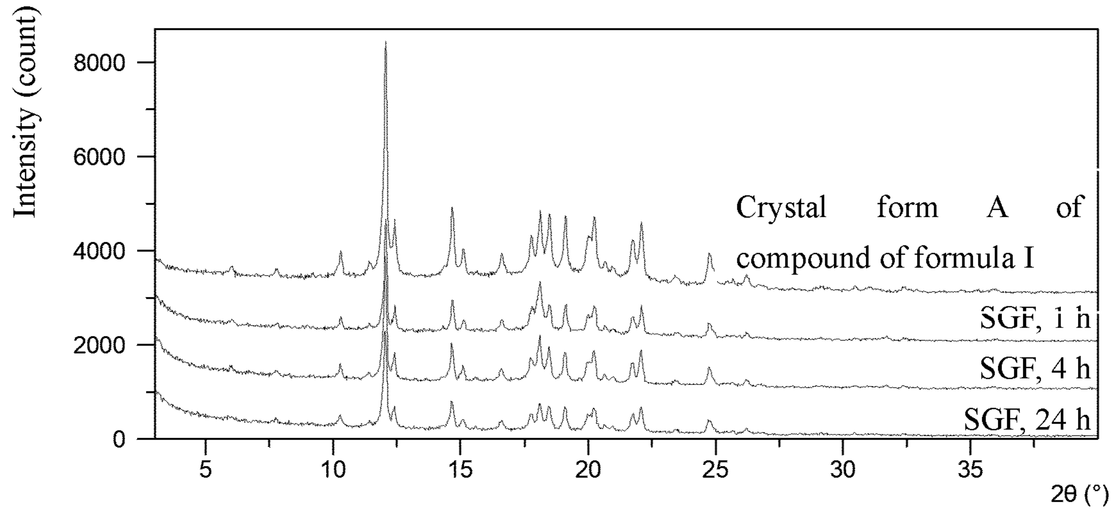


FIG. 97

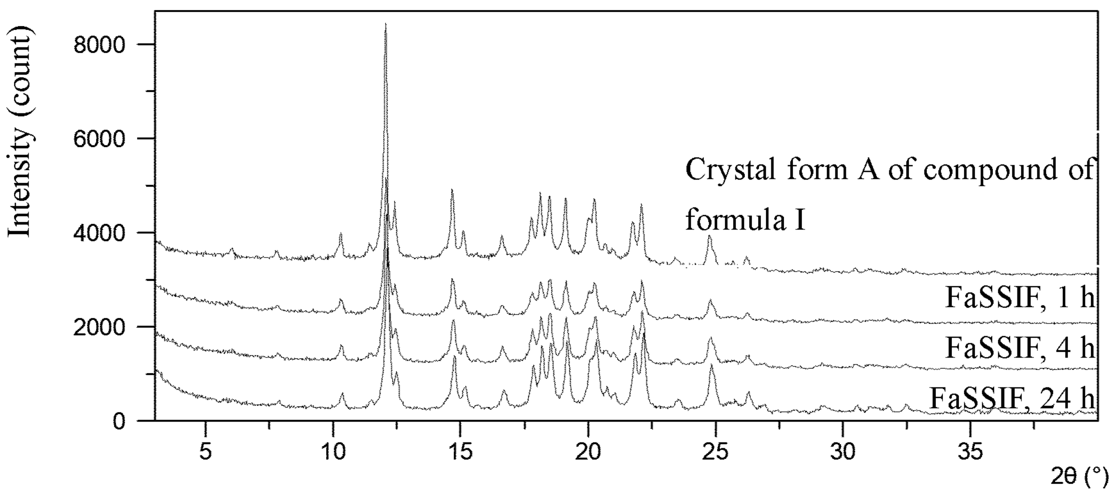


FIG. 98

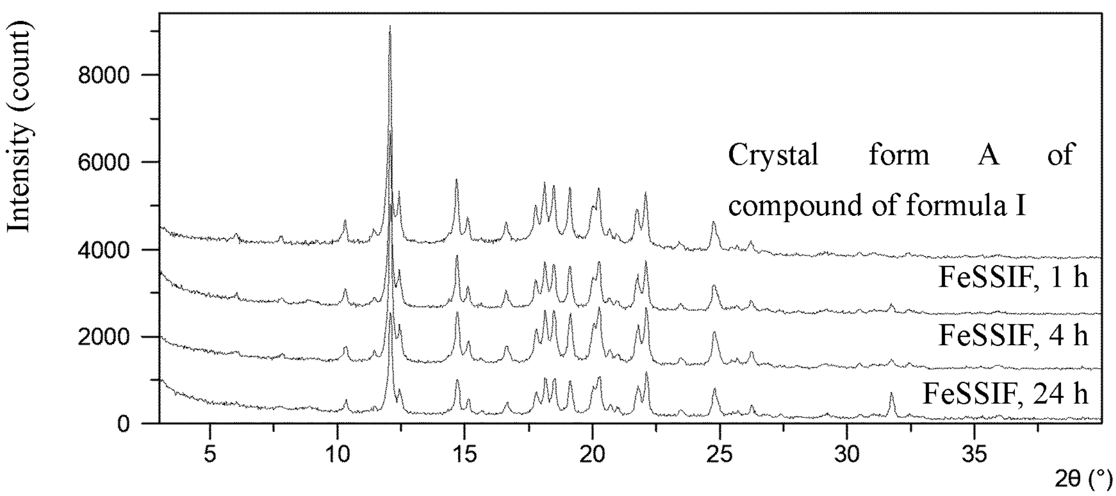


FIG. 99

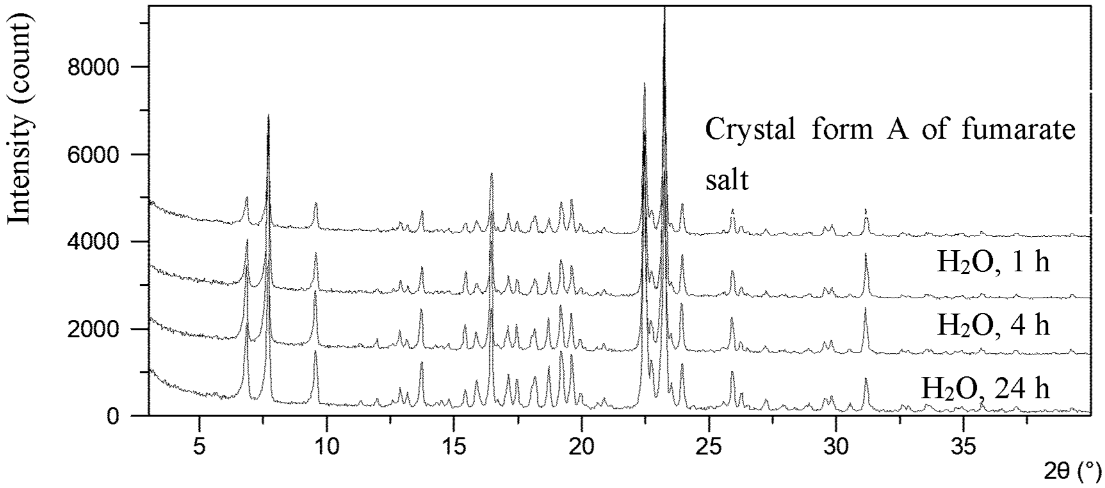


FIG. 100

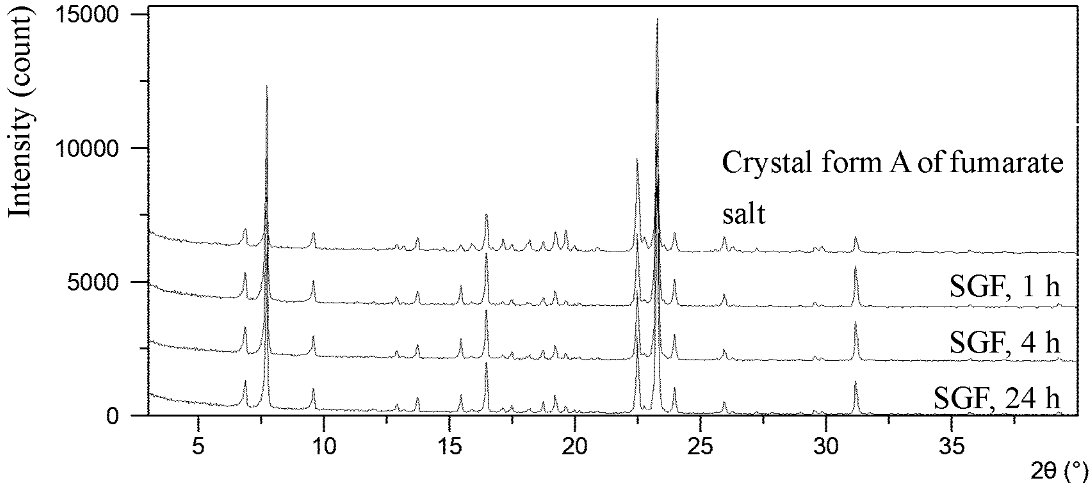


FIG. 101

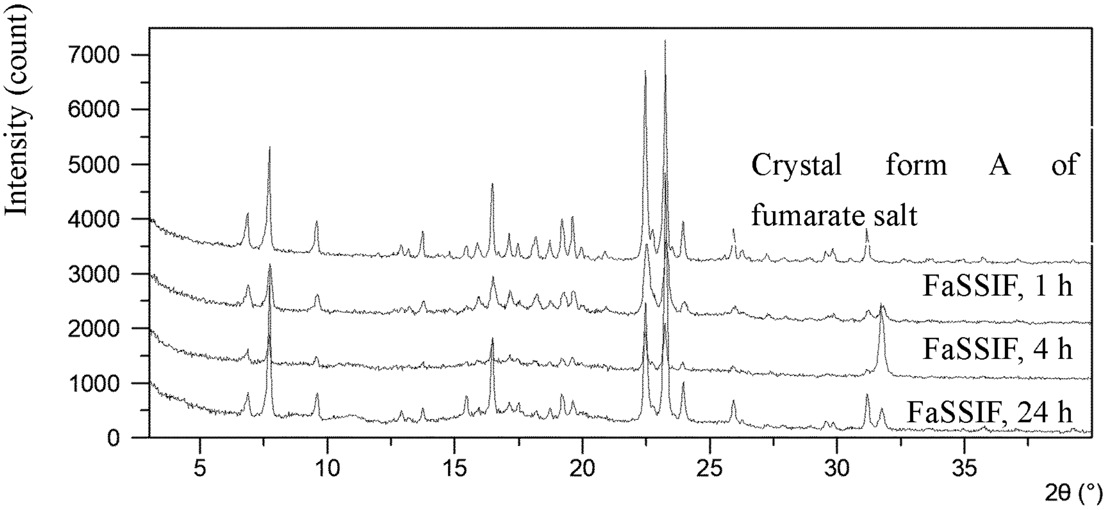


FIG. 102

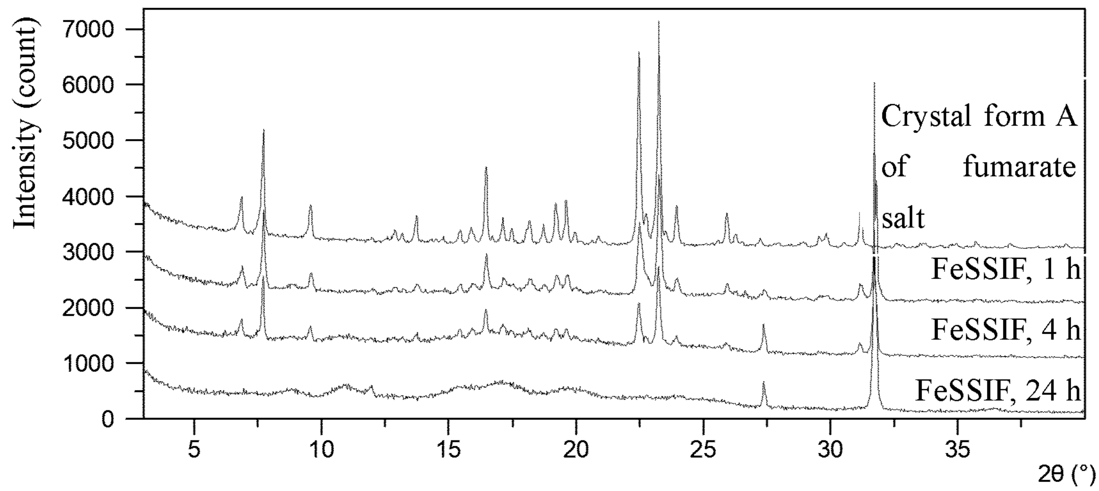


FIG. 103

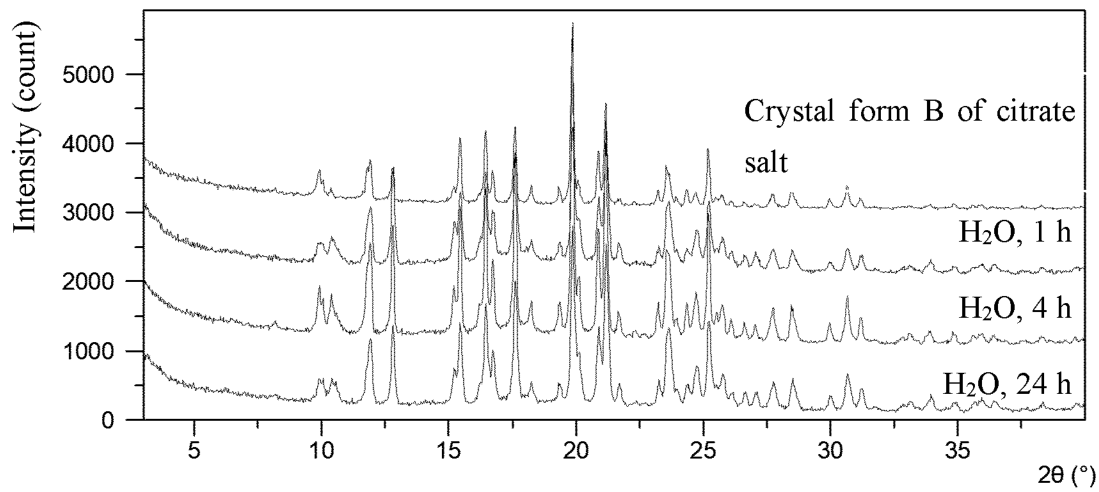


FIG. 104

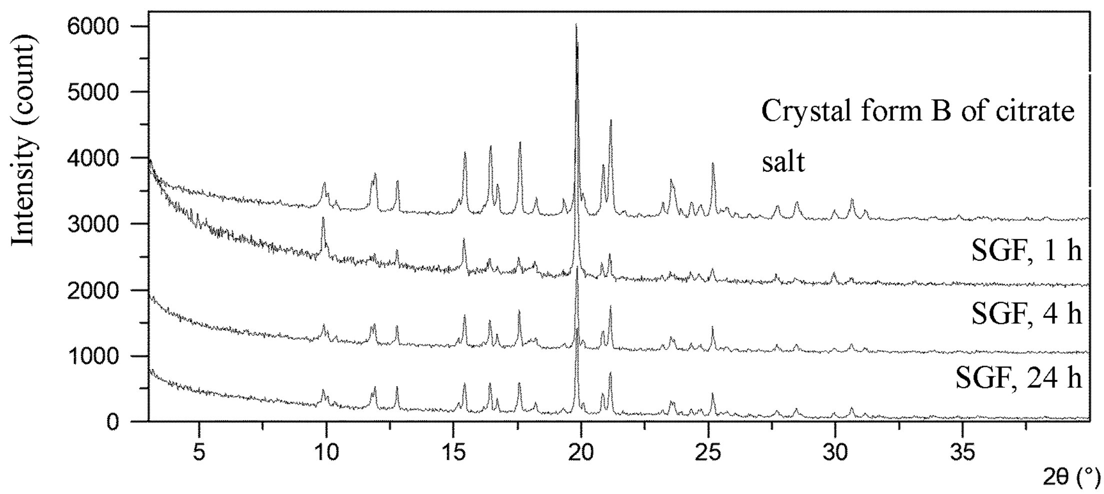


FIG. 105

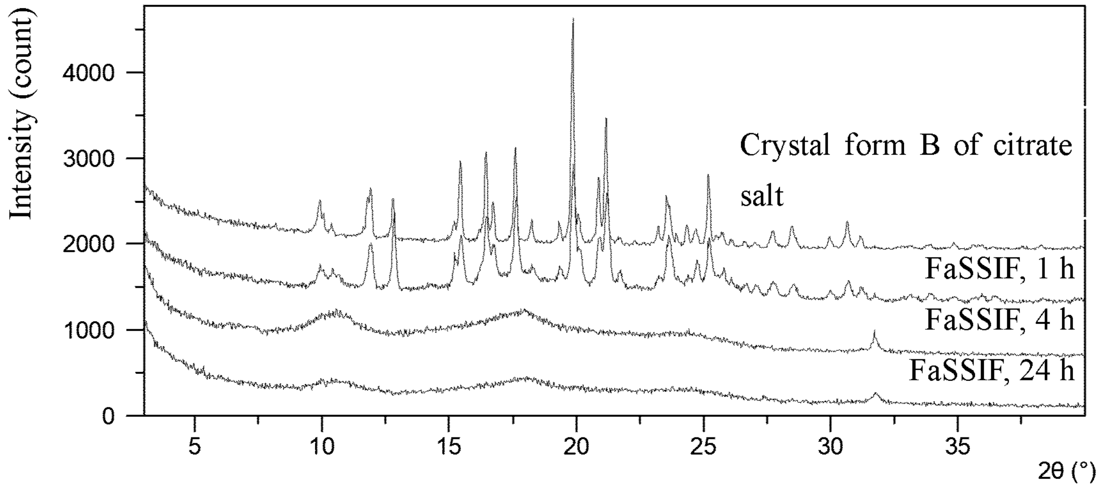


FIG. 106

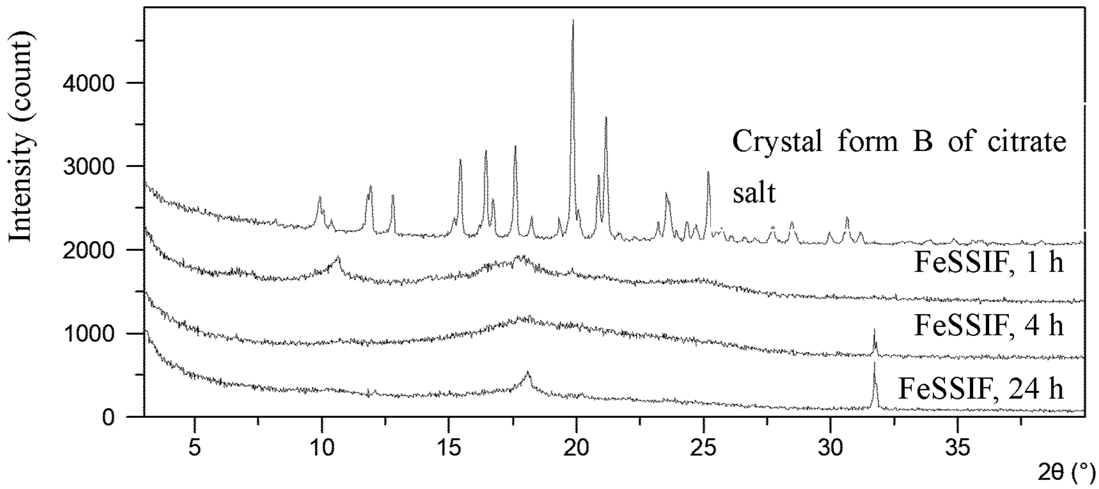


FIG. 107

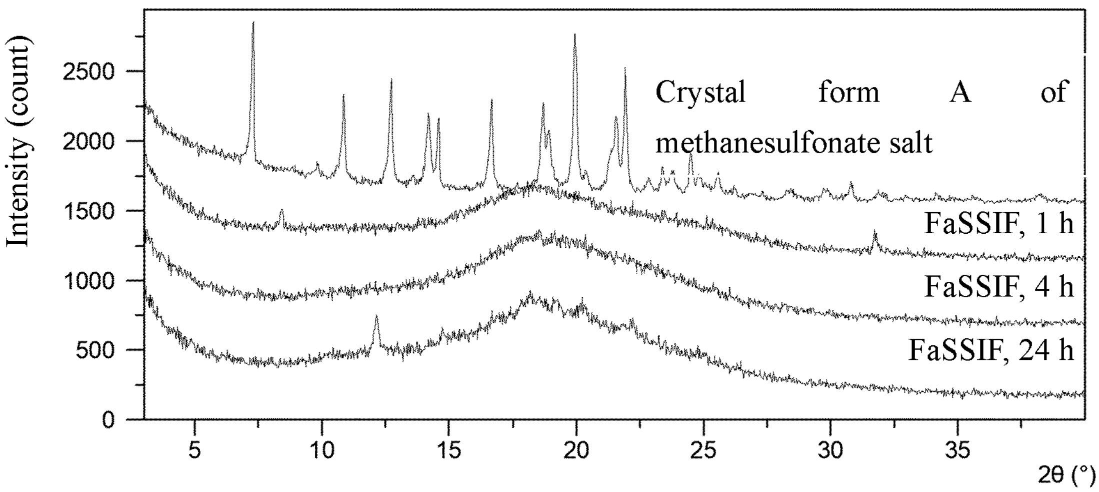


FIG. 108

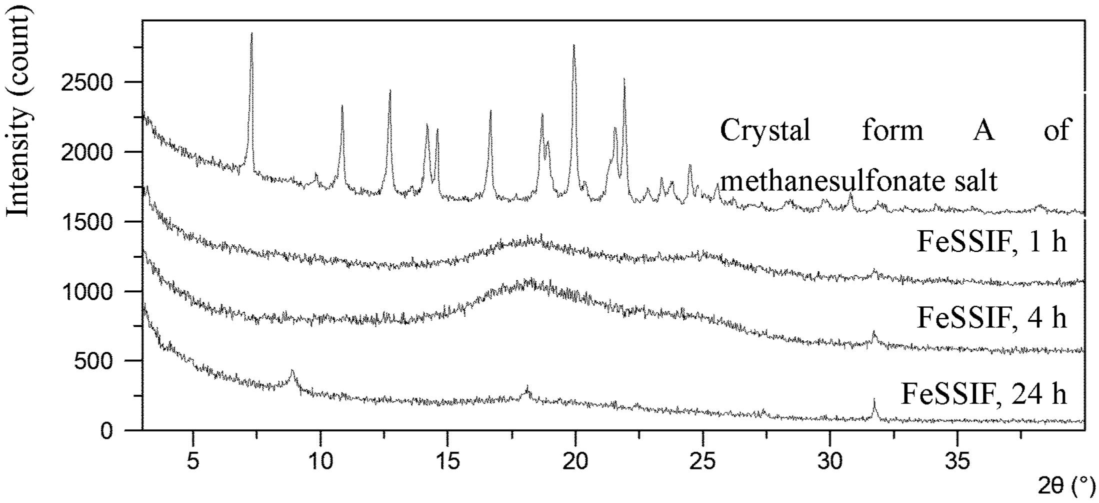


FIG. 109

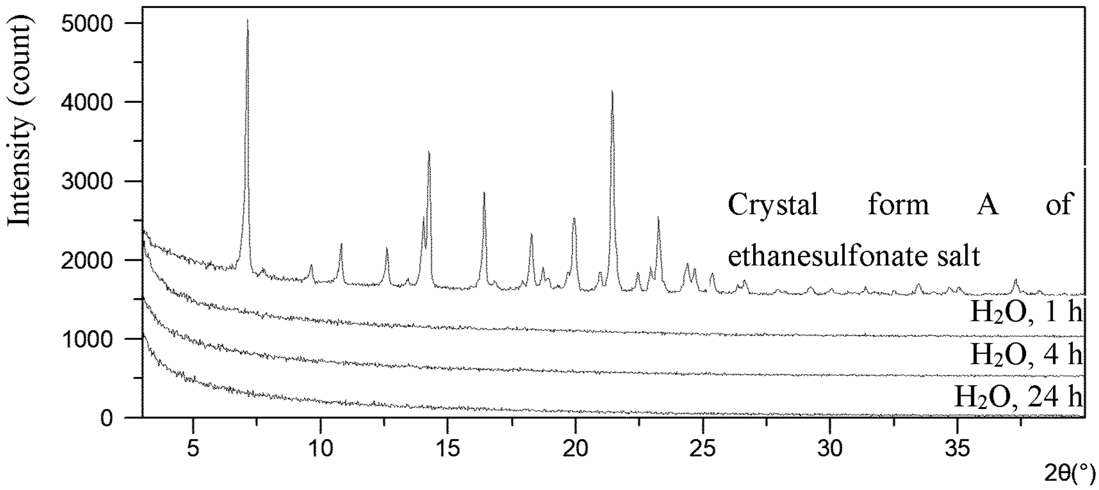


FIG. 110

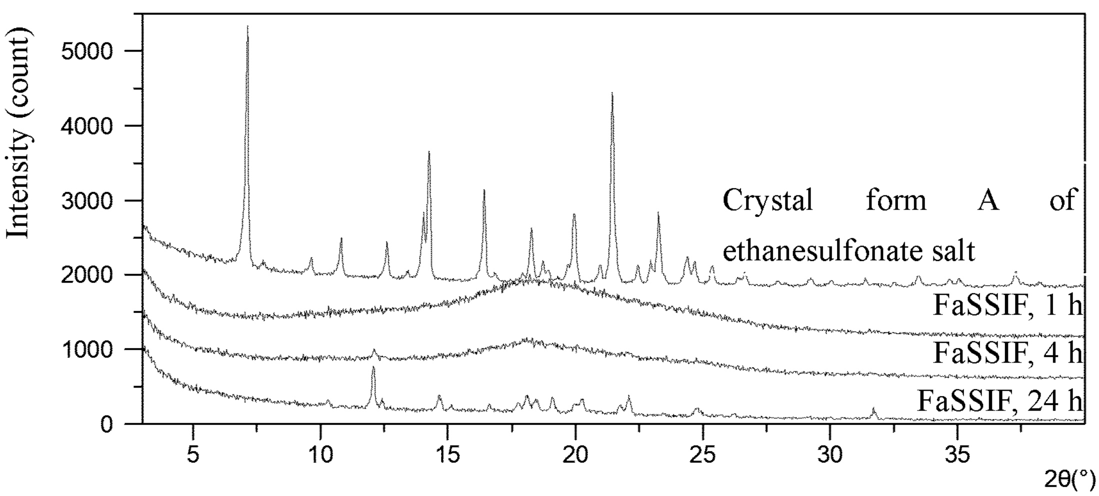


FIG. 111

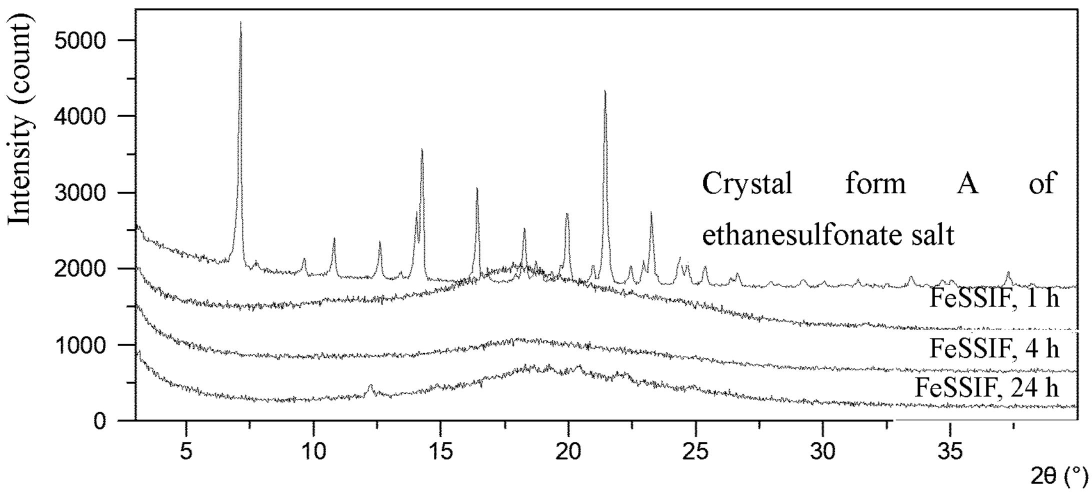


FIG. 112

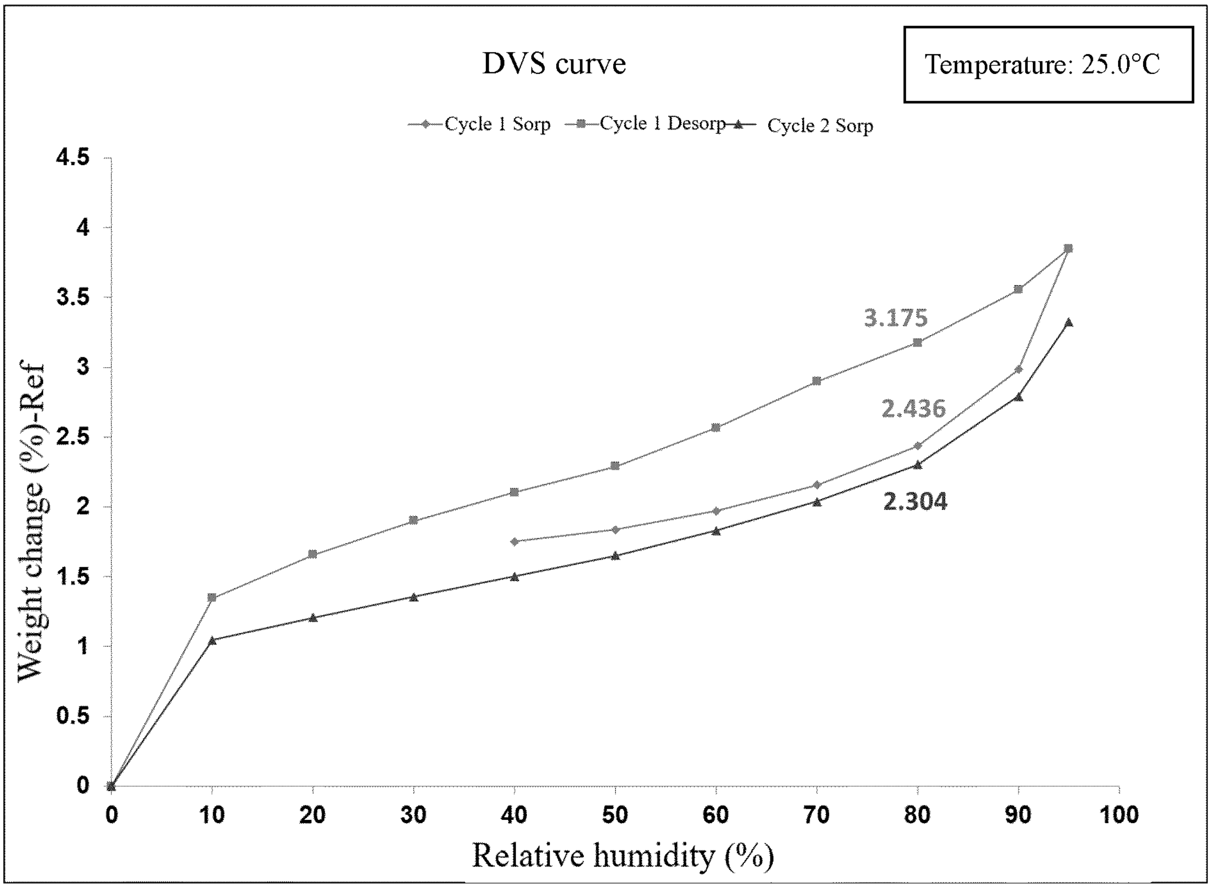


FIG. 113

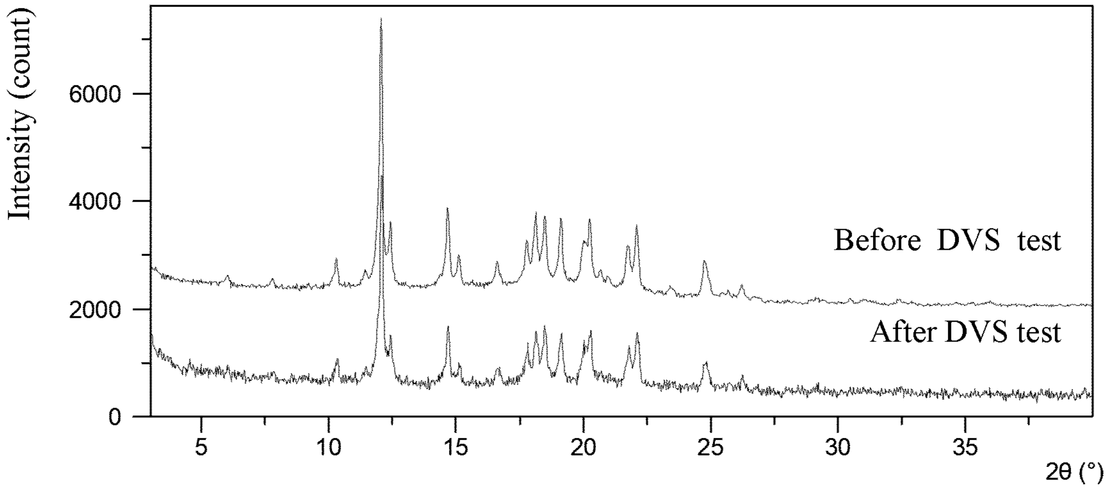


FIG. 114

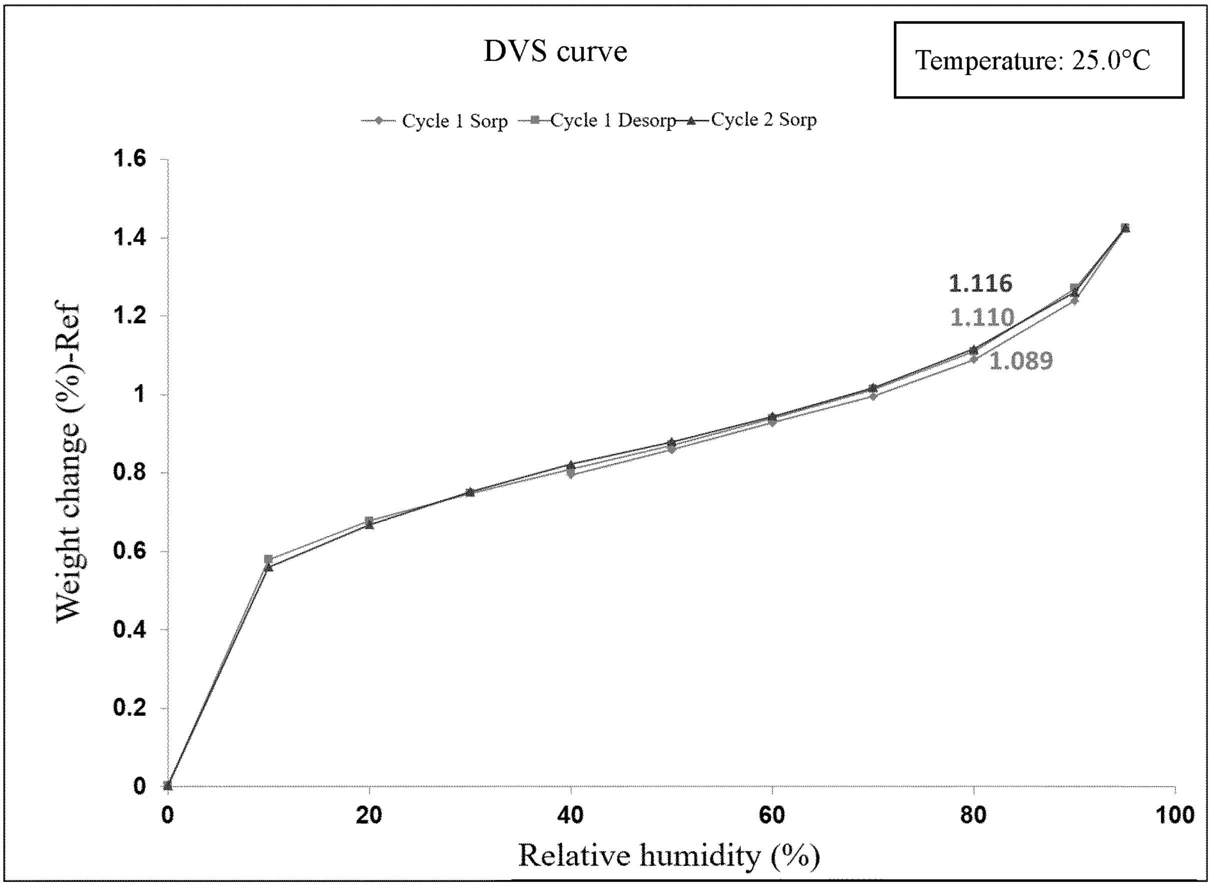


FIG. 115

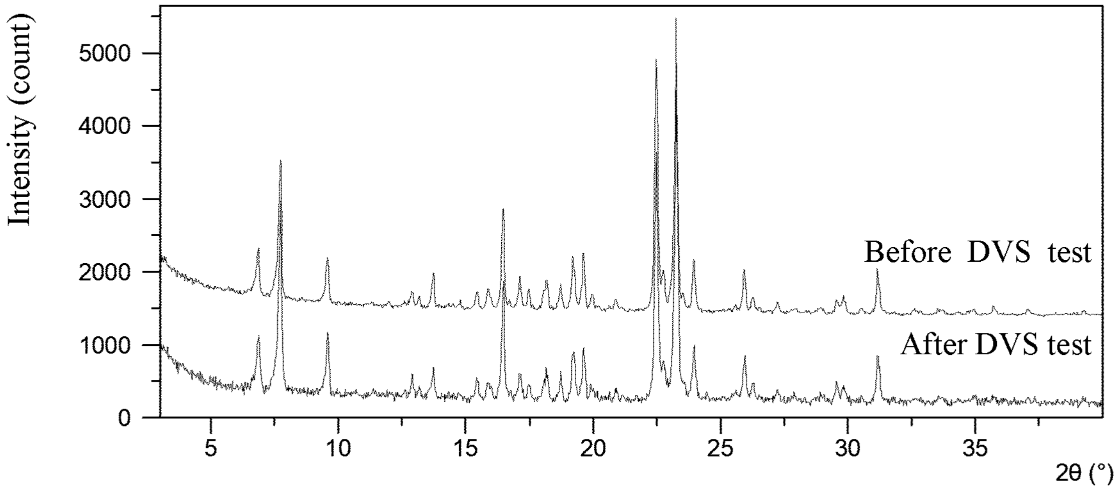


FIG. 116

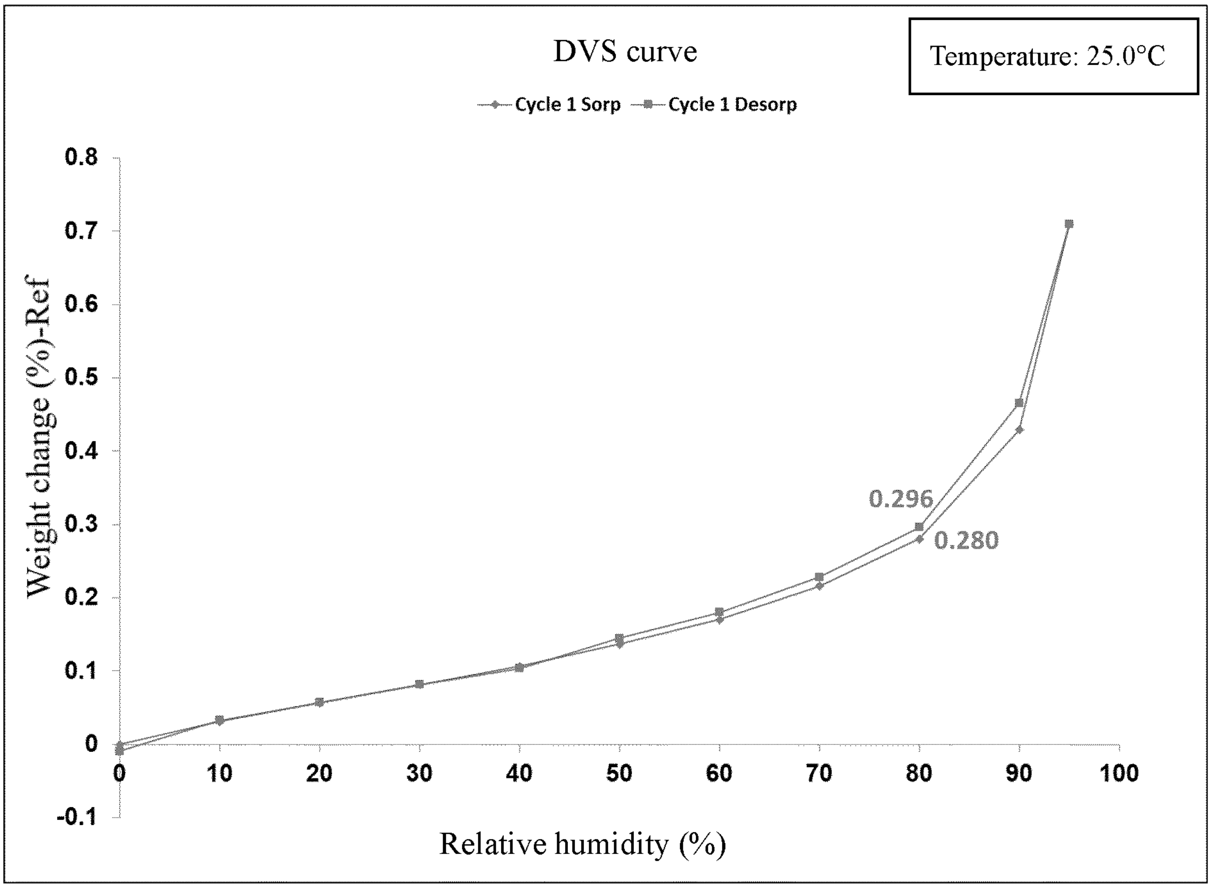


FIG. 117

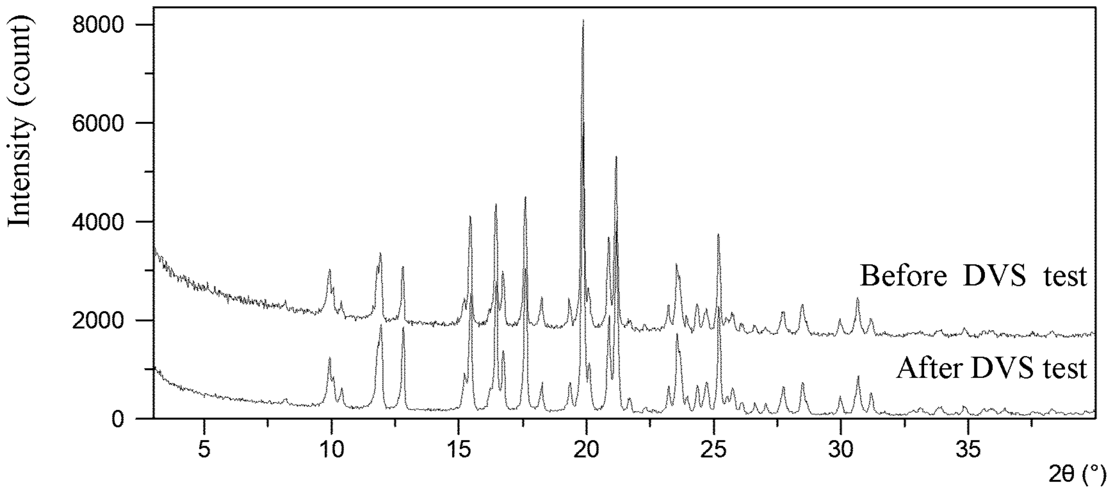


FIG. 118

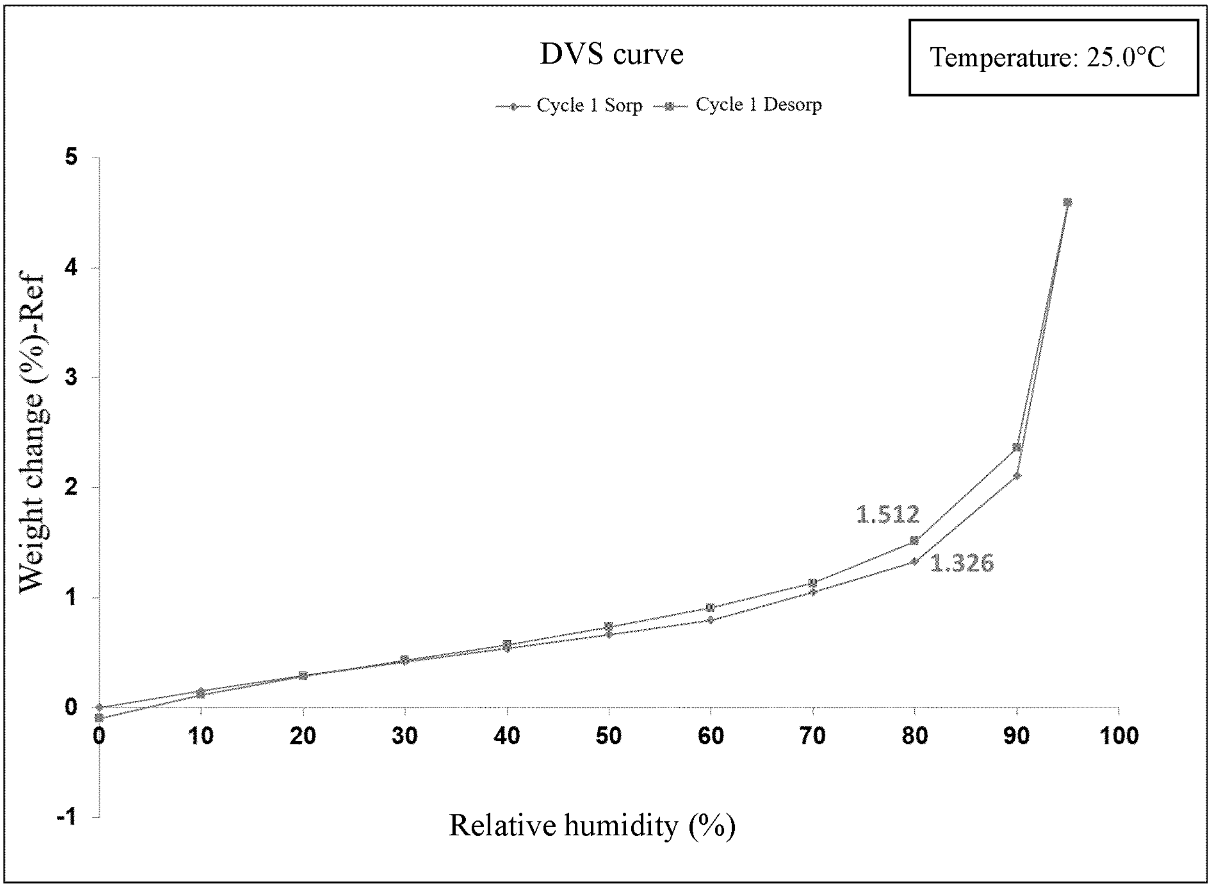


FIG. 119

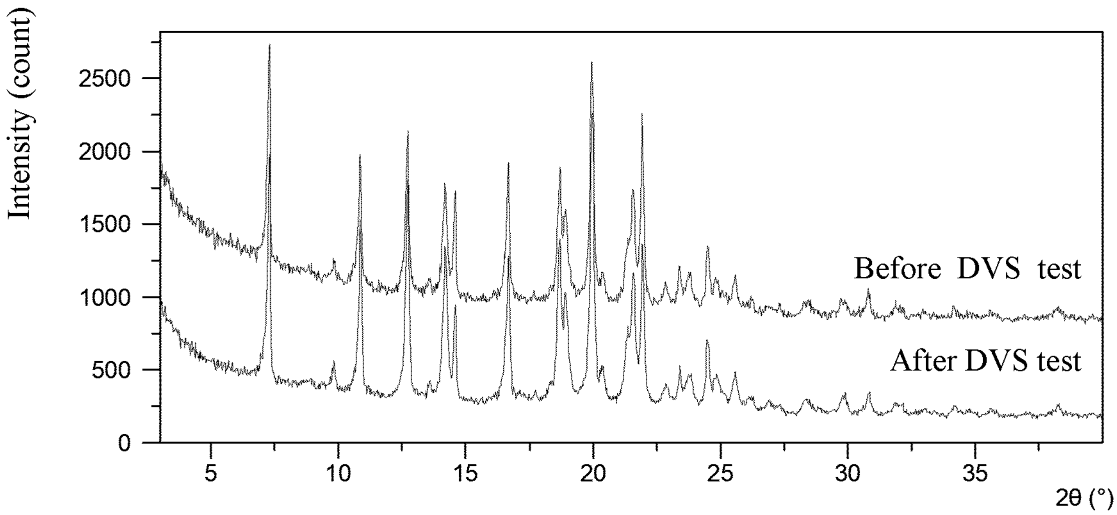


FIG. 120

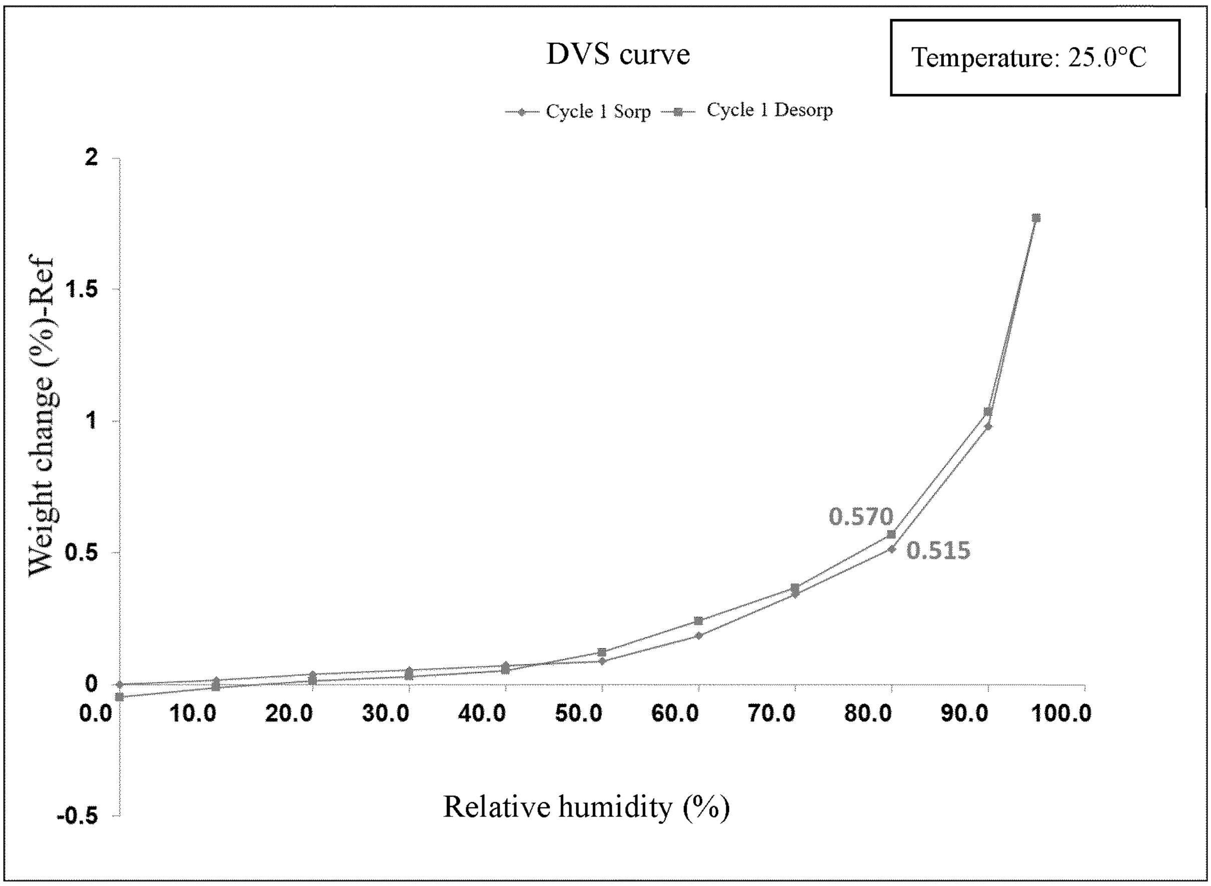


FIG. 121

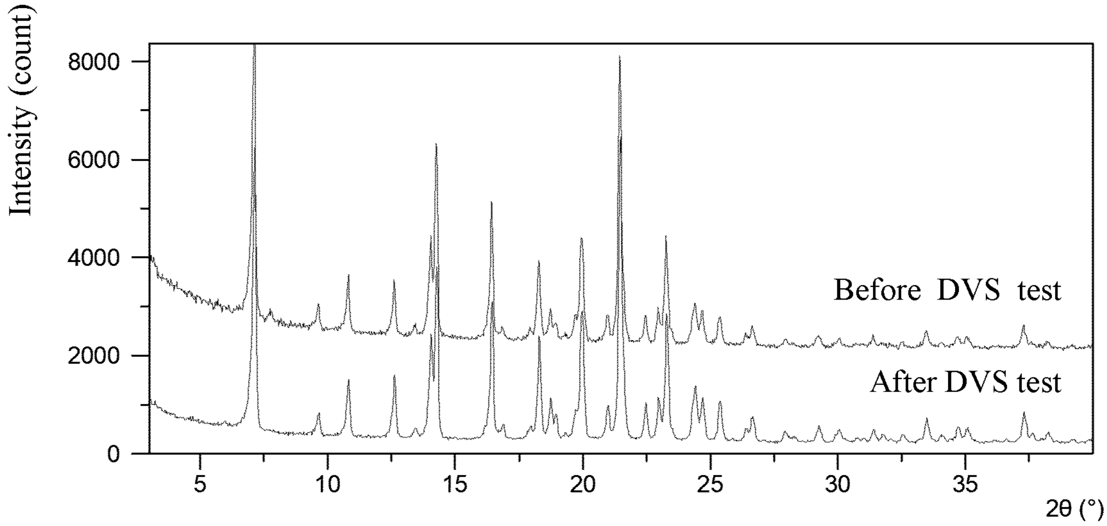


FIG. 122

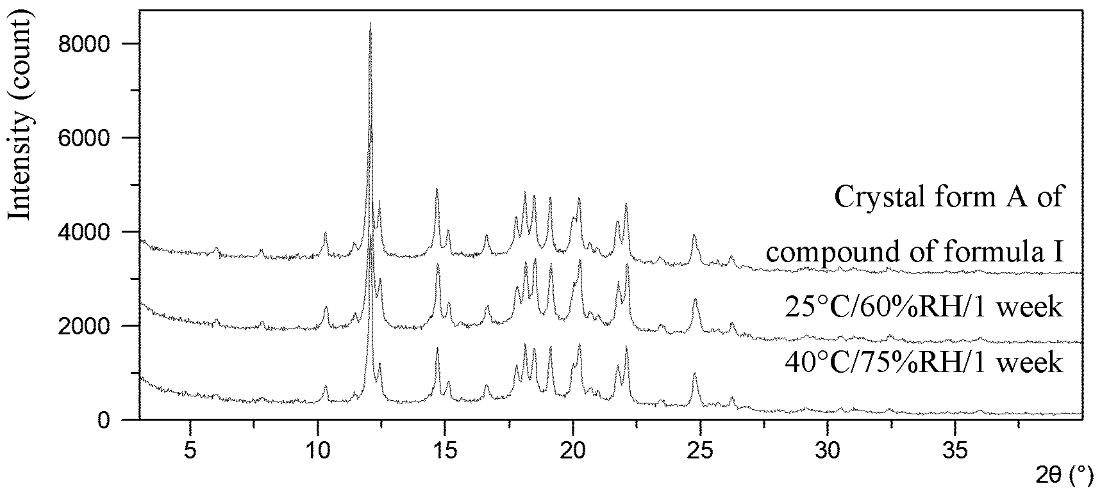


FIG. 123

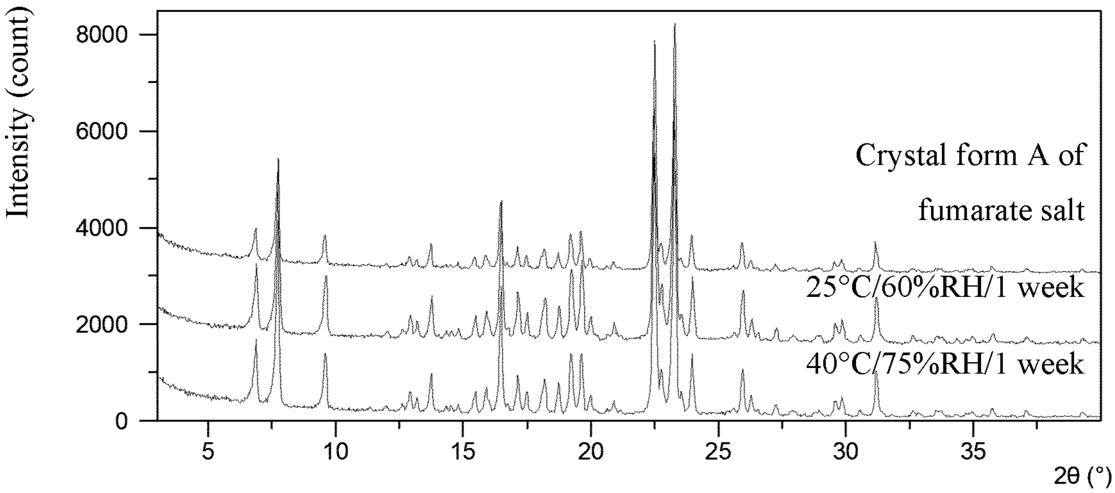


FIG. 124

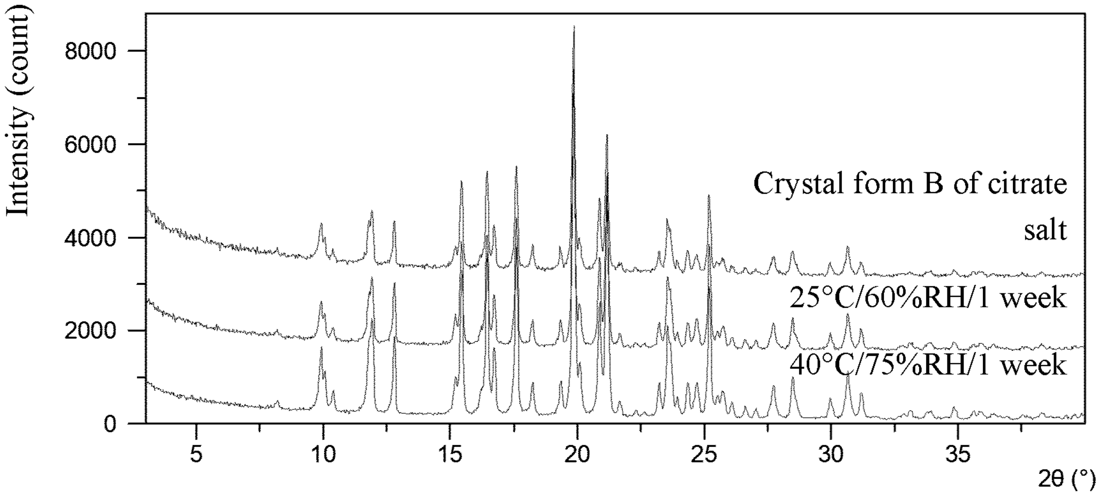


FIG. 125

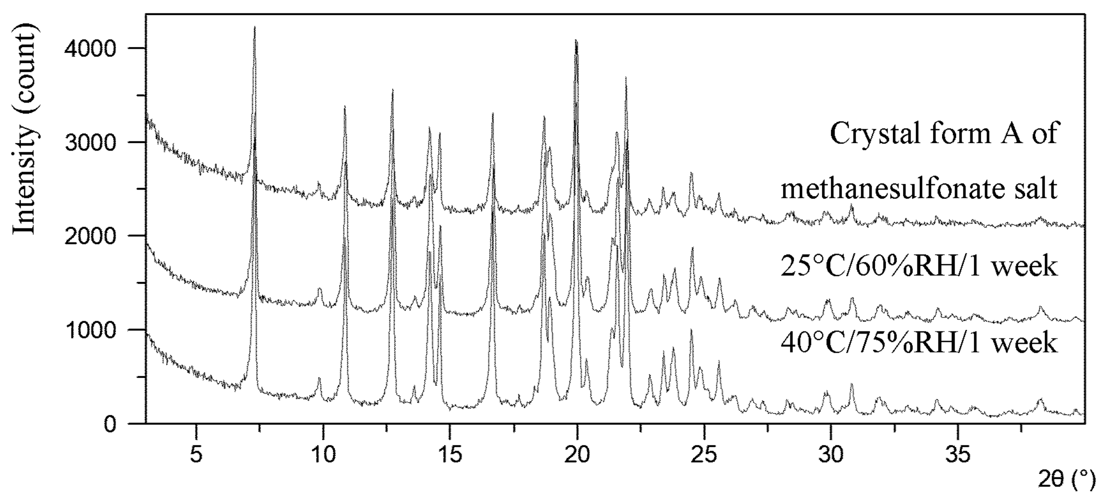


FIG. 126

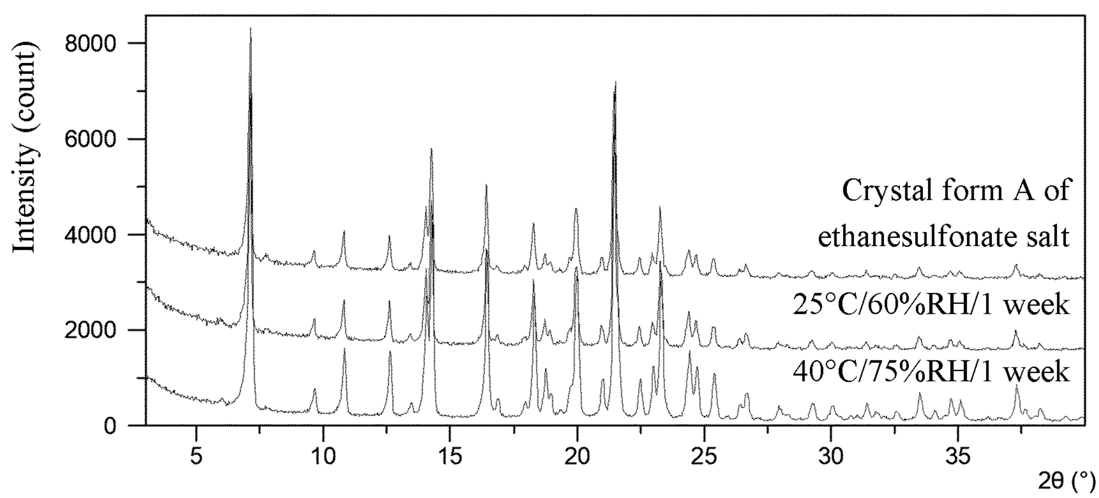


FIG. 127

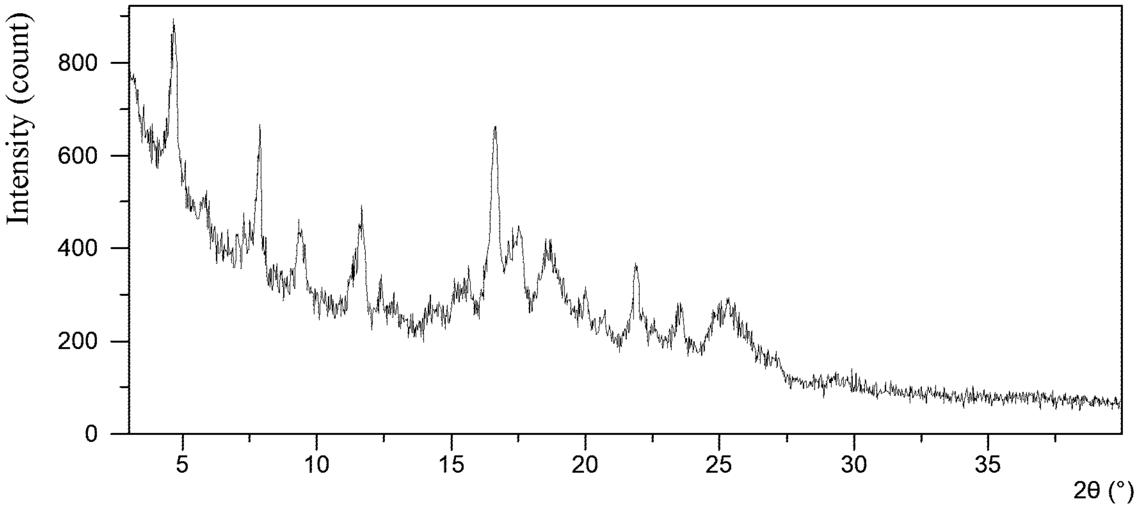


FIG. 128

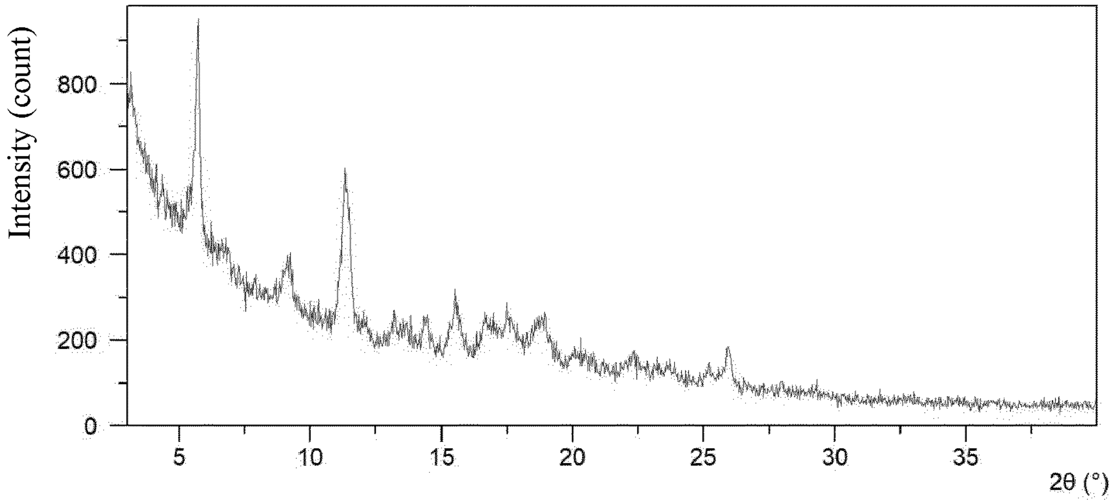


FIG. 129

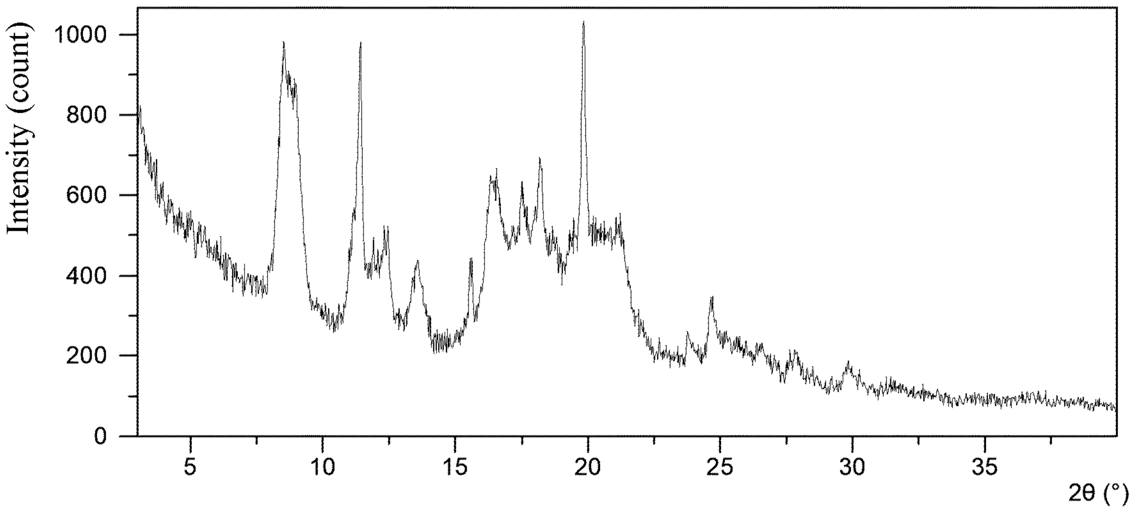


FIG. 130

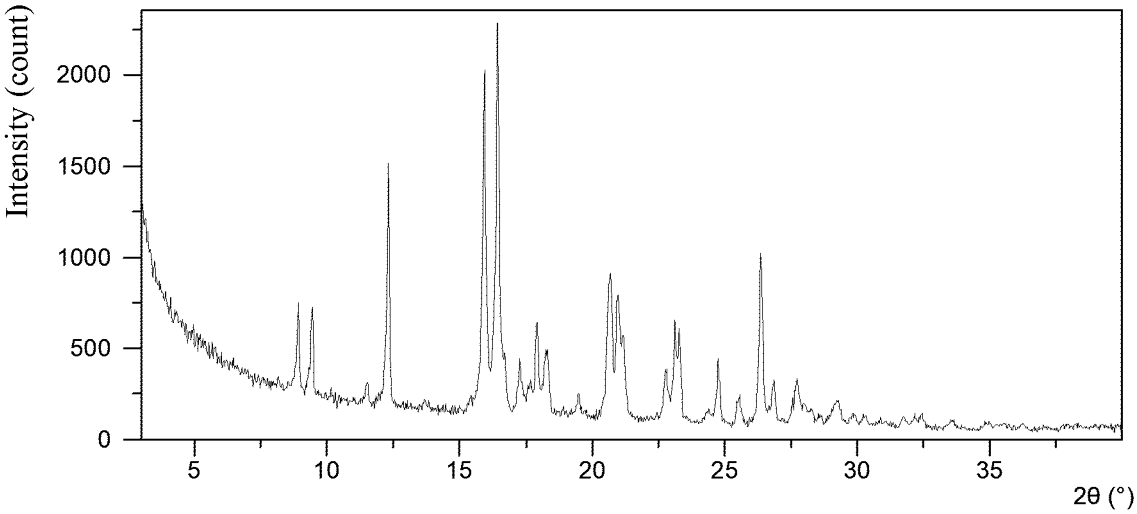


FIG. 131

## INTERNATIONAL SEARCH REPORT

International application No.
**PCT/CN2023/111760**

**A. CLASSIFICATION OF SUBJECT MATTER**

C07D471/04(2006.01)i； A61K31/519(2006.01)i； A61P35/00(2006.01)i

According to International Patent Classification (IPC) or to both national classification and IPC

**B. FIELDS SEARCHED**

Minimum documentation searched (classification system followed by classification symbols)

IPC:C07D,A61K,A61P

Documentation searched other than minimum documentation to the extent that such documents are included in the fields searched

Electronic data base consulted during the international search (name of data base and, where practicable, search terms used)

CNABS; DWPI; CNKI; Web of science; REGISTRY (STN); CAPLUS(STN): 人福创新, 张学军, 臧杨, 李群, 吡啶, 嘧啶酮, 晶体, 晶型, pyrido, pyrimidinone, crystal

**C. DOCUMENTS CONSIDERED TO BE RELEVANT**

| Category* | Citation of document, with indication, where appropriate, of the relevant passages | Relevant to claim No. |
|---|---|---|
| PX | CN 114907341 A (WUHAN RENFU INNOVATIVE DRUG R&D CENTER CO., LTD. et al.) 16 August 2022 (2022-08-16) description, pages 2 and 31-33, and claims 29-32 | 1-18 |
| PX | WO 2023041049 A1 (JIANGSU SIMCERE PHARM CO., LTD.) 23 March 2023 (2023-03-23) claims 1-17 | 1-18 |
| A | CN 111372932 A (BOEHRINGER INGELHEIM INTERNATIONAL GMBH) 03 July 2020 (2020-07-03) description, pages 4, 156 | 1-18 |
| A | CN 113200981 A (HANGZHOU INNOGATE PHARMA CO., LTD.) 03 August 2021 (2021-08-03) claims 1-17 | 1-18 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **16 November 2023** | **22 November 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/111760**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **16-17**
because they relate to subject matter not required to be searched by this Authority, namely:

The technical solutions of claims 16-17 comprise a disease treatment method as defined in PCT Rule 39.1. The present search report is provided on the basis of the technical solution of a corresponding pharmaceutical use.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/111760**

| Patent document cited in search report | Publication date (day/month/year) | Patent family member(s) | Publication date (day/month/year) |
|---|---|---|---|
| CN 114907341 A | 16 August 2022 | WO 2022166974 A1 | 11 August 2022 |
| | | KR 20230144065 A | 13 October 2023 |
| | | CA 3207590 A1 | 11 August 2022 |
| | | AU 2022217353 A1 | 14 September 2023 |
| WO 2023041049 A1 | 23 March 2023 | None | |
| CN 111372932 A | 03 July 2020 | WO 2019122129 A1 | 27 June 2019 |
| | | US 2021009588 A1 | 14 January 2021 |
| | | US 2019194192 A1 | 27 June 2019 |
| | | US 10829487 B2 | 10 November 2020 |
| | | KR 20200111163 A | 28 September 2020 |
| | | TW 201938557 A | 01 October 2019 |
| | | EP 4219493 A1 | 02 August 2023 |
| | | JP 2021506864 A | 22 February 2021 |
| | | JP 7189956 B2 | 14 December 2022 |
| | | EP 3728254 A1 | 28 October 2020 |
| | | EP 3728254 B1 | 15 February 2023 |
| CN 113200981 A | 03 August 2021 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2022109460522
- CN 202210117751
- WO 2019122129 A1